(19) Europäisches Patentamt
European Patent Office
Office européen des brevets

(11) **EP 3 154 982 B1**

(12) **EUROPEAN PATENT SPECIFICATION**

(45) Date of publication and mention of the grant of the patent:
**02.05.2018 Bulletin 2018/18**

(21) Application number: **15733915.1**

(22) Date of filing: **11.06.2015**

(51) Int Cl.:
***C07D 487/20*** *(2006.01)* ***C07D 487/10*** *(2006.01)*

(86) International application number:
**PCT/IB2015/054425**

(87) International publication number:
**WO 2015/189799 (17.12.2015 Gazette 2015/50)**

(54) **COMPOUNDS COMPRISING 1,1',2,5'-TETRAHYDROSPIRO[INDOLE-3,2'-PYRROLE]-2,5'-DIONE SYSTEM AS INHIBITORS P53-MDM2 PROTEIN-PROTEIN INTERACTION**

VERBINDUNGEN MIT 1,1',2,5'-TETRAHYDROSPIRO[INDOL-3,2'-PYRROL]-2,5'-DION-SYSTEM ALS INHIBITOREN DER P53-MDM2-PROTEIN-PROTEIN-INTERAKTION

COMPOSÉS COMPRENANT UN SYSTÈME 1,1',2,5'-TÉTRAHYDROSPIRO[INDOLE-3,2'-PYRROLE]-2,5'-DIONE EN TANT QU'INHIBITEURS DE L'INTERACTION PROTÉINE-PROTÉINE DE P53-MDM2

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **12.06.2014 PL 40854014**

(43) Date of publication of application:
**19.04.2017 Bulletin 2017/16**

(73) Proprietor: **Adamed sp. z o.o.
05-152 Czosnów k/Warszawy (PL)**

(72) Inventors:
- **FEDER, Marcin
PL-96-300 Zyrardow (PL)**
- **KALINOWSKA, Iwona
PL-08-400 Garwolin (PL)**
- **JASZCZEWSKA, Joanna Adriana
PL-32-045 Su oszowa (PL)**
- **BURCHARD, Ewa
PL-03-748 Warszawa (PL)**
- **LEWANDOWSKI, Wojciech
PL-05-600 Grojec (PL)**
- **BULKOWSKA, Urszula
PL-01-494 Warszawa (PL)**
- **MAZUR, Maria,
01-746 Warszawa (PL)**
- **WOS-LATOSI, Katarzyna
62-500 Konin (PL)**

(74) Representative: **Sulikowski, Daniel
Admarka sp. z o.o.
Pienków 149
05-152 Czosnów (PL)**

(56) References cited:
**WO-A1-2011/134925 WO-A2-2012/155066**

- **V L Gein ET AL: "THREE-COMPONENT SYNTHESIS OF 1-SUBSTITUTED 4-ACETYL-3-HYDROXYSPIRO[2,5-DIHYDRO-PYRROL-5,3'-INDOLE]-2,2'-DIONES", Chemistry of Heterocyclic Compounds, 19 March 2008 (2008-03-19), pages 786-787, XP055208702, Retrieved from the Internet: URL:http://download.springer.com/static/pdf/920/art%3A10.1007%2Fs10593-008-0085-6.pdf?originUrl=http://link.springer.com/article/10.1007/s10593-008-0085-6&token2=exp=1440063714~acl=/static/pdf/920/art%253A10.1007%252Fs10593-008-0085-6.pdf?originUrl=http%3A%2F%2Flink.springer.com%2Farticle%2F10 .1007%2Fs1 [retrieved on 2015-08-20]**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 154 982 B1

## Description

The present invention provides compounds comprising 1,1',2,5'-tetrahydrospiro[indole-3,2'-pyrrole]-2,5'-dione system having an activity of inhibiting p53-Mdm2 protein-protein interaction and these compounds for use as medicaments, especially for the treatment of diseases in which the p53/Mdm2 protein-protein interactions are disturbed and/or which are sensitive to inhibition of the p53/Mdm2 interactions, including proliferative diseases such as cancer. Furthermore, the present invention provides pharmaceutical compositions comprising the aforementioned compounds.

## Background of the invention

p53 is a transcription factor that responds to cellular stress by regulating the transcription of numerous genes that determine cells fate. In stress conditions p53 can trigger cell cycle arrest and DNA repair processes or cell death programs like apoptosis or senescence. The choice between these responses depends on the type and intensity of stress signals. In human cells p53 activity is strictly controlled by its negative regulator the protein named Mdm2. Mdm2 forms a tight complex with the p53 transactivation domain, blocking its ability to regulate target genes and to exert antiproliferative effects. Additionally, Mdm2 promotes the nuclear export and rapid degradation of p53 by the ubiquitin-proteasome system.

Being a key player in the cellular response to stress, p53 serves as the major obstruction for tumorigenesis. Patients with Li-Fraumeni syndrome which inherit mutated p53 are very susceptible to cancer. Mice with damaged p53 gene appear normal but are prone to the spontaneous development of a variety of neoplasms by 6 months of age. This prominent tumour suppressive role of p53 causes that its function is disabled in virtually all human cancers, either through mutation of the p53 gene or through aberrant expression of proteins acting as its negative regulators such as Mdm2.

Amplification of the Mdm2 gene is reported in more than 10% of 8000 various human cancers, including sarcomas, lung and stomach tumors, wherein p53 gene is not damaged. Multiple other tumors acquire a single nucleotide polymorphism in the Mdm2 promoter that leads to 2-3 fold increase in Mdm2 expression correlates with accelerated tumour formation. These alterations are perceived as the major mechanisms for inhibition of the p53 function in cancers retaining wild-type p53.

Functional genetic studies on mice have shown that restoration of inactivated p53 is sufficient to cause rapid regression of several different tumor types. Following this line, targeting the p53-Mdm2 interaction by small molecules to release and reactivate p53 has emerged as promising therapeutic strategy to treat human cancers that are p53 wild-type. Several groups of small-molecule non-peptide inhibitors of p53-Mdm2 interaction have been reported in recent years including nutlins, piperazine-4-phenyl derivatives, chalcones, sulphonamides, benzodiazepinediones, spiro-oxindoles. MDM2 inhibitors yield both common and different cellular responses in normal and tumor cells that are in agreement with the previous results from genetics studies. In normal cells, the activation of p53 by MDM2 inhibitors induces cell cycle arrest but not cell death. In tumor cells, the activation of p53 by the inhibitors induces not only cell cycle arrest but also cell death. This profile provides an outlook for high selectivity and low toxicity of the potential therapy. Nevertheless, none of these Mdm2 antagonists proved its effectiveness in human clinical trials. Thus, there is still a need to new compounds with increased potency and specificity.

The present invention provides a solution to this problem and satisfaction of this need by providing new compounds having the structure 1,1',2,5'-tetrahydrospiro[indole-3,2'-pyrrole]-2,5'-dione that show potent and specific antitumor activity in *in vitro* and *in vivo* studies.

Certain compounds comprising 1,1',2,5'-tetrahydrospiro[indole-3,2'-pyrrole]-2,5'-dione system are known in the art. They are disclosed, for example, in WO 2008/060789, WO 2008/046049, and WO 2006/110917. The documents, however, does not disclose the compounds of the present invention, neither report nor suggest that they have an activity inhibiting interaction with p53-Mdm2/4.

There are certain known compounds comprising spiro-oxoindole system that have an activity inhibiting interaction with p53-Mdm2/4. As an example, compounds disclosed in WO2012/155066, WO2012/121361, and WO2011/134925 can be named. None of the above-mentioned document discloses, however, compound of the present invention, and their similarity, beside spiro-oxoindole system, is rather low.

4-Acetyl-3-hydroxy-1-methylospiro[2,5-dihydropyrrol-5,3'-indole]-2,2'-dione and its synthesis is disclosed in V.L.Gein et al., Chemistry of Heterocyclic Compounds Vol. 44, No. 5, 2008, pp. 626-627. No data about utility of the compounds are reported.

## Description of the Invention

The invention relates to a compound represented by the formula selected from the group consisting of formula (IA) and (IB)

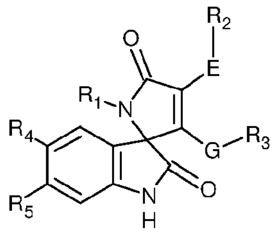

(IA)

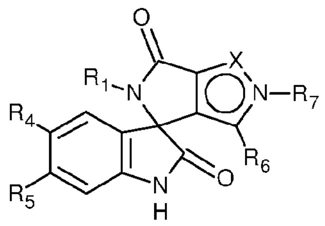

(IB)

wherein

$R_1$ is:

- $C_1$-$C_6$-alkyl unsubstituted or substituted by $C_3$-$C_6$-cycloalkyl,
- phenyl or 3-pyridyl that are unsubstituted or substituted by one or two substituents independently selected from the group consisting of halogen, -OH, -$NH_2$, -$NO_2$, -CN, $C_1$-$C_6$-alkyl, $C_2$-$C_6$-alkenyl, -O-($C_1$-$C_6$-alkyl), -O-($C_2$-$C_6$-alkenyl), -S-($C_1$-$C_6$-alkyl), -S-($C_2$-$C_6$-alkenyl), -C(O)O-($C_1$-$C_6$-alkyl),
- C(O)O-($C_2$-$C_6$-alkenyl), -C(O)$NH_2$, -C(O)NH($C_1$-$C_6$-alkyl), -C(O)N($C_1$-$C_6$-alkyl)$_2$, -C(O)NH($C_2$-$C_6$-alkenyl), -NH($C_1$-$C_6$-alkyl), -N($C_1$-$C_6$-alkyl)$_2$, -NH-phenyl, -NHC(O)-($C_1$-$C_6$-alkyl), -NHC(O)-($C_2$-$C_6$-alkenyl), -NHC(O)O-($C_1$-$C_6$-alkyl), and -$NHSO_2$-($C_1$-$C_6$-alkyl), and wherein said $C_1$-$C_6$-alkyl, $C_2$-$C_6$-alkenyl and phenyl are unsubstituted or further substituted by one or two substituents independently selected from the group consisting of halogen, -OH, -SH, -O-($C_1$-$C_6$-alkyl), -COOH, -C(O)O-($C_1$-$C_6$-alkyl), and -$SO_2$-($C_1$-$C_6$-alkyl),
- pyrazolyl unsubstituted or substituted by one or two substituents which are independently $C_1$-$C_6$-alkyl,
- 6-oxo-1,6-dihydropyridin-3-yl unsubstituted or substituted by one or two substituents independently selected from the group consisting of halogen and $C_1$-$C_6$-alkyl, or
- 2-oxo-1,2-dihydropyridin-3-yl unsubstituted or substituted by one or two substituents independently selected from the group consisting of halogen and $C_1$-$C_6$-alkyl;

$R_4$ and $R_5$ are independently H or halogen;

$R_2$ is hydrogen atom, ($C_1$-$C_6$-alkyl)sulfonyl, -($C_1$-$C_6$-alkyl), or -($C_1$-$C_6$-alkyl) terminally substituted by one substituent selected from the group consisting of -COOH, -$CONH_2$, -C(O)O-($C_1$-$C_6$-alkyl), -$NH_2$, NH($C_1$-$C_6$-alkyl), -N($C_1$-$C_6$-alkyl)$_2$, -NHC(O)($C_1$-$C_6$-alkyl), imidazole, tetrazole, and phenyl, wherein said phenyl is substituted by -($C_1$-$C_3$-alkyl), -O($C_1$-$C_3$-alkyl) or halogen;

$R_3$ is:

- $C_1$-$C_6$-alkyl,
- $C_3$-$C_6$-cycloalkyl unsubstituted or substituted by one $C_1$-$C_6$-alkyl,
- phenyl unsubstituted or substituted by one, two or three substituents independently selected from the group consisting of halogen, -OH, -$NH_2$, -$NO_2$, CN, $C_1$-$C_6$-alkyl, $C_2$-$C_6$-alkenyl, -O-($C_1$-$C_6$-alkyl), -O-($C_2$-$C_6$-alkenyl), -S-($C_1$-$C_6$-alkyl),
- S-($C_2$-$C_6$-alkenyl), -C(O)O-($C_1$-$C_6$-alkyl), -C(O)O-($C_2$-$C_6$-alkenyl), -C(O)$NH_2$, -(O)NH($C_1$-$C_6$-alkyl), -C(O)N($C_1$-$C_6$-alkyl)$_2$,
- C(O)NH($C_2$-$C_6$-alkenyl), -NH($C_1$-$C_6$-alkyl), -N($C_1$-$C_6$-alkyl)$_2$, -NH-phenyl, -NHC(O)-($C_1$-$C_6$-alkyl), -NHC(O)-($C_2$-$C_6$-alkenyl), and -NHC(O)O-($C_1$-$C_6$-alkyl), and wherein said $C_1$-$C_6$-alkyl, $C_2$-$C_6$-alkenyl and phenyl are unsubstituted or further substituted by one or two substituents independently selected from the group consisting of halogen, -OH, -SH, -O-($C_1$-$C_6$-alkyl), -COOH, and -C(O)O-($C_1$-$C_6$-alkyl), or

- 5- or 6-membered heteroaryl with one, two, three or four heteroatoms independently selected from N, 0, and S, wherein said heteroaryl is unsubstituted or substituted by one, two or three substituents independently selected from the group consisting of halogen, -OH, -$NH_2$, -$NO_2$, -CN, $C_1$-$C_6$-alkyl, $C_2$-$C_6$-alkenyl, -O-($C_1$-$C_6$-alkyl), -S-($C_1$-$C_6$-alkyl), -S-($C_2$-$C_6$-alkenyl), -C(O)O-($C_1$-$C_6$-alkyl), -C(O)O-($C_2$-$C_6$-alkenyl), -C(O)$NH_2$, -C(O)NH($C_1$-$C_6$-alkyl), -C(O)N($C_1$-$C_6$-alkyl)$_2$, -C(O)NH($C_2$-$C_6$-alkenyl), -NH($C_1$-$C_6$-alkyl), -N($C_1$-$C_6$-alkyl)$_2$, -NH-phenyl, -NHC(O)-($C_1$-$C_6$-alkyl), -NHC(O)-($C_2$-$C_6$-alkenyl), -NHC(O)O-($C_1$-$C_6$-alkyl), and 5- or 6-membered non-aromatic heterocyclyl with one or two heteroatoms selected from N and 0, and wherein said $C_1$-$C_6$-alkyl, $C_2$-$C_6$-alkenyl and phenyl are unsubstituted or further substituted by one or two substituents independently selected from the group consisting of halogen, -OH, -$NH_2$,-O-($C_1$-$C_6$-alkyl), -COOH, -C(O)O-($C_1$-$C_6$-alkyl), -NH-($C_1$-$C_6$-alkyl), and -N-($C_1$-$C_6$-alkyl)$_2$;

E is O, NH, or S;

G is S, carbonyl or a direct bond;

$R_6$ is:

- $C_1$-$C_6$-alkyl unsubstituted or substituted by one -O-($C_1$-$C_6$-alkyl), or
- $C_3$-$C_6$-cycloalkyl unsubstituted or substituted by one $C_1$-$C_6$-alkyl,

$R_7$ is:

- hydrogen atom,
- $C_1$-$C_3$-alkyl unsubstituted or substituted by one substituent selected from the group consisting of imidazole, tetrazole, and a 5- or 6-membered non-aromatic heterocyclyl comprising one or two heteroatoms selected from N and 0, wherein said non-aromatic heterocyclyl is unsubstituted or substituted on the nitrogen atom by substituent selected from the group consisting of -($C_1$-$C_6$-alkyl), -C(O)($C_1$-$C_6$-alkyl), and -C(O)N($C_1$-$C_6$-alkyl)$_2$,
- $C_1$-$C_6$-alkenyl,
- phenyl unsubstituted or substituted by one or two substituents independently selected from the group consisting of halogen, -OH, -$NH_2$, -$NO_2$, -CN, $C_1$-$C_6$-alkyl, $C_2$-$C_6$-alkenyl, -O-($C_1$-$C_6$-alkyl), -O-($C_2$-$C_6$-alkenyl), -S-($C_1$-$C_6$-alkyl), -S-($C_2$-$C_6$-alkenyl), -$CO_2$H, -C(O)O-($C_1$-$C_6$-alkyl), -C(O)O-($C_2$-$C_6$-alkenyl), -C(O)$NH_2$, -C(O)NH($C_1$-$C_6$-alkyl), and wherein said $C_1$-$C_6$-alkyl is unsubstituted or further substituted by -OH, -C(O)N($C_1$-$C_6$-alkyl)$_2$, -C(O)NH($C_2$-$C_6$-alkenyl), -NH($C_1$-$C_6$-alkyl), -N($C_1$-$C_6$-alkyl)$_2$, -NH-phenyl, -NHC(O)-($C_1$-$C_6$-alkyl), -NHC(O)-($C_2$-$C_6$-alkenyl), -NHC(O)O-($C_1$-$C_6$-alkyl), -$SO_2$N($C_1$-$C_6$-alkyl), or -$SO_2$-(5- or 6-membered heterocyclyl with one or two heteroatoms selected from N and O),
    - (3S,4R)-3-methoxypiperidin-4-yl, or
- 1-benzothiophen-3-yl, 2-oxo-2,3-dihydro-1H-1,3-benzodiazol-5-yl, 3-pyridyl, 4-pyridyl, 2H-1,3-benzodioxol-4-yl or 2-tiophenyl that are unsubstituted or substituted by $C_1$-$C_6$-alkyl, -O-($C_1$-$C_6$-alkyl) or halogen;

X is N or CH;

with the proviso that 4-acetyl-3-hydroxy-1-methylospiro[2,5-dihydropyrrol-5,3'-indole]-2,2'-dione is excluded;

and pharmaceutically acceptable salts, solvates, tautomers and stereoisomers thereof.

In the first variant of the invention, the compound is represented by formula (IA) wherein $R_1$, $R_2$, $R_3$, $R_4$, $R_5$, G and E are as defined as above.

In a preferred embodiment of the first variant, the compound of the invention is represented by formula (IA) and G is a carbonyl group.

In another preferred embodiment of the first variant, the compound of the invention is represented by formula

(IA) and G is -S-.

In another preferred embodiment of the first variant, the compound of the invention is represented by formula (IA) and G is a direct bond.

In another preferred embodiment of the first variant, the compound of the invention is represented by formula (IA) as defined in the embodiments presented above and

$R_1$ is:

- phenyl or 3-pyridyl that are unsubstituted or substituted by one or two substituents independently selected from the group consisting of halogen, -OH, -$NH_2$, -$NO_2$, -CN, $C_1$-$C_6$-alkyl, $C_2$-$C_6$-alkenyl, -O-($C_1$-$C_6$-alkyl), -O-($C_2$-$C_6$-alkenyl), -S-($C_1$-$C_6$-alkyl), -S-($C_2$-$C_6$-alkenyl), -C(O)O-($C_1$-$C_6$-alkyl), -C(O)O-($C_2$-$C_6$-alkenyl), -C(O)$NH_2$, -C(O)NH($C_1$-$C_6$-alkyl), -C(O)N($C_1$-$C_6$-alkyl)$_2$, -C(O)NH($C_2$-$C_6$-alkenyl), -NH($C_1$-$C_6$-alkyl), -N($C_1$-$C_6$-alkyl)$_2$, -NH-phenyl, -NHC(O)-($C_1$-$C_6$-alkyl), -NHC(O)-($C_2$-$C_6$-alkenyl), -NHC(O)O-($C_1$-$C_6$-alkyl), and $NHSO_2$-($C_1$-$C_6$-alkyl), and wherein said $C_1$-$C_6$-alkyl, $C_2$-$C_6$-alkenyl and phenyl are unsubstituted or further substituted by one or two substituents independently selected from the group consisting of halogen, -OH, -SH, -O-($C_1$-$C_6$-alkyl), -COOH, -C(O)O-($C_1$-$C_6$-alkyl), and -$SO_2$-($C_1$-$C_6$-alkyl),
- pyrazolyl unsubstituted or substituted by one or two substituents which are independently $C_1$-$C_6$-alkyl,
- 6-oxo-1,6-dihydropyridin-3-yl unsubstituted or substituted by one or two substituents independently selected from the group consisting of halogen and $C_1$-$C_6$-alkyl, or
- 2-oxo-1,2-dihydropyridin-3-yl unsubstituted or substituted by one or two substituents independently selected from the group consisting of halogen and $C_1$-$C_6$-alkyl,

$R_4$ is H, and

$R_5$ is Cl or F.

Preferably, the compound of the invention is represented by formula (IA) as defined in the embodiments of the formula (IA) presented above and

$R_1$ is:

- phenyl substituted with halogen at the meta position relative to the place of attachment to the pyrrolone ring nitrogen atom, or 3-pyridyl substituted with halogen at the position 5 relative to the place of attachment to the pyrrolone ring nitrogen atom, and said phenyl and 3-pyridyl are optionally further substituted by one or two substituents independently selected from the group consisting of halogen, -OH, -$NH_2$, -$NO_2$, -CN, $C_1$-$C_6$-alkyl, $C_2$-$C_6$-alkenyl, -O-($C_1$-$C_6$-alkyl), -O-($C_2$-$C_6$-alkenyl), -S-($C_1$-$C_6$-alkyl), -S-($C_2$-$C_6$-alkenyl), -C(O)O-($C_1$-$C_6$-alkyl), -C(O)O-($C_2$-$C_6$-alkenyl), -C(O)$NH_2$, -C(O)NH($C_1$-$C_6$-alkyl), -C(O)N($C_1$-$C_6$-alkyl)$_2$, -C(O)NH($C_2$-$C_6$-alkenyl), -NH($C_1$-$C_6$-alkyl), -N($C_1$-$C_6$-alkyl)$_2$, -NH-phenyl, -NHC(O)-($C_1$-$C_6$-alkyl), -NHC(O)-($C_2$-$C_6$-alkenyl), -NHC(O)O-($C_1$-$C_6$-alkyl), and $NHSO_2$-($C_1$-$C_6$-alkyl), and wherein said $C_1$-$C_6$-alkyl, $C_2$-$C_6$-alkenyl and phenyl are unsubstituted or further substituted by one or two substituents independently selected from the group consisting of halogen, -OH, -SH, -O-($C_1$-$C_6$-alkyl), -COOH, -C(O)O-($C_1$-$C_6$-alkyl), and -$SO_2$-($C_1$-$C_6$-alkyl).

Also preferably, the compound of the invention is represented by formula (IA) as defined in the embodiments of the formula (IA) presented above and $R_4$ is H, and $R_5$ is Cl or F.

In the second variant of the invention the compound is represented by the formula (IB)

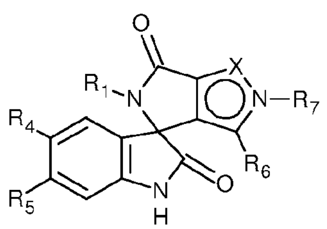

(IB)

wherein $R_4$, $R_5$, $R_6$, $R_7$ and X are defined as above.

It will be appreciated by a skilled person that formula (IB) corresponds to the formula (IA) wherein $R_2$ and $R_3$

together with the groups E and G are replaced with -X-N(R7)-CH(R6)- moiety to form with carbon atoms adjacent thereto a fused heterocyclic system.

In one sub-group of this second variant of formula (IB) X is N and the compound is represented by formula (IB-1)

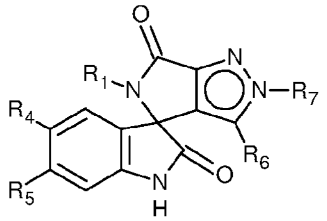

(IB-1).

Formula (IB-1) corresponds to the formula (IA) wherein $R_2$ and $R_3$ together with the groups E and G are replaced with -N-N(R7)-CH(R6)- moiety to form with carbon atoms adjacent thereto a fused heterocyclic pyrazole system.

In another sub-group of this second variant of formula (IB) X is CH and the compound is represented by formula (IB-2)

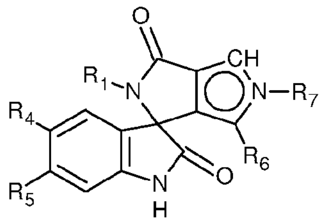

(IB-2).

Formula (IB-2) corresponds to the formula (IA) wherein $R_2$ and $R_3$ together with the groups E and G are replaced with -CH-N(R7)-CH(R6)- moiety to form with carbon atoms adjacent thereto a fused heterocyclic pyrrole system.

Preferably, in formula (IB), (IB-1) and (IB-2)

$R_1$ is:

- phenyl or 3-pyridyl that are unsubstituted or substituted by one or two substituents independently selected from the group consisting of halogen, -OH, $-NH_2$, $-NO_2$, -CN, $C_1$-$C_6$-alkyl, $C_2$-$C_6$-alkenyl, -O-($C_1$-$C_6$-alkyl), -O-($C_2$-$C_6$-alkenyl), -S-($C_1$-$C_6$-alkyl), -S-($C_2$-$C_6$-alkenyl), -C(O)O-($C_1$-$C_6$-alkyl), -C(O)O-($C_2$-$C_6$-alkenyl), $-C(O)NH_2$, -C(O)NH($C_1$-$C_6$-alkyl), -C(O)N($C_1$-$C_6$-alkyl)$_2$, -C(O)NH($C_2$-$C_6$-alkenyl), -NH($C_1$-$C_6$-alkyl), -N($C_1$-$C_6$-alkyl)$_2$, -NH-phenyl, -NHC(O)-($C_1$-$C_6$-alkyl), -NHC(O)-($C_2$-$C_6$-alkenyl), -NHC(O)O-($C_1$-$C_6$-alkyl), and $NHSO_2$-($C_1$-$C_6$-alkyl), and wherein said $C_1$-$C_6$-alkyl, $C_2$-$C_6$-alkenyl and phenyl are unsubstituted or further substituted by one or two substituents independently selected from the group consisting of halogen, -OH, -SH, -O-($C_1$-$C_6$-alkyl), -COOH, -C(O)O-($C_1$-$C_6$-alkyl), and $-SO_2$-($C_1$-$C_6$-alkyl),
- pyrazolyl unsubstituted or substituted by one or two substituents which are independently $C_1$-$C_6$-alkyl,
- 6-oxo-1,6-dihydropyridin-3-yl unsubstituted or substituted by one or two substituents independently selected from the group consisting of halogen and $C_1$-$C_6$-alkyl, or
- 2-oxo-1,2-dihydropyridin-3-yl unsubstituted or substituted by one or two substituents independently selected from the group consisting of halogen and $C_1$-$C_6$-alkyl.

Also preferably, in formula (IB), (IB-1) and (IB-2)

$R_1$ is:

- phenyl substituted with halogen at the meta position relative to the place of attachment to the pyrrolone ring nitrogen atom, or 3-pyridyl substituted with halogen at the position 5 relative to the place of attachment to the pyrrolone ring

nitrogen atom, and said phenyl and 3-pyridyl are optionally further substituted by one or two substituents independently selected from the group consisting of halogen, -OH, $-NH_2$, $-NO_2$, -CN, $C_1$-$C_6$-alkyl, $C_2$-$C_6$-alkenyl, -O-($C_1$-$C_6$-alkyl), -O-($C_2$-$C_6$-alkenyl), -S-($C_1$-$C_6$-alkyl), -S-($C_2$-$C_6$-alkenyl), -C(O)O-($C_1$-$C_6$-alkyl), -C(O)O-($C_2$-$C_6$-alkenyl), $-C(O)NH_2$, -C(O)NH($C_1$-$C_6$-alkyl), -C(O)N($C_1$-$C_6$-alkyl)$_2$, -C(O)NH($C_2$-$C_6$-alkenyl), -NH($C_1$-$C_6$-alkyl), -N($C_1$-$C_6$-alkyl)$_2$, -NH-phenyl, -NHC(O)-($C_1$-$C_6$-alkyl), -NHC(O)-($C_2$-$C_6$-alkenyl), -NHC(O)O-($C_1$-$C_6$-alkyl), and $NHSO_2$-($C_1$-$C_6$-alkyl), and wherein said $C_1$-$C_6$-alkyl, $C_2$-$C_6$-alkenyl and phenyl are unsubstituted or further substituted by one or two substituents independently selected from the group consisting of halogen, -OH, -SH, -O-($C_1$-$C_6$-alkyl), -COOH, -C(O)O-($C_1$-$C_6$-alkyl), and $-SO_2$-($C_1$-$C_6$-alkyl).

Also preferably, in formula (IB), (IB-1) and (IB-2) $R_4$ is H, and $R_5$ is Cl or F.

Also preferably, in formula (IB), (IB-1) and (IB-2) $R_1$ is:

- meta-chlorophenyl or 5-chloro-3-pyridyl that are optionally further substituted by one or two substituents independently selected from the group consisting of halogen, -OH, $-NH_2$, $-NO_2$, -CN, $C_1$-$C_6$-alkyl, $C_2$-$C_6$-alkenyl, -O-($C_1$-$C_6$-alkyl), -O-($C_2$-$C_6$-alkenyl), -S-($C_1$-$C_6$-alkyl), -S-($C_2$-$C_6$-alkenyl), -C(O)O-($C_1$-$C_6$-alkyl), -C(O)O-($C_2$-$C_6$-alkenyl), $-C(O)NH_2$, -C(O)NH($C_1$-$C_6$-alkyl), -C(O)N($C_1$-$C_6$-alkyl)$_2$, -C(O)NH($C_2$-$C_6$-alkenyl), -NH($C_1$-$C_6$-alkyl), -N($C_1$-$C_6$-alkyl)$_2$, -NH-phenyl, -NHC(O)-($C_1$-$C_6$-alkyl), -NHC(O)-($C_2$-$C_6$-alkenyl), -NHC(O)O-($C_1$-$C_6$-alkyl), and $NHSO_2$-($C_1$-$C_6$-alkyl), and wherein said $C_1$-$C_6$-alkyl, $C_2$-$C_6$-alkenyl and phenyl are unsubstituted or further substituted by one or two substituents independently selected from the group consisting of halogen, -OH, -SH, -O-($C_1$-$C_6$-alkyl), -COOH, -C(O)O-($C_1$-$C_6$-alkyl), and $-SO_2$-($C_1$-$C_6$-alkyl).

Also preferably, in formula (IB), (IB-1) and (IB-2) the absolute configuration at spiro carbon atom is S (configuration 3S).

As specific compounds of the invention, the following can be mentioned:

6-chloro-1'-(5-chloro-2-fluorophenyl)-4'-hydroxy-3'-(1-methylcyclopropane-carbonyl)-1,1',2,5'-tetrahydrospiro[indole-3,2'-pyrrole]-2,5'-dione,

6-chloro-1'-(5-chloro-2-methylphenyl)-4'-hydroxy-3'-(1-methylcyclopropane-carbonyl)-1,1',2,5'-tetrahydrospiro[indole-3,2'-pyrrole]-2,5'-dione,

3'-benzoyl-6-chloro-1'-(3-chlorophenyl)-4'-hydroxy-1,1',2,5'-tetrahydrospiro[indole-3,2'-pyrrole]-2,5'-dione,

3'-benzoyl-6-chloro-1'-(1,5-dimethyl-1H-pyrazol-3-yl)-4'-hydroxy-1,1',2,5'-tetrahydrospiro[indole-3,2'-pyrrole]-2,5'-dione,

6-chloro-1'-(3-chlorophenyl)-4'-hydroxy-3'-(1-methylcyclopropanecarbonyl)-1,1',2,5'-tetrahydrospiro[indole-3,2'-pyrrole]-2,5'-dione,

6-chloro-1'-(3-chlorophenyl)-3'-(2,2-tert-butanoyl)-4'-hydroxy-1,1',2,5'-tetrahydro-spiro[indole-3,2'-pyrrole]-2,5'-dione,

6-chloro-1'-(5-chloro-2-methoxyphenyl)-4'-hydroxy-3'-(1-methylcyclopropane-carbonyl)-1,1',2,5'-tetrahydrospiro[indole-3,2'-pyrrole]-2,5'-dione,

6-chloro-1'-(5-chloro-2-methylphenyl)-4'-hydroxy-3'-(iso-propanoyl)-1,1',2, 5'-tetrahydrospiro[indole-3,2'-pyrrole]-2,5'-dione,

6-chloro-1'-(5-chloro-2-hydroxyphenyl)-4'-hydroxy-3'-(iso-propanoyl)-1,1',2,5'-tetrahydrospiro[indole-3,2'-pyrrole]-2,5'-dione,

6-chloro-1'-(5-chloro-2-methoxyphenyl)-4'-hydroxy-3'-(iso-propanoyl)-1,1',2,5'-tetrahydrospiro[indole-3,2'-pyrrole]-2,5'-dione,

6-chloro-1'-(5-chloro-2-fluorophenyl)-4'-hydroxy-3'-(iso-propanoyl)-1,1',2, 5'-tetrahydrospiro[indole-3,2'-pyrrole]-2,5'-dione,

6-chloro-1'-(5-chloro-1-methyl-6-oxo-1,6-dihydropyridin-3-yl)-4'-hydroxy-3'-(isopropanoyl)-1,1',2,5'-tetrahydrospiro[indole-3,2'-pyrrole]-2,5'-dione,

6-chloro-1'-(3-chlorophenyl)-3'-[2,2-dimethyl-3-(propan-2-yloxy)propanoyl]-4'-hydroxy-1,1',2,5'-tetrahydrospiro[indole-3,2'-pyrrole]-2,5'-dione,

2-{[6-chloro-1'-(5-chloro-2-methylphenyl)-3'-(1-methylcyclopropanecarbonyl)-2,5'-dioxo-1,1',2,5'-tetrahydrospiro[indole-3,2'-pyrrole]-4'-yl]amino}acetamide,

ethyl 2-{[6-chloro-1'-(3-chlorophenyl)-3'-(1-methylcyclopropanecarbonyl)-2,5'-dioxo-1,1',2,5'-tetrahydrospiro[indole-3,2'-pyrrole]-4'-yl]amino}acetate,

2-{[6-chloro-1'-(3-chlorophenyl)-3'-(1-methylcyclopropanecarbonyl)-2,5'-dioxo-1,1',2,5'-tetrahydrospiro[indole-3,2'-pyrrole]-4'-yl]amino}acetamide,

ethyl (2E)-3-[(3-{[6-chloro-1'-(3-chlorophenyl)-4'-hydroxy-2,5'-dioxo-1,1',2,5'-tetra-hydrospiro[indole-3,2'-pyrrole]-3'-yl]carbonyl}phenyl)carbamoyl]prop-2-enoate,

6-chloro-5'-(5-chloro-2-methylphenyl)-3'-(1-methylcyclopropyl)-1,2,5',6'-tetrahydro-2'H-spiro[indole-3,4'-pyrrolo[3,4-c]pyrazole]-2,6'-dione,

6-chloro-5'-(3-chlorophenyl)-3'-(1-methylcyclopropyl)-1,2,5',6'-tetrahydro-2'H-spiro[indole-3,4'-pyrrolo[3,4-c]pyrazole]-2,6'-dione,

3'-tert-butyl-6-chloro-5'-(3-chlorophenyl)-1,2,5',6'-tetrahydro-2'H-spiro[indole-3,4'-pyrrolo[3,4-c]pyrazole]-2,6'-dione,

3'-tert-butyl-6-chloro-5'-(5-chloro-2-methoxyphenyl)-1,2,5',6'-tetrahydro-2'H-spiro[indole-3,4'-pyrrolo[3,4-c]pyrazole]-2,6'-dione,

6-chloro-5'-(5-chloro-2-methoxyphenyl)-3'-(1-methylcyclopropyl)-1,2, 5',6'-tetrahydro-2'H-spiro[indole-3,4'-pyrrolo[3,4-c]pyrazole]-2,6'-dione,

6-chloro-5'-(5-chloro-2-methylphenyl)-3'-(propan-2-yl)-1,2,5',6'-tetrahydro-2'H-spiro[indole-3,4'-pyrrolo[3,4-c]pyrazole]-2,6'-dione,

6-chloro-5'-(5-chloro-2-hydroxyphenyl)-3'-(propan-2-yl)-1,2,5',6'-tetrahydro-2'H-spiro[indole-3,4'-pyrrolo[3,4-c]pyrazole]-2,6'-dione,

6-chloro-5'-(5-chloro-2-methoxyphenyl)-3'-(propan-2-yl)-1,2,5',6'-tetrahydro-2'H-spiro[indole-3,4'-pyrrolo[3,4-c]pyrazole]-2,6'-dione,

6-chloro-5'-(3-chlorophenyl)-2'-(2-methoxyphenyl)-3'-(1-methylcyclopropyl)-1,2,5',6'-tetrahydro-2'H-spiro[indole-3,4'-pyrrolo[3,4-c]pyrazole]-2,6'-dione,

6-chloro-5'-(3-chlorophenyl)-2'-(2-methoxyphenyl)-3'-(propan-2-yl)-1,2,5',6'-tetrahydro-2'H-spiro[indole-3,4'-pyrrolo[3,4-c]pyrazole]-2,6'-dione,

6-chloro-5'-(3-chlorophenyl)-3'-(1-methylcyclopropyl)-2'-(pyrrolidin-2-ylmethyl)-1,2,5',6'-tetrahydro-2'H-spiro[indole-3,4'-pyrrolo[3,4-c]pyrazole]-2,6'-dione,

2-[6-chloro-5'-(3-chlorophenyl)-3'-(1-methylcyclopropyl)-2,6'-dioxo-1,2,5',6'-tetra-hydro-2'H-spiro[indole-3,4'-pyrrolo[3,4-c]pyrazole]-2'-ylmethyl]-N,N-dimethyl-cyclopentane-1-carboxamide,

6-chloro-5'-(5-chloro-2-methoxyphenyl)-2'-(2-methoxyphenyl)-3'-(propan-2-yl)-1,2,5',6'-tetrahydro-2'H-spiro[indole-3,4'-pyrrolo[3,4-c]pyrazole]-2,6'-dione,

6-chloro-5'-(5-chloro-2-methylphenyl)-2'-(2-methoxyphenyl)-3'-(propan-2-yl)-1,2,5',6'-tetrahydro-2'H-spiro[indole-3,4'-pyrrolo[3,4-c]pyrazole]-2,6'-dione,

6-chloro-5'-(5-chloro-2-methylphenyl)-1'-(2-methoxyphenyl)-3'-(propan-2-yl)-1,2,5',6'-tetrahydro-1'H-spiro[indole-3,4'-pyrrolo[3,4-c]pyrazole]-2,6'-dione,

6-chloro-5'-(5-chloro-2-hydroxyphenyl)-1'-(2-methoxyphenyl)-3'-(propan-2-yl)-1,2,5',6'-tetrahydro-1'H-spiro[indole-3,4'-pyrrolo[3,4-c]pyrazole]-2,6'-dione,

2-[6-chloro-5'-(3-chlorophenyl)-2,6'-dioxo-3'-(propan-2-yl)-1,2,5',6'-tetrahydro-2'H-spiro[indole-3,4'-pyrrolo[3,4-c]pyrazole]-2'-ylmethyl]-N,N-dimethylpyrrolidine-1-carboxamide,

6-chloro-1'-(2,2-dimethylpropyl)-4'-hydroxy-3'-phenyl-1,1',2,5'-tetrahydro-spiro[indole-3,2'-pyrrole]-2,5'-dione,

6-chloro-3'-(3-chlorophenyl)-1'-(2,2-dimethylpropyl)-4'-hydroxy-1,1',2,5'-tetra-hydrospiro[indole-3,2'-pyrrole]-2,5'-dione,

6-chloro-3'-(3-chlorophenyl)-4'-hydroxy-1'-phenyl-1,1',2,5'-tetrahydrospiro[indole-3,2'-pyrrole]-2,5'-dione,

5-chloro-1'-(3-chlorophenyl)-4'-hydroxy-3'-phenyl-1,1',2,5'-tetrahydrospiro[indole-3,2'-pyrrole]-2,5'-dione,

1'-(3-chlorophenyl)-4'-hydroxy-3'-phenyl-1,1',2,5'-tetrahydrospiro[indole-3,2'-pyrrole]-2,5'-dione,

6-chloro-1',3'-bis(3-chlorophenyl)-4'-hydroxy-1,1',2, 5'-tetrahydrospiro[indole-3,2'-pyrrole]-2,5'-dione,

6-chloro-1'-(3-chlorophenyl)-4'-hydroxy-3'-phenyl-1,1',2,5'-tetrahydrospiro[indole-3,2'-pyrrole]-2,5'-dione,

1'-(3-chlorophenyl)-6-fluoro-4'-hydroxy-3'-phenyl-1,1',2,5'-tetrahydrospiro[indole-3,2'-pyrrole]-2,5'-dione,

5,6-dichloro-1'-(3-chlorophenyl)-4'-hydroxy-3'-phenyl-1,1',2,5'-tetrahydrospiro-[indole-3,2'-pyrrole]-2,5'-dione,

6-chloro-1'-(3-chlorophenyl)-3'-cyclohexyl-4'-hydroxy-1,1',2,5'-tetrahydrospiro-[indole-3,2'-pyrrole]-2,5'-dione,

6-chloro-1'-(5-chloro-2-fluorophenyl)-4'-hydroxy-3'-phenyl-1,1',2,5'-tetrahydrospiro-[indole-3,2'-pyrrole]-2,5'-dione,

6-chloro-1'-(5-chloro-2-methoxyphenyl)-4'-hydroxy-3'-phenyl-1,1',2,5'-tetrahydro-spiro[indole-3,2'-pyrrole]-2,5'-dione,

6-chloro-1'-(5-chloro-2-methylphenyl)-4'-hydroxy-3'-phenyl-1,1',2,5'-tetrahydro-spiro[indole-3,2'-pyrrole]-2,5'-dione,

ethyl 2-{[6-chloro-1'-(3-chlorophenyl)-2,5'-dioxo-3'-phenyl-1,1',2,5'-tetrahydro-spiro[indole-3,2'-pyrrole]-4'-yl]amino}acetate,

2-{[6-chloro-1'-(3-chlorophenyl)-2,5'-dioxo-3'-phenyl-1,1',2,5'-tetrahydrospiro-[indole-3,2'-pyrrole]-4'-yl]amino}acetic acid,

methyl 2-[6-chloro-1'-(3-chlorophenyl)-2,5'-dioxo-3'-phenyl-1,1',2,5'-tetrahydro-spiro[indole-3,2'-pyrrole]-4'-ylsulfanyl]acetate,

2-[6-chloro-1'-(3-chlorophenyl)-2,5'-dioxo-3'-phenyl-1,1',2,5'-tetrahydrospiro-[indole-3,2'-pyrrole]-4'-ylsulfanyl]acetic acid,

6-chloro-1'-(3-chlorophenyl)-2,5'-dioxo-3'-phenyl-1,1',2,5'-tetrahydrospiro[indole-3,2'-pyrrole]-4'-yl methanesulfonate,

2-[6-chloro-1'-(3-chlorophenyl)-2,5'-dioxo-3'-phenyl-1,1',2,5'-tetrahydrospiro-[indole-3,2'-pyrrole]-4'-ylsulfanyl]acetamide,

6-chloro-1'-(3-chlorophenyl)-3'-[(4-chlorophenyl)sulfanyl]-4'-hydroxy-1,1',2,5'-tetrahydrospiro[indole-3,2'-pyrrole]-2,5'-dione,

3'-(butan-2-ylsulfanyl)-6-chloro-1'-(3-chlorophenyl)-4'-hydroxy-1,1',2,5'-tetrahydro-spiro[indole-3,2'-pyrrole]-2,5'-dione,

3'-(tert-butylsulfanyl)-6-chloro-1'-(3-chlorophenyl)-4'-hydroxy-1,1',2,5'-tetrahydro-spiro[indole-3,2'-pyrrole]-2,5'-dione,

6-chloro-1'-(3-chlorophenyl)-4'-hydroxy-3'-{[(4-methoxyphenyl)methyl]sulfanyl}-1,1',2,5'-tetrahydrospiro[indole-3,2'-pyrrole]-2,5'-dione,

6-chloro-1'-(3-chlorophenyl)-4'-hydroxy-3'-{[5-(morpholin-4-yl)-1,3,4-thiadiazol-2-yl]sulfanyl}-1,1',2,5'-tetrahydrospiro[indole-3,2'-pyrrole]-2,5'-dione,

6-chloro-3'-[(5-chloro-1,3-dimethyl-1H-pyrazol-4-yl)sulfanyl]-1'-(3-chlorophenyl)-4'-hydroxy-1,1',2,5'-tetrahydrospiro[indole-3,2'-pyrrole]-2,5'-dione,

methyl (2E)-4-{4-chloro-2-[6-chloro-2'-(2-methoxyphenyl)-2,6'-dioxo-3'-(propan-2-yl)-1,2,5',6'-tetrahydro-2'H-spiro[indole-3,4'-pyrrolo[3,4-c]pyrazole]-5'-yl]-phenoxy}but-2-enoate,

6-chloro-5'-(5-chloro-1-methyl-6-oxo-1,6-dihydropyridin-3-yl)-2'-(2-methoxyphenyl)-3'-(propan-2-yl)-1,2,5',6'-tetrahydro-2'H-spiro[indole-3,4'-pyrrolo[3,4-c]-pyrazole]-2,6'-dione,

6-chloro-5'-(5-chloro-2-methylphenyl)-2'-(3-methoxypyridin-4-yl)-3'-(propan-2-yl)-1,2,5',6'-tetrahydro-2'H-spiro[indole-3,4'-pyrrolo[3,4-c]pyrazole]-2,6'-dione,

6-chloro-5'-(5-chloro-2-methylphenyl)-2'-(3-ethylphenyl)-3'-(propan-2-yl)-1,2,5',6'-tetrahydro-2'H-spiro[indole-3,4'-pyrrolo[3,4-c]pyrazole]-2,6'-dione,

6-chloro-5'-(5-chloro-2-methylphenyl)-2'-(2-ethylphenyl)-3'-(propan-2-yl)-1,2,5',6'-tetrahydro-2'H-spiro[indole-3,4'-pyrrolo[3,4-c]pyrazole]-2,6'-dione,

6-chloro-5'-(5-chloro-2-methylphenyl)-2'-(2-methylphenyl)-3'-(propan-2-yl)-1,2,5',6'-tetrahydro-2'H-spiro[indole-3,4'-pyrrolo[3,4-c]pyrazole]-2,6'-dione,

6-chloro-5'-(5-chloro-2-methylphenyl)-2'-(3-methylphenyl)-3'-(propan-2-yl)-1,2,5',6'-tetrahydro-2'H-spiro[indole-3,4'-pyrrolo[3,4-c]pyrazole]-2,6'-dione,

6-chloro-5'-(5-chloro-2-methylphenyl)-2'-(2,6-dimethoxyphenyl)-3'-(propan-2-yl)-1,2,5',6'-tetrahydro-2'H-spiro[indole-3,4'-pyrrolo[3,4-c]pyrazole]-2,6'-dione,

6-chloro-5'-(5-chloro-2-methylpyridin-3-yl)-2'-(2-methoxyphenyl)-3'-(propan-2-yl)-1,2,5',6'-tetrahydro-2'H-spiro[indole-3,4'-pyrrolo[3,4-c]pyrazole]-2,6'-dione,

6-chloro-5'-(5-chloropyridin-3-yl)-2'-(2-methoxyphenyl)-3'-(propan-2-yl)-1,2,5',6'-tetrahydro-2'H-spiro[indole-3,4'-pyrrolo[3,4-c]pyrazole]-2,6'-dione,

6-chloro-5'-[5-chloro-2-(2-methanesulfonylethoxy)phenyl]-2'-(2-methoxyphenyl)-3'-(propan-2-yl)-1,2,5',6'-tetrahydro-2'H-spiro[indole-3,4'-pyrrolo[3,4-c]pyrazole]-2,6'-dione,

2'-(1-benzothiophen-3-yl)-6-chloro-5'-(5-chloro-2-methylphenyl)-3'-(propan-2-yl)-1,2,5',6'-tetrahydro-2'H-spiro[indole-3,4'-pyrrolo[3,4-c]pyrazole]-2,6'-dione,

3-[6-chloro-5'-(5-chloro-2-hydroxyphenyl)-2,6'-dioxo-3'-(propan-2-yl)-1,2,5',6'-tetrahydro-2'H-spiro[indole-3,4'-pyrrolo[3,4-c]pyrazole]-2'-yl]-4-methoxybenzamide,

3-[6-chloro-5'-(5-chloro-2-hydroxyphenyl)-2,6'-dioxo-3'-(propan-2-yl)-1,2,5',6'-tetrahydro-2'H-spiro[indole-3,4'-pyrrolo[3,4-c]pyrazole]-2'-yl]-4-methoxybenzoic acid,

6-chloro-5'-(5-chloro-2-methylphenyl)-2'-[2-(methylsulfanyl)phenyl]-3'-(propan-2-yl)-1,2,5',6'-tetrahydro-2'H-spiro[indole-3,4'-pyrrolo[3,4-c]pyrazole]-2,6'-dione,

methyl 3-[6-chloro-5'-(5-chloro-2-methylphenyl)-2,6'-dioxo-3'-(propan-2-yl)-1,2,5',6'-tetrahydro-2'H-spiro[indole-3,4'-pyrrolo[3,4-c]pyrazole]-2'-yl]-4-methoxybenzoate,

methyl 3-[6-chloro-5'-(5-chloro-2-hydroxyphenyl)-2,6'-dioxo-3'-(propan-2-yl)-1,2,5',6'-tetrahydro-2'H-spiro[indole-3,4'-pyrrolo[3,4-c]pyrazole]-2'-yl]-4-methoxybenzoate,

6-chloro-5'-(4-chloropyridin-2-yl)-3'-(propan-2-yl)-1,2,5',6'-tetrahydro-2'H-spiro-[indole-3,4'-pyrrolo[3,4-c]pyrazole]-2,6'-dione,

6-chloro-5'-(5-chloropyridin-3-yl)-3'-(propan-2-yl)-1,2,5',6'-tetrahydro-2'H-spiro-[indole-3,4'-pyrrolo[3,4-c]pyrazole]-2,6'-dione,

6-chloro-5'-(5-chloro-2-methylpyridin-3-yl)-3'-(propan-2-yl)-1,2,5',6'-tetrahydro-2'H-spiro[indole-3,4'-pyrrolo[3,4-c]pyrazole]-2,6'-dione,

6-chloro-5'-(5-chloro-2-methylphenyl)-3'-ethyl-2'-(2-ethylphenyl)-1,2,5',6'-tetrahydro-2'H-spiro[indole-3,4'-pyrrolo[3,4-c]pyrazole]-2,6'-dione,

6-chloro-5'-(cyclobutylmethyl)-2'-(2-methoxyphenyl)-3'-(propan-2-yl)-1,2,5',6'-tetrahydro-2'H-spiro[indole-3,4'-pyrrolo[3,4-c]pyrazole]-2,6'-dione,

6-chloro-5'-(2,2-dimethylpropyl)-2'-(2-methoxyphenyl)-3'-(propan-2-yl)-1,2,5',6'-tetrahydro-2'H-spiro[indole-3,4'-pyrrolo[3,4-c]pyrazole]-2,6'-dione,

6-chloro-2'-(3-chlorophenyl)-5'-(2-methoxyphenyl)-6'-(propan-2-yl)-1,2,3',5'-tetrahydro-2'H-spiro[indole-3,1'-pyrrolo[3,4-c]pyrrole]-2,3'-dione,

6-chloro-2'-(5-chloro-2-nitrophenyl)-5'-(2-methoxyphenyl)-6'-(propan-2-yl)-1,2,3',5'-tetrahydro-2'H-spiro[indole-3,1'-pyrrolo[3,4-c]pyrrole]-2,3'-dione,

2'-(2-amino-5-chlorophenyl)-6-chloro-5'-(2-methoxyphenyl)-6'-(propan-2-yl)-1,2,3',5'-tetrahydro-2'H-spiro[indole-3,1'-pyrrolo[3,4-c]pyrrole]-2,3'-dione,

N-{4-chloro-2-[6-chloro-5'-(2-methoxyphenyl)-2,3'-dioxo-6'-(propan-2-yl)-1,2,3',5'-tetrahydro-2'H-spiro[indole-3,1'-pyrrolo[3,4-c]pyrrole]-2'-yl]phenyl}methane-sulfonamide,

6-chloro-2'-(5-chloro-2-methylphenyl)-5'-(2,4-dimethoxyphenyl)-6'-(propan-2-yl)-1,2,3',5'-tetrahydro-2'H-spiro[indole-3,1'-pyrrolo[3,4-c]pyrrole]-2,3'-dione,

5'-(2H-1,3-benzodioxol-4-yl)-6-chloro-2'-(5-chloro-2-methylphenyl)-6'-(propan-2-yl)-1,2,3',5'-tetrahydro-2'H-spiro[indole-3,1'-pyrrolo[3,4-c]pyrrole]-2,3'-dione,

3-[6-chloro-2'-(5-chloro-2-methylphenyl)-2,3'-dioxo-6'-(propan-2-yl)-1,2,3',5'-tetrahydro-2'H-spiro[indole-3,1'-pyrrolo[3,4-c]pyrrole]-5'-yl]-4-methoxybenzamide,

5'-(5-amino-2-methoxyphenyl)-6-chloro-2'-(5-chloro-2-methylphenyl)-6'-(propan-2-yl)-1,2,3',5'-tetrahydro-2'H-spiro[indole-3,1'-pyrrolo[3,4-c]pyrrole]-2,3'-dione,

N-{3-[6-chloro-2'-(5-chloro-2-methylphenyl)-2,3'-dioxo-6'-(propan-2-yl)-1,2,3',5'-tetrahydro-2'H-spiro[indole-3,1'-pyrrolo[3,4-c]pyrrole]-5'-yl]-4-methoxyphenyl}-acetamide,

3-[6-chloro-2'-(5-chloro-2-methylphenyl)-2,3'-dioxo-6'-(propan-2-yl)-1,2,3',5'-tetra-hydro-2'H-spiro[indole-3,1'-pyrrolo[3,4-c]pyrrole]-5'-yl]-N,N-diethyl-4-methoxybenzene-1-sulfonamide,

4-chloro-2-[6-chloro-5'-(2-methoxyphenyl)-2,3'-dioxo-6'-(propan-2-yl)-1,2,3',5'-tetrahydro-2'H-spiro[indole-3,1'-pyrrolo[3,4-c]pyrrole]-2'-yl]benzamide,

3-[6-chloro-2'-(5-chloro-2-methylphenyl)-2,3'-dioxo-6'-(propan-2-yl)-1,2,3',5'-tetra-hydro-2'H-spiro[indole-3,1'-pyrrolo[3,4-c]pyrrole]-5'-yl]-N-(2-hydroxyethyl)-4-methoxybenzamide,

6-chloro-2'-(5-chloro-2-methylphenyl)-5'-(6-methoxy-2-oxo-2,3-dihydro-1H-1,3-benzodiazol-5-yl)-6'-(propan-2-yl)-1,2,3',5'-tetrahydro-2'H-spiro[indole-3,1'-pyrrolo-[3,4-c]pyrrole]-2,3'-dione,

N-{4-chloro-2-[(3S)-6-chloro-5'-(2-methoxyphenyl)-2,3'-dioxo-6'-(propan-2-yl)-1,2,3',5'-tetrahydro-2'H-spiro[indole-3,1'-pyrrolo[3,4-c]pyrrole]-2'-yl]phenyl}-methanesulphonamide,

6-chloro-2'-(5-chloro-2-methylpyridin-3-yl)-5'-(2,4-dimethoxyphenyl)-6'-(propan-2-yl)-1,2,3',5'-tetrahydro-2'H-spiro[indole-3,1'-pyrrolo[3,4-c]pyrrole]-2,3'-dione,

4-chloro-2-[6-chloro-5'-(2-methoxyphenyl)-2,3'-dioxo-6'-(propan-2-yl)-1,2,3',5'-tetrahydro-2'H-spiro[indole-3,1'-pyrrolo[3,4-c]pyrrole]-2'-yl]benzoic acid,

ethyl 4-chloro-2-[6-chloro-5'-(2-methoxyphenyl)-2,3'-dioxo-6'-(propan-2-yl)-1,2,3',5'-tetrahydro-2'H-spiro[indole-3,1'-pyrrolo[3,4-c]pyrrole]-2'-yl]benzoate,

6-chloro-2'-(5-chloro-2-methylphenyl)-5'-[2-methoxy-5-(morpholine-4-sulfonyl)-phenyl]-6'-(propan-2-yl)-1,2,3',5'-tetrahydro-2'H-spiro[indole-3,1'-pyrrolo[3,4-c]-pyrrole]-2,3'-dione,

6-chloro-2'-(5-chloro-2-methylphenyl)-5'-(2-methoxythiophen-3-yl)-6'-(propan-2-yl)-1,2,3',5'-tetrahydro-2'H-spiro[indole-3,1'-pyrrolo[3,4-c]pyrrole]-2,3'-dione, and

6-chloro-5'-(5-chloro-2-methylphenyl)-2'-(2,6-dimethoxypyridin-3-yl)-3'-(propan-2-yl)-1,2,5',6'-tetrahydro-2'H-spiro[indole-3,4'-pyrrolo[3,4-c]pyrazole]-2,6'-dione.

As specific compounds having configuration S at spiro carbon atom, the following can be mentioned:

(3S)-6-chloro-1'-(3-chlorophenyl)-3'-[(4-chlorophenyl)sulfanyl]-4'-hydroxy-1,1',2, 5'-tetrahydrospiro[indole-3,2'-pyrrole]-2,5'-dione,

(3S)-6-chloro-2'-(5-chloro-2-methylphenyl)-5'-(2,4-dimethoxyphenyl)-6'-(propan-2-yl)-1,2,3',5'-tetrahydro-2'H-spiro[indole-3,1'-pyrrolo[3,4-c]pyrrole]-2,3'-dione,

N-{3-[(3S)-6-chloro-2'-(5-chloro-2-methylphenyl)-2,3'-dioxo-6'-(propan-2-yl)-1,2,3',5'-tetrahydro-2'H-spiro[indole-3,1'-pyrrolo[3,4-c]pyrrole]-5'-yl]-4-methoxyphenyl}acetamide,

(3S)-5'-(5-amino-2-methoxyphenyl)-6-chloro-2'-(5-chloro-2-methylphenyl)-6'-(propan-2-yl)-1,2,3',5'-tetrahydro-2'H-spiro[indole-3,1'-pyrrolo[3,4-c]pyrrole]-2,3'-dione,

3-[(3S)-6-chloro-2'-(5-chloro-2-methylphenyl)-2,3'-dioxo-6'-(propan-2-yl)-1,2,3',5'-tetrahydro-2'H-spiro[indole-3,1'-pyrrolo[3,4-c]pyrrole]-5'-yl]-4-methoxybenzamide,

(3S)-6-chloro-2'-(5-chloro-2-methylpyridin-3-yl)-5'-(2,4-dimethoxyphenyl)-6'-(propan-2-yl)-1,2,3',5'-tetrahydro-2'H-spiro[indole-3,1'-pyrrolo[3,4-c]pyrrole]-2,3'-dione,

(3S)-6-chloro-2'-(5-chloro-2-methylphenyl)-5'-[(3S,4R)-3-methoxypiperidin-4-yl]-6'-(propan-2-yl)-1,2,3',5'-tetrahydro-2'H-spiro[indole-3,1'-pyrrolo[3,4-c]pyrrole]-2,3'-dione,

(3S)-6-chloro-5'-(5-chloropyridin-3-yl)-2'-(2-methoxyphenyl)-3'-(propan-2-yl)-1,2,5',6'-tetrahydro-2'H-spiro[indole-3,4'-pyrrolo[3,4-c]pyrazole]-2,6'-dione,

(3S)-6-chloro-5'-(5-chloro-2-methylpyridin-3-yl)-2'-(2-methoxyphenyl)-3'-(propan-2-yl)-1,2,5',6'-tetrahydro-2'H-spiro[indole-3,4'-pyrrolo[3,4-c]pyrazole]-2,6'-dione,

(3S)-6-chloro-5'-(5-chloropyridin-3-yl)-2'-(2-methoxyphenyl)-3'-(propan-2-yl)-1,2,5',6'-tetrahydro-2'H-spiro[indole-3,4'-pyrrolo[3,4-c]pyrazole]-2,6'-dione, and

(3S)-6-chloro-5'-(5-chloro-2-methylphenyl)-2'-(2-methoxyphenyl)-3'-(propan-2-yl)-1,2,5',6'-tetrahydro-2'H-spiro[indole-3,4'-pyrrolo[3,4-c]pyrazole]-2,6'-dione.

Another aspect of the invention relates to a compound of formula (IA) or (IB) for use as a medicament.

Preferably, the medicament is useful for the prevention and/or treatment of diseases selected from the group consisting of cancer, immune diseases, inflammatory conditions, allergic skin diseases associated with excessive proliferation, and viral infections.

The next aspect of the invention relates to a pharmaceutical composition comprising as an active ingredient a compound of formula (IA) or (IB) in combination with at least one pharmaceutically acceptable excipient.

The last aspect of the invention relates to compounds for use in a method of treatment and/or prevention of diseases selected from the group consisting of cancer, immune diseases, inflammatory conditions, allergic skin diseases associated with excessive proliferation, and viral infections, comprising administration of a therapeutically effective amount of a compound of formula (IA) or (IB) or a pharmaceutical composition as defined above.

The compounds of the invention may also exist in one or more tautomeric forms. Such forms although not explicitly indicated in the above formula are within the scope of the present invention. Accordingly, the compounds may be present as a mixture of tautomers or as individual tautomers.

The terms used in the present invention have the following meanings. Other terms not defined below have the meanings as those understood by those skilled in the art.

The term $C_1$-$C_6$-alkyl is a saturated, straight or branched chain hydrocarbon having 1 to 6 carbon atoms. Examples of $C_1$-$C_6$-alkyl are methyl, ethyl, n-propyl, iso-propyl, n-butyl, tert-butyl, sec-butyl, n-pentyl and n-hexyl. $C_1$-$C_6$-alkyl may be unsubstituted or substituted by substituents such as those indicated in the definition of the general formulas (IA) and (IB).

The term $C_2$-$C_6$-alkenyl is a saturated, straight or branched chain hydrocarbon radicals having from 2 to 6 carbon atoms having one double carbon-carbon bond. Examples of $C_2$-$C_6$-alkenyl are ethylene, n-propylene, n-butylene, n-pentylene and n-hexylene. $C_2$-$C_6$-alkenyl may be unsubstituted or substituted by substituents such as those indicated in the definition of the general formula (IA) or (IB).

The term "5- or 6-membered heteroaryl with one, two, three or four heteroatoms independently selected from N, O, and S" as used herein means a monocyclic heteroaromatic substituent with the specified kind and number of heteroatoms in the ring. Examples of 5- or 6-membered heteroaromatic substituent are pyrrole, thiophene, oxazole, thiazole, pyrazole, imidazole, 1,3,4-thiadiazole, tetrazole. Heteroaryl may be unsubstituted or substituted by substituents such as those indicated in the definition of general formulas (IA) and (IB).

The term "5- lub 6-membered heterocyclyl with one or two heteroatoms selected from N and O" as used herein comprises saturated or partially unsaturated heterocyclic ring of the indicated type and number of hetero atoms in the ring. Examples of 5- or 6-membered heterocyclyl are pyrrolidinyl, piperidinyl, piperazinyl, morpholinyl, thiomorpholinyl, oxazolinyl, oxazolidinyl. Such heterocyclyl may be unsubstituted or substituted by substituents such as those indicated in the definition of the general formulas (IA) and (IB).

The term "halogen" is selected from F, Cl, Br and I.

The term "adjacent" in relation to the atoms and groups, as used herein, means that the specified atom or group is located in the immediate vicinity of a second atom or group and is connected to it by not more than one bond.

Since the compounds of the invention may be acidic or basic they can form suitable acid addition salts with a base or an acid, respectively.

Pharmaceutically acceptable acid addition salt refers to those salts which retain the biological effectiveness of the free bases and which are not biologically undesirable. Acid addition salts may be formed with inorganic (mineral) acids or organic acids. As examples of acids, may be mentioned hydrochloric, hydrobromic, hydroiodic, phosphoric, sulfuric, nitric, carbonic, succinic, maleic, formic, acetic, propionic, fumaric, citric, tartaric, lactic, benzoic, salicylic, glutamic, aspartic, p-toluenesulfonic, benzenesulfonic, methanesulfonic, ethanesulfonic, naphthalenesulfonic such as 2-naphthalenesulfonic, pamoic, xinafoic, hexanoic acid.

Acid addition salt may be prepared in a simple manner by reacting a compound of formula (IA) or (IB) with a suitable inorganic or organic acid in an amount substantially equimolar to the compound (IA) or (IB), optionally in a suitable solvent such as an organic solvent to form a salt which is usually isolated for example by crystallisation and filtration. For example, the free bases of the compounds can be converted into the corresponding hydrochloride salts by treating a solution of the compound, for example, in methanol, with a stoichiometric amount of hydrochloric acid or hydrogen chloride in methanol, ethanol or diethyl ether, followed by evaporation of solvents.

Similarly, pharmaceutically acceptable base addition salts include salts derived from inorganic bases such as sodium, potassium, lithium, ammonium, calcium, magnesium, iron, zinc, copper, manganese, aluminum and the like. Salts derived from pharmaceutically acceptable non-toxic organic bases include salts of primary, secondary, and tertiary amines including naturally occurring substituted amines, cyclic amines and basic ion exchange resins, such as isopropylamine, trimethylamine, diethylamine, triethylamine, tripropylamine, and ethanolamine and triethanolamine.

Compounds of formula (IA) and (IB) can be obtained using respective methods which depend directly on the structure of the final product.

Generally, the 1,1',2,5'-tetrahydrospiro[indolo-3,2'-pirolo]-2,5'-dione core can be obtained according to the following Scheme A:

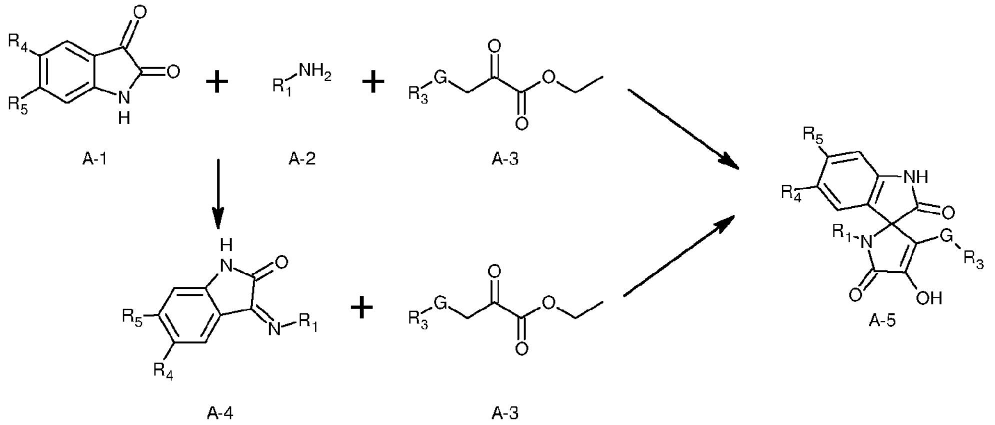

**Scheme A.**

General methodology for synthesis of compound A-5 is based on the cyclization reaction of respective imine derivative (A-4) with compound with $\alpha$-keto ester moiety (A-3). Imine derivatives can be obtained from the corresponding isatin (A-1) and amine (A-2), either in separate reaction or *in situ* during the cyclization reaction.

All cyclization reactions were carried out in suitable alcohol e.g. methanol, ethanol, propanol and/or isopropanol, optionally in the mixture of ether solvents e.g. THF, 1,4-dioxane, $Et_2O$, MTBE at the temperature range from room temperature to reflux of the solvent. The reaction was conducted in the presence of an acid (e.g. acetic and trifluoroacetic acid).

When G represents a carbonyl group (-C(O)-), the compound (A-3) can be added to reaction either in the form of diketo ester or as an enolate.

The compound A-3 with $\alpha$-keto ester group can be prepared using suitable methods known in organic synthesis.

When G represents a carbonyl group (-C(O)-), the compound (A-3) can be prepared in a Claisen type cross condensation reaction between the respective methyl ketone and diethyl oxalate in the presence of a base. The preparation of such keto esters is described in detail in Zhang, J. et al. Bioorganic & Medicinal Chemistry Letters, 2000, vol. 10, p. 2575-2578; Takeda Pharmaceutical Company Limited Patent: EP2005957 A1, 2008; Pei, Y. et al. Tetrahedron Letters, 1993, vol. 34, p. 7509-7512; Nagarapu, L. et al. European Journal of Medicinal Chemistry, 2010, vol. 45, p. 4720-4725; and Skinner, Ph. J. et al. Bioorganic and Medicinal Chemistry Letters, 2007, vol. 17, p. 5620-5623.

When G represents a single C-C bond, then the compound (A-3) can be prepared, for example, in a reaction between an aldehyde $R_3CHO$ and 1,4-diacetylpiperazine-2,5-dione or imidazolidine-2,4-dione in the presence of a base, followed by a hydrolysis of the intermediate in acidic or basic conditions. The preparation of such compounds is described in detail in Balducci, D. et. al Tetrahedron, 2012, vol. 68, p. 7374-7379; Kidwai, M., Mishrain, N. K. Green Chemistry - Environmentally Benign Approaches, InTech, Janeza Trdine, Croatia, 2012, vol. 23; Meiwes, J. et. al Tetrahedron Asymmetry, 1997, vol. 8, p. 527-536.

However, when G represents -S- (thioether), the respective compound (A-3) can be obtained by direct reaction between a thiol and ethyl bromopyruvate in the presence of an organic base. The preparation of such thio keto esters is described in detail in, for example, Hutchinson, J.H. et al. Tetrahedron Letters, 1992, vol. 33, p. 4713-4716; Beck. J. Tetrahedron, 1994 , vol. 50, p. 4691 - 4698; Wang, B. et al. US2003/13656 A1, 2003.

Compounds based on 1',2,5'-tetrahydrospiro[indolo-3,2'-pyrrolo]-2,5'-dione core fused with pyrazole ring (formula (IB) wherein X is N) can be obtained according to the following Scheme B:

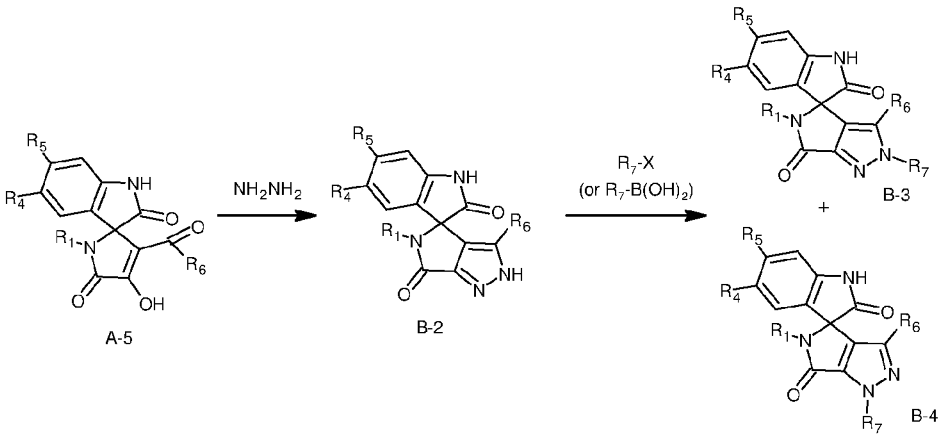


**Scheme B.**

First, Compound (A-5) (as obtained according to the method presented on Scheme A) was cyclized with hydrazine hydrate. Then, the resulted spirobicyclic compound (B-2) was subjected to an alkylation or arylation with $R_7$-X (X is halogen or tosylate group) in DMF in the presence of an inorganic base e.g. potassium carbonate or sodium hydride. Alternatively, the compound B-2 can be reacted with $R_7$-$B(OH)_2$ in the presence of copper (II) acetate and DMAP in DMF. Both methods provided the mixture of regioisomers (B-3) and (B-4) which were separated by chromatographic techniques to give desired regioisomer (B-3). [Lam, P.Y.S. et. al Tetrahedron Lett. 1998, vol. 39, p. 2941-2944]

Compounds based on 1',2,5'-tetrahydrospiro[indolo-3,2'-pyrrolo]-2,5'-dione core fused with pyrrole ring (formula (IB) wherein X is CH) may be obtained according to the Scheme C.

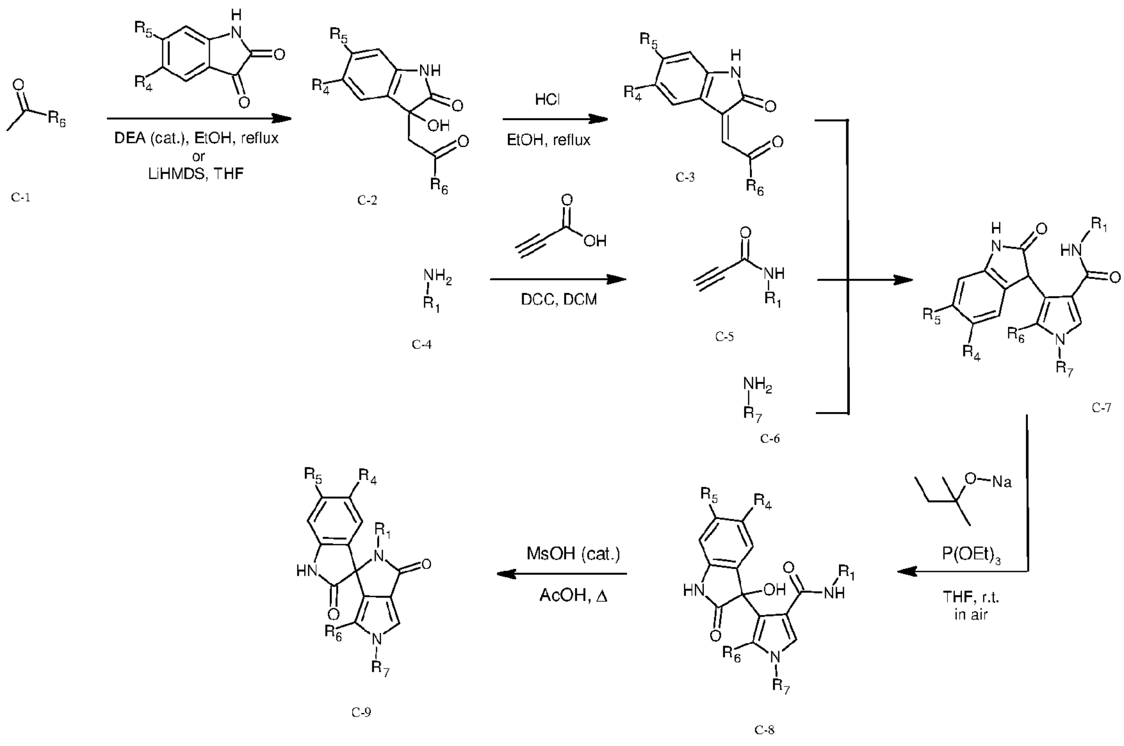


**Scheme C.**

Initially, methyl ketone (C-1) was treated with isatin in the presence of a base. The resulted aldol (C-2) was subsequently dehydrated providing $\alpha,\beta$-unsaturated carbonyl compound (C-3). In parallel, amide (C-5) was prepared by coupling of an amine C-4 with acetylenecarboxylic acid, preferably by the use of carbodiimides. Hydroamination of the alkyne C-5 with the amine C-6 and subsequent reaction with enone C-3 leads to substituted pyrrole C-7. Deprotonated

intermediate (C-7) was oxidized to 3-hydroxy-2-oxindole derivative with atmospheric oxygen. Such prepared compounds (C-8) were cyclized in acidic medium, giving the desired fused spirocyclic oxindoles. The final compound, depending on the structure of the moieties e.g. $R_1$, $R_7$ may be further modified by known methods in organic synthesis. [Popp, F. D. et al. J. Pharm. Sci., 1980, 69, p. 1235 - 1237; Asselin, Guinosso, Soll. J. Org. Chem. 1988, vol. 53, p. 2844-2847; Dong, Guang Ri et al. Synlett, 2013, vol. 24(15), p. 1993-1997; Dan Zhu, Jing Sun and Chao-Guo Yan. RSC Adv., 2014, 4, p. 62817; Han, Ying et al. Tetrahedron, 2012, vol. 68, p. 8256 - 8260; Shao, Li-Xiong et al. Org. Lett., 2013, vol. 15 (6), p. 1254-1257; Bailey, D. M., De Grazia C.G. Tetrahedron Lett., 1970, vol. 9, p. 633-636].

Furthermore, in some cases, in order to obtain compounds according to the invention, modifications of final structure were carried out according to known methods in organic chemistry, for example by introducing of conventional protecting groups, in order to fully control the desired course of the reaction. Extensive discussion on protecting groups can be found in Green, T.W. and P.G.M. Wuts, Greene's Protective Groups in Organic Synthesis (1999), 3rd Ed., Wiley.

Thus, for example, when it was desirable to obtain the final compound, wherein $R_3$ is a phenyl group substituted with acylamino group, it was necessary to prepare first a derivative wherein $R_3$ is a (BocNH)Ph group, and following deprotection, the desirable compound can be obtained by reactions with the corresponding anhydrides, acyl chlorides or acids in the presence of condensing agents such as DCC or EDCI and DMAP.

Compounds of the formula (IA), wherein -E-$R_2$ is different from -OH can be prepared as follows:

Compounds of the formula (IA), wherein -E- is -S- can be prepared from corresponding compound of formula (IA) wherein E is 0 and $R_2$ is H by nucleic substitution with corresponding thiol $R_2$-SH.

Compounds of the formula (IA), wherein -E- is -NH- can be prepared from corresponding compound of formula (IA) wherein E is 0 and $R_2$ is H by nucleic substitution with corresponding amine $R_2$-$NH_2$.

Said reactions of nucleophilic substitution reaction with thiol or amine can be carried out in a manner known in the art in the presence of an acid (acetic acid or trifluoroacetic acid) at room temperature or at elevated temperature. Such reactions are described in detail in Gein, V. L. et al. Russian Journal of Organic Chemistry, 2011, vol. 47, No. 1 p. 95-99.

As mentioned above, the compounds of the invention are for use as a medicament that is useful for the prevention and/or treatment of diseases selected from the group consisting of cancer, immune diseases, inflammatory conditions, allergic skin diseases associated with excessive proliferation, and viral infections.

In particular, the compounds according to the invention are useful for the prevention and/or treatment of diseases associated with dysregulation of the cell cycle and apoptosis, i.e. immune diseases such as for example autoimmune diseases and conditions associated with the rejection of tissue/organ transplant such as rheumatoid arthritis, graft-versus-host disease, systemic lupus erythematosus, Sjorgen's syndrome, multiple sclerosis, Hashimoto's thyreoiditis, polymyositis; chronic inflammatory conditions are asthma, osteoarthritis, atherosclerosis, Morbus Crohn; inflammatory or allergic conditions of the skin are psoriasis, contact dermatitis, atopic dermatitis, alopecia areata, erythema multiforma, dermatitis herpetiformis, scleroderma, vitiligo, hypersensitivity angiitis, urticarial, bullous pemphigoid, pemphigus, epidermolysis bullosa acquisita; hyperproliferative disorder is Li-Fraumeni syndrome; cancer or tumor diseases are benign or malignant tumors, sarcomas, such as rhabdomyosarcoma, bone cancer, e.g. osteosarcomas, carcinoma of the brain, e.g. soft tissue brain tumor, kidney, liver, adrenal gland, bladder, brest, stomach, gastric tumors, ovaries, colon, rectum, prostate, pancreas, lung, vagina or thyroid, glioblastomas, multiple myeloma, gastrointestinal cancer, especially colon carcinoma or colorectal adenoma, a tumor of the neck and head, melanoma, prostate hyperplasia, a neoplasia, a neoplasia of epithelial character, a mammary carcinoma, a leukemia, such as B- or T-cell lymphomas, adrenocortical carcinoma, including metastasis in other organs, respectively; viral infections are herpes, papilloma, HIV, hepatits.

In the treatment of the above-mentioned diseases, the compounds according to the invention can be administered as a chemical compound, but typically will be used in the form of pharmaceutical compositions, comprising a compound according to the invention or a pharmaceutically acceptable salt thereof as defined above as active ingredient, in combination with pharmaceutically acceptable carriers and excipients.

In the treatment of the abovementioned diseases, the pharmaceutical compositions of the invention they can be administered by any route, preferably orally or parenterally, and will have the form of a preparation intended for use in medicine, depending upon the intended route of administration.

Solid preparations can take the form of, for example, tablets or capsules prepared by conventional means with pharmaceutically acceptable inactive ingredients such as binding agents (eg., pregelatinized maize starch, polyvinylpyrrolidone or hydroxypropyl methylcellulose); fillers (eg. lactose, sucrose, carboxymethylcellulose, microcrystalline cellulose or calcium hydrogen phosphate); lubricants (eg. magnesium stearate, talc or silica); disintegrants (eg. crospovidone, potato starch or sodium starch glycolate); wetting agents (eg. sodium lauryl sulphate). The tablets may be coated according to methods well known in the art with conventional coatings, coatings for delaying/controlling release or enteric coatings. Liquid preparations for oral administration may take the form of, for example, solutions, syrups or suspensions, or may be presented as a dry product for reconstitution with water or other suitable vehicle before use. Such liquid

preparations may be prepared by conventional means with pharmaceutically acceptable inactive ingredients such as suspending agents (eg. sorbitol syrup, cellulose derivatives or hydrogenated edible fats); emulsifying agents (eg. lecithin or acacia); non-aqueous vehicles (np.olej almond oil, oily esters, ethyl alcohol or fractionated vegetable oils); and preservatives (eg. methyl p- or propyl hydroxybenzoate or sorbic acid). Preparations may also comprise suitable buffers, flavoring agents, coloring agents, and sweeteners.

Preparations for oral administration may be suitably formulated by methods known to those skilled in the art to obtain a controlled release of the active compound.

Parenteral administration includes administration by intramuscular and intravenous injection and infusion (infusion) intravenous. Formulations for parenteral administration may be in unit dosage form, for example, in ampoules or in multidose containers, with a preservative added. The compositions may take forms of suspensions, solutions or emulsions in oily or aqueous vehicles, and may contain formulating agents such as suspending, stabilizing and/or dispersing agents.

Alternatively, the active ingredient may be in powder form for reconstitution with a suitable vehicle, eg. sterile pyrogen-free water.

The method of treatment using the compounds of this invention will involve administration of a therapeutically effective amount of a compound of the invention, preferably in the form of a pharmaceutical composition to a subject in need of such treatment.

A proposed dose of the compounds of the present invention is from about 0.1 to about 1000 mg per day, in single or divided doses. The skilled person will appreciate that the selection of the dose required to achieve the desired biological effect will depend on a number of factors, for example the specific compound, the use, the mode of administration, the age and condition of the patient and the precise dosage will be ultimately determined at the discretion of the attendant physician.

## EXAMPLES

General information

UPLC/MS analyses were performed on a UPLC liquid chromatograph equipped with PDA detector and SQD MS detector, operating under ESI(+) or ESI(-) using C18 column, 2,1 mm x 50 mm, 1,7 μm (AQUITY UPLC BEH or equivalent). HPLC or LC/MS grade methanol, HPLC grade water, HPLC or LC/MS grade formic acid, p.a. grade 25% solution of ammonia and mixture of them were used as a mobile phase. Operating conditions were the following: mobile phase flow 0,35 ml/min, wavelength 210 - 400 nm, injection volume 1 μL, column temperature 60 °C, autosampler temperature 5 °C, gradient elution with a linear course:

| Time [min] | % A | % B | Gradient curve |
|---|---|---|---|
| 0.0 | 95 | 5 | - |
| 1.8 | 5 | 95 | linear (6) |
| 2.8 | 95 | 5 | immediate (11) |

The analysis was conducted 3.3 min + 0.5 min for "the delay of the next injection". The solutions were prepared as follows:

Preparation of the mobile phase A1 - basic gradient:

25 μl of formic acid and 250 μl of 25% ammonia solution were added to 250 ml of water. Degas using an ultrasonic bath for 10 min.

Preparation of the mobile phase A2 - acidic gradient:

50 μl of formic acid was added to 250 ml of water. Degas using an ultrasonic bath for 10 min.

Mobile phase B was Methanol Super Gradient.

**Example 1. Preparation of carbonyl compounds according to the invention**

**Example 1A. Preparation of keto esters (A-3, G=C(O)), enolate form or free keto ester**

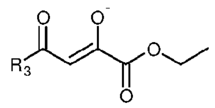

A solution of ketone (0.02 mol, 1 eq) in THF (40 ml) was cooled to -10 °C. Then, base was added portionwise: sodium t-pentoxide (0.022 mol, 1,1 eq) or potassium t-butoxide (0.02 mol, 1 eq). The reaction was carried out in the same temperature for about 1 hour. Next, diethyl oxalate was added dropwise (0.024 mol, 1.2 eq or 1 eq), and the reaction was stirred for another 5-20 hours at room temperature. After completion (TLC), the solvent was evaporated *in vacuo.* The crude product was used for further steps without purification. In some cases enol ester precipitated from reaction mixture. Using the method, sodium or potassium salt was prepared. Alternatively to isolate free ketoester, crude product was partitioned between 1N HCl and ethyl acetate. Organic phase was separated, dried and concentrated. The crude material was purified by silica gel chromatography.

**Example 1B. Preparation of pyruvic acid derivatives (A-3, G = single bond)**

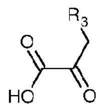

**Step 1.**

**Method 1A:** using N-acetylglycine: aldehyde derivative (6.6 mmol, 1 eq), N-acetyl glycine (1.4 eq) and sodium acetate (1.6 eq) were dissolved in 10 ml of acetic anhydride. The reaction was stirred for another 2-48 hours at reflux. The product was isolated after addition of water to the reaction mixture, followed by filtration. Then the precipitate was dissolved in 1,4-dioxane and hydrolysed using concentrated hydrochloric acid.

**Method 1B:** using 1,4-diacetyl-2,5-piperazinedione: 1,4-diacetyl-2,5-piperazinedione (8.9 mmol, 1 eq), t-BuOK (1 eq) and t-BuOH (4.5 ml) were added to aldehyde (8.9 mmol, 1 eq) dissolved in dry THF (9 ml). The reaction was stirred for 3 days at room temperature under argon. After completing of reaction the reaction mixture was washed with $NH_4Cl$, the product was extracted with AcOEt, dried over anhydrous magnesium sulfate ($MgSO_4$). The crude product was hydrolysed.

**Step 2. Hydrolysis:** The product obtained in step 1 (0.64 mmol, 1 eq) was dissolved in 1,4-dioxane (1 ml). Then 25 % HCl (3 ml) was added. The reaction was stirred at reflux for 20 hours. The product was extracted with DCM. Collected organic fractions were dried over an anhydrous $MgSO_4$, and evaporated in vacuo. The crude product was used in further step.

**Example 1C. Preparation of pyruvic acid derivatives (A-3, G = S)**

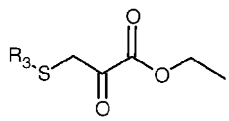

Appropriate thiol/thiophenol (20 mmol, 1 eq) was dissolved in 20 ml THF. The reaction mixture was cooled to 0 °C and base (pyridine or triethylamine) (20 mmol, 1 eq) was added. Then ethyl bromopyruvate (21 mmol, 1.05 eq) was added dropwise at 0 °C over 10 minutes. The reaction was stirred at the same temperature to its completion (by TLC), the mixture was diluted with water, acidified with 1N HCl and extracted with ethyl acetate. The combined organic

fractions were washed with brine, dried over anhydrous magnesium sulfate ($MgSO_4$) and evaporated to give a crude product, which was purified by flash column chromatography (silica gel; hexane : AcOEt; appropriate gradient) or used in the next step without further purification.

**Example 1D. Preparation of imine derivatives of isatin (A-4)**

**Method 1D-1:** A mixture of equimolar amounts of aniline (1.5 mmol, 1 eq) and isatin (1.5 mmol, 1 eq) was stirred in mixture EtOH (or 1,4-dioxane or THF): acetic acid 8:0,5 (v/v) (or p-toluenesulfonic acid 0.375 mmol) with molecular sieves 3Å. The reaction was carried out at 80 °C for 2-3 days. After cooling the imine precipitate was filtrated and used in further steps.

**Alternative method 1D-2:** A mixture of equimolar amounts of aniline (1.5 mmol, 1 eq) and isatin (1.5 mmol, 1 eq) was stirred with TBAB (0.225 mmol, 0.15 eq) in 50 ml $H_2O$. The reaction was carried out at 75 °C for 4-7 days. After cooling the product was precipitated from reaction mixture. Then, the imine was filtrated and washed several times with warm water and dried on air.

**Example 2A. Preparation of compound of formula (IA) wherein G = C(O)**

A mixture of imine (A-4) (1.25 mmol, 1 eq), keto ester (A-3, G=C(O)) (1.87 mmol, 1.5 eq) and AcOH (1.87 mmol, 1.5 eq) was stirred in 1,4-dioxane (2 ml) with molecular sieves 3Å. The reaction mixture was carried out at 70-90 °C for 20 hours. The solvent was evaporated and the residue was purified by silica gel column chromatography (hexane : AcOEt : MeOH or $CHCl_3$ : MeOH; appropriate gradient).

**Example 2A-1. Preparation of compound 13**

**Step 1. Preparation of 4-hydroxy-3,3-dimethylbutan-2-one**

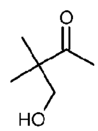

A mixture of 3,3-dimethyl-2-butanone (20 mmol, 1 eq) and paraformaldehyde (1.2 eq) in trifluoroacetic acid (TFA, 3.1 ml) was heated at 90 °C for 20 hours. After completing, the reaction mixture was cooled to room temperature and neutralized with 10% NaOH. The product was extracted with DCM. Combined organic phases were dried over anhydrous sodium sulfate ($Na_2SO_4$). After the solvent was evaporated, the crude product was used in further step.

**Step 2. Preparation of 3,3-dimethyl-4-{[(4-methylphenyl)carbonyl]oxy}butan-2-one**

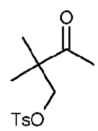

The product of step 1 (8.6 mmol, 1 eq) was dissolved in dichloromethane (17 ml) and cooled to 0 °C. Pyridine (7 eq) and p-toluenesulfonyl chloride (1.2 eq) were added at the same temperature. Then the reaction was carried out at room temperature for 20 hours. The product was purified by silica gel column chromatography (hexane : AcOEt 4:1 -> 0:1).

**Step 3. Preparation of 3,3-dimethyl-4-(propan-2-yloxy)butan-2-one**

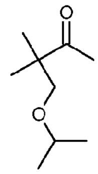

The compound of Step 2 (0.59 mmol, 1 eq) was dissolved in DCM (4 ml). Then, the triethylamine (2 eq) and 2-propanol (10 eq) was added. The reaction was carried out at room temperature for 24 hours. After completing of the reaction the solvents were evaporated and the crude product was using in further step.

The synthesis of sodium enolate and cyclic product was carried out using procedures respectively from Example 1A and 2A.

**Example 2A-2. Preparation of compound 17**

**Step 1. Synthesis of N-Boc-3-aminoacetophenone**

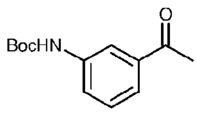

To a solution 1-(3-aminophenyl)ethan-1-one (37 mmol, 1 eq) in mixture 1,4-dioxane : water (40 : 20 ml) cooled to 0 °C, NaOH (2 eq) and di-tert-butyl dicarbonate (1.1 eq) were added. The reaction was carried out at room temperature for 12 hours. Then the reaction mixture was acidified with 3M hydrochloric acid or 2% citric acid and extracted with ethyl acetate. The organic layer was dried over anhydrous magnesium sulfate. The pure product was obtained after solvent evaporation. The preparation of enolate and cyclic compound was carried out using procedures of Examples 1A and 2A, respectively.

**Step 2. Synthesis N-Boc spirocompound**

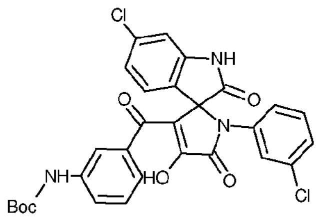

**Step 3. Cleavage of Boc group**

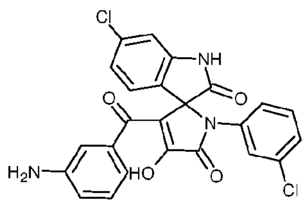

The compound of step 1 (0.21 mmol, 1 eq) was dissolved in TFA (1 ml). The reaction was carried out at room temperature for 2 hours. Then the reaction mixture was neutralized by addition of a saturated solution of sodium bicarbonate ($NaHCO_3$) and extracted with ethyl acetate. The combined organic layers were washed with sodium bicarbonate,

water and dried over anhydrous magnesium sulfate. The product was obtained after solvent evaporation.

**Step 4. Reaction of free amine group with ethyl (2E)-4-chloro-4-oxobut-2-enoate**

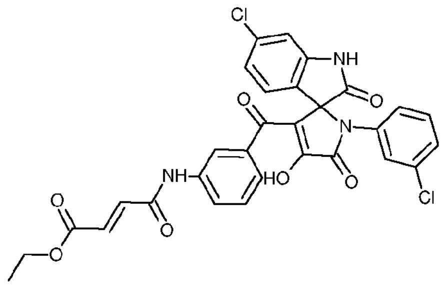

The compound of step 3 (0.19 mmol, 1 eq) was dissolved in 1,4-dioxane (1 ml) and ethyl (2E)-4-chloro-4-oxobut-2-enoate (0.19 mmol, 1 eq) in 1,4-dioxane (0.5 ml) was added.
The reaction was carried out at 60 °C for 24 hours. The product was precipitated after addition of DCM.
Using a similar approach, free amino group can be modified using other acylating agents in related conditions, i.e., using other acyl chlorides, or carboxylic acids in the presence of coupling agents, for example, carbodimides (dicyclohexylcarbodiimide).

**Example 2B. Preparation of compounds of formula (IA) wherein G=single bond**

A mixture of equimolar amounts of amine (A-2), isatin (A-1) and compound prepared in Example 1B (1 eq) was stirred in EtOH : AcOH mixture. The reaction was carried out at reflux for 24 hours. The product was purified by silica gel column chromatography (hexane : AcOEt: MeOH 2:1:0 -> 0:9:1 or $CHCl_3$ : MeOH 9:1).

**Example 2C. Preparation of compounds of formula (IA) wherein G=S**

The compound prepared in Example 1C (2-3 mmol, 1-1.5 eq) was dissolved in anhydrous THF (15 ml) and molecular sieves 4Å were added. The reaction mixture was cooled to - 20 °C. The N,N-diisopropylethylamine (1-3 eq) was added and then chlorotrimethylsilane (1-1.5 eq) was added dropwise. After stirring the mixture for 1 h at -20 °C the titanium tetrachloride (1-1.5 eq, pure or 1M solution in toluene) was added dropwise. The imine (A-4) (1 eq) was added and the reaction was carried out at 0 °C for 2 hours and then at room temperature or 40 °C for 1-2 days. The reaction mixture was diluted with ethyl acetate, washed with 5% sodium bicarbonate, brine and dried over anhydrous magnesium sulfate. The solvent was evaporated and the product was purified by using silica gel column chromatography (hexane : AcOEt; appropriate gradient).

**Example 3. Preparation of compounds with fused pyrazole ring** (formula (IB) wherein X is N)

**Example 3A. Preparation of compounds with fused unsubstituted pyrazole ring**

Compound B-1 (0.016 mol, 1 eq) was dissolved in 50 ml of acetic acid and hydrazine hydrate (2-20 eq) was added. The reaction was carried out at 70-120 °C for 1-20 hours. The solvent was evaporated and the residue was purified by using silica gel column chromatography (hexane : AcOEt or $CHCl_3$ : MeOH; appropriate gradient).

**Example 3B. Preparation of compounds with fused N-substituted pyrazole ring**

**Method 3B-1:** Coupling with boronic acid: the compound prepared in Example 3A (0.66 mmol, 1 eq) was dissolved in 10 ml DCM. The phenylboronic acid (1.5 eq), copper (II) acetate monohydrate (1.5 eq) and pyridine (2 eq) were added. The reaction was carried out at room temperature for 20 hours. The product was purified by silica gel column chromatography (hexane : AcOEt 4:1 -> 1:1). The final product was obtained as a mixture of isomers.
**Method 3B-2:** Coupling with boronic acid: the compound prepared in Example 3A (0.66 mmol, 1 eq) was dissolved in 5 ml DMF. The phenylboronic acid (2-4 eq), copper (II) acetate monohydrate (or copper acetylacetonate) (2-3 eq) and DMAP (2-4 eq) were added. The reaction was carried out at room temperature for 20 hours. The product

was purified by silica gel column chromatography (hexane : AcOEt or $CHCl_3$ : MeOH) or preparative HPLC (column: Gemini NX 5u C18 100x21,2 mm, acidic or basic gradient with MeOH or ACN).

**Method 3B-3:** The compound prepared in Example 3A (0.1 mmol, 1 eq) was dissolved in DMF (1 ml). 3-bromoprop-1-ene (1 eq), sodium hydride (2 eq) were added. The reaction was carried out at room temperature for 30 minutes. The reaction was diluted with water and extracted with ethyl acetate. The product was purified as above.

**Method 3B-4:** The compound prepared in Example 3A (0.1 mmol, 1 eq) was dissolved in 1 ml of DMF. O-alkylating agent (1-2 eq), potassium carbonate (2-5 eq) were added. The reaction was carried out at room temperature for 20 hours. The reaction was diluted with water and extracted with ethyl acetate. The product was purified as above.

The method was chosen depending on the availability of starting materials. In each of the reaction the mixture of regioisomers on pyrazole ring was obtained. The separation of regioisomers was carried out by using silica gel column chromatography or preparative HPLC (column: Gemini NX 5u C18 100x21.2 mm, MeOH or ACN with $H_2O$+HCOOH or $H_2O+HCOONH_4$).

**Example 3C: Preparation of compounds by direct modification on $R_1$**

**Method 3C-1: Preparation of Compound 62 (O-alkylation)**

The compound prepared in Example 3B (0.17 mmol, 1 eq) was dissolved in anhydrous ACN (3 ml). O-alkylating agent (1 eq) and cesium carbonate (1 eq) were added. The reaction was carried out at room temperature for 20 hours. After completion of the reaction, the cesium salt was filtered off. Crude product was concentrated *in vacuo* and purified by preparative HPLC (column: Gemini NX 5u C18 100x21.2 mm, MeOH or ACN with $H_2O$+HCOOH or $H_2O+HCOONH_4$).

**Method 3C-2: Preparation of compound 73**

The compound prepared in Example 2A (0.9 mmol, 1 eq) was dissolved in anhydrous DMF (10 ml). O-alkylating agent (1.2 eq), cesium carbonate (0.05 eq), tert-butyl alcohol (0.1 eq), were added. The reaction was stirred vigorously at 80 °C for 16 hours. The reaction was diluted with water and extracted with ethyl acetate. The crude product was purified by using silica gel column chromatography (hexane : AcOEt; appropriate gradient). Preparation of compounds with fused unsubstituted (compound B-2) and N-substituted (compound B-3) pyrazole ring were prepared according to examples 3A and 3 B.

**Example 4. Preparation of compounds with fused N-substituted pyrrole ring** (formula (IB) wherein X is C)

**Step 1. General procedures for the preparation of the intermediate (C-2)**

**Method 4A:**

To a stirred suspension of an appropriate isatin (50 mmol, 1 eq) in 100 ml of absolute ethanol, methyl ketone (C-1) (1-5 eq) and 0.05-0.1 eq of diethylamine were added. The mixture was refluxed for 1-3 days and then evaporated to dryness. The crude product was used in the next step without further purification.

**Method 4B:**

To a stirred solution of LiHMDS (2.2 eq; 1M sol. in THF) cooled to -70 °C, methyl ketone (C-1) (1.2 eq) was added dropwise. After stirring at -70 °C for 30 min, isatin (1 eq) was added portionwise. The reaction was stirred vigorously at -70 °C for about 30 min, then slowly warmed to r.t. and stirred until disappearance of the starting material. The reaction mixture was then cooled to -20 °C and quenched with an excess of acetic acid. The solution was diluted with 1N HCl and extracted with ethyl acetate. The combined organic fractions were washed with brine, dried over anhydrous $MgSO_4$ and evaporated to dryness. The product was used in the next step without further purification.

**Step 2. General procedure for the preparation of the intermediate (C-3)**

Intermediate C-2 (50 mmol, 1 eq) was suspended in 100 ml of absolute ethanol. 36% hydrochloric acid (1 eq) was added and the reaction mixture was stirred at reflux until disappearance of the starting material (UPLC/MS). The mixture was then cooled to 0 °C. The resulting precipitate was filtered, washed with a small amount of cold DCM or ethanol and dried under vacuum.

**Step 3. Procedures for the preparation of the intermediate (C-5)**

**Method 4C:**

To a solution of *N*,*N'*-Dicyclohexylcarbodiimide (10 mmol, 1.3 eq) in dry methylene chloride (100 ml) was added acetylenecarboxylic acid (1.3 eq) and the reaction mixture was stirred for 15 min at 0 °C. Then, amine (C-4) (1 eq) dissolved in 20 ml of DCM was added and the reaction mixture was stirred at room temperature. After its completion, the solid was filtered off and washed with DCM. The filtrate was concentrated under vacuum and the residue was purified by flash column chromatography.

*At this stage, commercially available amine (C-4) was used or was obtained by simple transformations, like: reduction of the corresponding nitro derivative or coupling. In some cases substrates with protected functional groups were used. For example: ethyl 2-amino-4-chlorobenzoate (Compounds: 106, 105, 99) and N-(2-amino-4-chlorophenyl)methanesulfonamide (Compound 102)

**Method 4D** (Compounds 90, 91):

5-chloro-2-nitroaniline (10 mmol, 1 eq) was dissolved in 25 ml of toluene. Next, phosphorus pentoxide (3 eq) and propiolic acid (1.5-2,0 eq) were added and the mixture was refluxed for 30 min. After cooling to room temperature, the solid was filtered off and washed with ethyl acetate. The filtrate was evaporated under vacuum. The residue was purified by column chromatography (hexane : AcOEt; gradient elution).

**Step 4. General procedure for the preparation of the intermediate C-7**

Amine (C-6) (3 mmol, 1 eq) and the intermediate (C-5) (1.0-1.1 eq) dissolved in absolute ethanol or toluene (20 ml), was stirred at 70-100 °C in a sealed tube. After 24-48 hours (enamine formation), intermediate (C-3) (1 eq) was added, and the reaction mixture was stirred again at 70-100 °C for 1 to 3 days. After completion of the reaction (monitored by UPLC/MS), the solvent was evaporated. The residue was purified by flash column chromatography (hexane : AcOEt; gradient elution) to give the final product.

**[0124]** * At this stage, commercially available amines (C-6) were used or were obtained by simple transformations like: reduction of the corresponding nitro derivatives or coupling. In some cases substrates with protected functional groups were utilized. For example: tert-butyl (3*S*,4*R*)-4-amino-3-methoxypiperidine-1-carboxylate (synthesis of Compound 103), 3-amino-N-(2-hydroxyethyl)-4-methoxybenzamide (synthesis of Compound 100).

**Step 5. General procedure for the preparation of the intermediate C-8**

Intermediate (C-7) (2 mmol, 1 eq) and triethyl phosphite (2 eq) were dissolved in 15 ml of dry THF and cooled to 0 °C. Then, sodium tert-pentoxide (3-5 eq) was added portionwise and the reaction was carried out in air, at room temperature. After 1-24 h, the mixture was cooled to 0 °C, diluted with water and acidified with 1N HCl. The postreaction mixture was extracted with ethyl acetate. The combined organic fractions were washed with brine, dried over anhydrous $MgSO_4$ and evaporated to dryness. The residue was purified by flash chromatography (hexane : AcOEt or $CHCl_3$ : MeOH; gradient elution) giving the desired product*.

**[0126]** * During the synthesis of Compounds 105 and 99, partial hydrolysis of ethyl ester was observed. Two intermediates were isolated and used separately in the next step.

**Step 6. General procedure for the preparation of the final product/ intermediate C-9**

Intermediate C-8 (1 mmol, 1 eq) was dissolved in 20 ml of glacial acetic acid. Then, 1 drop of methanesulfonic acid was added and the reaction was stirred for 1-24 h at 70-80 °C. Acetic acid was evaporated *in vocuo.* The residue was purified by flash chromatography (hexane : AcOEt or $CHCl_3$ : MeOH; gradient elution) or preparative RP-HPLC.

*During the synthesis of Compound 100, O-acetylation of free hydroxyl group was observed.

**Step 7. Specific procedures for selected Compounds**

**Method 4E: Preparation of Compound 96** (N-deacetylation)

Compound 97 (0.1 mmol, 1 eq) was dissolved in a mixture of ethanol and 3M NaOH (1:1, 10 ml). After 2 days at reflux the reaction mixture was diluted with water and extracted with ethyl acetate. The combined organic fractions were washed with brine, dried over anhydrous $MgSO_4$ and evaporated to dryness. The residue was purified by preparative

HPLC (Gemini NX C18 5u, 5 μm, 21.2x100mm; MeOH/$H_2O$+$HCO_2NH_4$; Flow: 30 mL/min, time: 10 min, UV=254).

**Method 4F: Preparation of Compound 91** (reduction of nitro group)

Compound 96 (0.1 mmol, 1 eq) was dissolved in 5 ml of methanol. Pd/C (10%) (0.1 eq) and hydrazine hydrate (5.0 eq) were added and the mixture was stirred for 1 hour at room temperature. The reaction mixture was then filtered through Celite and evaporated to dryness. The product was purified by preparative HPLC (Gemini NX C18 5u, 5 μm, 21.2x100mm; MeOH/$H_2O$+$HCO_2NH_4$; Flow: 30 mL/min, time: 10 min, UV=254).

**Method 4G: Preparation of Compound 100** (O-deacetylation)

The O-acetylated product (C-9) (Step 6) (1 mmol, 1 eq) was dissolved in 10 ml of methanol. 2 eq of potassium carbonate was added and the reaction was stirred for 24 h, at room temperature. After completion, 10 ml of water was added. The precipitate was collected, washed with water and dried under vacuum.

**Method 4H: Preparation of Compound 99** (synthesis of carboxamide)

TFFH (0.12 mmol, 1 eq) was added to a stirred solution of the acid 111 (0.12 mmol, 1 eq) and DIPEA (0.24 mmol, 2 eq) in DCM cooled to 0 °C. After 1 hour the reaction mixture was treated with concentrated aqueous ammonium hydroxide (1 ml), warmed to room temperature and stirred overnight. The reaction mixture was poured into water and extracted with DCM (30 ml). The organic layer was washed with brine (10 ml), dried over $MgSO_4$ and concentrated under vacuum. The crude residue was subjected to flash column chromatography (hexane : AcOEt).

All enantiomers were separated on preparative HPLC with chiral columns, according to the methods described in Example 6.

**Example 5. The preparation of compounds of formula (IA) with -E-$R_2$ other than -OH**

**Method 5A:**

**Step 1. Substitution with amine, for E-$R_2$=NH-$CH_2CO_2Et$:** Previously synthesized corresponding 1,1',2,5'-tetrahydrospiro[indolo-3,2'-pyrrolo]-2,5'-dione (3 mmol, 1 eq), ethyl 2-aminoacetate hydrochloride (5 eq) and TEA (10 eq) were dissolved in glacial acetic acid (10 ml). The reaction was carried out for 24 hours at reflux. After evaporation of the solvent, product was purified by silica gel column chromatography (hexane : AcOEt 4:1 -> 0:1).

**Optional step 2. Hydrolysis:** Compound obtained in step 1 (1 mmol, 1 eq) was dissolved in THF (5 ml). Then, 10% NaOH was added. The reaction was carried out at room temperature for 1 hour.

**Optional step 2. Ammonolysis:** Compound obtained in step 1 (0.04 mmol, 1 eq) was dissolved in t-BuOH (1 ml). Then, 25% aqueous solution of ammonia (5 eq) was added. The reaction was carried out at room temperature for 16 hours. The product was purified by silica gel column chromatography (hexane: AcOEt: MeOH 2:1:0 -> 0:9:1).

**Method 5B: (via derivative with -E-$R_2$=OMs)**

**Step 1.** Methanesulfonyl chloride (0.61 mmol, 1.5 eq) and TEA (0.68 mmol, 1.7 eq) were added to Compound 3 (0.4 mmol, 1 eq) dissolved in 5 ml DCM. The reaction was carried out at 0 °C for 30 minutes. Then, TEA (0.85 eq) and methanesulfonyl chloride (0.75 eq) were added twice to reaction mixture. After completion of reaction the mixture was rinsed with sodium bicarbonate. The organic layer was dried over $MgSO_4$ and concentated.

**Step 2.** Amine (for E=N) or thiol (for E=S) (0.4 mmol, 5 eq) and TEA (5 eq) were added to Compound 3 (1 eq) dissolved in 4 ml ACN. The reaction was carried out at 80 °C for 3-5 days. The product was purified by silica gel column chromatography (hexane : AcOEt 4:1 -> 0:1).

**Example 6. Separation of enantiomerically pure compounds**

All enantiomers were separated on preparative HPLC with chiral columns.

**Method 6A:** Column: Lux 5u Cellulose-1 AXIA Packed (and equivalent) 150x21.20mm with security guard PREP cartridge, column temperature: 35 °C, flow: 30ml/min, isocratic elution, mobile phase: MeOH/IPA 9:1 (v/v) with 0,1% (v)TFA, UV detection: A 240nm and 280 nm.

**Method 6B:** Column: Lux 5u Cellulose-1 AXIA Packed (or equivalent column) 150x21.20mm with security guard PREP cartridge, column temperature: 35 °C, flow: 30ml/min, gradient elution; A=MeOH, B=$H_2O$, UV detection: A 254nm.

| Time[min] | % A | % B | Gradient curve |
|---|---|---|---|
| 0.0 | 60 | 40 | - |
| 4 | 90 | 10 | linear (6) |
| 10 | 60 | 40 | immediate (11) |

**Method 6C:** Column: Lux 5u Cellulose-1 AXIA Packed (or equivalent column) 150x21.20mm with security guard PREP cartridge, column temperature: 35 °C, flow: 30 ml/min, gradient elution; A=ACN, B=$H_2O$, UV detection: λ 254nm.

| Time[min] | % A | % B | Gradient curve |
|---|---|---|---|
| 0.0 | 60 | 40 | - |
| 1.0 | 60 | 40 | linear (6) |
| 8.0 | 90 | 10 | linear (6) |
| 11.0 | 60 | 40 | immediate (11) |

**Method 6D:** Column: Lux 5u Cellulose-2 AXIA Packed (or equivalent column) 150x21.20mm with security guard PREP cartridge, column temperature: 35 °C, flow: 30 ml/min, gradient elution; A=ACN, B=$H_2O$, UV detection: λ 254nm.

| Time[min] | % A | % B | Gradient curve |
|---|---|---|---|
| 0.0 | 40 | 60 | - |
| 1.0 | 40 | 60 | linear (6) |
| 5.0 | 90 | 10 | linear (6) |
| 9.0 | 40 | 60 | immediate (11) |

**Method 6E:** Column: Lux 5u Amylose-2 AXIA Packed (or equivalent column) 150x21.20mm with security guard PREP cartridge, column temperature: 35 °C, flow: 30 ml/min, gradient elution; A=ACN, B=$H_2O$, UV detection: λ 254nm.

| Time[min] | % A | % B | Gradient curve |
|---|---|---|---|
| 0.0 | 60 | 40 | - |
| 4.0 | 90 | 10 | linear (6) |
| 10.0 | 40 | 60 | immediate (11) |

**Method 6F:** Column: Lux 5u Cellulose-4 AXIA Packed (and equivalent) 150x21.20mm with security guard PREP cartridge, column temperature: 35 °C, flow: 30 ml/min, gradient elution; A=ACN, B=$H_2O$, UV detection: λ 254nm.

| Time[min] | % A | % B | Gradient curve |
|---|---|---|---|
| 0.0 | 60 | 40 | - |
| 1.0 | 60 | 40 | linear (6) |
| 8.0 | 90 | 10 | linear (6) |
| 11.0 | 60 | 40 | immediate (11) |

**Method 6G:** Column: Lux 5u Cellulose-2 AXIA Packed (or equivalent column) 150x21.20mm with security guard PREP cartridge, column temperature: 35 °C, flow: 30 ml/min, gradient elution; A=ACN, B=$H_2O$+$HCO_2NH_4$, UV detection: A 254nm.

| Time[min] | % A | % B | Gradient curve |
|---|---|---|---|
| 0.0 | 25 | 75 | - |
| 1.0 | 25 | 75 | linear (6) |
| 12.0 | 90 | 10 | linear (6) |
| 14.0 | 25 | 75 | immediate (11) |

**Example 7. Preparation of capsule oral formulation (compound 93)**

Compound 93 (500 mg) was mixed with microcrystalline cellulose (800 mg), and magnesium stearate (15 mg) to homogeneity. Then, capsules was filled with the mixture, wherein each capsule received 131,5 mg of the mixture. As a result, a capsule containing 50 mg compound 93 was obtained.

The Compounds in Table 1 were obtained according to the above described methods (1-6).

Table 1

| Cpd No | Structure | Method | Name | Analytical Data |
|---|---|---|---|---|
| 1 | 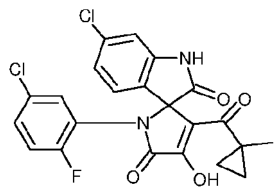 | 2A | 6-chloro-1'-(5-chloro-2-fluorophenyl)-4'-hydroxy-3'-(1-methylcyclopropanecarbonyl)-1,1',2,5'-tetrahydrospiro[indole-3,2'-pyrrole]-2,5'-dione | analysis in acidic gradient: 98%, 461 $[M+H]^+$, retention time: 1.9; analysis in basic gradient: 96%, 461 $[M+H]^+$, retention time: 1.7. |
| 2 | 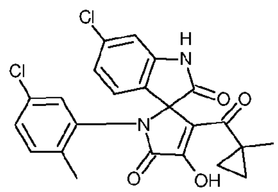 | 2A | 6-chloro-1'-(5-chloro-2-methylphenyl)-4'-hydroxy-3'-(1-methylcyclopropanecarbonyl)-1,1',2,5'-tetrahydrospiro[indole-3,2'-pyrrole]-2,5'-dione | analysis in acidic gradient: 92%, 457 $[M+H]^+$, retention time: 2.0; analysis in basic gradient: 86%, 457 $[M+H]^+$, retention time: 1.7. |

(continued)

| Cpd No | Structure | Method | Name | Analytical Data |
|---|---|---|---|---|
| 3 | 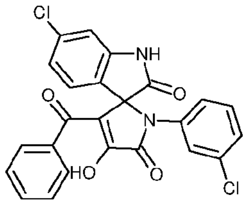 | 2A | 3'-benzoyl-6-chloro-1'-(3-chlorophenyl)-4'-hydroxy-1,1',2,5'-tetrahydrospiro [indole-3,2'-pyrrole]-2,5'-dione | analysis in acidic gradient: 94%, 465 $[M+H]^+$, retention time: 1.89; analysis in basic gradient: 91%, 465 $[M+H]^+$, retention time: 1.73. |
| 4 | 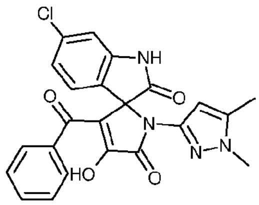 | 2A | 3'-benzoyl-6-chloro-1'-(1,5-dimethyl-1H-pyrazol-3-yl)-4'-hydroxy-1,1',2,5'-tetrahydrospiro[indole-3,2'-pyrrole]-2,5'-dione | analysis in acidic gradient: 93.7%, 449 $[M+H]^+$; analysis in basic gradient: 90%, 449 $[M+H]^+$. |
| 5 | 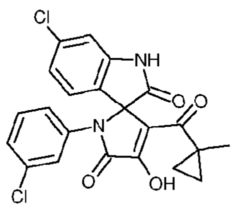 | 2A | 6-chloro-1'-(3-chlorophenyl)-4'-hydroxy-3'-(1-methylcyclopropanecarbonyl)-1,1', 2,5'-tetrahydrospiro[indole-3,2'-pyrrole]-2,5'-dione | analysis in acidic gradient: 99,1%, 443 $[M+H]^+$, retention time: 1.99; analysis in basic gradient: 90%, 443 $[M+H]^+$, retention time: 1.72. |

(continued)

| Cpd No | Structure | Method | Name | Analytical Data |
|---|---|---|---|---|
| 6 | 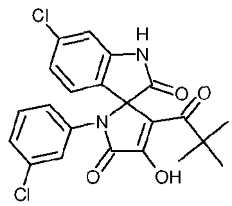 | 2A | 6-chloro-1'-(3-chlorophenyl)-3'-(2,2-tert-butanoyl)-4'-hydroxy-1,1',2,5'-tetrahydrospiro[indole-3,2'-pyrrole]-2,5'-dione | analysis in acidic gradient: 97%, 445 $[M+H]^+$, retention time: 2.05; analysis in basic gradient: 93,1%, 445 $[M+H]^+$, retention time: 1.76. |
| 7 | 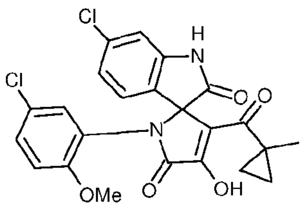 | 2A | 6-chloro-1'-(5-chloro-2-methoxyphenyl)-4'-hydroxy-3'-(1-methylcyclopropanecarbonyl)-1,1',2,5'-tetrahydrospiro[indole-3,2'-pyrrole]-2,5'-dione | analysis in acidic gradient: 99,7%, 473 $[M+H]^+$, retention time: 1.95; analysis in basic gradient: 96,7%, 473 $[M+H]^+$, retention time: 1.66. |
| 8 | 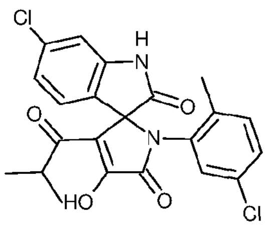 | 2A | 6-chloro-1'-(5-chloro-2-methylphenyl)-4'-hydroxy-3'-(*iso*-propanoyl)-1,1',2,5'-tetrahydrospiro[indole-3,2'-pyrrole]-2,5'-dione | analysis in acidic gradient: 100%, 445 $[M+H]^+$, retention time: 1.97; analysis in basic gradient: 100%, 445 $[M+H]^+$, retention time: 1.74. |

(continued)

| Cpd No | Structure | Method | Name | Analytical Data |
|---|---|---|---|---|
| 9 | 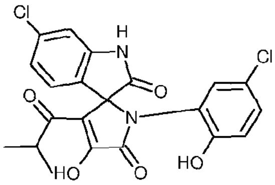 | 2A | 6-chloro-1'-(5-chloro-2-hydroxyphenyl)-4'-hydroxy-3'-(*iso*-propanoyl)-1,1',2,5'-tetrahydrospiro[indole-3,2'-pyrrole]-2,5'-dione | analysis in acidic gradient: 99,1%, 447 [M+H]+, retention time: 1.80; analysis in basic gradient: 94,9%, 447 [M+H]+, retention time: 1.51. |
| 10 | 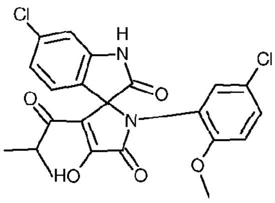 | 2A | 6-chloro-1'-(5-chloro-2-methoxyphenyl)-4'-hydroxy-3'-(*iso*-propanoyl)-1,1',2,5'-tetrahydrospiro[indole-3,2'-pyrrole]-2,5'-dione | analysis in acidic gradient: 98,4%, 461 [M+H]+, retention time: 1.87; analysis in basic gradient: 98,1%, 461 [M+H]+, retention time: 1.64. |
| 11 | 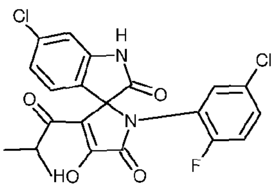 | 2A | 6-chloro-1'-(5-chloro-2-fluorophenyl)-4'-hydroxy-3'-(*iso*-propanoyl)-1,1',2,5'-tetrahydrospiro[indole-3,2'-pyrrole]-2,5'-dione | analysis in acidic gradient: 98.5%, 449 [M+H]+, retention time: 1.84; analysis in basic gradient: 99,5%, 449 [M+H]+, retention time: 1.64. |

(continued)

| Cpd No | Structure | Method | Name | Analytical Data |
|---|---|---|---|---|
| 12 | 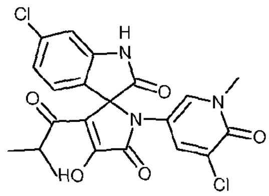 | 2A | 6-chloro-1'-(5-chloro-1-methyl-6-oxo-1,6-dihydropyridin-3-yl)-4'-hydroxy-3'-(*iso*propanoyl)-1,1',2,5'-tetrahydrospiro [indole-3,2'-pyrrole]-2,5'-dione | analysis in acidic gradient: 86,4%, 460 $[M+H]^+$, retention time: 1.62; analysis in basic gradient: 93,7%, 462 $[M+H]^+$, retention time: 1.42. |
| 13 | 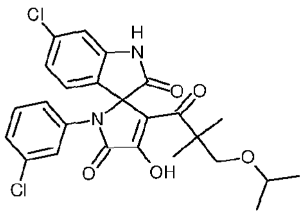 | 2A-1 | 6-chloro-1'-(3-chlorophenyl)-3'-[2,2-dimethyl-3-(propan-2-yloxy)propanoyl]-4'-hydroxy-1,1',2,5'-tetrahydrospiro[indole-3,2'-pyrrole]-2,5'-dione | analysis in acidic gradient: 99%, 503 $[M+H]^+$, retention time: 1.92; analysis in basic gradient: 98,5%, 503 $[M+H]^+$, retention time: 1.72. |
| 14 | 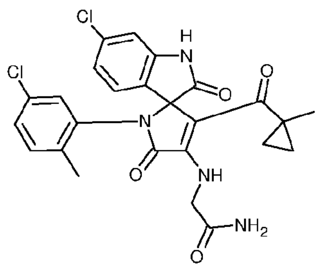 | 5 | 2-{[6-chloro-1'-(5-chloro-2-methylphenyl)-3'-(1-methylcyclopropanecarbonyl)-2,5'-dioxo-1,1',2,5'-tetrahydrospiro[indole-3,2'-pyrrole]-4'-yl]amino}acetamide | analysis in acidic gradient: 96%, 513 $[M+H]^+$, retention time: 1.96; analysis in basic gradient: 79%, 513 $[M+H]^+$, retention time: 1.96. |

(continued)

| Cpd No | Structure | Method | Name | Analytical Data |
|---|---|---|---|---|
| 15 | 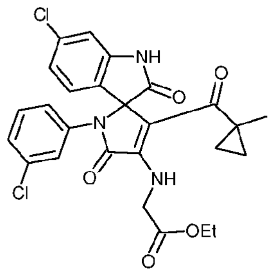 | 5 | ethyl 2-{[6-chloro-1'-(3-chlorophenyl)-3'-(1-methylcyclopropanecarbonyl)-2,5'-dioxo-1,1',2,5'-tetrahydrospiro[indole-3,2'-pyrrole]-4'-yl]amino}acetate | analysis in acidic gradient: 94.4%, 528 [M+H]+, retention time: 2.10; analysis in basic gradient: 85.2%, 528 [M+H]+, retention time: 2.10. |
| 16 | 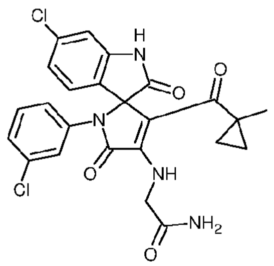 | 5 | 2-{[6-chloro-1'-(3-chlorophenyl)-3'-(1-methylcyclopropanecarbonyl)-2,5'-dioxo-1,1',2,5'-tetrahydrospiro[indole-3,2'-pyrrole]-4'-yl]amino}acetamide | analysis in acidic gradient: 90%, 499 [M+H]+, retention time: 1.95; analysis in basic gradient: 83%, 499 [M+H]+, retention time: 1.72. |
| 17 | 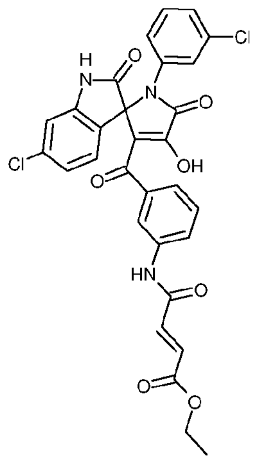 | 2A-2 | ethyl (2E)-3-[(3-{[6-chloro-1'-(3-chlorophenyl)-4'-hydroxy-2,5'-dioxo-1,1', 2,5'-tetrahydrospiro[indole-3,2'-pyrrole]-3'-yl]carbonyl}phenyl)carbamoyl]prop-2-enoate | analysis in acidic gradient: 100%, 606 [M+H]+, retention time: 1.95; analysis in basic gradient: 97,8%, 606 [M+H]+, retention time: 1.83. |

(continued)

| Cpd No | Structure | Method | Name | Analytical Data |
|---|---|---|---|---|
| 18 | 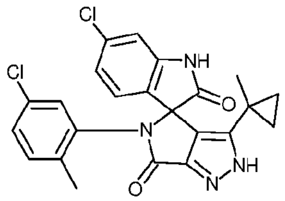 | 3A | 6-chloro-5'-(5-chloro-2-methylphenyl)-3'-(1-methylcyclopropyl)-1,2,5',6'-tetrahydro-2'H-spiro[indole-3,4'-pyrrolo [3,4-c]pyrazole]-2,6'-dione | analysis in acidic gradient: 95%, 453 $[M+H]^+$, retention time: 1.96; analysis in basic gradient: 96%, 453 $[M+H]^+$, retention time: 1.96. |
| 19 | 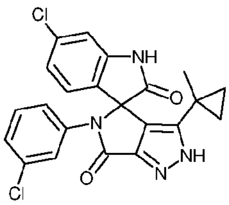 | 3A | 6-chloro-5'-(3-chlorophenyl)-3'-(1-methylcyclopropyl)-1,2,5',6'-tetrahydro-2'H-spiro[indole-3,4'-pyrrolo[3,4-c] pyrazole]-2,6'-dione | analysis in acidic gradient: 92%, 439 $[M+H]^+$, retention time: 1.98; analysis in basic gradient: 91%, 439 $[M+H]^+$, retention time: 1.98. |
| 20 | 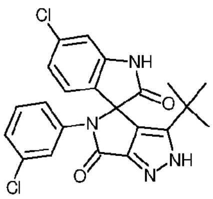 | 3A | 3'-*tert*-butyl-6-chloro-5'-(3-chlorophenyl)-1,2,5',6'-tetrahydro-2'H-spiro[indole-3,4'-pyrrolo[3,4-c]pyrazole]-2,6'-dione | analysis in acidic gradient: 98%, 441 $[M+H]^+$, retention time: 1.99; analysis in basic gradient: 95%, 441 $[M+H]^+$, retention time: 1.99. |

(continued)

| Cpd No | Structure | Method | Name | Analytical Data |
|---|---|---|---|---|
| 21 | 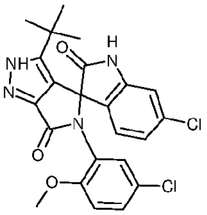 | 3A | 3'-*tert*-butyl-6-chloro-5'-(5-chloro-2-methoxyphenyl)-1,2,5',6'-tetrahydro-2'H-spiro[indole-3,4'-pyrrolo[3,4-c]pyrazole]-2,6'-dione | analysis in acidic gradient: 99,5%, 471 $[M+H]^+$, retention time: 1.96; analysis in basic gradient: 85,5%, 471 $[M+H]^+$, retention time: 1.99. |
| 22 | 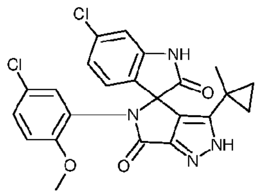 | 3A | 6-chloro-5'-(5-chloro-2-methoxyphenyl)-3'-(1-methylcyclopropyl)-1,2,5',6'-tetrahydro-2'H-spiro[indole-3,4-pyrrolo [3,4-c]pyrazole]-2,6-dione | analysis in acidic gradient: 92,9%, 469 $[M+H]^+$, retention time: 1.94; analysis in basic gradient: 92,5%, 469 $[M+H]^+$, retention time: 1.92. |
| 23 | 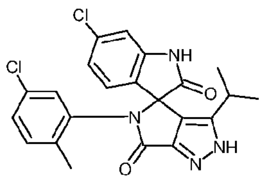 | 3A | 6-chloro-5'-(5-chloro-2-methylphenyl)-3'-(propan-2-yl)-1,2, 5',6'-tetrahydro-2'H-spiro[indole-3,4'-pyrrolo[3,4-c]pyrazole]-2,6'-dione | analysis in acidic gradient: 99.1%, 441 $[M+H]^+$, retention time: 1.99; analysis in basic gradient: 99.5%, 441 $[M+H]^+$, retention time: 1.98. |

(continued)

| Cpd No | Structure | Method | Name | Analytical Data |
|---|---|---|---|---|
| 24 | 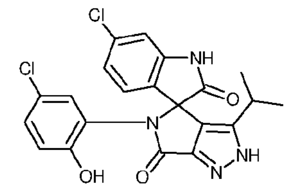 | 3A | 6-chloro-5'-(5-chloro-2-hydroxyphenyl)-3'-(propan-2-yl)-1,2, 5',6'-tetrahydro-2'H-spiro[indole-3,4'-pyrrolo[3,4-c]pyrazole]-2,6'-dione | analysis in acidic gradient: 99.7%, 443 $[M+H]^+$, retention time: 1.86; analysis in basic gradient: 94.1%, 443 $[M+H]^+$, retention time: 1.83. |
| 25 | 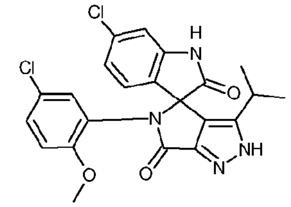 | 3A | 6-chloro-5'-(5-chloro-2-methoxyphenyl)-3'-(propan-2-yl)-1,2,5',6'-tetrahydro-2'H-spiro[indole-3,4'-pyrrolo[3,4-c]pyrazole]-2,6'-dione | analysis in acidic gradient: 99.9%, 457 $[M+H]^+$, retention time: 1.91; analysis in basic gradient: 92.3%, 457 $[M+H]^+$, retention time: 1.89. |
| 26 | 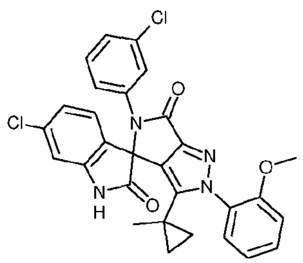 | 3B | 6-chloro-5'-(3-chlorophenyl)-2'-(2-methoxyphenyl)-3'-(1-methylcyclopropyl)-1,2,5',6'-tetrahydro-2'H-spiro[indole-3,4'-pyrrolo[3,4-c]pyrazole]-2,6'-dione | analysis in acidic gradient: 98.5%, 545 $[M+H]^+$, retention time: 2.08; analysis in basic gradient: 97.8%, 545 $[M+H]^+$, retention time: 2.08. |

(continued)

| Cpd No | Structure | Method | Name | Analytical Data |
|---|---|---|---|---|
| 27 | 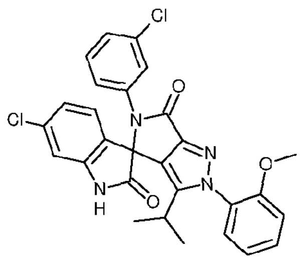 | 3B | 6-chloro-5'-(3-chlorophenyl)-2'-(2-methoxyphenyl)-3'-(propan-2-yl)-1,2,5',6'-tetrahydro-2'H-spiro[indole-3,4'-pyrrolo [3,4-c]pyrazole]-2,6'-dione | analysis in acidic gradient: 99.7%, 533 $[M+H]^+$, retention time: 2.07; analysis in basic gradient: 99.7%, 533 $[M+H]^+$, retention time: 2.06. |
| 28 | 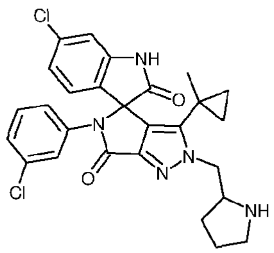 | 3B | 6-chloro-5'-(3-chlorophenyl)-3'-(1-methylcyclopropyl)-2'-(pyrrolidin-2-ylmethyl)-1,2,5',6'-tetrahydro-2'H-spiro [indole-3,4'-pyrrolo[3,4-c]pyrazole]-2,6'-dione | analysis in acidic gradient: 98%, 522 $[M+H]^+$, retention time: 1.70; analysis in basic gradient: 95%, 522 $[M+H]^+$, retention time: 2.04. |
| 29 | 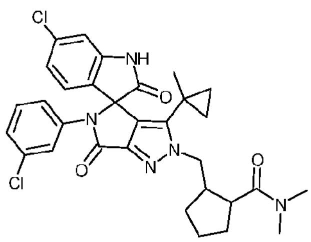 | 3B | 2-[6-chloro-5'-(3-chlorophenyl)-3'-(1-methylcyclopropyl)-2,6'-dioxo-1,2,5',6'-tetrahydro-2'H-spiro[indole-3,4'-pyrrolo [3,4-c]pyrazole]-2'-ylmethyl]-N,N-dimethylcyclopentane-1-carboxamide | analysis in acidic gradient: 96.8%, 592 $[M+H]^+$, retention time: 2.07; analysis in basic gradient: 95.7%, 592 $[M+H]^+$, retention time: 2.07. |

(continued)

| Cpd No | Structure | Method | Name | Analytical Data |
|---|---|---|---|---|
| 30 | 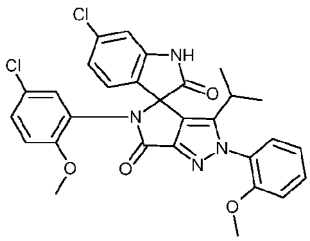 | 3B | 6-chloro-5'-(5-chloro-2-methoxyphenyl)-2'-(2-methoxyphenyl)-3'-(propan-2-yl)-1,2,5',6'-tetrahydro-2'H-spiro[indole-3,4'-pyrrolo[3,4-c]pyrazole]-2,6'-dione | analysis in acidic gradient: 97.6%, 563 $[M+H]^+$, retention time: 2.04; analysis in basic gradient: 92.4%, 563 $[M+H]^+$, retention time: 2.04. |
| 31 | 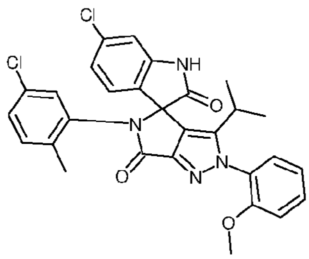 | 3B | 6-chloro-5'-(5-chloro-2-methylphenyl)-2'-(2-methoxyphenyl)-3'-(propan-2-yl)-1,2,5',6'-tetrahydro-2'H-spiro[indole-3,4'-pyrrolo[3,4-c]pyrazole]-2,6'-dione | analysis in acidic gradient: 100%, 547 $[M+H]^+$, retention time: 2.09; analysis in basic gradient: 98.2%, 547 $[M+H]^+$, retention time: 2.09. |
| 32 | 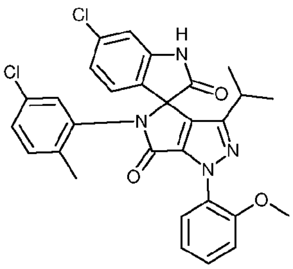 | 3B | 6-chloro-5'-(5-chloro-2-methylphenyl)-1'-(2-methoxyphenyl)-3'-(propan-2-yl)-1,2,5',6'-tetrahydro-1'H-spiro[indole-3,4'-pyrrolo[3,4-c]pyrazole]-2,6'-dione (not of the invention, comparative compound) | analysis in acidic gradient: 99%, 547 $[M+H]^+$, retention time: 2.17. |
| 33 | 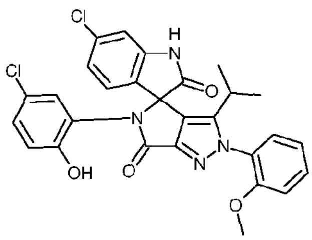 | 3B | 6-chloro-5'-(5-chloro-2-hydroxyphenyl)-1'-(2-methoxyphenyl)-3'-(propan-2-yl)-1,2,5',6'-tetrahydro-1'H-spiro[indole-3,4'-pyrrolo[3,4-c]pyrazole]-2,6'-dione | analysis in acidic gradient: 100%, 549 $[M+H]^+$, retention time: 1.99; analysis in basic gradient: 99.7%, 549 $[M+H]^+$, retention time: 1.99. |

(continued)

| Cpd No | Structure | Method | Name | Analytical Data |
|---|---|---|---|---|
| 34 | 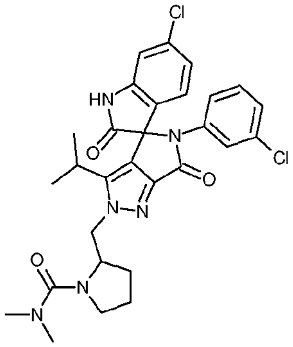 | 3B | 2-[6-chloro-5'-(3-chlorophenyl)-2,6'-dioxo-3'-(propan-2-yl)-1,2,5',6'-tetrahydro-2'H-spiro[indole-3,4'-pyrrolo[3,4-c]pyrazole]-2'-ylmethyl]-N,N-dimethylpyrrolidine-1-carboxamide | analysis in acidic gradient: 99.8%, 581 [M+H]+, analysis in basic gradient: 97.8%, 584 [M+H]+. |
| 35 | 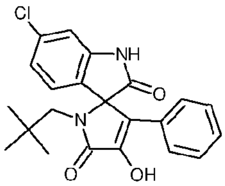 | 2B | 6-chloro-1'-(2,2-dimethylpropyl)-4'-hydroxy-3'-phenyl-1,1',2,5'-tetrahydrospiro[indole-3,2'-pyrrole]-2,5'-dione | analysis in acidic gradient: 98%, 397 [M+H]+, retention time: 2.09; analysis in basic gradient: 97%, 397 [M+H]+, retention time: 1.85. |
| 36 | 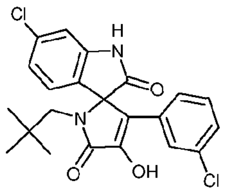 | 2B | 6-chloro-3'-(3-chlorophenyl)-1'-(2,2-dimethylpropyl)-4'-hydroxy-1,1',2,5'-tetrahydro-spiro[indole-3,2'-pyrrole]-2,5'-dione | analysis in acidic gradient: 96%, 431 [M+H]+, retention time: 2.20; analysis in basic gradient: 97%, 431 [M+H]+, retention time: 1.90. |

(continued)

| Cpd No | Structure | Method | Name | Analytical Data |
|---|---|---|---|---|
| 37 | 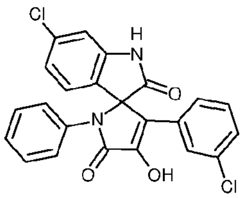 | 2B | 6-chloro-3'-(3-chlorophenyl)-4'-hydroxy-1'-phenyl-1,1',2,5'-tetrahydrospiro[indole-3,2'-pyrrole]-2,5'-dione | analysis in acidic gradient: 100%, 437 $[M+H]^+$, retention time: 2.10; analysis in basic gradient: 95%, 437 $[M+H]^+$, retention time: 1.80. |
| 38 | 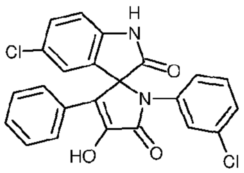 | 2B | 5-chloro-1'-(3-chlorophenyl)-4'-hydroxy-3'-phenyl-1,1',2,5'-tetrahydrospiro[indole-3,2'-pyrrole]-2,5'-dione | analysis in acidic gradient: 99%, 437 $[M+H]^+$, retention time: 2.00; analysis in basic gradient: 98%, 437 $[M+H]^+$, retention time: 1.70. |
| 39 | 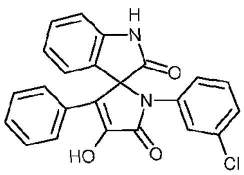 | 2B | 1'-(3-chlorophenyl)-4'-hydroxy-3'-phenyl-1,1',2,5'-tetrahydrospiro[indole-3,2'-pyrrole]-2,5'-dione | analysis in acidic gradient: 99%, 403 $[M+H]^+$, retention time: 1.90; analysis in basic gradient: 100%, 403 $[M+H]^+$, retention time: 1.60. |

(continued)

| Cpd No | Structure | Method | Name | Analytical Data |
|---|---|---|---|---|
| 40 | 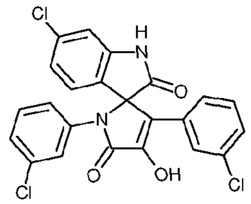 | 2B | 6-chloro-1',3'-bis(3-chlorophenyl)-4'-hydroxy-1,1',2,5'-tetrahydrospiro[indole-3,2'-pyrrole]-2,5'-dione | analysis in acidic gradient: 97%, 471 $[M+H]^+$, retention time: 2.10; analysis in basic gradient: 97%, 471 $[M+H]^+$, retention time: 1.80. |
| 41 | 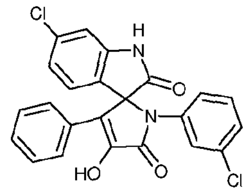 | 2B | 6-chloro-1'-(3-chlorophenyl)-4'-hydroxy-3'-phenyl-1,1',2,5'-tetrahydrospiro[indole-3,2'-pyrrole]-2,5'-dione | analysis in acidic gradient: 97%, 437 $[M+H]^+$, retention time: 2.10; analysis in basic gradient: 97%, 437 $[M+H]^+$, retention time: 1.80. |
| 42 | 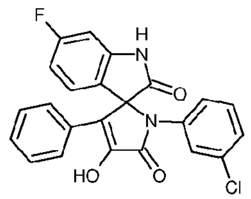 | 2B | 1'-(3-chlorophenyl)-6-fluoro-4'-hydroxy-3'-phenyl-1,1',2,5'-tetrahydrospiro[indole-3,2'-pyrrole]-2,5'-dione | analysis in acidic gradient: 98,8%, 421 $[M+H]^+$, retention time: 1.96; analysis in basic gradient: 98,2%, 421 $[M+H]^+$, retention time: 1.67. |

(continued)

| Cpd No | Structure | Method | Name | Analytical Data |
|---|---|---|---|---|
| 43 | 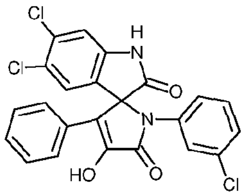 | 2B | 5,6-dichloro-1'-(3-chlorophenyl)-4'-hydroxy-3'-phenyl-1,1',2,5'-tetrahydrospiro[indole-3,2'-pyrrole]-2,5'-dione | analysis in acidic gradient: 95,8%, 471 [M+H]+, retention time: 2.13; analysis in basic gradient: 95,4%, 471 [M+H]+, retention time: 1.87. |
| 44 | 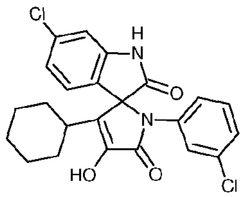 | 2B | 6-chloro-1'-(3-chlorophenyl)-3'-cyclohexyl-4'-hydroxy-1,1',2,5'-tetrahydrospiro[indole-3,2'-pyrrole]-2,5'-dione | analysis in acidic gradient: 97,8%, 443 [M+H]+, retention time: 2.18; analysis in basic gradient: 98,6%, 443 [M+H]+, retention time: 2.14. |
| 45 | 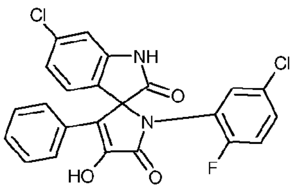 | 2B | 6-chloro-1'-(5-chloro-2-fluorophenyl)-4'-hydroxy-3'-phenyl-1,1',2,5'-tetrahydrospiro[indole-3,2'-pyrrole]-2,5'-dione | analysis in acidic gradient: 96,6%, 455 [M+H]+, retention time: 2.03; analysis in basic gradient: 96,9%, 455 [M+H]+, retention time: 1.74. |

(continued)

| Cpd No | Structure | Method | Name | Analytical Data |
|---|---|---|---|---|
| 46 | 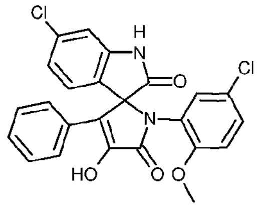 | 2B | 6-chloro-1'-(5-chloro-2-methoxyphenyl)-4'-hydroxy-3'-phenyl-1,1',2,5'-tetrahydrospiro[indole-3,2'-pyrrole]-2,5'-dione | analysis in acidic gradient: 100%, 467 [M+H]+, retention time: 1.99; analysis in basic gradient: 100%, 467 [M+H]+, retention time: 1.76. |
| 47 | 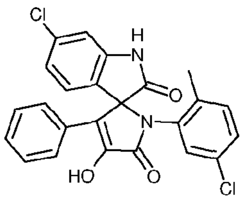 | 2B | 6-chloro-1'-(5-chloro-2-methylphenyl)-4'-hydroxy-3'-phenyl-1,1',2,5'-tetrahydrospiro[indole-3,2'-pyrrole]-2,5'-dione | analysis in acidic gradient: 100%, 451 [M+H]+, retention time: 2.06; analysis in basic gradient: 100%, 451 [M+H]+, retention time: 1.78. |
| 48 | 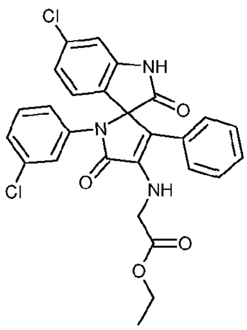 | 5 | ethyl 2-{[6-chloro-1'-(3-chlorophenyl)-2,5'-dioxo-3'-phenyl-1,1',2,5'-tetrahydrospiro [indole-3,2'-pyrrole]-4'-yl]amino}acetate | analysis in acidic gradient: 96%, 522 [M+H]+, retention time: 2.11; analysis in basic gradient: 95%, 522 [M+H]+, retention time: 2.11. |
| 49 | 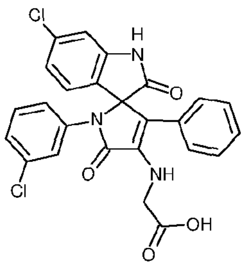 | 5 | 2-{[6-chloro-1'-(3-chlorophenyl)-2,5'-dioxo-3'-phenyl-1,1',2,5'-tetrahydrospiro [indole-3,2'-pyrrole]-4'-yl]amino}acetic acid | analysis in acidic gradient: 96%, 494 [M+H]+, retention time: 1.70. |

(continued)

| Cpd No | Structure | Method | Name | Analytical Data |
|---|---|---|---|---|
| 50 | 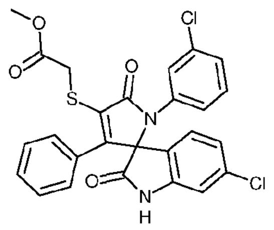 | 5 | methyl 2-[6-chloro-1'-(3-chlorophenyl)-2,5'-dioxo-3'-phenyl-1,1',2,5'-tetrahydro-spiro[indole-3,2'-pyrrole]-4'-ylsulfanyl] acetate | analysis in acidic gradient: 94.7%, 525 $[M+H]^+$, retention time: 2.10; analysis in basic gradient: 91.4%, 525 $[M+H]^+$, retention time: 2.10. |
| 51 | 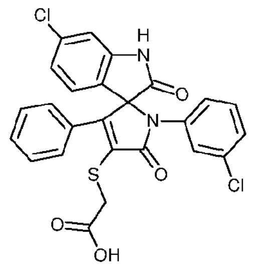 | 5 | 2-[6-chloro-1'-(3-chlorophenyl)-2,5'-dioxo-3'-phenyl-1,1',2,5'-tetrahydrospiro[indole-3,2'-pyrrole]-4'-ylsulfanyl]acetic acid | analysis in acidic gradient: 89.7%, 511 $[M+H]^+$, retention time: 1.98; analysis in basic gradient: 90,6%, 511 $[M+H]^+$, retention time: 1.71. |
| 52 | 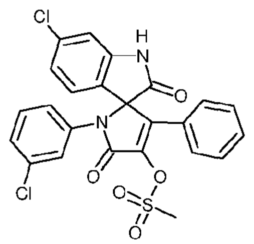 | 5 | 6-chloro-1(3-chlorophenyl)-2,5'-dioxo-3'-phenyl-1,1',2,5'-tetrahydrospiro[indole-3,2'-pyrrole]-4'-yl methanesulfonate | analysis in acidic gradient: 96.8%, 515 $[M+H]^+$, retention time: 2.02; analysis in basic gradient: 97,5%, 515 $[M+H]^+$, retention time: 2.05. |

(continued)

| Cpd No | Structure | Method | Name | Analytical Data |
|---|---|---|---|---|
| 53 | 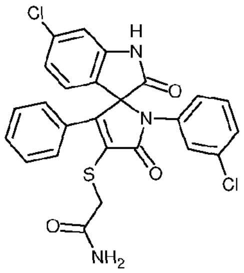 | 6 | 2-[6-chloro-1'-(3-chlorophenyl)-2,5'-dioxo-3'-phenyl-1,1',2,5'-tetrahydrospiro[indole-3,2'-pyrrole]-4'-ylsulfanyl]acetamide | analysis in acidic gradient: 98.4%, 510 $[M+H]^+$, retention time: 1.93; analysis in basic gradient: 100%, 510 $[M+H]^+$, retention time: 1.92. |
| 54 | 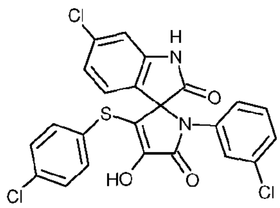 | 2C | 6-chloro-1'-(3-chlorophenyl)-3'-[(4-chlorophenyl)sulfanyl]-4'-hydroxy-1,1',2,5-tetrahydrospiro[indole-3,2'-pyrrole]-2,5'-dione | analysis in acidic gradient: 100%, 503 $[M+H]^+$, retention time: 2.16; analysis in basic gradient: 100%, 503 $[M+H]^+$, retention time: 1.90. |
| 55 | 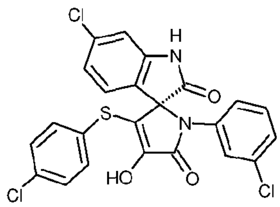 | 6A | (3*S*)-6-chloro-1'-(3-chlorophenyl)-3'-[(4-chlorophenyl)sulfanyl]-4'-hydroxy-1,1',2,5-tetrahydrospiro[indole-3,2'-pyrrole]-2,5-dione | chiral analysis: 100%, retention time: 1.60. |
| 56 | 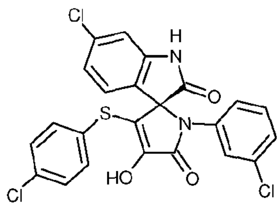 | 6A | (3*R*)-6-chloro-1'-(3-chlorophenyl)-3'-[(4-chlorophenyl)sulfanyl]-4-hydroxy-1,1',2,5'-tetrahydrospiro[indole-3,2'-pyrrole]-2,5'-dione | chiral analysis: 100%, retention time: 2.40. |

(continued)

| Cpd No | Structure | Method | Name | Analytical Data |
|---|---|---|---|---|
| 57 | 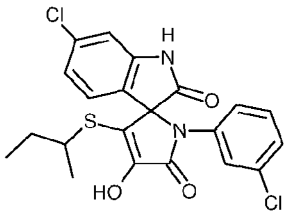 | 2C | 3'-(butan-2-ylsulfanyl)-6-chloro-1'-(3-chlorophenyl)-4'-hydroxy-1,1',2,5'-tetrahydrospiro[indole-3,2'-pyrrole]-2,5'-dione | analysis in acidic gradient: 99.6%, 449 $[M+H]^+$, retention time: 2.12; analysis in basic gradient: 98.7%, 449 $[M+H]^+$, retention time: 1.84. |
| 58 | 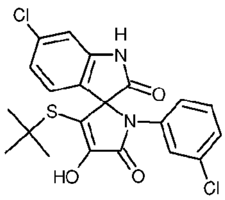 | 2C | 3'-(*tert*-butylsulfanyl)-6-chloro-1'-(3-chlorophenyl)-4'-hydroxy-1,1',2,5'-tetrahydrospiro[indole-3,2'-pyrrole]-2,5'-dione | analysis in acidic gradient: 99.5%, 449 $[M+H]^+$, retention time: 2.01; analysis in basic gradient: 99%, 449 $[M+H]^+$, retention time: 1.85. |
| 59 | 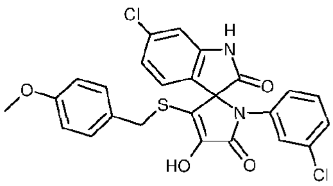 | 2C | 6-chloro-1'-(3-chlorophenyl)-4'-hydroxy-3'-{[(4-methoxyphenyl)methyl]sulfanyl}-1,1',2,5'-tetrahydrospiro[indole-3,2'-pyrrole]-2,5'-dione | analysis in acidic gradient: 99%, 513 $[M+H]^+$, retention time: 2.11; analysis in basic gradient: 98.9%, 513 $[M+H]^+$, retention time: 1.84. |

(continued)

| Cpd No | Structure | Method | Name | Analytical Data |
|---|---|---|---|---|
| 60 | 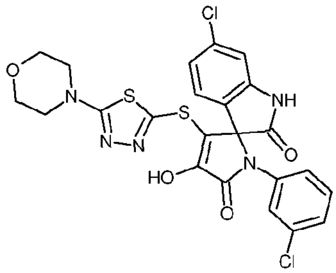 | 2C | 6-chloro-1'-(3-chlorophenyl)-4'-hydroxy-3'-{[5-(morpholin-4-yl)-1,3,4-thiadiazol-2-yl]sulfanyl}-1,1',2,5'-tetrahydrospiro [indole-3,2'-pyrrole]-2,5'-dione | analysis in acidic gradient: 100%, 562 $[M+H]^+$, retention time: 1.91; analysis in basic gradient: 95.9%, 562 $[M+H]^+$, retention time: 1.,68. |
| 61 | 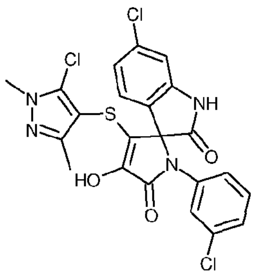 | 2C | 6-chloro-3'-[(5-chloro-1,3-dimethyl-1H-pyrazol-4-yl)sulfanyl]-1'-(3-chlorophenyl)-4'-hydrooy-1,1',2,5'-tetrahydrospiro [indole-3,2'-pyrrole]-2,5'-dione | analysis in acidic gradient: 95.3%, 521 $[M+H]^+$, retention time: 1.99; analysis in basic gradient: 92.3%, 521 $[M+H]^+$, retention time: 1.70. |
| 62 | 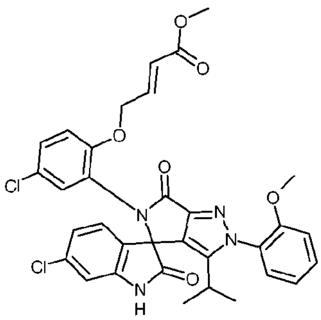 | 3C | methyl (2E)-4-{4-chloro-2-[6-chloro-2'-(2-methoxyphenyl)-2,6'-dioxo-3'-(propan-2-yl)-1,2,5',6'-tetrahydro-2'H-spiro[indole-3,4'-pyrrolo[3,4-c]pyrazole]-5'-yl]phenoxy} but-2-enoate | analysis in acidic gradient: 98.8%, 647 $[M+H]^+$, retention time: 2.03; analysis in basic gradient: 99.5%, 647 $[M+H]^+$, retention time: 2.04. |

(continued)

| Cpd No | Structure | Method | Name | Analytical Data |
|---|---|---|---|---|
| 63 | 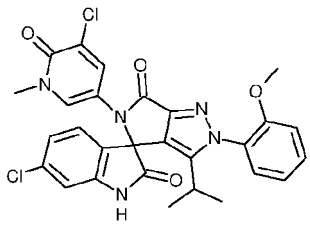 | 3B | 6-chloro-5'-(5-chloro-1-methyl-6-oxo-1,6-dihydropyridin-3-yl)-2'-(2-methoxyphenyl)-3'-(propan-2-yl)-1,2,5',6'-tetrahydro-2'H-spiro[indole-3,4'-pyrrolo[3,4-c]pyrazole]-2,6'-dione | analysis in acidic gradient: 93.9%, 564 [M+H]+, retention time: 1.93; analysis in basic gradient: 91.5%, 564 [M+H]+, retention time: 1.92. |
| 64 | 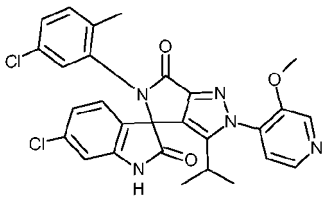 | 3B | 6-chloro-5'-(5-chloro-2-methylphenyl)-2'-(3-methoxypyridin-4-yl)-3'-(propan-2-yl)-1,2,5',6'-tetrahydro-2'H-spiro[indole-3,4'-pyrrolo[3,4-c]pyrazole]-2,6'-dione | analysis in acidic gradient: 100%, 548 $[M+H]^+$, retention time: 2.07; analysis in basic gradient: 98.9%, 548 $[M+H]^+$, retention time: 2.06. |
| 65 | 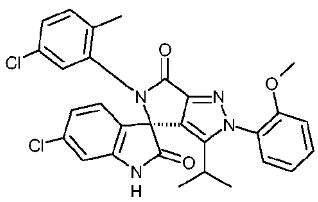 | 6B | (3S)-6-chloro-5'-(5-chloro-2-methylphenyl)-2'-(2-methoxyphenyl)-3'-(propan-2-yl)-1,2,5',6'-tetrahydro-2'H-spiro[indole-3,4'-pyrrolo[3,4-c]pyrazole]-2,6'-dione | analysis in acidic gradient: 99.8%, 547 $[M+H]^+$, retention time: 2.09; chiral analysis: 96.2%, retention time: 6.11. |

(continued)

| Cpd No | Structure | Method | Name | Analytical Data |
|---|---|---|---|---|
| 66 | 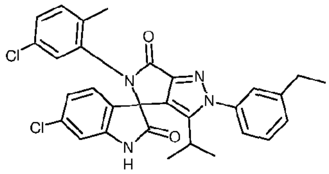 | 3B | 6-chloro-5'-(5-chloro-2-methylphenyl)-2'-(3-ethylphenyl)-3'-(propan-2-yl)-1,2,5',6'-tetrahydro-2'H-spiro[indole-3,4'-pyrrolo[3,4-c]pyrazole]-2,6'-dione | analysis in acidic gradient: 100%, 545 $[M+H]^+$, retention time: 2.18; analysis in basic gradient: 100%, 545 $[M+H]^+$, retention time: 2.18. |
| 67 | 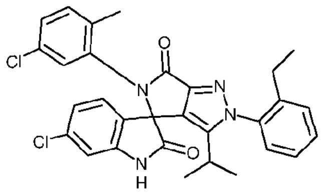 | 3B | 6-chloro-5'-(5-chloro-2-methylphenyl)-2'-(2-ethylphenyl)-3'-(propan-2-yl)-1,2,5',6'-tetrahydro-2'H-spiro[indole-3,4'-pyrrolo[3,4-c]pyrazole]-2,6'-dione | analysis in acidic gradient: 99.8%, 545 $[M+H]^+$, retention time: 2.21; analysis in basic gradient: 96.9%, 545 $[M+H]^+$, retention time: 2.21. |
| 68 | 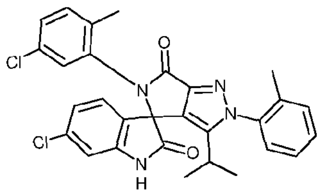 | 3B | 6-chloro-5'-(5-chloro-2-methylphenyl)-2'-(2-methylphenyl)-3'-(propan-2-yl)-1,2,5',6'-tetrahydro-2'H-spiro[indole-3,4'-pyrrolo[3,4-c]pyrazole]-2,6'-dione | analysis in acidic gradient: 97%, 531 $[M+H]^+$, retention time: 2.15; analysis in basic gradient: 96%, 531 $[M+H]^+$, retention time: 2.15. |

(continued)

| Cpd No | Structure | Method | Name | Analytical Data |
|---|---|---|---|---|
| 69 | 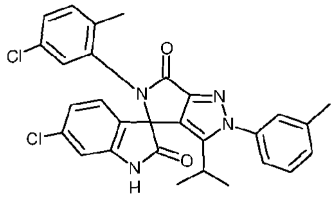 | 3B | 6-chloro-5'-(5-chloro-2-methylphenyl)-2'-(3-methylphenyl)-3'-(propan-2-yl)-1,2,5',6'-tetrahydro-2'H-spiro[indole-3,4'-pyrrolo[3,4-c]pyrazole]-2,6'-dione | analysis in acidic gradient: 93.9%, 531 [M+H]+, retention time: 2.17; analysis in basic gradient: 93%, 531 [M+H]+, retention time: 2.17. |
| 70 | 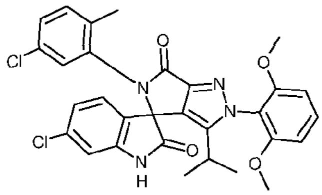 | 3B | 6-chloro-5'-(5-chloro-2-methylphenyl)-2'-(2,6-dimethoxyphenyl)-3'-(propan-2-yl)-1,2,5',6'-tetrahydro-2'H-spiro[indole-3,4'-pyrrolo[3,4-c]pyrazole]-2,6'-dione | analysis in acidic gradient: 99.4%, 577 [M+H]+, retention time: 2.06; analysis in basic gradient: 99.4%, 577 [M+H]+, retention time: 2.06. |
| 71 | 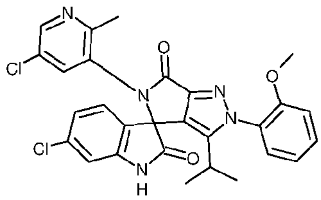 | 3B | 6-chloro-5'-(5-chloro-2-methylpyridin-3-yl)-2'-(2-methoxyphenyl)-3'-(propan-2-yl)-1,2,5',6'-tetrahydro-2'H-spiro[indole-3,4'-pyrrolo[3,4-c]pyrazole]-2,6'-dione | analysis in acidic gradient: 100%, 548 [M+H]+, retention time: 1.99; analysis in basic gradient: 100%, 548 [M+H]+, retention time: 1.99. |

(continued)

| Cpd No | Structure | Method | Name | Analytical Data |
|---|---|---|---|---|
| 72 | 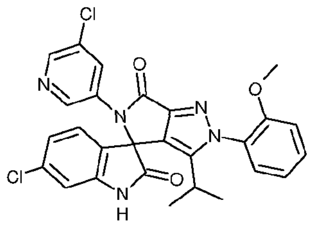 | 3B | 6-chloro-5'-(5-chloropyridin-3-yl)-2'-(2-methoxyphenyl)-3'-(propan-2-yl)-1,2,5',6'-tetrahydro-2'H-spiro[indole-3,4'-pyrrolo [3,4-c]pyrazole]-2,6'-dione | analysis in acidic gradient: 100%, 534 $[M+H]^+$, retention time: 1.97; analysis in basic gradient: 100%, 534 $[M+H]^+$, retention time: 1.97. |
| 73 | 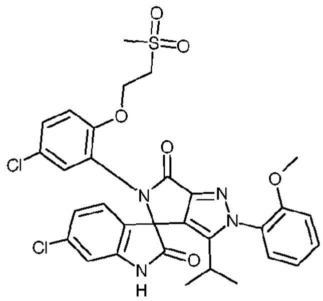 | 3C | 6-chloro-5'-[5-chloro-2-(2-methanesulfonylethoxy)phenyl]-2'-(2-methoxyphenyl)-3'-(propan-2-yl)-1,2,5',6'-tetrahydro-2'H-spiro[indole-3,4'-pyrrolo [3,4-c]pyrazole]-2,6'-dione | analysis in acidic gradient: 99.9%, 655 $[M+H]^+$, retention time: 1.92; |
| 74 | 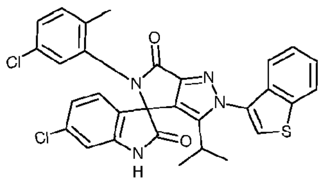 | 3B | 2'-(1-benzothiophen-3-yl)-6-chloro-5'-(5-chloro-2-methylphenyl)-3'-(propan-2-yl)-1,2,5',6'-tetrahydro-2'H-spiro[indole-3,4'-pyrrolo[3,4-c]pyrazole]-2,6'-dione | analysis in acidic gradient: 97%, 573 $[M+H]^+$, retention time: 2.15; analysis in basic gradient: 93%, 573 $[M+H]^+$, retention time: 2.14. |

(continued)

| Cpd No | Structure | Method | Name | Analytical Data |
|---|---|---|---|---|
| 75 | 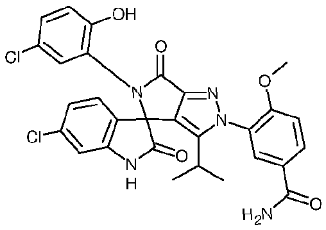 | 3B | 3-[6-chloro-5'-(5-chloro-2-hydroxyphenyl)-2,6'-dioxo-3'-(propan-2-yl)-1,2,5',6'-tetrahydro-2'H-spiro[indole-3,4'-pyrrolo [3,4-c]pyrazole]-2'-yl]-4-methoxybenzamide | analysis in acidic gradient: 100%, 592 $[M+H]^+$, retention time: 1.8; analysis in basic gradient: 98.2%, 592 $[M+H]^+$, retention time: 1.78. |
| 76 | 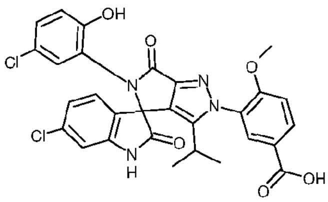 | 3B | 3-[6-chloro-5'-(5-chloro-2-hydroxyphenyl)-2,6'-dioxo-3'-(propan-2-yl)-1,2,5',6'-tetrahydro-2'H-spiro[indole-3,4'-pyrrolo [3,4-c]pyrazole]-2'-yl]-4-methoxybenzoic acid | analysis in acidic gradient: 99.4%, 593 $[M+H]^+$, retention time: 1.86; analysis in basic gradient: 97.8%, 593 $[M+H]^+$, retention time: 1.64. |
| 77 | 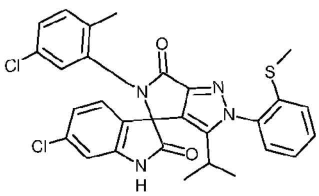 | 3B | 6-chloro-5'-(5-chloro-2-methylphenyl)-2'-[2-(methylsulfanyl)phenyl]-3'-(propan-2-yl)-1,2,5',6'-tetrahydro-2'H-spiro[indole-3,4'-pyrrolo[3,4-c]pyrazole]-2,6'-dione | analysis in acidic gradient: 100%, 563 $[M+H]^+$, retention time: 2.11; analysis in basic gradient: 100%, 563 $[M+H]^+$, retention time: 2.1. |

(continued)

| Cpd No | Structure | Method | Name | Analytical Data |
|---|---|---|---|---|
| 78 | 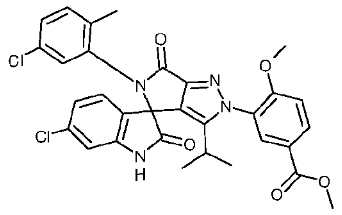 | 3B | methyl 3-[6-chloro-5'-(5-chloro-2-methylphenyl)-2,6'-dioxo-3'-(propan-2-yl)-1,2,5',6'-tetrahydro-2'H-spiro[indole-3,4'-pyrrolo[3,4-c]pyrazole]-2'-yl]-4-methoxybenzoate | analysis in acidic gradient: 100%, 605 [M+H]+, retention time: 2.08; analysis in basic gradient: 99.6%, 605 [M+H]+, retention time: 2.08. |
| 79 | 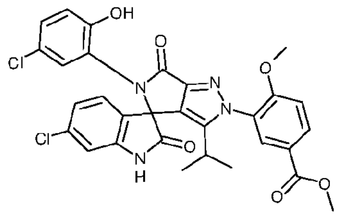 | 3B | methyl 3-[6-chloro-5'-(5-chloro-2-hydroxyphenyl)-2,6'-dioxo-3'-(propan-2-yl)-1,2,5',6'-tetrahydro-2'H-spiro[indole-3,4'-pyrrolo[3,4-c]pyrazole]-2'-yl]-4-methoxybenzoate | analysis in acidic gradient: 100%, 607 [M+H]+, retention time: 1.95; analysis in basic gradient: 100%, 607 [M+H]+, retention time: 1.95. |
| 80 | 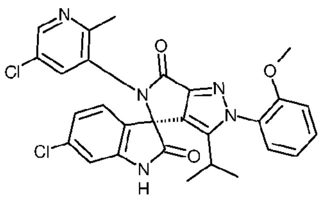 | 6C | (3S)-6-chloro-5'-(5-chloro-2-methylpyridin-3-yl)-2'-(2-methoxyphenyl)-3'-(propan-2-yl)-1,2,5',6'-tetrahydro-2'H-spiro[indole-3,4'-pyrrolo[3,4-c]pyrazole]-2,6'-dione | analysis in acidic gradient: 99.9%, 548 [M+H]+, retention time: 2.02; chiral analysis: 99.3%, retention time: 9.2. |

(continued)

| Cpd No | Structure | Method | Name | Analytical Data |
|---|---|---|---|---|
| 81 | 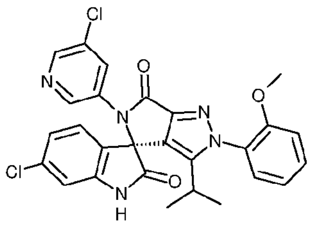 | 6D | (3S)-6-chloro-5'-(5-chloropyridin-3-yl)-2'-(2-methoxyphenyl)-3'-(propan-2-yl)-1,2,5',6'-tetrahydro-2'H-spiro[indole-3,4'-pyrrolo[3,4-c]pyrazole]-2,6'-dione | acidic gradient: 100%, 534 $[M+H]^+$, retention time: 2.0; analysis in basic gradient: 99.9%, 534 $[M+H]^+$, retention time: 1.99. chiral analysis: 99.1%, retention time: 8.46. |
| 82 | 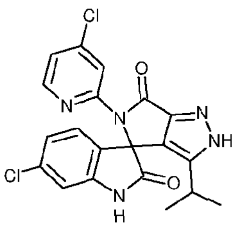 | 3A | 6-chloro-5'-(4-chloropyridin-2-yl)-3'-(propan-2-yl)-1,2,5',6'-tetrahydro-2'H-spiro[indole-3,4'-pyrrolo[3,4-c]pyrazole]-2,6'-dione | analysis in acidic gradient: 92.2%, 428 $[M+H]^+$, retention time: 2.0; analysis in basic gradient: 84%, 428 $[M+H]^+$, retention time: 2.0. |
| 83 | 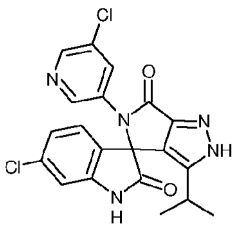 | 3A | 6-chloro-5'-(5-chloropyridin-3-yl)-3'-(propan-2-yl)-1,2, 5',6'-tetrahydro-2'H-spiro[indole-3,4'-pyrrolo[3,4-c]pyrazole]-2,6'-dione | analysis in acidic gradient: 91.6%, 428 $[M+H]^+$, retention time: 1.89; analysis in basic gradient: 91.2%, 428 $[M+H]^+$, retention time: 1.89. |

(continued)

| Cpd No | Structure | Method | Name | Analytical Data |
|---|---|---|---|---|
| 84 | 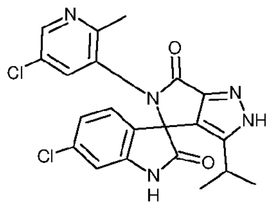 | 3A | 6-chloro-5'-(5-chloro-2-methylpyridin-3-yl)-3'-(propan-2-yl)-1,2,5',6'-tetrahydro-2'H-spiro[indole-3,4'-pyrrolo[3,4-c] pyrazole]-2,6'-dione | analysis in acidic gradient: 98.3%, 442 $[M+H]^+$, retention time: 1.9; analysis in basic gradient: 93%, 442 $[M+H]^+$, retention time: 1.9. |
| 85 | 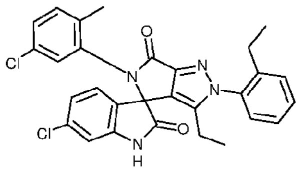 | 3B | 6-chloro-5'-(5-chloro-2-methylphenyl)-3'-ethyl-2'-(2-ethylphenyl)-1,2,5',6'-tetrahydro-2'H-spiro[indole-3,4'-pyrrolo [3,4-c]pyrazole]-2,6'-dione | analysis in acidic gradient: 98%, 531 $[M+H]^+$, retention time: 3.65; analysis in basic gradient: 98%, 531 $[M+H]^+$, retention time: 3.65. |
| 86 | 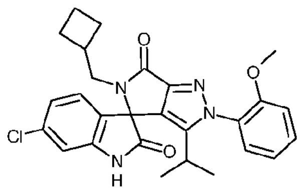 | 3B | 6-chloro-5'-(cyclobutylmethyl)-2'-(2-methoxyphenyl)-3'-(propan-2-yl)-1,2,5',6'-tetrahydro-2'H-spiro[indole-3,4'-pyrrolo [3,4-c]pyrazole]-2,6'-dione | analysis in acidic gradient: 84%, 491 $[M+H]^+$, retention time: 2.18. |
| 87 | 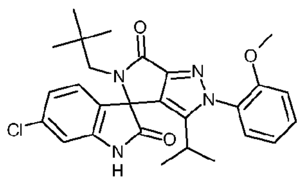 | 3B | 6-chloro-5'-(2,2-dimethylpropyl)-2'-(2-methoxyphenyl)-3'-(propan-2-yl)-1,2,5',6'-tetrahydro-2'H-spiro[indole-3,4'-pyrrolo [3,4-c]pyrazole]-2,6'-dione | analysis in acidic gradient: 95%, 493 $[M+H]^+$, retention time: 2.08. |
| 88 | 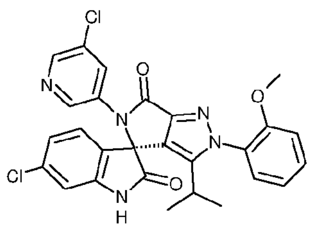 | 6C | (3S)-6-chloro-5'-(5-chloropyridin-3-yl)-2'-(2-methoxyphenyl)-3'-(propan-2-yl)-1,2,5',6'-tetrahydro-2'H-spiro[indole-3,4'-pyrrolo[3,4-c]pyrazole]-2,6'-dione | chiral analysis: 95.6%, retention time: 9.07. |

(continued)

| Cpd No | Structure | Method | Name | Analytical Data |
|---|---|---|---|---|
| 89 | 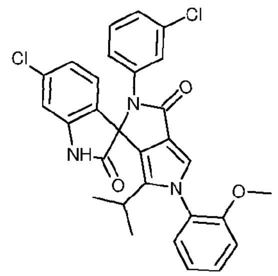 | 4 | 6-chloro-2'-(3-chlorophenyl)-5'-(2-methoxyphenyl)-6'-(propan-2-yl)-1,2,3',5'-tetrahydro-2'H-spiro[indole-3,1'-pyrrolo [3,4-c]pyrrole]-2,3'-dione | analysis in acidic gradient: 97.7%, 532 $[M+H]^+$, retention time: 2.17; analysis in basic gradient: 95.6%, 532 $[M+H]^+$, retention time: 2.16. |
| 90 | 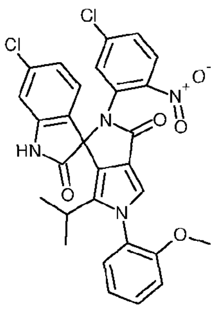 | 4 | 6-chloro-2'-(5-chloro-2-nitrophenyl)-5'-(2-methoxyphenyl)-6'-(propan-2-yl)-1,2,3',5'-tetrahydro-2'H-spiro[indole-3,1'-pyrrolo [3,4-c]pyrrole]-2,3'-dione | analysis in acidic gradient: 98.7%, 577 $[M+H]^+$, retention time: 2.12; analysis in basic gradient: 97.2%, 577 $[M+H]^+$, retention time: 2.,12. |
| 91 | 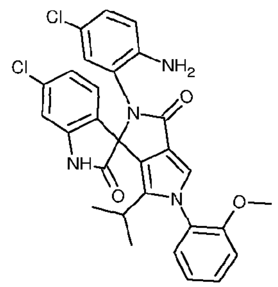 | 4 | 2'-(2-amino-5-chlorophenyl)-6-chloro-5'-(2-methoxyphenyl)-6'-(propan-2-yl)-1,2,3',5'-tetrahydro-2'H-spiro[indole-3,1'-pyrrolo[3,4-c]pyrrole]-2,3'-dione | analysis in acidic gradient: 99%, 547 $[M+H]^+$, retention time: 2,07; analysis in basic gradient: 94.3%, 547 $[M+H]^+$, retention time: 2.07. |

(continued)

| Cpd No | Structure | Method | Name | Analytical Data |
|---|---|---|---|---|
| 92 | 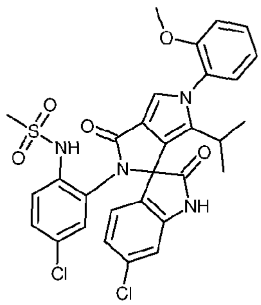 | 4 | N-{4-chloro-2-[6-chloro-5'-(2-methoxyphenyl)-2,3'-dioxo-6'-(propan-2-yl)-1,2,3',5'-tetrahydro-2'H-spiro[indole-3,1'-pyrrolo[3,4-c]pyrrole]-2'-yl]phenyl} methanesulfonamide | analysis in acidic gradient: 97.3%, 625 $[M+H]^+$, retention time: 2.12; analysis in basic gradient: 94.4%, 625 $[M+H]^+$, retention time: 2.11. |
| 93 | 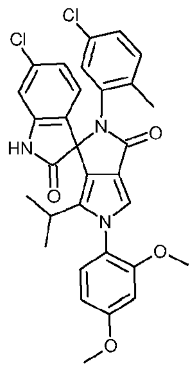 | 4 | 6-chloro-2'-(5-chloro-2-methylphenyl)-5'-(2,4-dimethoxyphenyl)-6'-(propan-2-yl)-1,2,3',5'-tetrahydro-2'H-spiro[indole-3,1'-pyrrolo[3,4-c]pyrrole]-2,3'-dione | analysis in acidic gradient: 99%, 576 $[M+H]^+$, retention time: 2.15; analysis in basic gradient: 98.9%, 576 $[M+H]^+$, retention time: 2.15. |
| 94 | 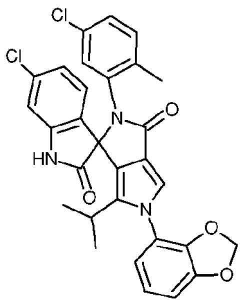 | 4 | 5'-(2H-1,3-benzodioxol-4-yl)-6-chloro-2'-(5-chloro-2-methylphenyl)-6'-(propan-2-yl)-1,2,3',5'-tetrahydro-2'H-spiro[indole-3,1'-pyrrolo[3,4-c]pyrrole]-2,3'-dione | analysis in acidic gradient: 99.5%, 560 $[M+H]^+$, retention time: 2.14; analysis in basic gradient: 99.4%, 560 $[M+H]^+$, retention time: 2.13. |

(continued)

| Cpd No | Structure | Method | Name | Analytical Data |
|---|---|---|---|---|
| 95 | 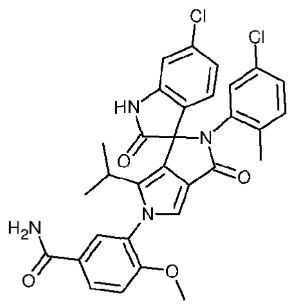 | 4 | 3-[6-chloro-2'-(5-chloro-2-methylphenyl)-2,3'-dioxo-6'-(propan-2-yl)-1,2,3',5'-tetrahydro-2'H-spiro[indole-3,1'-pyrrolo [3,4-c]pyrrole]-5'-yl]-4-methoxybenzamide | analysis in acidic gradient: 97%, 589 $[M+H]^+$, retention time: 2.01; analysis in basic gradient: 93.4%, 589 $[M+H]^+$, retention time: 2.0. |
| 96 | 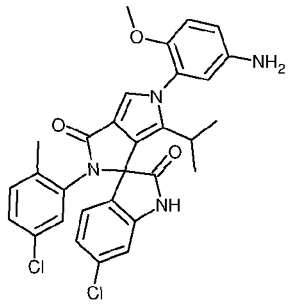 | 4 | 5'-(5-amino-2-methoxyphenyl)-6-chloro-2'-(5-chloro-2-methylphenyl)-6'-(propan-2-yl)-1,2,3',5'-tetrahydro-2'H-spiro[indole-3,1'-pyrrolo[3,4-c]pyrrole]-2,3'-dione | analysis in acidic gradient: 100%, 561 $[M+H]^+$, retention time: 2.0; analysis in basic gradient: 98.8%, 561 $[M+H]^+$, retention time: 2.0. |
| 97 | 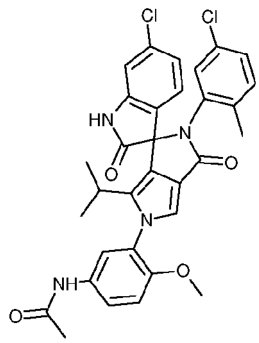 | 4 | N-{3-[6-chloro-2'-(5-chloro-2-methylphenyl)-2,3'-dioxo-6'-(propan-2-yl)-1,2,3',5'-tetrahydro-2'H-spiro[indole-3,1'-pyrrolo[3,4-c]pyrrole]-5'-yl]-4-methoxyphenyl}acetamide | analysis in acidic gradient: 100%, 603 $[M+H]^+$, retention time: 2.0; analysis in basic gradient: 99,9%, 603 $[M+H]^+$, retention time: 2.0. |

(continued)

| Cpd No | Structure | Method | Name | Analytical Data |
|---|---|---|---|---|
| 98 | 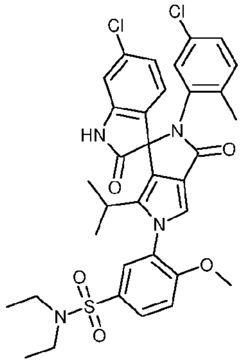 | 4 | 3-[6-chloro-2'-(5-chloro-2-methylphenyl)-2,3'-dioxo-6'-(propan-2-yl)-1,2,3',5'-tetrahydro-2'H-spiro[indole-3,1'-pyrrolo [3,4-c]pyrrole]-5'-yl]-N,N-diethyl-4-methoxybenzene-1-sulfonamide | analysis in acidic gradient: 99.5%, 681 $[M+H]^+$, retention time: 2.12; analysis in basic gradient: 97.8%, 681 $[M+H]^+$, retention time: 2.12. |
| 99 | 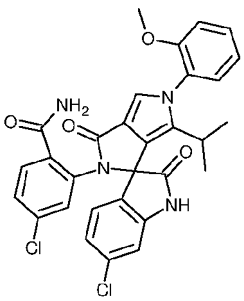 | 4 | 4-chloro-2-[6-chloro-5'-(2-methoxyphenyl)-2,3'-dioxo-6'-(propan-2-yl)-1,2,3',5'-tetrahydro-2'H-spiro[indole-3,1'-pyrrolo[3,4-c]pyrrole]-2'-yl]benzamide | analysis in acidic gradient: 98.1%, 575 $[M+H]^+$, retention time: 2.04; analysis in basic gradient: 98%, 575 $[M+H]^+$, retention time: 2.04. |
| 100 | 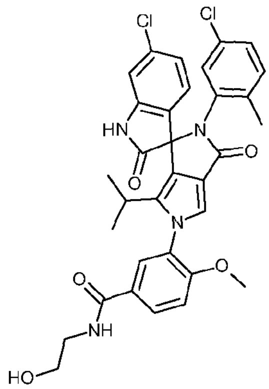 | 4 | 3-[6-chloro-2'-(5-chloro-2-methylphenyl)-2,3'-dioxo-6'-(propan-2-yl)-1,2,3',5'-tetrahydro-2'H-spiro[indole-3,1'-pyrrolo [3,4-c]pyrrole]-5'-yl]-N-(2-hydroxyethyl)-4-methoxybenzamide | analysis in acidic gradient: 100%, 633 $[M+H]^+$, retention time: 1.99; analysis in basic gradient: 98.7%, 633 $[M+H]^+$, retention time: 1.99. |

(continued)

| Cpd No | Structure | Method | Name | Analytical Data |
|---|---|---|---|---|
| 101 | 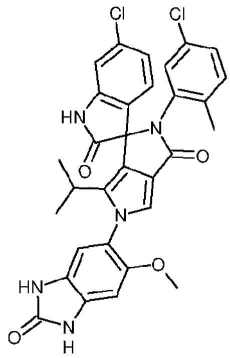 | 4 | 6-chloro-2'-(5-chloro-2-methylphenyl)-5'-(6-methoxy-2-oxo-2,3-dihydro-1H-1,3-benzodiazol-5-yl)-6'-(propan-2-yl)-1,2,3',5'-tetrahydro-2'H-spiro[indole-3,1'-pyrrolo[3,4-c]pyrrole]-2,3'-dione | analysis in acidic gradient: 97.7%, 602 $[M+H]^+$, retention time: 2.0; analysis in basic gradient: 94.3%, 602 $[M+H]^+$, retention time: 1.99. |
| 102 | 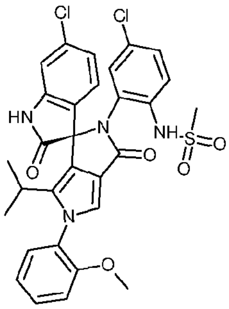 | 6C | N-{4-chloro-2-[(3S)-6-chloro-5'-(2-methoxy-phenyl)-2,3'-dioxo-6'-(propan-2-yl)-1,2,3',5'-tetrahydro-2'H-spiro[indole-3,1'-pyrrolo[3,4-c]pyrrole]-2'-yl]phenyl} methanesulfonamide | analysis in acidic gradient: 99.4%, 625 $[M+H]^+$, retention time: 2.04; chiral analysis: 96.2%, retention time: 4.29. |
| 103 | 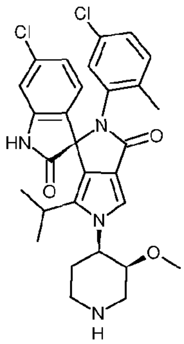 | 6G | (3S)-6-chloro-2'-(5-chloro-2-methylphenyl)-5'-[(3S,4R)-3-methoxypiperidin-4-yl]-6'-(propan-2-yl)-1,2,3',5'-tetrahydro-2'H-spiro[indole-3,1'-pyrrolo[3,4-c]pyrrole]-2,3'-dione | analysis in acidic gradient: 97.4%, 553 $[M+H]^+$, retention time: 1.72; chiral analysis: 96.8%, retention time: 9.71. |
| 104 | 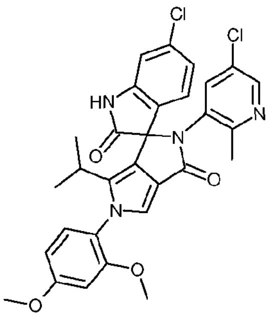 | 4 | 6-chloro-2'-(5-chloro-2-methylpyridin-3-yl)-5'-(2,4-dimethoxyphenyl)-6'-(propan-2-yl)-1,2,3',5'-tetrahydro-2'H-spiro[indole-3,1'-pyrrolo[3,4-c]pyrrole]-2,3'-dione | analysis in acidic gradient: 95.5%, 577 $[M+H]^+$, |

(continued)

| Cpd No | Structure | Method | Name | Analytical Data |
|---|---|---|---|---|
| 105 | 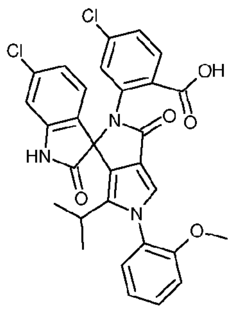 | 4 | 4-chloro-2-[6-chloro-5'-(2-methoxyphenyl)-2,3'-dioxo-6'-(propan-2-yl)-1,2,3',5'-tetrahydro-2'H-spiro[indole-3,1'-pyrrolo[3,4-c]pyrrole]-2'-yl]benzoic acid | analysis in acidic gradient: 99.2%, 576 $[M+H]^+$, retention time: 2.03; analysis in basic gradient: 97.4%, 576 $[M+H]^+$, retention time: 1.77. |
| 106 | 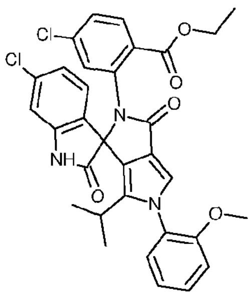 | 4 | ethyl 4-chloro-2-[6-chloro-5'-(2-methoxyphenyl)-2,3'-dioxo-6'-(propan-2-yl)-1,2,3',5'-tetrahydro-2'H-spiro[indole-3,1'-pyrrolo[3,4-c]pyrrole]-2'-yl]benzoate | analysis in acidic gradient: 97.5%, 604 $[M+H]^+$, retention time: 2.18; analysis in basic gradient: 96.3%, 604 $[M+H]^+$, retention time: 2.18. |
| 107 | 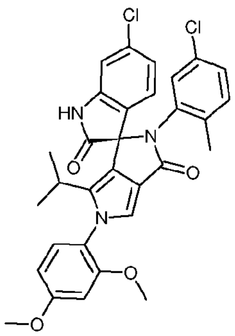 | 6E | (3S)-6-chloro-2'-(5-chloro-2-methylphenyl)-5'-(2,4-dimethoxyphenyl)-6'-(propan-2-yl)-1,2,3',5'-tetrahydro-2'H-spiro[indole-3,1'-pyrrolo[3,4-c]pyrrole]-2,3'-dione | chiral analysis: 94.4%, retention time: 5.16. |
| 108 | 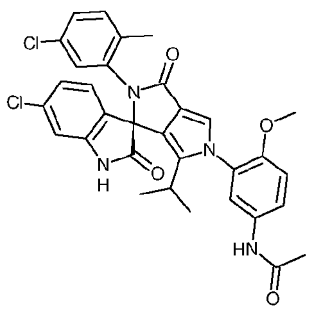 | 6F | N-{3-[(3S)-6-chloro-2'-(5-chloro-2-methylphenyl)-2,3'-dioxo-6'-(propan-2-yl)-1,2,3',5'-tetrahydro-2'H-spiro[indole-3,1'-pyrrolo[3,4-c]pyrrole]-5'-yl]-4-methoxyphenyl}acetamide | chiral analysis: 100%, retention time: 4.34. |

(continued)

| Cpd No | Structure | Method | Name | Analytical Data |
|---|---|---|---|---|
| 109 | 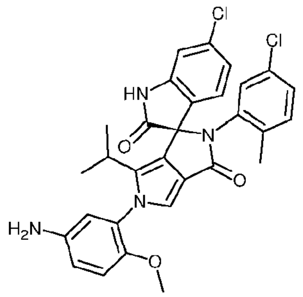 | 6F | (3S)-5'-(5-amino-2-methoxyphenyl)-6-chloro-2'-(5-chloro-2-methylphenyl)-6'-(propan-2-yl)-1,2,3',5'-tetrahydro-2'H-spiro[indole-3,1'-pyrrolo[3,4-c]pyrrole]-2,3'-dione | chiral analysis: 99.8%, retention time: 5.68. |
| 110 | 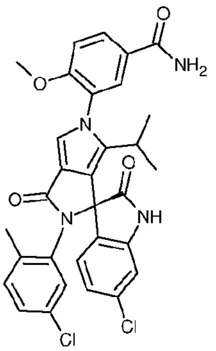 | 6D | 3-[(3S)-6-chloro-2'-(5-chloro-2-methylphenyl)-2,3'-dioxo-6'-(propan-2-yl)-1,2,3',5'-tetrahydro-2'H-spiro[indole-3,1'-pyrrolo[3,4-c]pyrrole]-5'-yl]-4-methoxybenzamide | analysis in acidic gradient: 97.9%, 589 $[M+H]^+$, retention time: 1.92; chiral analysis: 99.8%, retention time: 6.9. |
| 111 | 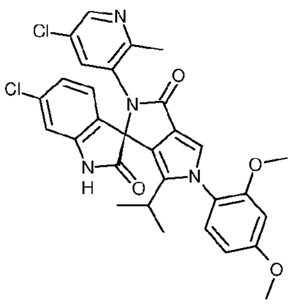 | 6C | (3S)-6-chloro-2'-(5-chloro-2-methylpyridin-3-yl)-5'-(2,4-dimethoxyphenyl)-6'-(propan-2-yl)-1,2,3',5'-tetrahydro-2'H-spiro[indole-3,1'-pyrrolo[3,4-c]pyrrole]-2,3'-dione | chiral analysis: 93.8%, retention time: 6.54. |
| 112 | 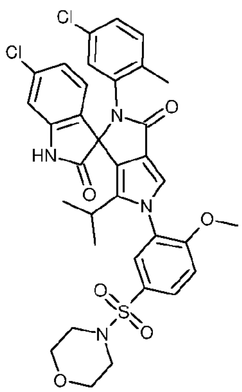 | 4 | 6-chloro-2'-(5-chloro-2-methylphenyl)-5'-[2-methoxy-5-(morpholine-4-sulfonyl)phenyl]-6'-(propan-2-yl)-1,2,3',5'-tetrahydro-2'H-spiro[indole-3,1'-pyrrolo[3,4-c]pyrrole]-2,3'-dione | analysis in acidic gradient: 97.9%, 696 $[M+H]^+$, retention time: 2.06; analysis in basic gradient: 96.7%, 696 $[M+H]^+$, retention time: 2.06. |

(continued)

| Cpd No | Structure | Method | Name | Analytical Data |
|---|---|---|---|---|
| 113 | 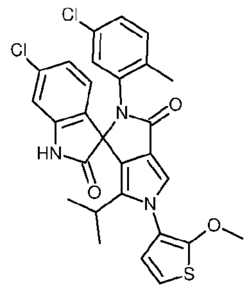 | 4 | 6-chloro-2'-(5-chloro-2-methylphenyl)-5'-(2-methoxythiophen-3-yl)-6'-(propan-2-yl)-1,2,3',5'-tetrahydro-2'H-spiro[indole-3,1'-pyrrolo[3,4-c]pyrrole]-2,3'-dione | analysis in acidic gradient: 100%, 552 $[M+H]^+$, retention time: 2.16; analysis in basic gradient: 100%, 552 $[M+H]^+$, retention time: 2.16. |
| 114 | 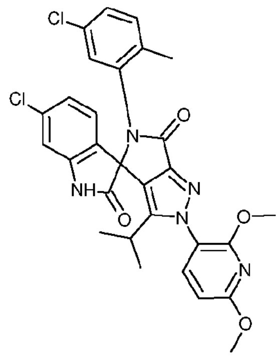 | 3B | 6-chloro-5'-(5-chloro-2-methylphenyl)-2'-(2,6-dimethoxypyridin-3-yl)-3'-(propan-2-yl)-1,2,5',6'-tetrahydro-2'H-spiro[indole-3,4'-pyrrolo[3,4-c]pyrazole]-2,6'-dione | analysis in acidic gradient: 94%, 578 $[M+H]^+$, retention time: 2.15; |

## Biological Examples:

### Example 8. Fluorescent Polarisation Assay

The inhibition of p53-Mdm2 interaction was measured using a fluorescence polarization (FP) binding assay. FP measures the rotational movement of molecules in a homogenous suspension. For this assay, N-terminal domain of Mdm2 protein (amino acids 1-111) is combined with a fluorescein-labelled (FAM) peptide derived from p53 transactivation domain. Upon excitation of the fluorescent ligand with linearly polarized light, the peptide rotates faster and emits light which is perpendicularly polarized. If the peptide is bound by Mdm2, rotation will slow down and the perpendicular component will decrease. Disruption of the formation of the peptide-Mdm2 complex due to an inhibitor molecule binding to the p53 binding site of Mdm2 results in faster rotation of the peptide.

Fluorescence polarization experiments were read on Tecan Infinite M1000 reader with the 470 nm excitation and 520 nm emission filters for fluorescein. The fluorescence polarization was measured in black 96-well plates (Corning, CLS3991) in room temperature. Purity of Mdm2 was controlled at >95%. Reaction buffer was optimized by adding 5 mM DTT and 0.1% zwitterionic detergent CHAPS to reduce effect of nonspecific interactions.

The test was performed by combining successive dilution of compounds diluted in dimethyl sulfoxide (DMSO, 5% final concentration) with 130 nM Mdm2 in reaction buffer (PBS, 0.1% CHAPS, 5 mM DTT (dithiothreitol)). After 15 minutes of incubation in room temperature 10 nm FAM-labelled peptide was added. Final reading was performed after 90 minutes of incubation. Dose-dependent binding curves and IC50 values were calculated using GraphPad Prism5 and next transformed to Ki values using Kenakin equation. IC50 values are presented in Table 2.

Table 2

| Compound | Ki (nM) p53-Mdm2 |
|---|---|
| 1 | 354 |

(continued)

| Compound | Ki (nM) p53-Mdm2 |
|---|---|
| 2 | 122 |
| 3 | 51 |
| 5 | 194 |
| 6 | 102 |
| 7 | 700 |
| 8 | 120 |
| 9 | 268 |
| 10 | 610 |
| 13 | 196 |
| 15 | 349 |
| 17 | 57 |
| 18 | 45 |
| 19 | 64 |
| 20 | 24 |
| 21 | 70 |
| 22 | 120 |
| 23 | 20 |
| 24 | 54 |
| 25 | 94 |
| 26 | 23 |
| 27 | 4.3 |
| 28 | 1460 |
| 29 | 358 |
| 30 | 8.2 |
| 31 | 4.7 |
| 32 | 1390 |
| 33 | 4.8 |
| 34 | 235 |
| 36 | 295 |
| 37 | 190 |
| 38 | 550 |
| 39 | 750 |
| 41 | 24 |
| 42 | 90 |
| 43 | 650 |
| 44 | 281 |
| 45 | 36 |
| 46 | 96 |

(continued)

| Compound | Ki (nM) p53-Mdm2 |
|---|---|
| 47 | 29 |
| 48 | 205 |
| 49 | 38 |
| 51 | 130 |
| 52 | 800 |
| 53 | 581 |
| 54 | 123 |
| 55 | 1910 |
| 56 | 100 |
| 57 | 79 |
| 58 | 82 |
| 59 | 178 |
| 62 | 11 |
| 63 | 2.4 |
| 65 | 1.9 |
| 66 | 141 |
| 67 | 86 |
| 68 | 46 |
| 69 | 67 |
| 70 | 120 |
| 71 | 4.3 |
| 72 | 5.6 |
| 73 | 42 |
| 75 | 3.6 |
| 76 | 3.6 |
| 77 | 30 |
| 78 | 3.4 |
| 79 | 5.7 |
| 80 | 1.9 |
| 81 | 2.2 |
| 82 | 425 |
| 83 | 152 |
| 84 | 152 |
| 85 | 86 |
| 88 | 1.9 |
| 89 | 5.3 |
| 90 | 33 |
| 91 | 4.5 |

(continued)

| Compound | Ki (nM) p53-Mdm2 |
|---|---|
| 92 | 6.6 |
| 93 | 3.8 |
| 94 | 9.6 |
| 95 | 1.8 |
| 96 | 4.0 |
| 97 | 3.4 |
| 98 | 4.6 |
| 99 | 6.7 |
| 100 | 3.5 |
| 101 | 4.7 |
| 102 | 2.9 |
| 103 | 4.5 |
| 104 | 3.1 |
| 105 | 4.3 |
| 106 | 4.2 |
| 107 | 1.9 |
| 108 | 1.6 |
| 109 | 1.3 |
| 110 | 1.7 |
| 111 | 2.8 |
| 112 | 3.5 |
| 113 | 3.4 |
| 114 | 3.3 |

Inspection of measured Ki values shows that substitution of the position 2' (in the pyrazole ring) is much more favourable than the position 1' (in pyrazole ring). As an example, Compound 31 substituted with orto-methoxy-phenyl in position 2' is 4 times more active with Ki=4,7 nM. On the contrary, an analogous substitution in position 1' significantly decreases the activity and Compound 32 has Ki=1390 nM, which means that it is almost 300 times less potent than Compound 31.

We also noticed that although the racemic mixtures of S and R stereoisomers can exhibit very high potency, most of the observed activity is caused by one isomer (isomer S for all compound IA and IB, except G=S, and isomer R for G=S (sulphur atom changes prioritisation around chiral center leading to configuration R)). For example, Compound 31 has Ki=4,7 nM and its isomer S has Ki=1,9nM. Such a relation has also been observed for other compound pairs from this group where, for example, enantiopure S isomers - Compounds 80, 107 and 81 are about 2 times more potent than the corresponding S/R (racemic) mixtures Compounds 71, 93 and 72, respectively. The same applies to Compound 55 (G=S) and its enantiomer Compound 56. Compound 55 is about 19 times less potent than Compound 56.

**Example 9. Cell viability assay**

The effect of the invented p53-Mdm2 inhibitors on cell viability has been assessed using MTT assay. It is a colorimetric assay that measures conversion of tetrazolium ring of the soluble yellow dye (MTT) into insoluble purple formazan. This process is catalysed solely in mitochondrial dehydrogenases of living cells. Dead cells do not cause this change. In order to measure the specific cytotoxicty of Mdm2-p53 inhibitors the MTT assay was performed with SJSA-1 osteosarcoma cell line that exhibits MDM2 gene amplification and the wild type p53.

Cells were seeded on 96-well plates and then treated with successive dilutions of tested compounds. After 72h incubation, MTT was added to the final concentration 0.5 mg/ml. The cells were further incubated for the next 4h. Then the solution was drained and the remaining formazan crystals were dissolved in 100 μl DMSO. The absorbance read-out was performed at 570 nm revealing the relative cell viability between cells treated with the assessed compounds and the DMSO control. All the MTT experiments were independently repeated 2-5 times. Dose-dependent binding curves and IC50 values were calculated using GraphPad Prism 5. IC50 values represent the average value from all the performed experiments and are presented in Table 3.

Table 3

| Compound | IC50 (μM) SJSA-1 |
|---|---|
| 18 | 10.8 |
| 19 | 22.3 |
| 20 | 21.2 |
| 21 | 15.1 |
| 22 | 6.95 |
| 23 | 10.2 |
| 24 | 33.5 |
| 25 | 10.2 |
| 26 | 4.91 |
| 27 | 1.07 |
| 28 | 32 |
| 29 | 48.8 |
| 30 | 1.36 |
| 31 | 0.53 |
| 33 | 1.21 |
| 34 | 24.2 |
| 41 | 21.4 |
| 43 | 43.8 |
| 44 | 18.1 |
| 45 | 29.9 |
| 46 | 32.3 |
| 47 | 44.5 |
| 48 | 28.0 |
| 52 | 39.6 |
| 53 | 29.6 |
| 59 | 37.5 |
| 62 | 2.23 |
| 65 | 0.22 |
| 66 | 14.7 |
| 67 | 12.4 |
| 68 | 6.35 |
| 69 | 4.04 |

(continued)

| Compound | IC50 (μM) SJSA-1 |
|---|---|
| 71 | 0.46 |
| 72 | 0.89 |
| 73 | 11.4 |
| 77 | 8.57 |
| 78 | 0.36 |
| 79 | 1.02 |
| 80 | 0.28 |
| 81 | 0.80 |
| 84 | 46.1 |
| 85 | 13.0 |
| 88 | 0.34 |
| 90 | 6.19 |
| 92 | 1.07 |
| 93 | 0.42 |
| 94 | 0.93 |
| 95 | 0.30 |
| 96 | 0.30 |
| 97 | 0.30 |
| 98 | 0.38 |
| 99 | 0.65 |
| 100 | 1.21 |
| 101 | 1.77 |
| 102 | 0.66 |
| 103 | 2.20 |
| 104 | 0.16 |
| 105 | 9.98 |
| 106 | 0.78 |
| 107 | 0.06 |
| 108 | 0.35 |
| 109 | 0.07 |
| 110 | 0.35 |
| 111 | 0.12 |
| 112 | 0.15 |
| 113 | 0.69 |
| 114 | 0.29 |

**Example 10. In vivo efficacy in the SJSA-1 xenograft model in mice**

The experiment was conducted on female mice from the Crl:SHO-*Prkdc*$^{scid}$*Hr*$^{hr}$ strain. Mice were inoculated

subcutaneously in the right flank with cancer cell line SJSA-1 in the amount of 3 mln cells suspended in 100 μl HBSS : Matrigel matrix in a 3:1 ratio per mouse. On the 16th day after inoculation mice were divided into groups, so that in each group the mean tumor volume was similar and averaged around 160 $mm^3$. Two experiment groups were selected, each consisting of 5 mice: Control NaCl 0,9% and compound 93. The compound 93 was dissolved in 15% PEG400, 10% Cremophore EL, 75% $H_2O$.

Mice used in the experiment were administered per os (p.o.) with compounds or NaCl 0.9% in a q1dx14 schedule (14 doses, daily). During the course of experiment mice were weighed before each administration, - twice/thrice a week. Animal welfare was monitored daily. No significant difference in body weight or welfare was observed between experiment groups during and at the end of study.

Change in tumor volume was monitored twice/thrice a week starting from the first day of administration. Tumor volume was calculated based on its length and width measured with an electronic calipers:

$$V\ [mm^3] = d^2 x\ D/2$$

where d - width, D - length.

The tumor volume in the compound 93 group was measured up to 68 days after inoculation (39 days after last administration). Results of the experiment were expressed as mean values of tumor volume ± SEM. All calculations and graphs were performed using GraphPad Prism 5 software. The results of efficacy testing of the compound 93 at 100 mg/kg p.o. in this experiment are presented on Fig.1.

## Claims

1. A compound represented by the formula selected from the group consisting of formula (IA) and (IB)

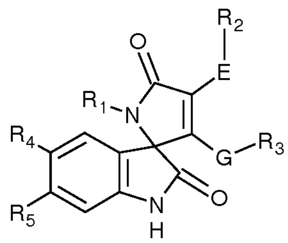

(IA)

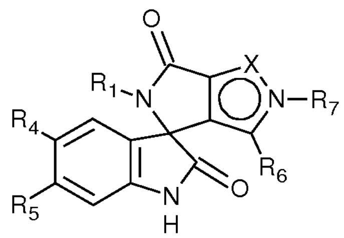

(IB)

wherein

$R_1$ is:

- $C_1$-$C_6$-alkyl unsubstituted or substituted by $C_3$-$C_6$-cycloalkyl,
- phenyl or 3-pyridyl that are unsubstituted or substituted by one or two substituents independently selected from the group consisting of halogen, -OH, $-NH_2$, $-NO_2$, -CN, $C_1$-$C_6$-alkyl, $C_2$-$C_6$-alkenyl, -O-($C_1$-$C_6$-alkyl), -O-($C_2$-$C_6$-alkenyt), -S-($C_1$-$C_6$-alkyl), -S-($C_2$-$C_6$-alkenyl), -C(O)O-($C_1$-$C_6$-alkyl), -C(O)O-($C_2$-$C_6$-alkenyl), $-C(O)NH_2$, -C(O)NH($C_1$-$C_6$-alkyl), -C(O)N($C_1$-$C_6$-alkyl)$_2$, -C(O)NH($C_2$-$C_6$-alkenyl), -NH($C_1$-$C_6$-alkyl), -N($C_1$-$C_6$-alkyl)$_2$, -NH-phenyl, -NHC(O)-($C_1$-$C_6$-alkyl), -NHC(O)-($C_2$-$C_6$-alkenyl), -NHC(O)O-($C_1$-$C_6$-alkyl), and $-NHSO_2$-($C_1$-$C_6$-alkyl), and wherein said $C_1$-$C_6$-alkyl, $C_2$-$C_6$-alkenyl and phenyl are unsubstituted or further substituted by one or two substituents independently selected from the group consisting of halogen, -OH, -SH, -O-($C_1$-$C_6$-alkyl), -COOH, -C(O)O-($C_1$-$C_6$-alkyl), and $-SO_2$-($C_1$-$C_6$-alkyl),
- pyrazolyl unsubstituted or substituted by one or two substituents which are independently $C_1$-$C_6$-alkyl,
- 6-oxo-1,6-dihydropyridin-3-yl unsubstituted or substituted by one or two substituents independently selected from the group consisting of halogen and $C_1$-$C_6$-alkyl, or
- 2-oxo-1,2-dihydropyridin-3-yl unsubstituted or substituted by one or two substituents independently selected from the group consisting of halogen and $C_1$-$C_6$-alkyl;

$R_4$ and $R_5$ are independently H or halogen;
$R_2$ is hydrogen atom, ($C_1$-$C_6$-alkyl)sulfonyl, -($C_1$-$C_6$-alkyl), or -($C_1$-$C_6$-alkyl) terminally substituted by one substituent selected from the group consisting of -COOH, -$CONH_2$, -C(O)O-($C_1$-$C_6$-alkyl), -$NH_2$, NH($C_1$-$C_6$-alkyl), -N($C_1$-$C_6$-alkyl)$_2$, -NHC(O)($C_1$-$C_6$-alkyl), imidazole, tetrazole, and phenyl, wherein said phenyl is substituted by -($C_1$-$C_3$-alkyl), -O($C_1$-$C_3$-alkyl) or halogen;
$R_3$ is:

- $C_1$-$C_6$-alkyl,
- $C_3$-$C_6$-cycloalkyl unsubstituted or substituted by one $C_1$-$C_6$-alkyl,
- phenyl unsubstituted or substituted by one, two or three substituents independently selected from the group consisting of halogen, -OH, -$NH_2$, -$NO_2$, -CN, $C_1$-$C_6$-alkyl, $C_2$-$C_6$-alkenyl, -O-($C_1$-$C_6$-alkyl), -O-($C_2$-$C_6$-alkenyl), -S-($C_1$-$C_6$-alkyl), -S-($C_2$-$C_6$-alkenyl), -C(O)O-($C_1$-$C_6$-alkyl), -C(O)O-($C_2$-$C_6$-alkenyl), -C(O)$NH_2$, -(O)NH($C_1$-$C_6$-alkyl), -C(O)N($C_1$-$C_6$-alkyl)$_2$, -C(O)NH($C_2$-$C_6$-alkenyl), -NH($C_1$-$C_6$-alkyl), -N($C_1$-$C_6$-alkyl)$_2$, -NH-phenyl, -NHC(O)-($C_1$-$C_6$-alkyl), -NHC(O)-($C_2$-$C_6$-alkenyl), and -NHC(O)O-($C_1$-$C_6$-alkyl), and wherein said $C_1$-$C_6$-alkyl, $C_2$-$C_6$-alkenyl and phenyl are unsubstituted or further substituted by one or two substituents independently selected from the group consisting of halogen, -OH, -SH, -O-($C_1$-$C_6$-alkyl), -COOH, and -C(O)O-($C_1$-$C_6$-alkyl), or
- 5- or 6-membered heteroaryl with one, two, three or four heteroatoms independently selected from N, O, and S, wherein said heteroaryl is unsubstituted or substituted by one, two or three substituents independently selected from the group consisting of halogen, -OH, -$NH_2$, -$NO_2$, -CN, $C_1$-$C_6$-alkyl, $C_2$-$C_6$-alkenyl, -O-($C_1$-$C_6$-alkyl), -S-($C_1$-$C_6$-alkyl), -S-($C_2$-$C_6$-alkenyl), -C(O)O-($C_1$-$C_6$-alkyl), -C(O)O-($C_2$-$C_6$-alkenyl), -C(O)$NH_2$, -C(O)NH($C_1$-$C_6$-alkyl), -C(O)N($C_1$-$C_6$-alkyl)$_2$, -C(O)NH($C_2$-$C_6$-alkenyl), -NH($C_1$-$C_6$-alkyl), -N($C_1$-$C_6$-alkyl)$_2$, -NH-phenyl, -NHC(O)-($C_1$-$C_6$-alkyl), -NHC(O)-($C_2$-$C_6$-alkenyl), -NHC(O)O-($C_1$-$C_6$-alkyl), and 5- or 6-membered non-aromatic heterocyclyl with one or two heteroatoms selected from N and 0, and wherein said $C_1$-$C_6$-alkyl, $C_2$-$C_6$-alkenyl and phenyl are unsubstituted or further substituted by one or two substituents independently selected from the group consisting of halogen, -OH, -$NH_2$,-O-($C_1$-$C_6$-alkyl), -COOH, -C(O)O-($C_1$-$C_6$-alkyl), -NH-($C_1$-$C_6$-alkyl), and -N-($C_1$-$C_6$-alkyl)$_2$;

E is O, NH, or S;
G is S, carbonyl or a direct bond;
$R_6$ is:

- $C_1$-$C_6$-alkyl unsubstituted or substituted by one -O-($C_1$-$C_6$-alkyl), or
- $C_3$-$C_6$-cycloalkyl unsubstituted or substituted by one $C_1$-$C_6$-alkyl,

$R_7$ is:

- hydrogen atom,
- $C_1$-$C_3$-alkyl unsubstituted or substituted by one substituent selected from the group consisting of imidazole, tetrazole, and a 5- or 6-membered non-aromatic heterocyclyl comprising one or two heteroatoms selected from N and 0, wherein said non-aromatic heterocyclyl is unsubstituted or substituted on the nitrogen atom by substituent selected from the group consisting of -($C_1$-$C_6$-alkyl), -C(O)($C_1$-$C_6$-alkyl), and -C(O)N($C_1$-$C_6$-alkyl)$_2$,
- $C_1$-$C_6$-alkenyl,
- phenyl unsubstituted or substituted by one or two substituents independently selected from the group consisting of halogen, -OH, -$NH_2$, -$NO_2$, -CN, $C_1$-$C_6$-alkyl, $C_2$-$C_6$-alkenyl, -O-($C_1$-$C_6$-alkyl), -O-($C_2$-$C_6$-alkenyl), -S-($C_1$-$C_6$-alkyl), -S-($C_2$-$C_6$-alkenyl), -$CO_2$H, -C(O)O-($C_1$-$C_6$-alkyl), -C(O)O-($C_2$-$C_6$-alkenyl), -C(O)$NH_2$, -C(O)NH($C_1$-$C_6$-alkyl), and wherein said $C_1$-$C_6$-alkyl is unsubstituted or further substituted by -OH, -C(O)N($C_1$-$C_6$-alkyl)$_2$, -C(O)NH($C_2$-$C_6$-alkenyl), -NH($C_1$-$C_6$-alkyl), -N($C_1$-$C_6$-alkyl)$_2$, -NH-phenyl, -NHC(O)-($C_1$-$C_6$-alkyl), -NHC(O)-($C_2$-$C_6$-alkenyl), -NHC(O)O-($C_1$-$C_6$-alkyl), -$SO_2$N($C_1$-$C_6$-alkyl), or -$SO_2$-(5- or 6-membered heterocyclyl with one or two heteroatoms selected from N and 0),

    - (3S,4R)-3-methoxypiperidin-4-yl, or

- 1-benzothiophen-3-yl, 2-oxo-2,3-dihydro-1H-1,3-benzodiazol-5-yl, 3-pyridyl, 4-pyridyl, 2H-1,3-benzodioxol-4-yl or 2-tiophenyl that are unsubstituted or substituted by $C_1$-$C_6$-alkyl, -O-($C_1$-$C_6$-alkyl) or halogen;

X is N or CH;
with the proviso that 4-acetyl-3-hydroxy-1-methylospiro[2,5-dihydropyrrol-5,3'-indole]-2,2'-dione is excluded; and pharmaceutically acceptable salts, solvates, tautomers and stereoisomers thereof.

2. The compound of claim 1, represented by formula (IA).

3. The compound of claim 2, wherein G is carbonyl group.

4. The compound of claim 2, wherein G is -S-.

5. The compound of claim 2, wherein G is direct bond.

6. The compound of any one of claims 1 to 5, wherein
$R_1$ is:

- phenyl or 3-pyridyl that are unsubstituted or substituted by one or two substituents independently selected from the group consisting of halogen, -OH, $-NH_2$, $-NO_2$, -CN, $C_1$-$C_6$-alkyl, $C_2$-$C_6$-alkenyl, -O-($C_1$-$C_6$-alkyl), -O-($C_2$-$C_6$-alkenyl), -S-($C_1$-$C_6$-alkyl), -S-($C_2$-$C_6$-alkenyl), -C(O)O-($C_1$-$C_6$-alkyl),
- C(O)O-($C_2$-$C_6$-alkenyl), $-C(O)NH_2$, -C(O)NH($C_1$-$C_6$-alkyl), -C(O)N($C_1$-$C_6$-alkyl)$_2$, -C(O)NH($C_2$-$C_6$-alkenyl), -NH($C_1$-$C_6$-alkyl), -N($C_1$-$C_6$-alkyl)$_2$, -NH-phenyl, -NHC(O)-($C_1$-$C_6$-alkyl), -NHC(O)-($C_2$-$C_6$-alkenyl), -NHC(O)O-($C_1$-$C_6$-alkyl), and $NHSO_2$-($C_1$-$C_6$-alkyl), and wherein said $C_1$-$C_6$-alkyl, $C_2$-$C_6$-alkenyl and phenyl are unsubstituted or further substituted by one or two substituents independently selected from the group consisting of halogen, -OH, -SH, -O-($C_1$-$C_6$-alkyl), -COOH, -C(O)O-($C_1$-$C_6$-alkyl), and $-SO_2$-($C_1$-$C_6$-alkyl),
- pyrazolyl unsubstituted or substituted by one or two substituents which are independently $C_1$-$C_6$-alkyl,
- 6-oxo-1,6-dihydropyridin-3-yl unsubstituted or substituted by one or two substituents independently selected from the group consisting of halogen and $C_1$-$C_6$-alkyl, or
- 2-oxo-1,2-dihydropyridin-3-yl unsubstituted or substituted by one or two substituents independently selected from the group consisting of halogen and $C_1$-$C_6$-alkyl,

$R_4$ is H, and
$R_5$ is Cl or F.

7. The compound of claim 1, represented by formula (IB).

8. The compound of claim 7, wherein X is N and the compound is represented by formula (IB-1)

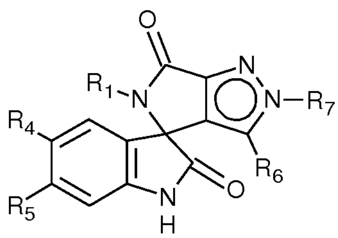

(IB-1).

9. The compound of claim 7, wherein X is CH and the compound is represented by formula (IB-2)

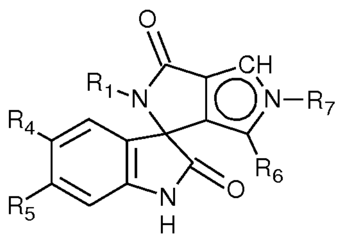

(IB-2).

10. The compound of any one of claims 7 to 9, wherein
$R_1$ is:

- phenyl or 3-pyridyl that are unsubstituted or substituted by one or two substituents independently selected from the group consisting of halogen, -OH, -$NH_2$, -$NO_2$, -CN, $C_1$-$C_6$-alkyl, $C_2$-$C_6$-alkenyl, -O-($C_1$-$C_6$-alkyl), -O-($C_2$-$C_6$-alkenyl), -S-($C_1$-$C_6$-alkyl), -S-($C_2$-$C_6$-alkenyl), -C(O)O-($C_1$-$C_6$-alkyl), -C(O)O-($C_2$-$C_6$-alkenyl), -C(O)$NH_2$, -C(O)NH($C_1$-$C_6$-alkyl), -C(O)N($C_1$-$C_6$-alkyl)$_2$, -C(O)NH($C_2$-$C_6$-alkenyl), -NH($C_1$-$C_6$-alkyl), -N($C_1$-$C_6$-alkyl)$_2$, -NH-phenyl, -NHC(O)-($C_1$-$C_6$-alkyl), -NHC(O)-($C_2$-$C_6$-alkenyl), -NHC(O)O-($C_1$-$C_6$-alkyl), and $NHSO_2$-($C_1$-$C_6$-alkyl), and wherein said $C_1$-$C_6$-alkyl, $C_2$-$C_6$-alkenyl and phenyl are unsubstituted or further substituted by one or two substituents independently selected from the group consisting of halogen, -OH, -SH, -O-($C_1$-$C_6$-alkyl), -COOH, -C(O)O-($C_1$-$C_6$-alkyl), and -$SO_2$-($C_1$-$C_6$-alkyl),
- pyrazolyl unsubstituted or substituted by one or two substituents which are independently $C_1$-$C_6$-alkyl,
- 6-oxo-1,6-dihydropyridin-3-yl unsubstituted or substituted by one or two substituents independently selected from the group consisting of halogen and $C_1$-$C_6$-alkyl, or
- 2-oxo-1,2-dihydropyridin-3-yl unsubstituted or substituted by one or two substituents independently selected from the group consisting of halogen and $C_1$-$C_6$-alkyl.

**11.** The compound of claim 10, wherein $R_1$ is:

- phenyl substituted with halogen at the meta position relative to the place of attachment to the pyrrolone ring nitrogen atom, or 3-pyridyl substituted with halogen at the position 5 relative to the place of attachment to the pyrrolone ring nitrogen atom, and said phenyl and 3-pyridyl are optionally further substituted by one or two substituents independently selected from the group consisting of halogen, -OH, -$NH_2$, -$NO_2$, -CN, $C_1$-$C_6$-alkyl, $C_2$-$C_6$-alkenyl, -O-($C_1$-$C_6$-alkyl), -O-($C_2$-$C_6$-alkenyl), -S-($C_1$-$C_6$-alkyl), -S-($C_2$-$C_6$-alkenyl), -C(O)O-($C_1$-$C_6$-alkyl), -C(O)O-($C_2$-$C_6$-alkenyl), -C(O)$NH_2$, -C(O)NH($C_1$-$C_6$-alkyl), -C(O)N($C_1$-$C_6$-alkyl)$_2$, -C(O)NH($C_2$-$C_6$-alkenyl), -NH($C_1$-$C_6$-alkyl), -N($C_1$-$C_6$-alkyl)$_2$, -NH-phenyl, -NHC(O)-($C_1$-$C_6$-alkyl), -NHC(O)-($C_2$-$C_6$-alkenyl), -NHC(O)O-($C_1$-$C_6$-alkyl), and $NHSO_2$-($C_1$-$C_6$-alkyl), and wherein said $C_1$-$C_6$-alkyl, $C_2$-$C_6$-alkenyl and phenyl are unsubstituted or further substituted by one or two substituents independently selected from the group consisting of halogen, -OH, -SH, -O-($C_1$-$C_6$-alkyl), -COOH, -C(O)O-($C_1$-$C_6$-alkyl), and -$SO_2$-($C_1$-$C_6$-alkyl).

**12.** The compound of any one of claims 7 to 11, wherein $R_4$ is H, and $R_5$ is Cl or F.

**13.** The compound of claim 12, wherein $R_1$ is:

- meta-chlorophenyl or 5-chloro-3-pyridyl that are optionally further substituted by one or two substituents independently selected from the group consisting of halogen, -OH, -$NH_2$, -$NO_2$, -CN, $C_1$-$C_6$-alkyl, $C_2$-$C_6$-alkenyl, -O-($C_1$-$C_6$-alkyl), -O-($C_2$-$C_6$-alkenyl), -S-($C_1$-$C_6$-alkyl), -S-($C_2$-$C_6$-alkenyl), -C(O)O-($C_1$-$C_6$-alkyl), -C(O)O-($C_2$-$C_6$-alkenyl), -C(O)$NH_2$, -C(O)NH($C_1$-$C_6$-alkyl), -C(O)N($C_1$-$C_6$-alkyl)$_2$, -C(O)NH($C_2$-$C_6$-alkenyl), -NH($C_1$-$C_6$-alkyl), -N($C_1$-$C_6$-alkyl)$_2$, -NH-phenyl, -NHC(O)-($C_1$-$C_6$-alkyl), -NHC(O)-($C_2$-$C_6$-alkenyl), -NHC(O)O-($C_1$-$C_6$-alkyl), and $NHSO_2$-($C_1$-$C_6$-alkyl), and wherein said $C_1$-$C_6$-alkyl, $C_2$-$C_6$-alkenyl and phenyl are unsubstituted or further substituted by one or two substituents independently selected from the group consisting of halogen, -OH, -SH, -O-($C_1$-$C_6$-alkyl), -COOH, -C(O)O-($C_1$-$C_6$-alkyl), and -$SO_2$-($C_1$-$C_6$-alkyl).

**14.** The compound of any one of claims 7 to 13 wherein the absolute configuration at spiro carbon atom is S (configuration 3*S*).

**15.** The compound of claim 1 selected from the following group:

6-chloro-1'-(5-chloro-2-fluorophenyl)-4'-hydroxy-3'-(1-methylcyclopropanecarbonyl)-1,1',2,5'-tetrahydrospiro[indole-3,2'-pyrrole]-2,5'-dione,
6-chloro-1'-(5-chloro-2-methylphenyl)-4'-hydroxy-3'-(1-methylcyclopropanecarbonyl)-1,1',2,5'-tetrahydrospiro[indole-3,2'-pyrrole]-2,5'-dione,
3'-benzoyl-6-chloro-1'-(3-chlorophenyl)-4'-hydroxy-1,1',2,5'-tetrahydrospiro[indole-3,2'-pyrrole]-2,5'-dione,
3'-benzoyl-6-chloro-1'-(1,5-dimethyl-1H-pyrazol-3-yl)-4'-hydroxy-1,1',2,5'-tetrahydro-spiro[indole-3,2'-pyrrole]-2,5'-dione,
6-chloro-1'-(3-chlorophenyl)-4'-hydroxy-3'-(1-methylcyclopropanecarbonyl)-1,1',2,5'-tetrahydrospiro[indole-3,2'-pyrrole]-2,5'-dione,
6-chloro-1'-(3-chlorophenyl)-3'-(2,2-tert-butanoyl)-4'-hydroxy-1,1',2,5'-tetrahydrospiro[indole-3,2'-pyrrole]-2,5'-dione,
6-chloro-1'-(5-chloro-2-methoxyphenyl)-4'-hydroxy-3'-(1-methylcyclopropane-carbonyl)-1,1',2,5'-tetrahydrospiro[indole-3,2'-pyrrole]-2,5'-dione,
6-chloro-1'-(5-chloro-2-methylphenyl)-4'-hydroxy-3'-(iso-propanoyl)-1,1',2,5'-tetrahydrospiro[indole-3,2'-pyrrole]-2,5'-dione,
6-chloro-1'-(5-chloro-2-hydroxyphenyl)-4'-hydroxy-3'-(iso-propanoyl)-1,1',2,5'-tetrahydrospiro[indole-3,2'-pyrrole]-2,5'-dione,
6-chloro-1'-(5-chloro-2-methoxyphenyl)-4'-hydroxy-3'-(iso-propanoyl)-1,1',2,5'-tetrahydrospiro[indole-3,2'-pyrrole]-2,5'-dione,
6-chloro-1'-(5-chloro-2-fluorophenyl)-4'-hydroxy-3'-(iso-propanoyl)-1,1',2,5'-tetrahydrospiro[indole-3,2'-pyrrole]-2,5'-dione,
6-chloro-1'-(5-chloro-1-methyl-6-oxo-1,6-dihydropyridin-3-yl)-4'-hydroxy-3'-(isopropanoyl)-1,1',2,5'-tetrahydrospiro[indole-3,2'-pyrrole]-2,5'-dione,
6-chloro-1'-(3-chlorophenyl)-3'-[2,2-dimethyl-3-(propan-2-yloxy)propanoyl]-4'-hydroxy-1,1',2,5'-tetrahydrospiro[indole-3,2'-pyrrole]-2,5'-dione,
2-{[6-chloro-1'-(5-chloro-2-methylphenyl)-3'-(1-methylcyclopropanecarbonyl)-2,5'-dioxo-1,1',2,5'-tetrahydrospiro[indole-3,2'-pyrrole]-4'-yl]amino}acetamide,
ethyl 2-{[6-chloro-1'-(3-chlorophenyl)-3'-(1-methylcyclopropanecarbonyl)-2,5'-dioxo-1,1',2,5'-tetrahydrospiro[indole-3,2'-pyrrole]-4'-yl]amino}acetate,
2-{[6-chloro-1'-(3-chlorophenyl)-3'-(1-methylcyclopropanecarbonyl)-2,5'-dioxo-1,1',2,5'-tetrahydrospiro[indole-3,2'-pyrrole]-4'-yl]amino}acetamide,
ethyl (2E)-3-[(3-{[6-chloro-1'-(3-chlorophenyl)-4'-hydroxy-2,5'-dioxo-1,1',2,5'-tetrahydrospiro[indole-3,2'-pyrrole]-3'-yl]carbonyl}phenyl)carbamoyl]prop-2-enoate,
6-chloro-5'-(5-chloro-2-methylphenyl)-3'-(1-methylcyclopropyl)-1,2,5',6'-tetrahydro-2'H-spiro[indole-3,4'-pyrrolo[3,4-c]pyrazole]-2,6'-dione,
6-chloro-5'-(3-chlorophenyl)-3'-(1-methylcyclopropyl)-1,2,5',6'-tetrahydro-2'H-spiro[indole-3,4'-pyrrolo[3,4-c]pyrazole]-2,6'-dione,
3'-tert-butyl-6-chloro-5'-(3-chlorophenyl)-1,2,5',6'-tetrahydro-2'H-spiro[indole-3,4'-pyrrolo[3,4-c]pyrazole]-2,6'-dione,
3'-tert-butyl-6-chloro-5'-(5-chloro-2-methoxyphenyl)-1,2,5',6'-tetrahydro-2'H-spiro[indole-3,4'-pyrrolo[3,4-c]pyrazole]-2,6'-dione,
6-chloro-5'-(5-chloro-2-methoxyphenyl)-3'-(1-methylcyclopropyl)-1,2,5',6'-tetrahydro-2'H-spiro[indole-3,4'-pyrrolo[3,4-c]pyrazole]-2,6'-dione,
6-chloro-5'-(5-chloro-2-methylphenyl)-3'-(propan-2-yl)-1,2,5',6'-tetrahydro-2'H-spiro[indole-3,4'-pyrrolo[3,4-c]pyrazole]-2,6'-dione,
6-chloro-5'-(5-chloro-2-hydroxyphenyl)-3'-(propan-2-yl)-1,2,5',6'-tetrahydro-2'H-spiro[indole-3,4'-pyrrolo[3,4-c]pyrazole]-2,6'-dione,
6-chloro-5'-(5-chloro-2-methoxyphenyl)-3'-(propan-2-yl)-1,2,5',6'-tetrahydro-2'H-spiro[indole-3,4'-pyrrolo[3,4-c]pyrazole]-2,6'-dione,
6-chloro-5'-(3-chlorophenyl)-2'-(2-methoxyphenyl)-3'-(1-methylcyclopropyl)-1,2,5',6'-tetrahydro-2'H-spiro[indole-3,4'-pyrrolo[3,4-c]pyrazole]-2,6'-dione,
6-chloro-5'-(3-chlorophenyl)-2'-(2-methoxyphenyl)-3'-(propan-2-yl)-1,2,5',6'-tetrahydro-2'H-spiro[indole-3,4'-pyrrolo[3,4-c]pyrazole]-2,6'-dione,
6-chloro-5'-(3-chlorophenyl)-3'-(1-methylcyclopropyl)-2'-(pyrrolidin-2-ylmethyl)-1,2,5',6'-tetrahydro-2'H-spiro[indole-3,4'-pyrrolo[3,4-c]pyrazole]-2,6'-dione,
2-[6-chloro-5'-(3-chlorophenyl)-3'-(1-methylcyclopropyl)-2,6'-dioxo-1,2,5',6'-tetrahydro-2'H-spiro[indole-3,4'-pyrrolo[3,4-c]pyrazole]-2'-ylmethyl]-N,N-dimethylcyclopentane-1-carboxamide,
6-chloro-5'-(5-chloro-2-methoxyphenyl)-2'-(2-methoxyphenyl)-3'-(propan-2-yl)-1,2,5',6'-tetrahydro-2'H-spiro[indole-3,4'-pyrrolo[3,4-c]pyrazole]-2,6'-dione,
6-chloro-5'-(5-chloro-2-methylphenyl)-2'-(2-methoxyphenyl)-3'-(propan-2-yl)-1,2,5',6'-tetrahydro-2'H-spiro[indole-3,4'-pyrrolo[3,4-c]pyrazole]-2,6'-dione,
6-chloro-5'-(5-chloro-2-methylphenyl)-1'-(2-methoxyphenyl)-3'-(propan-2-yl)-1,2,5',6'-tetrahydro-1'H-spiro[indole-3,4'-pyrrolo[3,4-c]pyrazole]-2,6'-dione,
2-[6-chloro-5'-(3-chlorophenyl)-2,6'-dioxo-3'-(propan-2-yl)-1,2,5',6'-tetrahydro-2'H-spiro[indole-3,4'-pyrrolo[3,4-c]pyrazole]-2'-ylmethyl]-N,N-dimethylpyrrolidine-1-carboxamide,
6-chloro-1'-(2,2-dimethylpropyl)-4'-hydroxy-3'-phenyl-1,1',2,5'-tetrahydrospiro[indole-3,2'-pyrrole]-2,5'-dione,
6-chloro-3'-(3-chlorophenyl)-1'-(2,2-dimethylpropyl)-4'-hydroxy-1,1',2,5'-tetrahydrospiro[indole-3,2'-pyrrole]-2,5'-dione,
6-chloro-3'-(3-chlorophenyl)-4'-hydroxy-1'-phenyl-1,1',2,5'-tetrahydrospiro[indole-3,2'-pyrrole]-2,5'-dione,
5-chloro-1'-(3-chlorophenyl)-4'-hydroxy-3'-phenyl-1,1',2,5'-tetrahydrospiro[indole-3,2'-pyrrole]-2,5'-dione,

1'-(3-chlorophenyl)-4'-hydroxy-3'-phenyl-1,1',2,5'-tetrahydrospiro[indole-3,2'-pyrrole]-2,5'-dione,
6-chloro-1',3'-bis(3-chlorophenyl)-4'-hydroxy-1,1',2,5'-tetrahydrospiro[indole-3,2'-pyrrole]-2,5'-dione,
6-chloro-1'-(3-chlorophenyl)-4'-hydroxy-3'-phenyl-1,1',2,5'-tetrahydrospiro[indole-3,2'-pyrrole]-2,5'-dione,
1'-(3-chlorophenyl)-6-fluoro-4'-hydroxy-3'-phenyl-1,1',2,5'-tetrahydrospiro[indole-3,2'-pyrrole]-2,5'-dione,
5,6-dichloro-1'-(3-chlorophenyl)-4'-hydroxy-3'-phenyl-1,1',2,5'-tetrahydrospiro[indole-3,2'-pyrrole]-2,5'-dione,
6-chloro-1'-(3-chlorophenyl)-3'-cyclohexyl-4'-hydroxy-1,1',2,5'-tetrahydrospiro[indole-3,2'-pyrrole]-2,5'-dione,
6-chloro-1'-(5-chloro-2-fluorophenyl)-4'-hydroxy-3'-phenyl-1,1',2,5'-tetrahydrospiro-[indole-3,2'-pyrrole]-2,5'-dione,
6-chloro-1'-(5-chloro-2-methoxyphenyl)-4'-hydroxy-3'-phenyl-1,1',2,5'-tetrahydrospiro-[indole-3,2'-pyrrole]-2,5'-dione,
6-chloro-1'-(5-chloro-2-methylphenyl)-4'-hydroxy-3'-phenyl-1,1',2,5'-tetrahydro-spiro[indole-3,2'-pyrrole]-2,5'-dione,
ethyl 2-{[6-chloro-1'-(3-chlorophenyl)-2,5'-dioxo-3'-phenyl-1,1',2,5'-tetrahydro-spiro[indole-3,2'-pyrrole]-4'-yl]amino}acetate,
2-{[6-chloro-1'-(3-chlorophenyl)-2,5'-dioxo-3'-phenyl-1,1',2,5'-tetrahydrospiro[indole-3,2'-pyrrole]-4'-yl]amino}acetic acid,
methyl 2-[6-chloro-1'-(3-chlorophenyl)-2,5'-dioxo-3'-phenyl-1,1',2,5'-tetrahydro-spiro[indole-3,2'-pyrrole]-4'-ylsulfanyl]acetate,
2-[6-chloro-1'-(3-chlorophenyl)-2,5'-dioxo-3'-phenyl-1,1',2,5'-tetrahydrospiro[indole-3,2'-pyrrole]-4'-ylsulfanyl]acetic acid,
6-chloro-1'-(3-chlorophenyl)-2,5'-dioxo-3'-phenyl-1,1',2,5'-tetrahydrospiro[indole-3,2'-pyrrole]-4'-yl methanesulfonate,
2-[6-chloro-1'-(3-chlorophenyl)-2,5'-dioxo-3'-phenyl-1,1',2,5'-tetrahydrospiro[indole-3,2'-pyrrole]-4'-ylsulfanyl]acetamide,
(3S)-6-chloro-1'-(3-chlorophenyl)-3'-[(4-chlorophenyl)sulfanyl]-4'-hydroxy-1,1',2,5'-tetrahydrospiro[indole-3,2'-pyrrole]-2,5'-dione,
6-chloro-1'-(3-chlorophenyl)-3'-[(4-chlorophenyl)sulfanyl]-4'-hydroxy-1,1',2,5'-tetrahydrospiro[indole-3,2'-pyrrole]-2,5'-dione,
3'-(butan-2-ylsulfanyl)-6-chloro-1'-(3-chlorophenyl)-4'-hydroxy-1,1',2,5'-tetrahydro-spiro[indole-3,2'-pyrrole]-2,5'-dione,
3'-(tert-butylsulfanyl)-6-chloro-1'-(3-chlorophenyl)-4'-hydroxy-1,1',2,5'-tetrahydro-spiro[indole-3,2'-pyrrole]-2,5'-dione,
6-chloro-1'-(3-chlorophenyl)-4'-hydroxy-3'-{[(4-methoxyphenyl)methyl]sulfanyl}-1,1',2,5'-tetrahydrospiro[indole-3,2'-pyrrole]-2,5'-dione,
6-chloro-1'-(3-chlorophenyl)-4'-hydroxy-3'-{[5-(morpholin-4-yl)-1,3,4-thiadiazol-2-yl]sulfanyl}-1,1',2,5'-tetrahydrospiro[indole-3,2'-pyrrole]-2,5'-dione,
6-chloro-3'-[(5-chloro-1,3-dimethyl-1H-pyrazol-4-yl)sulfanyl]-1'-(3-chlorophenyl)-4'-hydroxy-1,1',2,5'-tetrahydrospiro[indole-3,2'-pyrrole]-2,5'-dione,
methyl (2E)-4-{4-chloro-2-[6-chloro-2'-(2-methoxyphenyl)-2,6'-dioxo-3'-(propan-2-yl)-1,2,5',6'-tetrahydro-2'H-spiro[indole-3,4'-pyrrolo[3,4-c]pyrazole]-5'-yl]phenoxy}but-2-enoate,
6-chloro-5'-(5-chloro-1-methyl-6-oxo-1,6-dihydropyridin-3-yl)-2'-(2-methoxyphenyl)-3'-(propan-2-yl)-1,2,5',6'-tetrahydro-2'H-spiro[indole-3,4'-pyrrolo[3,4-c]pyrazole]-2,6'-dione,
6-chloro-5'-(5-chloro-2-methylphenyl)-2'-(3-methoxypyridin-4-yl)-3'-(propan-2-yl)-1,2,5',6'-tetrahydro-2'H-spiro[indole-3,4'-pyrrolo[3,4-c]pyrazole]-2,6'-dione,
(3S)-6-chloro-5'-(5-chloro-2-methylphenyl)-2'-(2-methoxyphenyl)-3'-(propan-2-yl)-1,2,5',6'-tetrahydro-2'H-spiro[indole-3,4'-pyrrolo[3,4-c]pyrazole]-2,6'-dione,
6-chloro-5'-(5-chloro-2-methylphenyl)-2'-(3-ethylphenyl)-3'-(propan-2-yl)-1,2,5',6'-tetrahydro-2'H-spiro[indole-3,4'-pyrrolo[3,4-c]pyrazole]-2,6'-dione,
6-chloro-5'-(5-chloro-2-methylphenyl)-2'-(2-ethylphenyl)-3'-(propan-2-yl)-1,2,5',6'-tetrahydro-2'H-spiro[indole-3,4'-pyrrolo[3,4-c]pyrazole]-2,6'-dione,
6-chloro-5'-(5-chloro-2-methylphenyl)-2'-(2-methylphenyl)-3'-(propan-2-yl)-1,2,5',6'-tetrahydro-2'H-spiro[indole-3,4'-pyrrolo[3,4-c]pyrazole]-2,6'-dione,
6-chloro-5'-(5-chloro-2-methylphenyl)-2'-(3-methylphenyl)-3'-(propan-2-yl)-1,2,5',6'-tetrahydro-2'H-spiro[indole-3,4'-pyrrolo[3,4-c]pyrazole]-2,6'-dione,
6-chloro-5'-(5-chloro-2-methylphenyl)-2'-(2,6-dimethoxyphenyl)-3'-(propan-2-yl)-1,2,5',6'-tetrahydro-2'H-spiro[indole-3,4'-pyrrolo[3,4-c]pyrazole]-2,6'-dione,
6-chloro-5'-(5-chloro-2-methylpyridin-3-yl)-2'-(2-methoxyphenyl)-3'-(propan-2-yl)-1,2,5',6'-tetrahydro-2'H-spiro[indole-3,4'-pyrrolo[3,4-c]pyrazole]-2,6'-dione,

6-chloro-5'-(5-chloropyridin-3-yl)-2'-(2-methoxyphenyl)-3'-(propan-2-yl)-1,2,5',6'-tetrahydro-2'H-spiro[indole-3,4'-pyrrolo[3,4-c]pyrazole]-2,6'-dione,
6-chloro-5'-[5-chloro-2-(2-methanesulfonylethoxy)phenyl]-2'-(2-methoxyphenyl)-3'-(propan-2-yl)-1,2,5',6'-tetrahydro-2'H-spiro[indole-3,4'-pyrrolo[3,4-c]pyrazole]-2,6'-dione,
2'-(1-benzothiophen-3-yl)-6-chloro-5'-(5-chloro-2-methylphenyl)-3'-(propan-2-yl)-1,2,5',6'-tetrahydro-2'H-spiro[indole-3,4'-pyrrolo[3,4-c]pyrazole]-2,6'-dione,
3-[6-chloro-5'-(5-chloro-2-hydroxyphenyl)-2,6'-dioxo-3'-(propan-2-yl)-1,2,5',6'-tetrahydro-2'H-spiro[indole-3,4'-pyrrolo[3,4-c]pyrazole]-2'-yl]-4-methoxybenzamide,
3-[6-chloro-5'-(5-chloro-2-hydroxyphenyl)-2,6'-dioxo-3'-(propan-2-yl)-1,2,5',6'-tetra-hydro-2'H-spiro[indole-3,4'-pyrrolo[3,4-c]pyrazole]-2'-yl]-4-methoxybenzoic acid,
6-chloro-5'-(5-chloro-2-methylphenyl)-2'-[2-(methylsulfanyl)phenyl]-3'-(propan-2-yl)-1,2,5',6'-tetrahydro-2'H-spiro[indole-3,4'-pyrrolo[3,4-c]pyrazole]-2,6'-dione,
methyl 3-[6-chloro-5'-(5-chloro-2-methylphenyl)-2,6'-dioxo-3'-(propan-2-yl)-1,2,5',6'-tetrahydro-2'H-spiro[indole-3,4'-pyrrolo[3,4-c]pyrazole]-2'-yl]-4-methoxybenzoate,
methyl 3-[6-chloro-5'-(5-chloro-2-hydroxyphenyl)-2,6'-dioxo-3'-(propan-2-yl)-1,2,5',6'-tetrahydro-2'H-spiro[indole-3,4'-pyrrolo[3,4-c]pyrazole]-2'-yl]-4-methoxybenzoate,
(3S)-6-chloro-5'-(5-chloro-2-methylpyridin-3-yl)-2'-(2-methoxyphenyl)-3'-(propan-2-yl)-1,2,5',6'-tetrahydro-2'H-spiro[indole-3,4'-pyrrolo[3,4-c]pyrazole]-2,6'-dione,
(3S)-6-chloro-5'-(5-chloropyridin-3-yl)-2'-(2-methoxyphenyl)-3'-(propan-2-yl)-1,2,5',6'-tetrahydro-2'H-spiro[indole-3,4'-pyrrolo[3,4-c]pyrazole]-2,6'-dione,
6-chloro-5'-(4-chloropyridin-2-yl)-3'-(propan-2-yl)-1,2,5',6'-tetrahydro-2'H-spiro[indole-3,4'-pyrrolo[3,4-c]pyrazole]-2,6'-dione,
6-chloro-5'-(5-chloropyridin-3-yl)-3'-(propan-2-yl)-1,2,5',6'-tetrahydro-2'H-spiro-[indole-3,4'-pyrrolo[3,4-c]pyrazole]-2,6'-dione,
6-chloro-5'-(5-chloro-2-methylpyridin-3-yl)-3'-(propan-2-yl)-1,2,5',6'-tetrahydro-2'H-spiro[indole-3,4'-pyrrolo[3,4-c]pyrazole]-2,6'-dione,
6-chloro-5'-(5-chloro-2-methylphenyl)-3'-ethyl-2'-(2-ethylphenyl)-1,2,5',6'-tetrahydro-2'H-spiro[indole-3,4'-pyrrolo[3,4-c]pyrazole]-2,6'-dione,
6-chloro-5'-(cyclobutylmethyl)-2'-(2-methoxyphenyl)-3'-(propan-2-yl)-1,2,5',6'-tetra-hydro-2'H-spiro[indole-3,4'-pyrrolo[3,4-c]pyrazole]-2,6'-dione,
6-chloro-5'-(2,2-dimethylpropyl)-2'-(2-methoxyphenyl)-3'-(propan-2-yl)-1,2,5',6'-tetrahydro-2'H-spiro[indole-3,4'-pyrrolo[3,4-c]pyrazole]-2,6'-dione,
(3S)-6-chloro-5'-(5-chloropyridin-3-yl)-2'-(2-methoxyphenyl)-3'-(propan-2-yl)-1,2,5',6'-tetrahydro-2'H-spiro[indole-3,4'-pyrrolo[3,4-c]pyrazole]-2,6'-dione,
6-chloro-2'-(3-chlorophenyl)-5'-(2-methoxyphenyl)-6'-(propan-2-yl)-1,2,3',5'-tetrahydro-2'H-spiro[indole-3,1'-pyrrolo[3,4-c]pyrrole]-2,3'-dione,
6-chloro-2'-(5-chloro-2-nitrophenyl)-5'-(2-methoxyphenyl)-6'-(propan-2-yl)-1,2,3',5'-tetrahydro-2'H-spiro[indole-3,1'-pyrrolo[3,4-c]pyrrole]-2,3'-dione,
2'-(2-amino-5-chlorophenyl)-6-chloro-5'-(2-methoxyphenyl)-6'-(propan-2-yl)-1,2,3',5'-tetrahydro-2'H-spiro[indole-3,1'-pyrrolo[3,4-c]pyrrole]-2,3'-dione,
N-{4-chloro-2-[6-chloro-5'-(2-methoxyphenyl)-2,3'-dioxo-6'-(propan-2-yl)-1,2,3',5'-tetrahydro-2'H-spiro[indole-3,1'-pyrrolo[3,4-c]pyrrole]-2'-yl]phenyl}methane-sulfonamide,
6-chloro-2'-(5-chloro-2-methylphenyl)-5'-(2,4-dimethoxyphenyl)-6'-(propan-2-yl)-1,2,3',5'-tetrahydro-2'H-spiro[indole-3,1'-pyrrolo[3,4-c]pyrrole]-2,3'-dione,
5'-(2H-1,3-benzodioxol-4-yl)-6-chloro-2'-(5-chloro-2-methylphenyl)-6'-(propan-2-yl)-1,2,3',5'-tetrahydro-2'H-spiro[indole-3,1'-pyrrolo[3,4-c]pyrrole]-2,3'-dione,
3-[6-chloro-2'-(5-chloro-2-methylphenyl)-2,3'-dioxo-6'-(propan-2-yl)-1,2,3',5'-tetrahydro-2'H-spiro[indole-3,1'-pyrrolo[3,4-c]pyrrole]-5'-yl]-4-methoxybenzamide,
5'-(5-amino-2-methoxyphenyl)-6-chloro-2'-(5-chloro-2-methylphenyl)-6'-(propan-2-yl)-1,2,3',5'-tetrahydro-2'H-spiro[indole-3,1'-pyrrolo[3,4-c]pyrrole]-2,3'-dione,
N-{3-[6-chloro-2'-(5-chloro-2-methylphenyl)-2,3'-dioxo-6'-(propan-2-yl)-1,2,3',5'-tetra-hydro-2'H-spiro[indole-3,1'-pyrrolo[3,4-c]pyrrole]-5'-yl]-4-methoxyphenyl}acetamide,
3-[6-chloro-2'-(5-chloro-2-methylphenyl)-2,3'-dioxo-6'-(propan-2-yl)-1,2,3',5'-tetra-hydro-2'H-spiro[indole-3,1'-pyrrolo[3,4-c]pyrrole]-5'-yl]-N,N-diethyl-4-methoxybenzene-1-sulfonamide,
4-chloro-2-[6-chloro-5'-(2-methoxyphenyl)-2,3'-dioxo-6'-(propan-2-yl)-1,2,3',5'-tetrahydro-2'H-spiro[indole-3,1'-pyrrolo[3,4-c]pyrrole]-2'-yl]benzamide,
3-[6-chloro-2'-(5-chloro-2-methylphenyl)-2,3'-dioxo-6'-(propan-2-yl)-1,2,3',5'-tetra-hydro-2'H-spiro[indole-3,1'-pyrrolo[3,4-c]pyrrole]-5'-yl]-N-(2-hydroxyethyl)-4-methoxybenzamide,

6-chloro-2'-(5-chloro-2-methylphenyl)-5'-(6-methoxy-2-oxo-2,3-dihydro-1H-1,3-benzo-diazol-5-yl)-6'-(propan-2-yl)-1,2,3',5'-tetrahydro-2'H-spiro[indole-3,1'-pyrrolo[3,4-c]-pyrrole]-2,3'-dione,
N-{4-chloro-2-[(3S)-6-chloro-5'-(2-methoxyphenyl)-2,3'-dioxo-6'-(propan-2-yl)-1,2,3',5'-tetrahydro-2'H-spiro[indole-3,1'-pyrrolo[3,4-c]pyrrole]-2'-yl]phenyl}methanesulphonamide,
(3S)-6-chloro-2'-(5-chloro-2-methylphenyl)-5'-[(3S,4R)-3-methoxypiperidin-4-yl]-6'-(propan-2-yl)-1,2,3',5'-tetrahydro-2'H-spiro[indole-3,1'-pyrrolo[3,4-c]pyrrole]-2,3'-dione,
6-chloro-2'-(5-chloro-2-methylpyridin-3-yl)-5'-(2,4-dimethoxyphenyl)-6'-(propan-2-yl)-1,2,3',5'-tetrahydro-2'H-spiro[indole-3,1'-pyrrolo[3,4-c]pyrrole]-2,3'-dione,
4-chloro-2-[6-chloro-5'-(2-methoxyphenyl)-2,3'-dioxo-6'-(propan-2-yl)-1,2,3',5'-tetrahydro-2'H-spiro[indole-3,1'-pyrrolo[3,4-c]pyrrole]-2'-yl]benzoic acid,
ethyl 4-chloro-2-[6-chloro-5'-(2-methoxyphenyl)-2,3'-dioxo-6'-(propan-2-yl)-1,2,3',5'-tetrahydro-2'H-spiro[indole-3,1'-pyrrolo[3,4-c]pyrrole]-2'-yl]benzoate,
(3S)-6-chloro-2'-(5-chloro-2-methylphenyl)-5'-(2,4-dimethoxyphenyl)-6'-(propan-2-yl)-1,2,3',5'-tetrahydro-2'H-spiro[indole-3,1'-pyrrolo[3,4-c]pyrrole]-2,3'-dione,
N-{3-[(3S)-6-chloro-2'-(5-chloro-2-methylphenyl)-2,3'-dioxo-6'-(propan-2-yl)-1,2,3',5'-tetrahydro-2'H-spiro[indole-3,1'-pyrrolo[3,4-c]pyrrole]-5'-yl]-4-methoxyphenyl}-acetamide,
(3S)-5'-(5-amino-2-methoxyphenyl)-6-chloro-2'-(5-chloro-2-methylphenyl)-6'-(propan-2-yl)-1,2,3',5'-tetrahydro-2'H-spiro[indole-3,1'-pyrrolo[3,4-c]pyrrole]-2,3'-dione,
3-[(3S)-6-chloro-2'-(5-chloro-2-methylphenyl)-2,3'-dioxo-6'-(propan-2-yl)-1,2,3',5'-tetrahydro-2'H-spiro[indole-3,1'-pyrrolo[3,4-c]pyrrole]-5'-yl]-4-methoxybenzamide,
(3S)-6-chloro-2'-(5-chloro-2-methylpyridin-3-yl)-5'-(2,4-dimethoxyphenyl)-6'-(propan-2-yl)-1,2,3',5'-tetrahydro-2'H-spiro[indole-3,1'-pyrrolo[3,4-c]pyrrole]-2,3'-dione,
6-chloro-2'-(5-chloro-2-methylphenyl)-5'-[2-methoxy-5-(morpholine-4-sulfonyl)-phenyl]-6'-(propan-2-yl)-1,2,3',5'-tetrahydro-2'H-spiro[indole-3,1'-pyrrolo[3,4-c]-pyrrole]-2,3'-dione,
6-chloro-2'-(5-chloro-2-methylphenyl)-5'-(2-methoxythiophen-3-yl)-6'-(propan-2-yl)-1,2,3',5'-tetrahydro-2'H-spiro[indole-3,1'-pyrrolo[3,4-c]pyrrole]-2,3'-dione, and
6-chloro-5'-(5-chloro-2-methylphenyl)-2'-(2,6-dimethoxypyridin-3-yl)-3'-(propan-2-yl)-1,2,5',6'-tetrahydro-2'H-spiro[indole-3,4'-pyrrolo[3,4-c]pyrazole]-2,6'-dione.

**16.** The compound of claim 15 selected from the following group:

(3S)-6-chloro-2'-(5-chloro-2-methylphenyl)-5'-(2,4-dimethoxyphenyl)-6'-(propan-2-yl)-1,2,3',5'-tetrahydro-2'H-spiro[indole-3,1'-pyrrolo[3,4-c]pyrrole]-2,3'-dione,
N-{3-[(3S)-6-chloro-2'-(5-chloro-2-methylphenyl)-2,3'-dioxo-6'-(propan-2-yl)-1,2,3',5'-tetrahydro-2'H-spiro[indole-3,1'-pyrrolo[3,4-c]pyrrole]-5'-yl]-4-methoxyphenyl}-acetamide,
(3S)-5'-(5-amino-2-methoxyphenyl)-6-chloro-2'-(5-chloro-2-methylphenyl)-6'-(propan-2-yl)-1,2,3',5'-tetrahydro-2'H-spiro[indole-3,1'-pyrrolo[3,4-c]pyrrole]-2,3'-dione,
3-[(3S)-6-chloro-2'-(5-chloro-2-methylphenyl)-2,3'-dioxo-6'-(propan-2-yl)-1,2,3',5'-tetrahydro-2'H-spiro[indole-3,1'-pyrrolo[3,4-c]pyrrole]-5'-yl]-4-methoxybenzamide,
(3S)-6-chloro-2'-(5-chloro-2-methylpyridin-3-yl)-5'-(2,4-dimethoxyphenyl)-6'-(propan-2-yl)-1,2,3',5'-tetrahydro-2'H-spiro[indole-3,1'-pyrrolo[3,4-c]pyrrole]-2,3'-dione,
(3S)-6-chloro-2'-(5-chloro-2-methylphenyl)-5'-[(3S,4R)-3-methoxypiperidin-4-yl]-6'-(propan-2-yl)-1,2,3',5'-tetrahydro-2'H-spiro[indole-3,1'-pyrrolo[3,4-c]pyrrole]-2,3'-dione,
(3S)-6-chloro-5'-(5-chloropyridin-3-yl)-2'-(2-methoxyphenyl)-3'-(propan-2-yl)-1,2,5',6'-tetrahydro-2'H-spiro[indole-3,4'-pyrrolo[3,4-c]pyrazole]-2,6'-dione,
(3S)-6-chloro-5'-(5-chloro-2-methylpyridin-3-yl)-2'-(2-methoxyphenyl)-3'-(propan-2-yl)-1,2,5',6'-tetrahydro-2'H-spiro[indole-3,4'-pyrrolo[3,4-c]pyrazole]-2,6'-dione,
(3S)-6-chloro-5'-(5-chloropyridin-3-yl)-2'-(2-methoxyphenyl)-3'-(propan-2-yl)-1,2,5',6'-tetrahydro-2'H-spiro[indole-3,4'-pyrrolo[3,4-c]pyrazole]-2,6'-dione, and
(3S)-6-chloro-5'-(5-chloro-2-methylphenyl)-2'-(2-methoxyphenyl)-3'-(propan-2-yl)-1,2,5',6'-tetrahydro-2'H-spiro[indole-3,4'-pyrrolo[3,4-c]pyrazole]-2,6'-dione.

**17.** The compound of claim 15 which is (3S)-6-chloro-1'-(3-chlorophenyl)-3'-[(4-chlorophenyl)sulfanyl]-4'-hydroxy-1,1',2,5'-tetrahydrospiro[indole-3,2'-pyrrole]-2,5'-dione.

**18.** The compound of claim 15 selected from the following group:

6-chloro-5'-(5-chloro-2-methylphenyl)-3'-(1-methylcyclopropyl)-1,2,5',6'-tetrahydro-2'H-spiro[indole-3,4'-pyrrolo[3,4-c]pyrazole]-2,6'-dione,
6-chloro-5'-(3-chlorophenyl)-3'-(1-methylcyclopropyl)-1,2,5',6'-tetrahydro-2'H-spiro[indole-3,4'-pyrrolo[3,4-c]pyrazole]-2,6'-dione,
3'-tert-butyl-6-chloro-5'-(3-chlorophenyl)-1,2,5',6'-tetrahydro-2'H-spiro[indole-3,4'-pyrrolo[3,4-c]pyrazole]-2,6'-dione,
3'-tert-butyl-6-chloro-5'-(5-chloro-2-methoxyphenyl)-1,2,5',6'-tetrahydro-2'H-spiro[indole-3,4'-pyrrolo[3,4-c]pyrazole]-2,6'-dione,
6-chloro-5'-(5-chloro-2-methoxyphenyl)-3'-(1-methylcyclopropyl)-1,2,5',6'-tetrahydro-2'H-spiro[indole-3,4'-pyrrolo[3,4-c]pyrazole]-2,6'-dione,
6-chloro-5'-(5-chloro-2-methylphenyl)-3'-(propan-2-yl)-1,2,5',6'-tetrahydro-2'H-spiro[indole-3,4'-pyrrolo[3,4-c]pyrazole]-2,6'-dione,
6-chloro-5'-(5-chloro-2-hydroxyphenyl)-3'-(propan-2-yl)-1,2,5',6'-tetrahydro-2'H-spiro[indole-3,4'-pyrrolo[3,4-c]pyrazole]-2,6'-dione,
6-chloro-5'-(5-chloro-2-methoxyphenyl)-3'-(propan-2-yl)-1,2,5',6'-tetrahydro-2'H-spiro[indole-3,4'-pyrrolo[3,4-c]pyrazole]-2,6'-dione,
6-chloro-5'-(3-chlorophenyl)-2'-(2-methoxyphenyl)-3'-(1-methylcyclopropyl)-1,2,5',6'-tetrahydro-2'H-spiro[indole-3,4'-pyrrolo[3,4-c]pyrazole]-2,6'-dione,
6-chloro-5'-(3-chlorophenyl)-2'-(2-methoxyphenyl)-3'-(propan-2-yl)-1,2,5',6'-tetrahydro-2'H-spiro[indole-3,4'-pyrrolo[3,4-c]pyrazole]-2,6'-dione,
6-chloro-5'-(3-chlorophenyl)-3'-(1-methylcyclopropyl)-2'-(pyrrolidin-2-ylmethyl)-1,2,5',6'-tetrahydro-2'H-spiro[indole-3,4'-pyrrolo[3,4-c]pyrazole]-2,6'-dione,
2-[6-chloro-5'-(3-chlorophenyl)-3'-(1-methylcyclopropyl)-2,6'-dioxo-1,2,5',6'-tetrahydro-2'H-spiro[indole-3,4'-pyrrolo[3,4-c]pyrazole]-2'-ylmethyl]-N,N-dimethylcyclopentane-1-carboxamide,
6-chloro-5'-(5-chloro-2-methoxyphenyl)-2'-(2-methoxyphenyl)-3'-(propan-2-yl)-1,2,5',6'-tetrahydro-2'H-spiro[indole-3,4'-pyrrolo[3,4-c]pyrazole]-2,6'-dione,
6-chloro-5'-(5-chloro-2-methylphenyl)-2'-(2-methoxyphenyl)-3'-(propan-2-yl)-1,2,5',6'-tetrahydro-2'H-spiro[indole-3,4'-pyrrolo[3,4-c]pyrazole]-2,6'-dione,
6-chloro-5'-(5-chloro-2-methylphenyl)-1'-(2-methoxyphenyl)-3'-(propan-2-yl)-1,2,5',6'-tetrahydro-1'H-spiro[indole-3,4'-pyrrolo[3,4-c]pyrazole]-2,6'-dione, and
2-[6-chloro-5'-(3-chlorophenyl)-2,6'-dioxo-3'-(propan-2-yl)-1,2,5',6'-tetrahydro-2'H-spiro[indole-3,4'-pyrrolo[3,4-c]pyrazole]-2'-ylmethyl]-N,N-dimethylpyrrolidine-1-carboxamide.

**19.** The compound of claim 15 selected from the following group:

methyl (2E)-4-{4-chloro-2-[6-chloro-2'-(2-methoxyphenyl)-2,6'-dioxo-3'-(propan-2-yl)-1,2,5',6'-tetrahydro-2'H-spiro[indole-3,4'-pyrrolo[3,4-c]pyrazole]-5'-yl]phenoxy}but-2-enoate,
6-chloro-5'-(5-chloro-1-methyl-6-oxo-1,6-dihydropyridin-3-yl)-2'-(2-methoxyphenyl)-3'-(propan-2-yl)-1,2,5',6'-tetrahydro-2'H-spiro[indole-3,4'-pyrrolo[3,4-c]pyrazole]-2,6'-dione,
6-chloro-5'-(5-chloro-2-methylphenyl)-2'-(3-methoxypyridin-4-yl)-3'-(propan-2-yl)-1,2,5',6'-tetrahydro-2'H-spiro[indole-3,4'-pyrrolo[3,4-c]pyrazole]-2,6'-dione,
(3S)-6-chloro-5'-(5-chloro-2-methylphenyl)-2'-(2-methoxyphenyl)-3'-(propan-2-yl)-1,2,5',6'-tetrahydro-2'H-spiro[indole-3,4'-pyrrolo[3,4-c]pyrazole]-2,6'-dione,
6-chloro-5'-(5-chloro-2-methylphenyl)-2'-(3-ethylphenyl)-3'-(propan-2-yl)-1,2,5',6'-tetrahydro-2'H-spiro[indole-3,4'-pyrrolo[3,4-c]pyrazole]-2,6'-dione,
6-chloro-5'-(5-chloro-2-methylphenyl)-2'-(2-ethylphenyl)-3'-(propan-2-yl)-1,2,5',6'-tetrahydro-2'H-spiro[indole-3,4'-pyrrolo[3,4-c]pyrazole]-2,6'-dione,
6-chloro-5'-(5-chloro-2-methylphenyl)-2'-(2-methylphenyl)-3'-(propan-2-yl)-1,2,5',6'-tetrahydro-2'H-spiro[indole-3,4'-pyrrolo[3,4-c]pyrazole]-2,6'-dione,
6-chloro-5'-(5-chloro-2-methylphenyl)-2'-(3-methylphenyl)-3'-(propan-2-yl)-1,2,5',6'-tetrahydro-2'H-spiro[indole-3,4'-pyrrolo[3,4-c]pyrazole]-2,6'-dione,
6-chloro-5'-(5-chloro-2-methylphenyl)-2'-(2,6-dimethoxyphenyl)-3'-(propan-2-yl)-1,2,5',6'-tetrahydro-2'H-spiro[indole-3,4'-pyrrolo[3,4-c]pyrazole]-2,6'-dione,
6-chloro-5'-(5-chloro-2-methylpyridin-3-yl)-2'-(2-methoxyphenyl)-3'-(propan-2-yl)-1,2,5',6'-tetrahydro-2'H-spiro[indole-3,4'-pyrrolo[3,4-c]pyrazole]-2,6'-dione,
6-chloro-5'-(5-chloropyridin-3-yl)-2'-(2-methoxyphenyl)-3'-(propan-2-yl)-1,2,5',6'-tetrahydro-2'H-spiro[indole-3,4'-pyrrolo[3,4-c]pyrazole]-2,6'-dione,
6-chloro-5'-[5-chloro-2-(2-methanesulfonylethoxy)phenyl]-2'-(2-methoxyphenyl)-3'-(propan-2-yl)-1,2,5',6'-tetrahydro-2'H-spiro[indole-3,4'-pyrrolo[3,4-c]pyrazole]-2,6'-dione,

2'-(1-benzothiophen-3-yl)-6-chloro-5'-(5-chloro-2-methylphenyl)-3'-(propan-2-yl)-1,2,5',6'-tetrahydro-2'H-spiro[indole-3,4'-pyrrolo[3,4-c]pyrazole]-2,6'-dione,
3-[6-chloro-5'-(5-chloro-2-hydroxyphenyl)-2,6'-dioxo-3'-(propan-2-yl)-1,2,5',6'-tetrahydro-2'H-spiro[indole-3,4'-pyrrolo[3,4-c]pyrazole]-2'-yl]-4-methoxybenzamide,
3-[6-chloro-5'-(5-chloro-2-hydroxyphenyl)-2,6'-dioxo-3'-(propan-2-yl)-1,2,5',6'-tetra-hydro-2'H-spiro[indole-3,4'-pyrrolo[3,4-c]pyrazole]-2'-yl]-4-methoxybenzoic acid
6-chloro-5'-(5-chloro-2-methylphenyl)-2'-[2-(methylsulfanyl)phenyl]-3'-(propan-2-yl)-1,2,5',6'-tetrahydro-2'H-spiro[indole-3,4'-pyrrolo[3,4-c]pyrazole]-2,6'-dione,
methyl 3-[6-chloro-5'-(5-chloro-2-methylphenyl)-2,6'-dioxo-3'-(propan-2-yl)-1,2,5',6'-tetrahydro-2'H-spiro[indole-3,4'-pyrrolo[3,4-c]pyrazole]-2'-yl]-4-methoxybenzoate,
methyl 3-[6-chloro-5'-(5-chloro-2-hydroxyphenyl)-2,6'-dioxo-3'-(propan-2-yl)-1,2,5',6'-tetrahydro-2'H-spiro[indole-3,4'-pyrrolo[3,4-c]pyrazole]-2'-yl]-4-methoxybenzoate,
(3S)-6-chloro-5'-(5-chloro-2-methylpyridin-3-yl)-2'-(2-methoxyphenyl)-3'-(propan-2-yl)-1,2,5',6'-tetrahydro-2'H-spiro[indole-3,4'-pyrrolo[3,4-c]pyrazole]-2,6'-dione,
(3S)-6-chloro-5'-(5-chloropyridin-3-yl)-2'-(2-methoxyphenyl)-3'-(propan-2-yl)-1,2,5',6'-tetrahydro-2'H-spiro[indole-3,4'-pyrrolo[3,4-c]pyrazole]-2,6'-dione,
6-chloro-5'-(4-chloropyridin-2-yl)-3'-(propan-2-yl)-1,2,5',6'-tetrahydro-2'H-spiro[indole-3,4'-pyrrolo[3,4-c]pyrazole]-2,6'-dione,
6-chloro-5'-(5-chloropyridin-3-yl)-3'-(propan-2-yl)-1,2,5',6'-tetrahydro-2'H-spiro-[indole-3,4'-pyrrolo[3,4-c]pyrazole]-2,6'-dione,
6-chloro-5'-(5-chloro-2-methylpyridin-3-yl)-3'-(propan-2-yl)-1,2,5',6'-tetrahydro-2'H-spiro[indole-3,4'-pyrrolo[3,4-c]pyrazole]-2,6'-dione
6-chloro-5'-(5-chloro-2-methylphenyl)-3'-ethyl-2'-(2-ethylphenyl)-1,2,5',6'-tetrahydro-2'H-spiro[indole-3,4'-pyrrolo[3,4-c]pyrazole]-2,6'-dione,
6-chloro-5'-(cyclobutylmethyl)-2'-(2-methoxyphenyl)-3'-(propan-2-yl)-1,2,5',6'-tetra-hydro-2'H-spiro[indole-3,4'-pyrrolo[3,4-c]pyrazole]-2,6'-dione,
6-chloro-5'-(2,2-dimethylpropyl)-2'-(2-methoxyphenyl)-3'-(propan-2-yl)-1,2,5',6'-tetrahydro-2'H-spiro[indole-3,4'-pyrrolo[3,4-c]pyrazole]-2,6'-dione,
(3S)-6-chloro-5'-(5-chloropyridin-3-yl)-2'-(2-methoxyphenyl)-3'-(propan-2-yl)-1,2,5',6'-tetrahydro-2'H-spiro[indole-3,4'-pyrrolo[3,4-c]pyrazole]-2,6'-dione,
6-chloro-2'-(3-chlorophenyl)-5'-(2-methoxyphenyl)-6'-(propan-2-yl)-1,2,3',5'-tetrahydro-2'H-spiro[indole-3,1'-pyrrolo[3,4-c]pyrrole]-2,3'-dione,
6-chloro-2'-(5-chloro-2-nitrophenyl)-5'-(2-methoxyphenyl)-6'-(propan-2-yl)-1,2,3',5'-tetrahydro-2'H-spiro[indole-3,1'-pyrrolo[3,4-c]pyrrole]-2,3'-dione,
2'-(2-amino-5-chlorophenyl)-6-chloro-5'-(2-methoxyphenyl)-6'-(propan-2-yl)-1,2,3',5'-tetrahydro-2'H-spiro[indole-3,1'-pyrrolo[3,4-c]pyrrole]-2,3'-dione,
N-{4-chloro-2-[6-chloro-5'-(2-methoxyphenyl)-2,3'-dioxo-6'-(propan-2-yl)-1,2,3',5'-tetrahydro-2'H-spiro[indole-3,1'-pyrrolo[3,4-c]pyrrole]-2'-yl]phenyl}methane-sulfonamide,
6-chloro-2'-(5-chloro-2-methylphenyl)-5'-(2,4-dimethoxyphenyl)-6'-(propan-2-yl)-1,2,3',5'-tetrahydro-2'H-spiro[indole-3,1'-pyrrolo[3,4-c]pyrrole]-2,3'-dione,
5'-(2H-1,3-benzodioxol-4-yl)-6-chloro-2'-(5-chloro-2-methylphenyl)-6'-(propan-2-yl)-1,2,3',5'-tetrahydro-2'H-spiro[indole-3,1'-pyrrolo[3,4-c]pyrrole]-2,3'-dione,
3-[6-chloro-2'-(5-chloro-2-methylphenyl)-2,3'-dioxo-6'-(propan-2-yl)-1,2,3',5'-tetrahydro-2'H-spiro[indole-3,1'-pyrrolo[3,4-c]pyrrole]-5'-yl]-4-methoxybenzamide,
5'-(5-amino-2-methoxyphenyl)-6-chloro-2'-(5-chloro-2-methylphenyl)-6'-(propan-2-yl)-1,2,3',5'-tetrahydro-2'H-spiro[indole-3,1'-pyrrolo[3,4-c]pyrrole]-2,3'-dione,
N-{3-[6-chloro-2'-(5-chloro-2-methylphenyl)-2,3'-dioxo-6'-(propan-2-yl)-1,2,3',5'-tetra-hydro-2'H-spiro[indole-3,1'-pyrrolo[3,4-c]pyrrole]-5'-yl]-4-methoxyphenyl}acetamide,
3-[6-chloro-2'-(5-chloro-2-methylphenyl)-2,3'-dioxo-6'-(propan-2-yl)-1,2,3',5'-tetra-hydro-2'H-spiro[indole-3,1'-pyrrolo[3,4-c]pyrrole]-5'-yl]-N,N-diethyl-4-methoxybenzene-1-sulfonamide,
4-chloro-2-[6-chloro-5'-(2-methoxyphenyl)-2,3'-dioxo-6'-(propan-2-yl)-1,2,3',5'-tetrahydro-2'H-spiro[indole-3,1'-pyrrolo[3,4-c]pyrrole]-2'-yl]benzamide,
3-[6-chloro-2'-(5-chloro-2-methylphenyl)-2,3'-dioxo-6'-(propan-2-yl)-1,2,3',5'-tetra-hydro-2'H-spiro[indole-3,1'-pyrrolo[3,4-c]pyrrole]-5'-yl]-N-(2-hydroxyethyl)-4-methoxybenzamide,
6-chloro-2'-(5-chloro-2-methylphenyl)-5'-(6-methoxy-2-oxo-2,3-dihydro-1H-1,3-benzodiazol-5-yl)-6'-(propan-2-yl)-1,2,3',5'-tetrahydro-2'H-spiro[indole-3,1'-pyrrolo[3,4-c]-pyrrole]-2,3'-dione,
N-{4-chloro-2-[(3S)-6-chloro-5'-(2-methoxyphenyl)-2,3'-dioxo-6'-(propan-2-yl)-1,2,3',5'-tetrahydro-2'H-spiro[indole-3,1'-pyrrolo[3,4-c]pyrrole]-2'-yl]phenyl}methanesulphonamide,

(3S)-6-chloro-2'-(5-chloro-2-methylphenyl)-5'-[(3S,4R)-3-methoxypiperidin-4-yl]-6'-(propan-2-yl)-1,2,3',5'-tetrahydro-2'H-spiro[indole-3,1'-pyrrolo[3,4-c]pyrrole]-2,3'-dione,
6-chloro-2'-(5-chloro-2-methylpyridin-3-yl)-5'-(2,4-dimethoxyphenyl)-6'-(propan-2-yl)-1,2,3',5'-tetrahydro-2'H-spiro[indole-3,1'-pyrrolo[3,4-c]pyrrole]-2,3'-dione,
4-chloro-2-[6-chloro-5'-(2-methoxyphenyl)-2,3'-dioxo-6'-(propan-2-yl)-1,2,3',5'-tetrahydro-2'H-spiro[indole-3,1'-pyrrolo[3,4-c]pyrrole]-2'-yl]benzoic acid,
ethyl 4-chloro-2-[6-chloro-5'-(2-methoxyphenyl)-2,3'-dioxo-6'-(propan-2-yl)-1,2,3',5'-tetrahydro-2'H-spiro[indole-3,1'-pyrrolo[3,4-c]pyrrole]-2'-yl]benzoate,
(3S)-6-chloro-2'-(5-chloro-2-methylphenyl)-5'-(2,4-dimethoxyphenyl)-6'-(propan-2-yl)-1,2,3',5'-tetrahydro-2'H-spiro[indole-3,1'-pyrrolo[3,4-c]pyrrole]-2,3'-dione,
N-{3-[(3S)-6-chloro-2'-(5-chloro-2-methylphenyl)-2,3'-dioxo-6'-(propan-2-yl)-1,2,3',5'-tetrahydro-2'H-spiro[indole-3,1'-pyrrolo[3,4-c]pyrrole]-5'-yl]-4-methoxyphenyl}-acetamide,
(3S)-5'-(5-amino-2-methoxyphenyl)-6-chloro-2'-(5-chloro-2-methylphenyl)-6'-(propan-2-yl)-1,2,3',5'-tetrahydro-2'H-spiro[indole-3,1'-pyrrolo[3,4-c]pyrrole]-2,3'-dione,
3-[(3S)-6-chloro-2'-(5-chloro-2-methylphenyl)-2,3'-dioxo-6'-(propan-2-yl)-1,2,3',5'-tetrahydro-2'H-spiro[indole-3,1'-pyrrolo[3,4-c]pyrrole]-5'-yl]-4-methoxybenzamide,
(3S)-6-chloro-2'-(5-chloro-2-methylpyridin-3-yl)-5'-(2,4-dimethoxyphenyl)-6'-(propan-2-yl)-1,2,3',5'-tetrahydro-2'H-spiro[indole-3,1'-pyrrolo[3,4-c]pyrrole]-2,3'-dione,
6-chloro-2'-(5-chloro-2-methylphenyl)-5'-[2-methoxy-5-(morpholine-4-sulfonyl)-phenyl]-6'-(propan-2-yl)-1,2,3',5'-tetrahydro-2'H-spiro[indole-3,1'-pyrrolo[3,4-c]-pyrrole]-2,3'-dione,
6-chloro-2'-(5-chloro-2-methylphenyl)-5'-(2-methoxythiophen-3-yl)-6'-(propan-2-yl)-1,2,3',5'-tetrahydro-2'H-spiro[indole-3,1'-pyrrolo[3,4-c]pyrrole]-2,3'-dione, and
6-chloro-5'-(5-chloro-2-methylphenyl)-2'-(2,6-dimethoxypyridin-3-yl)-3'-(propan-2-yl)-1,2,5',6'-tetrahydro-2'H-spiro[indole-3,4'-pyrrolo[3,4-c]pyrazole]-2,6'-dione.

**20.** A compound according to any one of claims 1 to 19 for use as a medicament.

**21.** The compound for use of claim 20, for use in a method of prevention and/or treatment of diseases selected from the group consisting of cancer, immune diseases, inflammatory conditions, allergic skin diseases associated with excessive proliferation, and viral infections.

**22.** A pharmaceutical composition comprising as an active ingredient a compound as defined in any one of claims 1 to 19, in combination with at least one pharmaceutically acceptable excipient.

## Patentansprüche

**1.** Eine Verbindung, dargestellt durch die Formel, ausgewählt aus der Gruppe bestehend aus Formel (IA) und (IB)

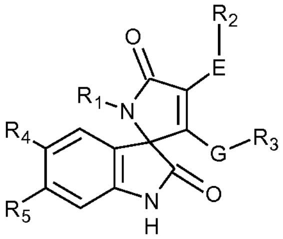

(IA)

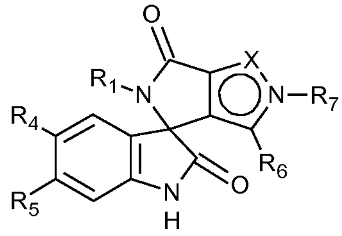

(IB)

wobei

$R_1$ steht für:

- $C_1$-$C_6$-Alkyl, das unsubstituiert oder durch $C_3$-$C_6$-Cycloalkyl substituiert ist,
- Phenyl oder 3-Pyridyl, das unsubstituiert oder durch einen oder zwei Substituenten substituiert ist, die unabhängig voneinander aus der Gruppe bestehend aus Halogen, -OH, $-NH_2$, $-NO_2$, -CN, $C_1$-$C_6$-Alkyl, $C_2$-$C_6$-Alkenyl, -O-($C_1$-$C_6$-Alkyl), -O-($C_2$-$C_6$-Alkenyl), -S-($C_1$-$C_6$-Alkyl), -S-($C_2$-$C_6$-Alkenyl),

-C(O)O-($C_1$-$C_6$-Alkyl), -C(O)O-($C_2$-$C_6$-Alkenyl), -C(O)$NH_2$, -C(O)NH($C_1$-$C_6$-Alkyl), -C(O)N($C_1$-$C_6$-Alkyl)$_2$, -C(O)NH($C_2$-$C_6$-Alkenyl), -NH($C_1$-$C_6$-Alkyl), -N($C_1$-$C_6$-Alkyl)$_2$, -NH-Phenyl, -NHC(O)-($C_1$-$C_6$-Alkyl), -NHC(O)-($C_2$-$C_6$-Alkenyl), -NHC(O)O-($C_1$-$C_6$-Alkyl) und -$NHSO_2$-($C_1$-$C_6$-Alkyl) ausgewählt sind, wobei das $C_1$-$C_6$-Alkyl, $C_2$-$C_6$-Alkenyl und Phenyl unsubstituiert oder weiter durch einen oder zwei Substituenten substituiert sind, die unabhängig voneinander aus der Gruppe bestehend aus Halogen, -OH, -SH, -O-($C_1$-$C_6$-Alkyl), -COOH, -C(O)O-($C_1$-$C_6$-Alkyl) und -$SO_2$-($C_1$-$C_6$-Alkyl) ausgewählt sind,

- Pyrazolyl, das unsubstituiert oder mit einem oder zwei Substituenten substituiert ist, die unabhängig voneinander $C_1$-$C_6$-Alkyl sind,
- 6-Oxo-1,6-Dihydropyridin-3-yl, das unsubstituiert oder durch einen oder zwei Substituenten substituiert ist, die unabhängig voneinander aus der Gruppe ausgewählt bestehend aus Halogen und $C_1$-$C_6$-Alkyl sind, oder
- 2-Oxo-1,2-Dihydropyridin-3-yl, das unsubstituiert oder durch einen oder zwei Substituenten substituiert ist, die unabhängig voneinander aus der Gruppe bestehend aus Halogen und $C_1$-$C_6$-Alkyl ausgewählt sind;

$R_4$ und $R_5$ stehen unabhängig voneinander für H oder Halogen;
$R_2$ steht für ein Wassestoffatom, ($C_1$-$C_6$-Alkyl)Sulfonyl, -($C_1$-$C_6$-Alkyl), oder -($C_1$-$C_6$-Alkyl), das endständig mit einem Substituenten substituiert ist, der aus der Gruppe bestehend aus -COOH, -$CONH_2$, -C(O)O-($C_1$-$C_6$-Alkyl), -$NH_2$, NH($C_1$-$C_6$-Alkyl), -N($C_1$-$C_6$-Alkyl)$_2$, -NHC(O)($C_1$-$C_6$-Alkyl), Imidazol, Tetrazol, und Phenyl, ausgewählt ist, worin das Phenyl durch -($C_1$-$C_3$-Alkyl), -O($C_1$-$C_3$-Alkyl) oder Halogen substituiert ist;
$R_3$ steht für:

- $C_1$-$C_6$-Alkyl,
- $C_3$-$C_6$-Cycloalkyl, das unsubstituiert oder durch $C_1$-$C_6$-Alkyl substituiert ist,
- Phenyl, das unsubstituiert oder durch eine, zwei oder drei Substituenten substituiert ist, die unabhängig aus der Gruppe bestehend aus Halogen, -OH, -$NH_2$, - $NO_2$, -CN, $C_1$-$C_6$-Alkyl, $C_2$-$C_6$-Alkenyl, -O-($C_1$-$C_6$-Alkyl), -O-($C_2$-$C_6$-Alkenyl), -S-($C_1$-$C_6$-Alkyl), -S-($C_2$-$C_6$-Alkenyl), -C(O)O-($C_1$-$C_6$-Alkyl), -C(O)O-($C_2$-$C_6$-Alkenyl), -C(O)$NH_2$, -(O)NH($C_1$-$C_6$-Alkyl), -C(O)N($C_1$-$C_6$-Alkyl)$_2$, -C(O)NH($C_2$-$C_6$-Alkenyl), -NH($C_1$-$C_6$-Alkyl), -N($C_1$-$C_6$-Alkyl)$_2$, -NH-Phenyl, -NHC(O)-($C_1$-$C_6$-Alkyl), -NHC(O)-($C_2$-$C_6$-Alkenyl), und -NHC(O)O-($C_1$-$C_6$-Alkyl) ausgewählt sind, und worin das $C_1$-$C_6$-Alkyl, $C_2$-$C_6$-Alkenyl und Phenyl unsubstituiert oder durch einen oder zwei Substituenten weiter substituiert ist, die unabhängig voneinander aus der Gruppe bestehend aus Halogen, -OH, -SH, -O-($C_1$-$C_6$-Alkyl), -COOH, und -C(O)O-($C_1$-$C_6$-Alkyl) ausgewählt sind, oder
- 5- oder 6-gliedriges Heteroaryl mit einem, zwei, drei oder vier Heteroatomen, die unabhängig voneinander aus N, 0 und S ausgewählt sind, worin das Heteroaryl unsubstituiert oder durch einen, zwei oder drei Substituenten substituiert ist, die unabhängig aus einer Gruppe bestehend aus Halogen, -OH, -$NH_2$, -$NO_2$, -CN, $C_1$-$C_6$-Alkyl, $C_2$-$C_6$-Alkenyl, -O-($C_1$-$C_6$-Alkyl), -S-($C_1$-$C_6$-Alkyl), -S-($C_2$-$C_6$-Alkenyl), -C(O)O-($C_1$-$C_6$-Alkyl), -C(O)O-($C_2$-$C_6$-Alkenyl), -C(O)$NH_2$, -C(O)NH($C_1$-$C_6$-Alkyl), -C(O)N($C_1$-$C_6$-Alkyl)$_2$, -C(O)NH($C_2$-$C_6$-Alkenyl), -NH($C_1$-$C_6$-Alkyl), -N($C_1$-$C_6$-Alkyl)$_2$, -NH-Phenyl, -NHC(O)-($C_1$-$C_6$-Alkyl), -NHC(O)-($C_2$-$C_6$-Alkenyl), -NHC(O)O-($C_1$-$C_6$-Alkyl) ausgewählt sind, und 5- oder 6-gliedriges nicht-aromatisches Heterocyclyl mit einem oder zwei Heteroatomen, die aus N und 0 ausgewählt sind, und worin das $C_1$-$C_6$-Alkyl, $C_2$-$C_6$-Alkenyl und Phenyl unsubstituiert oder durch einen oder zwei Substituenten substituiert ist, die unabhängig aus einer Gruppe bestehend aus Halogen, -OH, -$NH_2$, -O-($C_1$-$C_6$-Alkyl), -COOH, -C(O)O-($C_1$-$C_6$-Alkyl), -NH-($C_1$-$C_6$-Alkyl), und -N-($C_1$-$C_6$-Alkyl)$_2$ ausgewählt ist,

E steht für 0, NH oder S;
G steht für S, Carbonyl oder eine direkte Bindung;
$R_6$ steht für:

- $C_1$-$C_6$-Alkyl, das unsubstituiert oder durch -O-($C_1$-$C_6$-Alkyl) substituiert ist, oder
- $C_3$-$C_6$-Cycloalkyl, das unsubstituiert oder durch $C_1$-$C_6$-Alkyl substituiert ist,

$R_7$ steht für:

- ein Wasserstoffatom,
- $C_1$-$C_3$-Alkyl, das unsubstituiert oder mit einem Substituenten substituiert ist, der aus der Gruppe bestehend aus Imidazol, Tetrazol und einem 5- oder 6-gliedrigen nicht-aromatischen Heterocyclyl ausgewählt ist, das ein oder zwei Heteroatomen umfasst, die aus N und 0 ausgewählt sind, worin das nicht-aromatische Heterocyclyl auf dem Stickstoffatom unsubstituiert ist oder mit einem ausgewählten Substituenten aus der

Gruppe bestehend aus -($C_1$-$C_6$-Alkyl), -C(O)($C_1$-$C_6$-Alkyl), und -C(O)N($C_1$-$C_6$-Alkyl)$_2$, substituiert ist.
- $C_1$-$C_6$-Alkenyl,
- Phenyl, das unsubstituiert oder durch einen oder zwei Substituenten substituiert ist, die unabhängig voneinander aus der Gruppe bestehend aus Halogen, -OH, - $NH_2$, -$NO_2$, -CN, $C_1$-$C_6$-Alkyl, $C_2$-$C_6$-Alkenyl, -O-($C_1$-$C_6$-Alkyl), -O-($C_2$-$C_6$-Alkenyl), -S-($C_1$-$C_6$-Alkyl), -S-($C_2$-$C_6$-Alkenyl), -$CO_2$H, -C(O)O-($C_1$-$C_6$-Alkyl), -C(O)O-($C_2$-$C_6$-Alkenyl), -C(O)$NH_2$, -C(O)NH($C_1$-$C_6$-Alkyl) ausgewählt sind, und worin das $C_1$-$C_6$-Alkyl unsubstituiert oder weiter durch -OH, -C(O)N($C_1$-$C_6$-Alkyl)$_2$, -C(O)NH($C_2$-$C_6$-Alkenyl), -NH($C_1$-$C_6$-Alkyl), -N($C_1$-$C_6$-Alkyl)$_2$, -NH-Phenyl, -NHC(O)-($C_1$-$C_6$-Alkyl), -NHC(O)-($C_2$-$C_6$-Alkenyl), -NHC(O)O-($C_1$-$C_6$-Alkyl), -$SO_2$N($C_1$-$C_6$-Alkyl), oder -$SO_2$-(5- oder 6-gliedriges Heterocyclyl mit einem oder zwei Heteroatomen, ausgewählt aus N und 0) substituiert ist,
- (3S,4R)-3-Methoxypiperidin-4-yl, oder
- 1-Benzothiophen-3-yl, 2-Oxo-2,3-Dihydro-1H-1,3-Benzodiazol-5-yl, 3-Pyridyl, 4-Pyridyl, 2H-1,3-Benzodioxol-4-yl oder 2-Thiophenyl, die unsubstituiert oder mit $C_1$-$C_6$-Alkyl, -O-($C_1$-$C_6$-Alkyl) oder Halogen substituiert sind;

X steht für N oder CH;
Mit der Vorgabe, dass 4-Acetyl-3-Hydroxy-1-Methylspiro[2,5-Dihydropyrrol-5,3'-indol]-2,2'-dion nicht enthalten ist;
und pharmazeutisch verwendbare Salze, Solvate, Tautomere und Stereoisomere davon.

**2.** Die Verbindung nach Anspruch 1, dargestellt durch die Formel (IA).

**3.** Die Verbindung nach Anspruch 2, worin G eine Carbonylgruppe ist.

**4.** Die Verbindung nach Anspruch 2, worin G -S- ist.

**5.** Die Verbindung nach Anspruch 2, worin G ist eine direkte Bindung ist.

**6.** Die Verbindung nach den Ansprüchen 1 bis 5, worin:

$R_1$ steht für:

- Phenyl oder 3-Pyridyl, die unsubstituiert oder durch einen oder zwei Substituenten substituiert sind, die unabhängig voneinander aus der Gruppe bestehend aus Halogen, -OH, -$NH_2$, -$NO_2$, -CN, $C_1$-$C_6$-Alkyl, $C_2$-$C_6$-Alkenyl, -O-($C_1$-$C_6$-Alkyl), -O-($C_2$-$C_6$-Alkenyl), -S-($C_1$-$C_6$-Alkyl), -S-($C_2$-$C_6$-Alkenyl), -C(O)O-($C_1$-$C_6$-Alkyl), -C(O)O-($C_2$-$C_6$-Alkenyl), -C(O)$NH_2$, -C(O)NH($C_1$-$C_6$-Alkyl), -C(O)N($C_1$-$C_6$-Alkyl)$_2$, -C(O)NH($C_2$-$C_6$-Alkenyl), -NH($C_1$-$C_6$-Alkyl), -N($C_1$-$C_6$-Alkyl)$_2$, -NH-Phenyl, -NHC(O)-($C_1$-$C_6$-Alkyl), -NHC(O)-($C_2$-$C_6$-Alkenyl), -NHC(O)O-($C_1$-$C_6$-Alkyl), und $NHSO_2$-($C_1$-$C_6$-alkyl) ausgewählt sind, und worin das $C_1$-$C_6$-Alkyl, $C_2$-$C_6$-Alkenyl und Phenyl unabhängig voneinander durch einen oder zwei Substituenten aus der Gruppe bestehend aus Halogen, -OH, -SH, -O-($C_1$-$C_6$-Alkyl), -COOH, -C(O)O-($C_1$-$C_6$-Alkyl), und -$SO_2$-($C_1$-$C_6$-Alkyl) ausgewählt ist,
- Pyrazolyl, das unsubstituiert oder mit einem oder zwei Substituenten substituiert ist, die unabhängig voneinander $C_1$-$C_6$-Alkyl sind,
- 6-Oxo-1,6-Dihydropyridin-3-yl, das unsubstituiert oder durch einen oder zwei Substituenten substituiert ist, die unabhängig aus der Gruppe bestehend aus Halogen und $C_1$-$C_6$-Alkyl ausgewählt sind, oder
- 2-Oxo-1,2-Dihydropyridin-3-yl, das unsubstituiert oder durch einen oder zwei Substituenten substituiert ist, die unabhängig aus der Gruppe bestehend aus Halogen und $C_1$-$C_6$-Alkyl ausgewählt sind, oder

$R_4$ steht für H, und
$R_5$ steht für Cl oder F.

**7.** Die Verbindung nach Anspruch 1, dargestellt durch die Formel (IB).

**8.** Die Verbindung nach Anspruch 7, worin X für N steht und die Verbindung durch die Formel (IB-1) dargestellt wird.

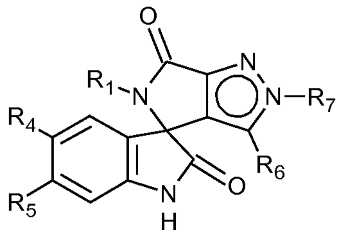

(IB-1).

**9.** Die Verbindung nach Anspruch 7, worin X für CH steht und die Verbindung durch die Formel (IB-2) dargestellt wird.

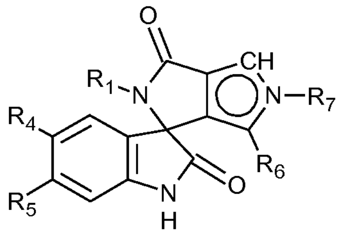

(IB-2).

**10.** Die Verbindung nach den Ansprüchen 7 bis 9, worin
$R_1$ steht für:

- Phenyl oder 3-Pyridyl, die unsubstituiert oder durch einen oder zwei Substituenten substituiert sind, die unabhängig voneinander aus der Gruppe bestehend aus Halogen, -OH, -$NH_2$, -$NO_2$, -CN, $C_1$-$C_6$-Alkyl, $C_2$-$C_6$-Alkenyl, -O-($C_1$-$C_6$-Alkyl), -O-($C_2$-$C_6$-Alkenyl), -S-($C_1$-$C_6$-Alkyl), -S-($C_2$-$C_6$-Alkenyl), -C(O)O-($C_1$-$C_6$-Alkyl), -C(O)O-($C_2$-$C_6$-Alkenyl), -C(O)$NH_2$, -C(O)NH($C_1$-$C_6$-Alkyl), -C(O)N($C_1$-$C_6$-Alkyl)$_2$, -C(O)NH($C_2$-$C_6$-Alkenyl), -NH($C_1$-$C_6$-Alkyl), -N($C_1$-$C_6$-Alkyl)$_2$, -NH-Phenyl, -NHC(O)-($C_1$-$C_6$-Alkyl), -NHC(O)-($C_2$-$C_6$-Alkenyl), -NHC(O)O-($C_1$-$C_6$-Alkyl), und $NHSO_2$-($C_1$-$C_6$-alkyl) ausgewählt sind, und worin das $C_1$-$C_6$-Alkyl, $C_2$-$C_6$-Alkenyl und Phenyl unabhängig voneinander durch einen oder zwei Substituenten substituiert sind, die aus der Gruppe bestehend aus Halogen -OH, -SH, -O-($C_1$-$C_6$-Alkyl), -COOH, -C(O)O-($C_1$-$C_6$-Alkyl), und -$SO_2$-($C_1$-$C_6$-Alkyl) ausgewählt sind,
- Pyrazolyl, das unsubstituiert oder mit einem oder zwei Substituenten substituiert ist, die unabhängig voneinander $C_1$-$C_6$-Alkyl sind,
- 6-Oxo-1,6-Dihydropyridin-3-yl, das unsubstituiert oder durch einen oder zwei Substituenten substituiert ist, die unabhängig aus der Gruppe bestehend aus Halogen und $C_1$-$C_6$-Alkyl ausgewählt sind, oder
- 2-Oxo-1,2-Dihydropyridin-3-yl, das unsubstituiert oder durch einen oder zwei Substituenten substituiert ist, die unabhängig voneinander aus der Gruppe bestehend aus Halogen und $C_1$-$C_6$-Alkyl ausgewählt sind.

**11.** Die Verbindung nach Anspruch 10, wobei:

$R_1$ stehnt für:

- Phenyl, das mit Halogen an der Meta-Position, relativ zum Bindungsort, an das Stickstoffatom des Pyrrolonrings substituiert ist, oder 3-Pyridyl, das mit Halogen an der Position 5 relativ zum Bindungsort an das Stickstoffatom des Pyrrolonrings substituiert ist, und wobei das Phenyl und 3-Pyridyl optional weiter mit einem oder zwei Substituenten substituiert ist, die unabhängig voneinander aus der Gruppe bestehend aus Halogen, -OH, -$NH_2$, -$NO_2$, -CN, $C_1$-$C_6$-Alkyl, $C_2$-$C_6$-Alkenyl, -O-($C_1$-$C_6$-Alkyl), -O-($C_2$-$C_6$-Alkenyl), -S-($C_1$-$C_6$-Alkyl), -S-($C_2$-$C_6$-Alkenyl), -C(O)O-($C_1$-$C_6$-Alkyl), -C(O)O-($C_2$-$C_6$-Alkenyl), -C(O)$NH_2$, -C(O)NH($C_1$-$C_6$-Alkyl), -C(O)N($C_1$-$C_6$-Alkyl)$_2$, -C(O)NH($C_2$-$C_6$-Alkenyl), -NH($C_1$-$C_6$-Alkyl), -N($C_1$-$C_6$-Alkyl)$_2$, -NH-Phenyl, -NHC(O)-($C_1$-$C_6$-Alkyl), -NHC(O)-($C_2$-$C_6$-Alkenyl), -NHC(O)O-($C_1$-$C_6$-Alkyl), und $NHSO_2$-($C_1$-$C_6$-Alkyl) ausgewählt sind, und worin das $C_1$-$C_6$-Alkyl, $C_2$-$C_6$-Alkenyl und Phenyl unabhängig voneinander durch einen oder zwei Substituenten substituiert ist, die aus der Gruppe bestehend aus Halogen, -OH, -SH, -O-($C_1$-$C_6$-Alkyl), -COOH, -C(O)O-($C_1$-$C_6$-Alkyl), und -$SO_2$-($C_1$-$C_6$-Alkyl) ausgewählt sind.

**12.** Die Verbindung nach den Ansprüchen 7 bis 11, wobei $R_4$ für H und $R_5$ für Cl oder F steht.

**13.** Die Verbindung nach Anspruch 12, wobei:

$R_1$ steht für:

- Meta-Chlorphenyl oder 5-Chlor-3-Pyridyl, die optional unabhängig voneinander durch einen oder zwei Substituenten substituiert sind, die aus der Gruppe bestehend aus Halogen, -OH, $-NH_2$, $-NO_2$, -CN, $C_1$-$C_6$-Alkyl, $C_2$-$C_6$-Alkenyl, -O-($C_1$-$C_6$-Alkyl), -O-($C_2$-$C_6$-Alkenyl), -S-($C_1$-$C_6$-Alkyl), -S-($C_2$-$C_6$-Alkenyl), -C(O)O-($C_1$-$C_6$-Alkyl), -C(O)O-($C_2$-$C_6$-Alkenyl), $-C(O)NH_2$, -C(O)NH($C_1$-$C_6$-Alkyl), -C(O)N($C_1$-$C_6$-Alkyl)$_2$, -C(O)NH($C_2$-$C_6$-Alkenyl), -NH($C_1$-$C_6$-Alkyl), -N($C_1$-$C_6$-Alkyl)$_2$, -NH-Phenyl, -NHC(O)-($C_1$-$C_6$-Alkyl), -NHC(O)-($C_2$-$C_6$-Alkenyl), -NHC(O)O-($C_1$-$C_6$-Alkyl), und $NHSO_2$-($C_1$-$C_6$-Alkyl) ausgewählt sind, und wobei das $C_1$-$C_6$-Alkyl, $C_2$-$C_6$-Alkenyl und Phenyl unabhängig voneinander durch einen oder zwei Substituenten substituiert sind, die aus der Gruppe bestehend aus Halogen, -OH, -SH, -O-($C_1$-$C_6$-Alkyl), -COOH, -C(O)O-($C_1$-$C_6$-Alkyl), und $-SO_2$-($C_1$-$C_6$-Alkyl) ausgewählt sind.

**14.** Die Verbindung nach den Ansprüchen 7 bis 13, wobei die absolute Konfiguration am Spiro-Kohlenstoffatom S ist (Konfiguration 3S).

**15.** Die Verbindung nach Anspruch 1, ausgewählt aus der folgenden Gruppe:

6-Chlor-1'-(5-chlor-2-fluorphenyl)-4'-hydroxy-3'-(1-methylcyclopropancarbonyl)-1,1',2,5'-tetrahydrospiro[indol-3,2'-pyrrol]-2,5'-dion,
6-Chlor-1'-(5-chlor-2-methylphenyl)-4'-hydroxy-3'-(1-methylcyclopropancarbonyl)-1,1',2,5'-tetrahydrospiro[indol-3,2'-pyrrol]-2,5'-dion,
3'-Benzoyl-6-chlor-1'-(3-chlorphenyl)-4'-hydroxy-1,1',2,5'-tetrahydrospiro[indol-3,2'-pyrrol]-2,5'-dion,
3'-Benzoyl-6-chlor-1'-(1,5-dimethyl-1H-pyrazol-3-yl)-4'-hydroxy-1,1',2,5'-tetrahydrospiro[indol-3,2'-pyrrol]-2,5'-dion,
6-Chlor-1'-(3-chlorphenyl)-4'-hydroxy-3'-(1-methylcyclopropancarbonyl)-1,1',2,5'-tetrahydrospiro[indol-3,2'-pyrrol]-2,5'-dion,
6-Chlor-1'-(3-chlorphenyl)-3'-(2,2-tert-butanoyl)-4'-hydroxy-1,1',2,5'-tetrahydrospiro[indol-3,2'-pyrrol]-2,5'-dion,
6-Chlor-1'-(5-chlor-2-methoxyphenyl)-4'-hydroxy-3'-(1-methylcyclopropancarbonyl)-1,1',2,5'-tetrahydrospiro[indol-3,2'-pyrrol]-2,5'-dion,
6-Chlor-1'-(5-chlor-2-methylphenyl)-4'-hydroxy-3'-(iso-propanoyl)-1,1',2,5'-tetrahydrospiro[indol-3,2'-pyrrol]-2,5'-dion,
6-Chlor-1'-(5-chlor-2-hydroxyphenyl)-4'-hydroxy-3'-(iso-propanoyl)-1,1',2,5'-tetrahydrospiro[indol-3,2'-pyrrol]-2,5'-dion,
6-Chlor-1'-(5-chlor-2-methoxyphenyl)-4'-hydroxy-3'-(iso-propanoyl)-1,1',2,5'-tetrahydrospiro[indol-3,2'-pyrrol]-2,5'-dion,
6-Chlor-1'-(5-chlor-2-fluorphenyl)-4'-hydroxy-3'-(iso-propanoyl)-1,1',2,5'-tetrahydrospiro[indol-3,2'-pyrrol]-2,5'-dion,
6-Chlor-1'-(5-chlor-1-methyl-6-oxo-1,6-dihydropyridin-3-yl)-4'-hydroxy-3'-(iso-propanoyl)-1,1',2,5'-tetrahydrospiro[indol-3,2'-pyrrol]-2,5'-dion,
6-Chlor-1'-(3-chlorphenyl)-3'-[2,2-dimethyl-3-(propan-2-yloxy)propanoyl]-4'-hydroxy-1,1',2,5'-tetrahydrospiro[indol-3,2'-pyrrol]-2,5'-dion,
2-{[6-Chlor-1'-(5-chlor-2-methylphenyl)-3'-(1-methylcyclopropancarbonyl)-2,5'-dioxo-1,1',2,5'-tetrahydrospiro[indol-3,2'-pyrrol]-4'-yl]amino}acetamid,
Ethyl 2-{[6-Chlor-1'-(3-chlorphenyl)-3'-(1-methylcyclopropancarbonyl)-2,5'-dioxo-1,1',2,5'-tetrahydrospiro[indol-3,2'-pyrrol]-4'-yl]amino}acetat,
2-{[6-Chlor-1'-(3-chlorphenyl)-3'-(1-methylcyclopropancarbonyl)-2,5'-dioxo-1,1',2,5'-tetrahydrospiro[indol-3,2'-pyrrol]-4'-yl]amino}acetamid,
Ethyl (2E)-3-[(3-{[6-Chlor-1'-(3-chlorphenyl)-4'-hydroxy-2,5'-dioxo-1,1',2,5'-tetrahydrospiro[indol-3,2'-pyrrol]-3'-yl]carbonyl}phenyl)carbamoyl]prop-2-enoat,
6-Chlor-5'-(5-chlor-2-methylphenyl)-3'-(1-methylcyclopropyl)-1,2,5',6'-tetrahydro-2'H-spiro[indol-3,4'-pyrrolo[3,4-c]pyrazol]-2,6'-dion,
6-Chlor-5'-(3-chlorphenyl)-3'-(1-methylcyclopropyl)-1,2,5',6'-tetrahydro-2'H-spiro[indol-3,4'-pyrrolo[3,4-c]pyrazol]-2,6'-dion,

3'-tert-Butyl-6-chlor-5'-(3-chlorphenyl)-1,2,5',6'-tetrahydro-2'H-spiro[indol-3,4'-pyrrolo[3,4-c]pyrazol]-2,6'-dion,
3'-tert-Butyl-6-chlor-5'-(5-chlor-2-methoxyphenyl)-1,2,5',6'-tetrahydro-2'H-spiro[indol-3,4'-pyrrolo[3,4-c]pyrazol]-2,6'-dion,
6-Chlor-5'-(5-chlor-2-methoxyphenyl)-3'-(1-methylcyclopropyl)-1,2,5',6'-tetrahydro-2'H-spiro[indol-3,4'-pyrrolo[3,4-c]pyrazol]-2,6'-dion,
6-Chlor-5'-(5-chlor-2-methylphenyl)-3'-(propan-2-yl)-1,2,5',6'-tetrahydro-2'H-spiro[indol-3,4'-pyrrolo[3,4-c]pyrazol]-2,6'-dion,
6-Chlor-5'-(5-chlor-2-hydroxyphenyl)-3'-(propan-2-yl)-1,2,5',6'-tetrahydro-2'H-spiro[indol-3,4'-pyrrolo[3,4-c]pyrazol]-2,6'-dion,
6-Chlor-5'-(5-chlor-2-methoxyphenyl)-3'-(propan-2-yl)-1,2,5',6'-tetrahydro-2'H-spiro[indol-3,4'-pyrrolo[3,4-c]pyrazol]-2,6'-dion,
6-Chlor-5'-(3-chlorphenyl)-2'-(2-methoxyphenyl)-3'-(1-methylcyclopropyl)-1,2,5',6'-tetrahydro-2'H-spiro[indol-3,4'-pyrrolo[3,4-c]pyrazol]-2,6'-dion,
6-Chlor-5'-(3-chlorphenyl)-2'-(2-methoxyphenyl)-3'-(propan-2-yl)-1,2,5',6'-tetrahydro-2'H-spiro[indol-3,4'-pyrrolo[3,4-c]pyrazol]-2,6'-dion,
6-Chlor-5'-(3-chlorphenyl)-3'-(1-methylcyclopropyl)-2'-(pyrrolidin-2-ylmethyl)-1,2,5',6'-tetrahydro-2'H-spiro[indol-3,4'-pyrrolo[3,4-c]pyrazol]-2,6'-dion,
2-[6-Chlor-5'-(3-chlorphenyl)-3'-(1-methylcyclopropyl)-2,6'-dioxo-1,2,5',6'-tetrahydro-2'H-spiro[indol-3,4'-pyrrolo[3,4-c]pyrazol]-2'-ylmethyl]-N,N-dimethylcyclopentan-1-carboxamid,
6-Chlor-5'-(5-chlor-2-methoxyphenyl)-2'-(2-methoxyphenyl)-3'-(propan-2-yl)-1,2,5',6'-tetrahydro-2'H-spiro[indol-3,4'-pyrrolo[3,4-c]pyrazol]-2,6'-dion,
6-Chlor-5'-(5-chlor-2-methylphenyl)-2'-(2-methoxyphenyl)-3'-(propan-2-yl)-1,2,5',6'-tetrahydro-2'H-spiro[indol-3,4'-pyrrolo[3,4-c]pyrazol]-2,6'-dion,
6-Chlor-5'-(5-chlor-2-methylphenyl)-1'-(2-methoxyphenyl)-3'-(propan-2-yl)-1,2,5',6'-tetrahydro-1'H-spiro[indol-3,4'-pyrrolo[3,4-c]pyrazol]-2,6'-dion,
2-[6-Chlor-5'-(3-chlorphenyl)-2,6'-dioxo-3'-(propan-2-yl)-1,2,5',6'-tetrahydro-2'H-spiro[indol-3,4'-pyrrolo[3,4-c]pyrazol]-2'-ylmethyl]-N,N-dimethylpyrrolidin-1-carboxamid,
6-Chlor-1'-(2,2-dimethylpropyl)-4'-hydroxy-3'-phenyl-1,1',2,5'-tetrahydrospiro[indol-3,2'-pyrrol]-2,5'-dion,
6-Chlor-3'-(3-chlorphenyl)-1'-(2,2-dimethylpropyl)-4'-hydroxy-1,1',2,5'-tetrahydrospiro[indol-3,2'-pyrrol]-2,5'-dion,
6-Chlor-3'-(3-chlorphenyl)-4'-hydroxy-1'-phenyl-1,1',2,5'-tetrahydrospiro[indol-3,2'-pyrrol]-2,5'-dion,
5-Chlor-1'-(3-chlorphenyl)-4'-hydroxy-3'-phenyl-1,1',2,5'-tetrahydrospiro[indol-3,2'-pyrrol]-2,5'-dion,
1'-(3-Chlorphenyl)-4'-hydroxy-3'-phenyl-1,1',2,5'-tetrahydrospiro[indol-3,2'-pyrrol]-2,5'-dion,
6-Chlor-1',3'-bis(3-chlorphenyl)-4'-hydroxy-1,1',2,5'-tetrahydrospiro[indol-3,2'-pyrrol]-2,5'-dion,
6-Chlor-1'-(3-chlorphenyl)-4'-hydroxy-3'-phenyl-1,1',2,5'-tetrahydrospiro[indol-3,2'-pyrrol]-2,5'-dion,
1'-(3-Chlorphenyl)-6-fluor-4'-hydroxy-3'-phenyl-1,1',2,5'-tetrahydrospiro[indol-3,2'-pyrrol]-2,5'-dion,
5,6-Dichlor-1'-(3-chlorphenyl)-4'-hydroxy-3'-phenyl-1,1',2,5'-tetrahydrospiro[indol-3,2'-pyrrol]-2,5'-dion,
6-Chlor-1'-(3-chlorphenyl)-3'-cyclohexyl-4'-hydroxy-1,1',2,5'-tetrahydrospiro[indol-3,2'-pyrrol]-2,5'-dion,
6-Chlor-1'-(5-chlor-2-fluorphenyl)-4'-hydroxy-3'-phenyl-1,1',2,5'-tetrahydrospiro[indol-3,2'-pyrrol]-2,5'-dion,
6-Chlor-1'-(5-chlor-2-methoxyphenyl)-4'-hydroxy-3'-phenyl-1,1',2,5'-tetrahydrospiro[indol-3,2'-pyrrol]-2,5'-dion,
6-Chlor-1'-(5-chlor-2-methylphenyl)-4'-hydroxy-3'-phenyl-1,1',2,5'-tetrahydrospiro[indol-3,2'-pyrrol]-2,5'-dion,
Ethyl 2-{[6-Chlor-1'-(3-chlorphenyl)-2,5'-dioxo-3'-phenyl-1,1',2,5'-tetrahydrospiro[indol-3,2'-pyrrol]-4'-yl]amino}acetat,
2-{[6-Chlor-1'-(3-chlorphenyl)-2,5'-dioxo-3'-phenyl-1,1',2,5'-tetrahydrospiro[indol-3,2'-pyrrol]-4'-yl]amino}essigsäure,
Methyl 2-[6-Chlor-1'-(3-chlorphenyl)-2,5'-dioxo-3'-phenyl-1,1',2,5'-tetrahydrospiro[indol-3,2'-pyrrol]-4'-ylsulfanyl]acetat,
2-[6-Chlor-1'-(3-chlorphenyl)-2,5'-dioxo-3'-phenyl-1,1',2,5'-Tetrahydrospiro[indol-3,2'-pyrrol]-4'-ylsulfanyl] Essigsäure,
6-Chlor-1'-(3-chlorphenyl)-2,5'-dioxo-3'-phenyl-1,1',2,5'-tetrahydrospiro[indol-3,2'-pyrrole]-4'-yl methansulfonat,
2-[6-Chlor-1'-(3-chlorphenyl)-2,5'-dioxo-3'-phenyl-1,1',2,5'-tetrahydrospiro[indol-3,2'-pyrrol]-4'-ylsulfanyl]acetamid,
(3S)-6-Chlor-1'-(3-chlorphenyl)-3'-[(4-chlorphenyl)sulfanyl]-4'-hydroxy-1,1',2,5'-tetrahydrospiro[indol-3,2'-pyrrol]-2,5'-dion,
6-Chlor-1'-(3-chlorphenyl)-3'-[(4-chlorphenyl)sulfanyl]-4'-hydroxy-1,1',2,5'-tetrahydrospiro[indol-3,2'-pyrrol]-2,5'-dion,

3’-(Butan-2-ylsulfanyl)-6-chlor-1’-(3-chlorphenyl)-4’-hydroxy-1,1’,2,5’-tetrahydrospiro[indol-3,2’-pyrrol]-2,5’-dion,
3’-(tert-Butylsulfanyl)-6-chlor-1’-(3-chlorphenyl)-4’-hydroxy-1,1’,2,5’-tetrahydrospiro[indol-3,2’-pyrrol]-2,5’-dion,
6-Chlor-1’-(3-chlorphenyl)-4’-hydroxy-3’-{[(4-methoxyphenyl)methyl]sulfanyl}-1,1’,2,5’-tetrahydrospiro[indol-3,2’-pyrrol]-2,5’-dion,
6-Chlor-1’-(3-chlorphenyl)-4’-hydroxy-3’-{[5-(morpholin-4-yl)-1,3,4-thiadiazol-2-yl]sulfanyl}-1,1’,2,5’-tetrahydrospiro[indol-3,2’-pyrrol]-2,5’-dion,
6-Chlor-3’-[(5-chlor-1,3-dimethyl-1H-pyrazol-4-yl)sulfanyl]-1’-(3-chlorphenyl)-4’-hydroxy-1,1’,2,5’-tetrahydrospiro[indol-3,2’-pyrrol]-2,5’-dion,
Methyl (2E)-4-{4-chlor-2-[6-chlor-2’-(2-methoxyphenyl)-2,6’-dioxo-3’-(propan-2-yl)-1,2,5’,6’-tetrahydro-2’H-spiro[indol-3,4’-pyrrolo[3,4-c]pyrazol]-5’-yl]phenoxy}but-2-enoat,
6-Chlor-5’-(5-chlor-1-methyl-6-oxo-1,6-dihydropyridin-3-yl)-2’-(2-methoxyphenyl)-3’-(propan-2-yl)-1,2,5’,6’-tetrahydro-2’H-spiro[indol-3,4’-pyrrolo[3,4-c]pyrazol]-2,6’-dion,
6-Chlor-5’-(5-chlor-2-methylphenyl)-2’-(3-methoxypyridin-4-yl)-3’-(propan-2-yl)-1,2,5’,6’-tetrahydro-2’H-spiro[indol-3,4’-pyrrolo[3,4-c]pyrazol]-2,6’-dion,
(3S)-6-Chlor-5’-(5-chlor-2-methylphenyl)-2’-(2-methoxyphenyl)-3’-(propan-2-yl)-1,2,5’,6’-tetrahydro-2’H-spiro[indol-3,4’-pyrrolo[3,4-c]pyrazol]-2,6’-dion,
6-Chlor-5’-(5-chlor-2-methylphenyl)-2’-(3-ethylphenyl)-3’-(propan-2-yl)-1,2,5’,6’-tetrahydro-2’H-spiro[indol-3,4’-pyrrolo[3,4-c]pyrazol]-2,6’-dion,
6-Chlor-5’-(5-chlor-2-methylphenyl)-2’-(2-ethylphenyl)-3’-(propan-2-yl)-1,2,5’,6’-tetrahydro-2’H-spiro[indol-3,4’-pyrrolo[3,4-c]pyrazol]-2,6’-dion,
6-Chlor-5’-(5-chlor-2-methylphenyl)-2’-(2-methylphenyl)-3’-(propan-2-yl)-1,2,5’,6’-tetrahydro-2’H-spiro[indol-3,4’-pyrrolo[3,4-c]pyrazol]-2,6’-dion,
6-Chlor-5’-(5-chlor-2-methylphenyl)-2’-(3-methylphenyl)-3’-(propan-2-yl)-1,2,5’,6’-tetrahydro-2’H-spiro[indol-3,4’-pyrrolo[3,4-c]pyrazol]-2,6’-dion,
6-Chlor-5’-(5-chlor-2-methylphenyl)-2’-(2,6-dimethoxyphenyl)-3’-(propan-2-yl)-1,2,5’,6’-tetrahydro-2’H-spiro[indol-3,4’-pyrrolo[3,4-c]pyrazol]-2,6’-dion,
6-Chlor-5’-(5-chlor-2-methylpyridin-3-yl)-2’-(2-methoxyphenyl)-3’-(propan-2-yl)-1,2,5’,6’-tetrahydro-2’H-spiro[indol-3,4’-pyrrolo[3,4-c]pyrazol]-2,6’-dion,
6-Chlor-5’-(5-chlorpyridin-3-yl)-2’-(2-methoxyphenyl)-3’-(propan-2-yl)-1,2,5’,6’-tetrahydro-2’H-spiro[indol-3,4’-pyrrolo[3,4-c]pyrazol]-2,6’-dion,
6-Chlor-5’-[5-chlor-2-(2-methansulfonylethoxy)phenyl]-2’-(2-methoxyphenyl)-3’-(propan-2-yl)-1,2,5’,6’-tetrahydro-2’H-spiro[indol-3,4’-pyrrolo[3,4-c]pyrazol]-2,6’-dion,
2’-(1-Benzothiophen-3-yl)-6-chlor-5’-(5-chlor-2-methylphenyl)-3’-(propan-2-yl)-1,2,5’,6’-tetrahydro-2’H-spiro[indol-3,4’-pyrrolo[3,4-c]pyrazol]-2,6’-dion,
3-[6-Chlor-5’-(5-chlor-2-hydroxyphenyl)-2,6’-dioxo-3’-(propan-2-yl)-1,2,5’,6’-tetrahydro-2’H-spiro[indol-3,4’-pyrrolo[3,4-c]pyrazol]-2’-yl]-4-methoxybenzamid,
3-[6-Chlor-5’-(5-chlor-2-hydroxyphenyl)-2,6’-dioxo-3’-(propan-2-yl)-1,2,5’,6’-tetrahydro-2’H-spiro[indol-3,4’-pyrrolo[3,4-c]pyrazol]-2’-yl]-4-methoxybenzoesäure,
6-Chlor-5’-(5-chlor-2-methylphenyl)-2’-[2-(methylsulfanyl)phenyl]-3’-(propan-2-yl)-1,2,5’,6’-tetrahydro-2’H-spiro[indol-3,4’-pyrrolo[3,4-c]pyrazol]-2,6’-dion,
Methyl 3-[6-Chlor-5’-(5-chlor-2-methylphenyl)-2,6’-dioxo-3’-(propan-2-yl)-1,2,5’,6’-tetrahydro-2’H-spiro[indol-3,4’-pyrrolo[3,4-c]pyrazol]-2’-yl]-4-methoxybenzoat,
Methyl 3-[6-Chlor-5’-(5-chlor-2-hydroxyphenyl)-2,6’-dioxo-3’-(propan-2-yl)-1,2,5’,6’-tetrahydro-2’H-spiro[indol-3,4’-pyrrolo[3,4-c]pyrazol]-2’-yl]-4-methoxybenzoat,
(3S)-6-Chlor-5’-(5-chlor-2-methylpyridin-3-yl)-2’-(2-methoxyphenyl)-3’-(propan-2-yl)-1,2,5’,6’-tetrahydro-2’H-spiro[indol-3,4’-pyrrolo[3,4-c]pyrazol]-2,6’-dion,
(3S)-6-Chlor-5’-(5-chlorpyridin-3-yl)-2’-(2-methoxyphenyl)-3’-(propan-2-yl)-1,2,5’,6’-tetrahydro-2’H-spiro[indol-3,4’-pyrrolo[3,4-c]pyrazol]-2,6’-dion,
6-Chlor-5’-(4-chlorpyridin-2-yl)-3’-(propan-2-yl)-1,2,5’,6’-tetrahydro-2’H-spiro[indol-3,4’-pyrrolo[3,4-c]pyrazol]-2,6’-dion,
6-Chlor-5’-(5-chlorpyridin-3-yl)-3’-(propan-2-yl)-1,2,5’,6’-tetrahydro-2’H-spiro[indol-3,4’-pyrrolo[3,4-c]pyrazol]-2,6’-dion,
6-Chlor-5’-(5-chlor-2-methylpyridin-3-yl)-3’-(propan-2-yl)-1,2,5’,6’-tetrahydro-2’H-spiro[indol-3,4’-pyrrolo[3,4-c]pyrazol]-2,6’-dion,
6-Chlor-5’-(5-chlor-2-methylphenyl)-3’-ethyl-2’-(2-ethylphenyl)-1,2,5’,6’-tetrahydro-2’H-spiro[indol-3,4’-pyrrolo[3,4-c]pyrazol]-2,6’-dion,

6-Chlor-5’-(cyclobutylmethyl)-2’-(2-methoxyphenyl)-3’-(propan-2-yl)-1,2,5’,6’-tetrahydro-2’H-spiro[indol-3,4’-pyrrolo[3,4-c]pyrazol]-2,6’-dion,
6-Chlor-5’-(2,2-dimethylpropyl)-2’-(2-methoxyphenyl)-3’-(propan-2-yl)-1,2,5’,6’-tetrahydro-2’H-spiro[indol-3,4’-pyrrolo[3,4-c]pyrazol]-2,6’-dion,
(3S)-6-Chlor-5’-(5-chlorpyridin-3-yl)-2’-(2-methoxyphenyl)-3’-(propan-2-yl)-1,2,5’,6’-tetrahydro-2’H-spiro[indol-3,4’-pyrrolo[3,4-c]pyrazol]-2,6’-dion,
6-Chlor-2’-(3-chlorphenyl)-5’-(2-methoxyphenyl)-6’-(propan-2-yl)-1,2,3’,5’-tetrahydro-2’H-spiro[indol-3,1’-pyrrolo[3,4-c]pyrrol]-2,3’-dion,
6-Chlor-2’-(5-chlor-2-nitrophenyl)-5’-(2-methoxyphenyl)-6’-(propan-2-yl)-1,2,3’,5’-tetrahydro-2’H-spiro[indol-3,1’-pyrrolo[3,4-c]pyrrol]-2,3’-dion,
2’-(2-Amino-5-chlorphenyl)-6-chlor-5’-(2-methoxyphenyl)-6’-(propan-2-yl)-1,2,3’,5’-tetrahydro-2’H-spiro[indol-3,1’-pyrrolo[3,4-c]pyrrol]-2,3’-dion,
N-{4-Chlor-2-[6-chlor-5’-(2-methoxyphenyl)-2,3’-dioxo-6’-(propan-2-yl)-1,2,3’,5’-tetrahydro-2’H-spiro[indol-3,1’-pyrrolo[3,4-c]pyrrol]-2’-yl]phenyl}methansulfonamid,
6-Chlor-2’-(5-chlor-2-methylphenyl)-5’-(2,4-dimethoxyphenyl)-6’-(propan-2-yl)-1,2,3’,5’-tetrahydro-2’H-spiro[indol-3,1’-pyrrolo[3,4-c]pyrrol]-2,3’-dion,
5’-(2H-1,3-Benzodioxol-4-yl)-6-chlor-2’-(5-chlor-2-methylphenyl)-6’-(propan-2-yl)-1,2,3’,5’-tetrahydro-2’H-spiro[indol-3,1’-pyrrolo[3,4-c]pyrrol]-2,3’-dion,
3-[6-Chlor-2’-(5-chlor-2-methylphenyl)-2,3’-dioxo-6’-(propan-2-yl)-1,2,3’,5’-tetrahydro-2’H-spiro[indol-3,1’-pyrrolo[3,4-c]pyrrol]-5’-yl]-4-methoxybenzamid,
5’-(5-Amino-2-methoxyphenyl)-6-chlor-2’-(5-chlor-2-methylphenyl)-6’-(propan-2-yl)-1,2,3’,5’-tetrahydro-2’H-spiro[indol-3,1’-pyrrolo[3,4-c]pyrrol]-2,3’-dion,
N-{3-[6-Chlor-2’-(5-chlor-2-methylphenyl)-2,3’-dioxo-6’-(propan-2-yl)-1,2,3’,5’-tetrahydro-2’H-spiro[indol-3,1’-pyrrolo[3,4-c]pyrrol]-5’-yl]-4-methoxyphenyl}acetamid,
3-[6-Chlor-2’-(5-chlor-2-methylphenyl)-2,3’-dioxo-6’-(propan-2-yl)-1,2,3’,5’-tetrahydro-2’H-spiro[indol-3,1’-pyrrolo[3,4-c]pyrrol]-5’-yl]-N,N-diethyl-4-methoxybenzol-1-sulfonamid,
4-Chlor-2-[6-chlor-5’-(2-methoxyphenyl)-2,3’-dioxo-6’-(propan-2-yl)-1,2,3’,5’-tetrahydro-2’H-spiro[indol-3,1’-pyrrolo[3,4-c]pyrrol]-2’-yl]benzamid,
3-[6-Chlor-2’-(5-chlor-2-methylphenyl)-2,3’-dioxo-6’-(propan-2-yl)-1,2,3’,5’-tetrahydro-2’H-spiro[indol-3,1’-pyrrolo[3,4-c]pyrrol]-5’-yl]-N-(2-hydroxyethyl)-4-methoxybenzamid,
6-Chlor-2’-(5-chlor-2-methylphenyl)-5’-(6-methoxy-2-oxo-2,3-dihydro-1H-1,3-benzodiazol-5-yl)-6’-(propan-2-yl)-1,2,3’,5’-tetrahydro-2’H-spiro[indol-3,1’-pyrrolo[3,4-c]pyrrol]-2,3’-dion,
N-{4-Chlor-2-[(3S)-6-chlor-5’-(2-methoxyphenyl)-2,3’-dioxo-6’-(propan-2-yl)-1,2,3’,5’-tetrahydro-2’H-spiro[indol-3,1’-pyrrolo[3,4-c]pyrrol]-2’-yl]phenyl}methansulphonamid,
(3S)-6-Chlor-2’-(5-chlor-2-methylphenyl)-5’-[(3S,4R)-3-methoxypiperidin-4-yl]-6’-(propan-2-yl)-1,2,3’,5’-tetrahydro-2’H-spiro[indol-3,1’-pyrrolo[3,4-c]pyrrol]-2,3’-dion,
6-Chlor-2’-(5-chlor-2-methylpyridin-3-yl)-5’-(2,4-dimethoxyphenyl)-6’-(propan-2-yl)-1,2,3’,5’-tetrahydro-2’H-spiro[indol-3,1’-pyrrolo[3,4-c]pyrrol]-2,3’-dion,
4-Chlor-2-[6-chlor-5’-(2-methoxyphenyl)-2,3’-dioxo-6’-(propan-2-yl)-1,2,3’,5’-tetrahydro-2’H-spiro[indol-3,1’-pyrrolo[3,4-c]pyrrol]-2’-yl]benzoesäure,
Ethyl 4-Chlor-2-[6-chlor-5’-(2-methoxyphenyl)-2,3’-dioxo-6’-(propan-2-yl)-1,2,3’,5’-tetrahydro-2’H-spiro[indol-3,1’-pyrrolo[3,4-c]pyrrol]-2’-yl]benzoat,
(3S)-6-Chlor-2’-(5-chlor-2-methylphenyl)-5’-(2,4-dimethoxyphenyl)-6’-(propan-2-yl)-1,2,3’,5’-tetrahydro-2’H-spiro[indol-3,1’-pyrrolo[3,4-c]pyrrol]-2,3’-dion,
N-{3-[(3S)-6-Chlor-2’-(5-chlor-2-methylphenyl)-2,3’-dioxo-6’-(propan-2-yl)-1,2,3’,5’-tetrahydro-2’H-spiro[indol-3,1’-pyrrolo[3,4-c]pyrrol]-5’-yl]-4-methoxyphenyl}acetamid,
(3S)-5’-(5-Amino-2-methoxyphenyl)-6-chlor-2’-(5-chlor-2-methylphenyl)-6’-(propan-2-yl)-1,2,3’,5’-tetrahydro-2’H-spiro[indol-3,1’-pyrrolo[3,4-c]pyrrol]-2,3’-dion,
3-[(3S)-6-Chlor-2’-(5-chlor-2-methylphenyl)-2,3’-dioxo-6’-(propan-2-yl)-1,2,3’,5’-tetrahydro-2’H-spiro[indol-3,1’-pyrrolo[3,4-c]pyrrol]-5’-yl]-4-methoxybenzamid,
(3S)-6-Chlor-2’-(5-chlor-2-methylpyridin-3-yl)-5’-(2,4-dimethoxyphenyl)-6’-(propan-2-yl)-1,2,3’,5’-tetrahydro-2’H-spiro[indol-3,1’-pyrrolo[3,4-c]pyrrol]-2,3’-dion,
6-Chlor-2’-(5-chlor-2-methylphenyl)-5’-[2-methoxy-5-(morpholin-4-sulfonyl)phenyl]-6’-(propan-2-yl)-1,2,3’,5’-tetrahydro-2’H-spiro[indol-3,1’-pyrrolo[3,4-c]pyrrol]-2,3’-dion,
6-Chlor-2’-(5-chlor-2-methylphenyl)-5’-(2-methoxythiophen-3-yl)-6’-(propan-2-yl)-1,2,3’,5’-tetrahydro-2’H-spiro[indol-3,1’-pyrrolo[3,4-c]pyrrol]-2,3’-dion, und
6-Chlor-5’-(5-chlor-2-methylphenyl)-2’-(2,6-dimethoxypyridin-3-yl)-3’-(propan-2-yl)-1,2,5’,6’-tetrahydro-2’H-spiro[indol-3,4’-pyrrolo[3,4-c]pyrazol]-2,6’-dion.

**16.** Die Verbindung nach Anspruch 15, ausgewählt aus der folgenden Gruppe:

(3S)-6-Chlor-2'-(5-chlor-2-methylphenyl)-5'-(2,4-dimethoxyphenyl)-6'-(propan-2-yl)-1,2,3',5'-tetrahydro-2'H-spiro[indol-3,1'-pyrrolo[3,4-c]pyrrol]-2,3'-dion,
N-{3-[(3S)-6-Chlor-2'-(5-chlor-2-methylphenyl)-2,3'-dioxo-6'-(propan-2-yl)-1,2,3',5'-tetrahydro-2'H-spiro[indol-3,1'-pyrrolo[3,4-c]pyrrol]-5'-yl]-4-methoxyphenyl}acetamid,
(3S)-5'-(5-Amino-2-methoxyphenyl)-6-chlor-2'-(5-chlor-2-methylphenyl)-6'-(propan-2-yl)-1,2,3',5'-tetrahydro-2'H-spiro[indol-3,1'-pyrrolo[3,4-c]pyrrol]-2,3'-dion,
3-[(3S)-6-Chlor-2'-(5-chlor-2-methylphenyl)-2,3'-dioxo-6'-(propan-2-yl)-1,2,3',5'-tetrahydro-2'H-spiro[indol-3,1'-pyrrolo[3,4-c]pyrrol]-5'-yl]-4-methoxybenzamid,
(3S)-6-Chlor-2'-(5-chlor-2-methylpyridin-3-yl)-5'-(2,4-dimethoxyphenyl)-6'-(propan-2-yl)-1,2,3',5'-tetrahydro-2'H-spiro[indol-3,1'-pyrrolo[3,4-c]pyrrol]-2,3'-dion,
(3S)-6-Chlor-2'-(5-chlor-2-methylphenyl)-5'-[(3S,4R)-3-methoxypiperidin-4-yl]-6'-(propan-2-yl)-1,2,3',5'-tetrahydro-2'H-spiro[indol-3,1'-pyrrolo[3,4-c]pyrrol]-2,3'-dion,
(3S)-6-Chlor-5'-(5-chlorpyridin-3-yl)-2'-(2-methoxyphenyl)-3'-(propan-2-yl)-1,2,5',6'-tetrahydro-2'H-spiro[indol-3,4'-pyrrolo[3,4-c]pyrazol]-2,6'-dion,
(3S)-6-Chlor-5'-(5-chlor-2-methylpyridin-3-yl)-2'-(2-methoxyphenyl)-3'-(propan-2-yl)-1,2,5',6'-tetrahydro-2'H-spiro[indol-3,4'-pyrrolo[3,4-c]pyrazol]-2,6'-dion,
(3S)-6-Chlor-5'-(5-chlorpyridin-3-yl)-2'-(2-methoxyphenyl)-3'-(propan-2-yl)-1,2,5',6'-tetrahydro-2'H-spiro[indol-3,4'-pyrrolo[3,4-c]pyrazol]-2,6'-dion, und
(3S)-6-Chlor-5'-(5-chlor-2-methylphenyl)-2'-(2-methoxyphenyl)-3'-(propan-2-yl)-1,2,5',6'-tetrahydro-2'H-spiro[indol-3,4'-pyrrolo[3,4-c]pyrazol]-2,6'-dion.

**17.** Die Verbindung nach Anspruch 15, wobei sie (3S)-6-chloro-1'-(3-chlorophenyl)-3'-[(4-chlorophenyl)sulfanyl]-4'-hydroxy-1,1',2,5'-tetrahydrospiro[indol-3,2'-pyrrol]-2,5'-dion ist.

**18.** Die Verbindung nach Anspruch 15, ausgewählt aus der folgenden Gruppe:

6-Chlor-5'-(5-chlor-2-methylphenyl)-3'-(1-methylcyclopropyl)-1,2,5',6'-tetrahydro-2'H-spiro[indol-3,4'-pyrrolo[3,4-c]pyrazol]-2,6'-dion,
6-Chlor-5'-(3-chlorphenyl)-3'-(1-methylcyclopropyl)-1,2,5',6'-tetrahydro-2'H-spiro[indol-3,4'-pyrrolo[3,4-c]pyrazol]-2,6'-dion,
3'-tert-Butyl-6-chlor-5'-(3-chlorphenyl)-1,2,5',6'-tetrahydro-2'H-spiro[indol-3,4'-pyrrolo[3,4-c]pyrazol]-2,6'-dion,
3'-tert-Butyl-6-chlor-5'-(5-chlor-2-methoxyphenyl)-1,2,5',6'-tetrahydro-2'H-spiro[indol-3,4'-pyrrolo[3,4-c]pyrazol]-2,6'-dion,
6-Chlor-5'-(5-chlor-2-methoxyphenyl)-3'-(1-methylcyclopropyl)-1,2,5',6'-tetrahydro-2'H-spiro[indol-3,4'-pyrrolo[3,4-c]pyrazol]-2,6'-dion,
6-Chlor-5'-(5-chlor-2-methylphenyl)-3'-(propan-2-yl)-1,2,5',6'-tetrahydro-2'H-spiro[indol-3,4'-pyrrolo[3,4-c]pyrazol]-2,6'-dion,
6-Chlor-5'-(5-chlor-2-hydroxyphenyl)-3'-(propan-2-yl)-1,2,5',6'-tetrahydro-2'H-spiro[indol-3,4'-pyrrolo[3,4-c]pyrazol]-2,6'-dion,
6-Chlor-5'-(5-chlor-2-methoxyphenyl)-3'-(propan-2-yl)-1,2,5',6'-tetrahydro-2'H-spiro[indol-3,4'-pyrrolo[3,4-c]pyrazol]-2,6'-dion,
6-Chlor-5'-(3-chlorphenyl)-2'-(2-methoxyphenyl)-3'-(1-methylcyclopropyl)-1,2,5',6'-tetrahydro-2'H-spiro[indol-3,4'-pyrrolo[3,4-c]pyrazol]-2,6'-dion,
6-Chlor-5'-(3-chlorphenyl)-2'-(2-methoxyphenyl)-3'-(propan-2-yl)-1,2,5',6'-tetrahydro-2'H-spiro[indol-3,4'-pyrrolo[3,4-c]pyrazol]-2,6'-dion,
6-Chlor-5'-(3-chlorphenyl)-3'-(1-methylcyclopropyl)-2'-(pyrrolidin-2-ylmethyl)-1,2,5',6'-tetrahydro-2'H-spiro[indol-3,4'-pyrrolo[3,4-c]pyrazol]-2,6'-dion,
2-[6-Chlor-5'-(3-chlorphenyl)-3'-(1-methylcyclopropyl)-2,6'-dioxo-1,2,5',6'-tetrahydro-2'H-spiro[indol-3,4'-pyrrolo[3,4-c]pyrazol]-2'-ylmethyl]-N,N-dimethylcyclopentan-1-carboxamid,
6-Chlor-5'-(5-chlor-2-methoxyphenyl)-2'-(2-methoxyphenyl)-3'-(propan-2-yl)-1,2,5',6'-tetrahydro-2'H-spiro[indol-3,4'-pyrrolo[3,4-c]pyrazol]-2,6'-dion,
6-Chlor-5'-(5-chlor-2-methylphenyl)-2'-(2-methoxyphenyl)-3'-(propan-2-yl)-1,2,5',6'-tetrahydro-2'H-spiro[indol-3,4'-pyrrolo[3,4-c]pyrazol]-2,6'-dion,
6-Chlor-5'-(5-chlor-2-methylphenyl)-1'-(2-methoxyphenyl)-3'-(propan-2-yl)-1,2,5',6'-tetrahydro-1'H-spiro[indol-3,4'-pyrrolo[3,4-c]pyrazol]-2,6'-dion, und
2-[6-Chlor-5'-(3-chlorphenyl)-2,6'-dioxo-3'-(propan-2-yl)-1,2,5',6'-tetrahydro-2'H-spiro[indol-3,4'-pyrrolo[3,4-c]pyrazol]-2'-ylmethyl]-N,N-dimethylpyrrolidin-1-carboxamid.

**19.** Die Verbindung nach Anspruch 15, ausgewählt aus der folgenden Gruppe:

Methyl (2E)-4-{4-chlor-2-[6-chlor-2'-(2-methoxyphenyl)-2,6'-dioxo-3'-(propan-2-yl)-1,2,5',6'-tetrahydro-2'H-spiro[indol-3,4'-pyrrolo[3,4-c]pyrazol]-5'-yl]phenoxy}but-2-enoat,
6-Chlor-5'-(5-chlor-1-methyl-6-oxo-1,6-dihydropyridin-3-yl)-2'-(2-methoxyphenyl)-3'-(propan-2-yl)-1,2,5',6'-tetrahydro-2'H-spiro[indol-3,4'-pyrrolo[3,4-c]pyrazol]-2,6'-dion,
6-Chlor-5'-(5-chlor-2-methylphenyl)-2'-(3-methoxypyridin-4-yl)-3'-(propan-2-yl)-1,2,5',6'-tetrahydro-2'H-spiro[indol-3,4'-pyrrolo[3,4-c]pyrazol]-2,6'-dion,
(3S)-6-Chlor-5'-(5-chlor-2-methylphenyl)-2'-(2-methoxyphenyl)-3'-(propan-2-yl)-1,2,5',6'-tetrahydro-2'H-spiro[indol-3,4'-pyrrolo[3,4-c]pyrazol]-2,6'-dion,
6-Chlor-5'-(5-chlor-2-methylphenyl)-2'-(3-ethylphenyl)-3'-(propan-2-yl)-1,2,5',6'-tetrahydro-2'H-spiro[indol-3,4'-pyrrolo[3,4-c]pyrazol]-2,6'-dion,
6-Chlor-5'-(5-chlor-2-methylphenyl)-2'-(2-ethylphenyl)-3'-(propan-2-yl)-1,2,5',6'-tetrahydro-2'H-spiro[indol-3,4'-pyrrolo[3,4-c]pyrazol]-2,6'-dion,
6-Chlor-5'-(5-chlor-2-methylphenyl)-2'-(2-methylphenyl)-3'-(propan-2-yl)-1,2,5',6'-tetrahydro-2'H-spiro[indol-3,4'-pyrrolo[3,4-c]pyrazol]-2,6'-dion,
6-Chlor-5'-(5-chlor-2-methylphenyl)-2'-(3-methylphenyl)-3'-(propan-2-yl)-1,2,5',6'-tetrahydro-2'H-spiro[indol-3,4'-pyrrolo[3,4-c]pyrazol]-2,6'-dion,
6-Chlor-5'-(5-chlor-2-methylphenyl)-2'-(2,6-dimethoxyphenyl)-3'-(propan-2-yl)-1,2,5',6'-tetrahydro-2'H-spiro[indol-3,4'-pyrrolo[3,4-c]pyrazol]-2,6'-dion,
6-Chlor-5'-(5-chlor-2-methylpyridin-3-yl)-2'-(2-methoxyphenyl)-3'-(propan-2-yl)-1,2,5',6'-tetrahydro-2'H-spiro[indol-3,4'-pyrrolo[3,4-c]pyrazol]-2,6'-dion,
6-Chlor-5'-(5-chlorpyridin-3-yl)-2'-(2-methoxyphenyl)-3'-(propan-2-yl)-1,2,5',6'-tetrahydro-2'H-spiro[indol-3,4'-pyrrolo[3,4-c]pyrazol]-2,6'-dion,
6-Chlor-5'-[5-chlor-2-(2-methansulfonylethoxy)phenyl]-2'-(2-methoxyphenyl)-3'-(propan-2-yl)-1,2,5',6'-tetrahydro-2'H-spiro[indol-3,4'-pyrrolo[3,4-c]pyrazol]-2,6'-dion,
2'-(1-Benzothiophen-3-yl)-6-chlor-5'-(5-chlor-2-methylphenyl)-3'-(propan-2-yl)-1,2,5',6'-tetrahydro-2'H-spiro[indol-3,4'-pyrrolo[3,4-c]pyrazol]-2,6'-dion,
3-[6-Chlor-5'-(5-chlor-2-hydroxyphenyl)-2,6'-dioxo-3'-(propan-2-yl)-1,2,5',6'-tetrahydro-2'H-spiro[indol-3,4'-pyrrolo[3,4-c]pyrazol]-2'-yl]-4-methoxybenzamid,
3-[6-Chlor-5'-(5-chlor-2-hydroxyphenyl)-2,6'-dioxo-3'-(propan-2-yl)-1,2,5',6'-tetrahydro-2'H-spiro[indol-3,4'-pyrrolo[3,4-c]pyrazol]-2'-yl]-4-methoxybenzoesäure.
6-Chlor-5'-(5-chlor-2-methylphenyl)-2'-[2-(methylsulfanyl)phenyl]-3'-(propan-2-yl)-1,2,5',6'-tetrahydro-2'H-spiro[indol-3,4'-pyrrolo[3,4-c]pyrazol]-2,6'-dion,
Methyl 3-[6-Chlor-5'-(5-chlor-2-methylphenyl)-2,6'-dioxo-3'-(propan-2-yl)-1,2,5',6'-tetrahydro-2'H-spiro[indol-3,4'-pyrrolo[3,4-c]pyrazol]-2'-yl]-4-methoxybenzoat,
Methyl 3-[6-Chlor-5'-(5-chlor-2-hydroxyphenyl)-2,6'-dioxo-3'-(propan-2-yl)-1,2,5',6'-tetrahydro-2'H-spiro[indol-3,4'-pyrrolo[3,4-c]pyrazol]-2'-yl]-4-methoxybenzoat,
(3S)-6-Chlor-5'-(5-chlor-2-methylpyridin-3-yl)-2'-(2-methoxyphenyl)-3'-(propan-2-yl)-1,2,5',6'-tetrahydro-2'H-spiro[indol-3,4'-pyrrolo[3,4-c]pyrazol]-2,6'-dion,
(3S)-6-Chlor-5'-(5-chlorpyridin-3-yl)-2'-(2-methoxyphenyl)-3'-(propan-2-yl)-1,2,5',6'-tetrahydro-2'H-spiro[indol-3,4'-pyrrolo[3,4-c]pyrazol]-2,6'-dion,
6-Chlor-5'-(4-chlorpyridin-2-yl)-3'-(propan-2-yl)-1,2,5',6'-tetrahydro-2'H-spiro[indol-3,4'-pyrrolo[3,4-c]pyrazol]-2,6'-dion,
6-Chlor-5'-(5-chlorpyridin-3-yl)-3'-(propan-2-yl)-1,2,5',6'-tetrahydro-2'H-spiro[indol-3,4'-pyrrolo[3,4-c]pyrazol]-2,6'-dion,
6-Chlor-5'-(5-chlor-2-methylpyridin-3-yl)-3'-(propan-2-yl)-1,2,5',6'-tetrahydro-2'H-spiro[indol-3,4'-pyrrolo[3,4-c]pyrazol]-2,6'-dion
6-Chlor-5'-(5-chlor-2-methylphenyl)-3'-ethyl-2'-(2-ethylphenyl)-1,2,5',6'-tetrahydro-2'H-spiro[indol-3,4'-pyrrolo[3,4-c]pyrazol]-2,6'-dion,
6-Chlor-5'-(cyclobutylmethyl)-2'-(2-methoxyphenyl)-3'-(propan-2-yl)-1,2,5',6'-tetrahydro-2'H-spiro[indol-3,4'-pyrrolo[3,4-c]pyrazol]-2,6'-dion,
6-Chlor-5'-(2,2-dimethylpropyl)-2'-(2-methoxyphenyl)-3'-(propan-2-yl)-1,2,5',6'-tetrahydro-2'H-spiro[indol-3,4'-pyrrolo[3,4-c]pyrazol]-2,6'-dion,
(3S)-6-Chlor-5'-(5-chlorpyridin-3-yl)-2'-(2-methoxyphenyl)-3'-(propan-2-yl)-1,2,5',6'-tetrahydro-2'H-spiro[indol-3,4'-pyrrolo[3,4-c]pyrazol]-2,6'-dion,

6-Chlor-2'-(3-chlorphenyl)-5'-(2-methoxyphenyl)-6'-(propan-2-yl)-1,2,3',5'-tetrahydro-2'H-spiro[indol-3,1'-pyrrolo[3,4-c]pyrrol]-2,3'-dion,
6-Chlor-2'-(5-chlor-2-nitrophenyl)-5'-(2-methoxyphenyl)-6'-(propan-2-yl)-1,2,3',5'-tetrahydro-2'H-spiro[indol-3,1'-pyrrolo[3,4-c]pyrrol]-2,3'-dion,
2'-(2-Amino-5-chlorphenyl)-6-chlor-5'-(2-methoxyphenyl)-6'-(propan-2-yl)-1,2,3',5'-tetrahydro-2'H-spiro[indol-3,1'-pyrrolo[3,4-c]pyrrol]-2,3'-dion,
N-{4-Chlor-2-[6-chlor-5'-(2-methoxyphenyl)-2,3'-dioxo-6'-(propan-2-yl)-1,2,3',5'-tetrahydro-2'H-spiro[indol-3,1'-pyrrolo[3,4-c]pyrrol]-2'-yl]phenyl}methansulfonamid,
6-Chlor-2'-(5-chlor-2-methylphenyl)-5'-(2,4-dimethoxyphenyl)-6'-(propan-2-yl)-1,2,3',5'-tetrahydro-2'H-spiro[indol-3,1'-pyrrolo[3,4-c]pyrrol]-2,3'-dion,
5'-(2H-1,3-Benzodioxol-4-yl)-6-chlor-2'-(5-chlor-2-methylphenyl)-6'-(propan-2-yl)-1,2,3',5'-tetrahydro-2'H-spiro[indol-3,1'-pyrrolo[3,4-c]pyrrol]-2,3'-dion,
3-[6-Chlor-2'-(5-chlor-2-methylphenyl)-2,3'-dioxo-6'-(propan-2-yl)-1,2,3',5'-tetrahydro-2'H-spiro[indol-3,1'-pyrrolo[3,4-c]pyrrol]-5'-yl]-4-methoxybenzamid,
5'-(5-Amino-2-methoxyphenyl)-6-chlor-2'-(5-chlor-2-methylphenyl)-6'-(propan-2-yl)-1,2,3',5'-tetrahydro-2'H-spiro[indol-3,1'-pyrrolo[3,4-c]pyrrol]-2,3'-dion,
N-{3-[6-Chlor-2'-(5-chlor-2-methylphenyl)-2,3'-dioxo-6'-(propan-2-yl)-1,2,3',5'-tetrahydro-2'H-spiro[indol-3,1'-pyrrolo[3,4-c]pyrrol]-5'-yl]-4-methoxyphenyl}acetamid,
3-[6-Chlor-2'-(5-chlor-2-methylphenyl)-2,3'-dioxo-6'-(propan-2-yl)-1,2,3',5'-tetrahydro-2'H-spiro[indol-3,1'-pyrrolo[3,4-c]pyrrol]-5'-yl]-N,N-diethyl-4-methoxybenzol-1-sulfonamid,
4-Chlor-2-[6-chlor-5'-(2-methoxyphenyl)-2,3'-dioxo-6'-(propan-2-yl)-1,2,3',5'-tetrahydro-2'H-spiro[indol-3,1'-pyrrolo[3,4-c]pyrrol]-2'-yl]benzamid,
3-[6-Chlor-2'-(5-chlor-2-methylphenyl)-2,3'-dioxo-6'-(propan-2-yl)-1,2,3',5'-tetrahydro-2'H-spiro[indol-3,1'-pyrrolo[3,4-c]pyrrol]-5'-yl]-N-(2-hydroxyethyl)-4-methoxybenzamid,
6-Chlor-2'-(5-chlor-2-methylphenyl)-5'-(6-methoxy-2-oxo-2,3-dihydro-1H-1,3-benzodiazol-5-yl)-6'-(propan-2-yl)-1,2,3',5'-tetrahydro-2'H-spiro[indol-3,1'-pyrrolo[3,4-c]pyrrol]-2,3'-dion,
N-{4-Chlor-2-[(3S)-6-chlor-5'-(2-methoxyphenyl)-2,3'-dioxo-6'-(propan-2-yl)-1,2,3',5'-tetrahydro-2'H-spiro[indol-3,1'-pyrrolo[3,4-c]pyrrol]-2'-yl]phenyl}methansulphonamid,
(3S)-6-Chlor-2'-(5-chlor-2-methylphenyl)-5'-[(3S,4R)-3-methoxypiperidin-4-yl]-6'-(propan-2-yl)-1,2,3',5'-tetrahydro-2'H-spiro[indol-3,1'-pyrrolo[3,4-c]pyrrol]-2,3'-dion,
6-Chlor-2'-(5-chlor-2-methylpyridin-3-yl)-5'-(2,4-dimethoxyphenyl)-6'-(propan-2-yl)-1,2,3',5'-tetrahydro-2'H-spiro[indol-3,1'-pyrrolo[3,4-c]pyrrol]-2,3'-dion,
4-Chlor-2-[6-chlor-5'-(2-methoxyphenyl)-2,3'-dioxo-6'-(propan-2-yl)-1,2,3',5'-tetrahydro-2'H-spiro[indol-3,1'-pyrrolo[3,4-c]pyrrol]-2'-yl]benzoesäure,
Ethyl 4-Chlor-2-[6-chlor-5'-(2-methoxyphenyl)-2,3'-dioxo-6'-(propan-2-yl)-1,2,3',5'-tetrahydro-2'H-spiro[indol-3,1'-pyrrolo[3,4-c]pyrrol]-2'-yl]benzoat,
(3S)-6-Chlor-2'-(5-chlor-2-methylphenyl)-5'-(2,4-dimethoxyphenyl)-6'-(propan-2-yl)-1,2,3',5'-tetrahydro-2'H-spiro[indol-3,1'-pyrrolo[3,4-c]pyrrol]-2,3'-dion,
N-{3-[(3S)-6-Chlor-2'-(5-chlor-2-methylphenyl)-2,3'-dioxo-6'-(propan-2-yl)-1,2,3',5'-tetrahydro-2'H-spiro[indol-3,1'-pyrrolo[3,4-c]pyrrol]-5'-yl]-4-methoxyphenyl}acetamid,
(3S)-5'-(5-Amino-2-methoxyphenyl)-6-chlor-2'-(5-chlor-2-methylphenyl)-6'-(propan-2-yl)-1,2,3',5'-tetrahydro-2'H-spiro[indol-3,1'-pyrrolo[3,4-c]pyrrol]-2,3'-dion,
3-[(3S)-6-Chlor-2'-(5-chlor-2-methylphenyl)-2,3'-dioxo-6'-(propan-2-yl)-1,2,3',5'-tetrahydro-2'H-spiro[indol-3,1'-pyrrolo[3,4-c]pyrrol]-5'-yl]-4-methoxybenzamid,
(3S)-6-Chlor-2'-(5-chlor-2-methylpyridin-3-yl)-5'-(2,4-dimethoxyphenyl)-6'-(propan-2-yl)-1,2,3',5'-tetrahydro-2'H-spiro[indol-3,1'-pyrrolo[3,4-c]pyrrol]-2,3'-dion,
6-Chlor-2'-(5-chlor-2-methylphenyl)-5'-[2-methoxy-5-(morpholin-4-sulfonyl)phenyl]-6'-(propan-2-yl)-1,2,3',5'-tetrahydro-2'H-spiro[indol-3,1'-pyrrolo[3,4-c]pyrrol]-2,3'-dion,
6-Chlor-2'-(5-chlor-2-methylphenyl)-5'-(2-methoxythiophen-3-yl)-6'-(propan-2-yl)-1,2,3',5'-tetrahydro-2'H-spiro[indol-3,1'-pyrrolo[3,4-c]pyrrol]-2,3'-dion, und
6-Chlor-5'-(5-chlor-2-methylphenyl)-2'-(2,6-dimethoxypyridin-3-yl)-3'-(propan-2-yl)-1,2,5',6'-tetrahydro-2'H-spiro[indol-3,4'-pyrrolo[3,4-c]pyrazol]-2,6'-dion.

**20.** Verbindung nach einem der Ansprüche 1 bis 19 zur Verwendung als Medikament.

**21.** Verbindung zur Verwendung nach Anspruch 20 zur Verwendung in einem Verfahren zur Vorbeugung und/oder Behandlung von Krankheiten, ausgewählt aus der Gruppe bestehend aus Krebs, Immunerkrankungen, entzündlichen Zuständen, allergischen Hauterkrankungen, die mit übermäßiger Proliferation assoziiert sind, und Virusinfektionen.

22. Pharmazeutische Zusammensetzung, umfassend als aktiven Bestandteil eine Verbindung nach einem der Ansprüche 1 bis 19, in Kombination mit mindestens einem pharmazeutisch verträglichen Hilfsstoff.

**Revendications**

1. Un composé représenté par la formule sélectionnée dans le groupe constitué par la formule (IA) et (IB)

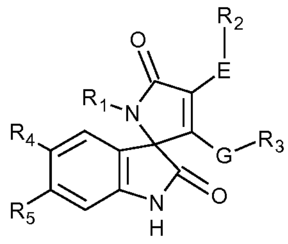

(IA)

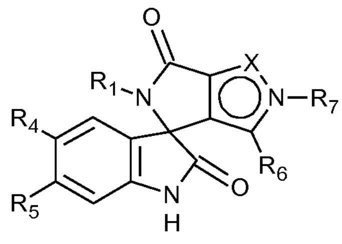

(IB)

où

$R_1$ est :

- alkyle $C_1$-$C_6$ non substitué ou substitué par cycloalkyle $C_3$-$C_6$,
- phényle ou 3-pyridyle substitués ou non substitués par un ou deux substituants indépendamment sélectionnés parmi le groupe consistant en halogène, -OH, -$NH_2$, -$NO_2$, -CN, alkyle $C_1$-$C_6$, alcényle $C_2$-$C_6$, -O-(alkyle $C_1$-$C_6$), -O-(alcényle $C_2$-$C_6$), -S-(alkyle $C_1$-$C_6$), -S-(alcényle $C_2$-$C_6$), -C(O)O-(alkyle $C_1$-$C_6$), -C(O)O-(alcényle $C_2$-$C_6$), -C(O)$NH_2$, -C(O)NH(alkyle $C_1$-$C_6$), -C(O)N(alkyle $C_1$-$C_6$)$_2$, -C(O)NH(alcényle $C_2$-$C_6$), -NH(alkyle $C_1$-$C_6$), -N(alkyle $C_1$-$C_6$)$_2$, -NH-phényl, -NHC(O)-(alkyle $C_1$-$C_6$), -NHC(O)-(alcényle $C_2$-$C_6$), -NHC(O)O-(alkyle $C_1$-$C_6$), et -$NHSO_2$-(alkyle $C_1$-$C_6$), et où lesdits alkyle $C_1$-$C_6$, alcényle $C_2$-$C_6$ et phényle sont non substitués ou en outre substitués par un ou deux substituants indépendamment sélectionnés parmi le groupe consistant en halogène, -OH, -SH, -O-(alkyle $C_1$-$C_6$), -COOH, -C(O)O-(alkyle $C_1$-$C_6$), et -$SO_2$-(alkyle $C_1$-$C_6$),
- pyrazolyle non substitué ou substitué par un ou deux substituants alkyle $C_1$-$C_6$ indépendamment sélectionnés,
- 6-oxo-1,6-dihydropyridine-3-yle non substitué ou substitué par un ou deux substituants indépendamment sélectionnés parmi le groupe consistant en halogène et alkyle $C_1$-$C_6$, ou
- 2-oxo-1,2-dihydropyridine-3-yle non substitué ou substitué par un ou deux substituants indépendamment sélectionnés parmi le groupe consistant en halogène et alkyle $C_1$-$C_6$;

$R_4$ et $R_5$ sont indépendamment H ou halogène ;
$R_2$ est un atome d'hydrogène, (alkyle $C_1$-$C_6$)sulfonyle, -(alkyle $C_1$-$C_6$), ou -(alkyle $C_1$-$C_6$) substitué par un substituant terminal sélectionné parmi le groupe consistant en -COOH, -$CONH_2$, -C(O)O-($C_1$-$C_6$-alkyl), -$NH_2$, NH(alkyle $C_1$-$C_6$), -N(alkyle $C_1$-$C_6$)$_2$, -NHC(O)(alkyle $C_1$-$C_6$), imidazole, tétrazole et phényle, où ledit phényle est substitué par -(alkyle $C_1$-$C_3$), -O(alkyle $C_1$-$C_3$) ou un halogène ;
$R_3$ est :

- alkyle $C_1$-$C_6$,
- cycloalkyle $C_3$-$C_6$, non substitué ou substitué par un alkyle $C_1$-$C_6$,
- phényle non substitué ou substitué par un, deux ou trois substituants indépendamment sélectionnés parmi le groupe consistant en halogène, -OH, -$NH_2$, - $NO_2$, -CN, alkyle $C_1$-$C_6$, alcényle $C_2$-$C_6$, -O-(alkyle $C_1$-$C_6$), -O-(alcényle $C_2$-$C_6$), -S-(alkyle $C_1$-$C_6$),
-S-(alcényle $C_2$-$C_6$), -C(O)O-(alkyle $C_1$-$C_6$), -C(O)O-(alcényle $C_2$-$C_6$), -C(O)$NH_2$, -(O)NH(alkyle $C_1$-$C_6$), -C(O)N(alkyle $C_1$-$C_6$)$_2$, -C(O)NH(alcényle $C_2$-$C_6$), -NH(alkyle $C_1$-$C_6$), -N(alkyle $C_1$-$C_6$)$_2$, -NH-phényl, -NHC(O)-(alkyle $C_1$-$C_6$), -NHC(O)-(alcényle $C_2$-$C_6$), et -NHC(O)O-(alkyle $C_1$-$C_6$), et où lesdits alkyle $C_1$-$C_6$, alcényle $C_2$-$C_6$ et phényle sont non substitués ou en outre substitués par un ou deux substituants indépendamment sélectionnés parmi le groupe consistant en halogène, -OH, -SH, -O-(alkyle $C_1$-$C_6$), -COOH, et -C(O)O-(alkyle $C_1$-$C_6$), ou

- hétéroaryle à 5 ou 6 chaînons avec un, deux, trois ou quatre hétéroatomes indépendamment sélectionnés parmi N, O, et S, où ledit hétéroaryle est non substitué ou substitué par un, deux ou trois substituants indépendamment sélectionnés parmi le groupe consistant en halogène, -OH, $-NH_2$, $-NO_2$, -CN, alkyle $C_1$-$C_6$l, alcényle $C_2$-$C_6$, -O-(alkyle $C_1$-$C_6$), -S-(alkyle $C_1$-$C_6$), -S-(alcényle $C_2$-$C_6$), -C(O)O-(alkyle $C_1$-$C_6$), -C(O)O-(alcényle $C_2$-$C_6$), $-C(O)NH_2$, -C(O)NH(alkyle $C_1$-$C_6$), -C(O)N(alkyle $C_1$-$C_6$)$_2$, -C(O)NH(alcényle $C_2$-$C_6$), -NH(alkyle $C_1$-$C_6$), -N(alkyle $C_1$-$C_6$)$_2$, -NH-phényl, -NHC(O)-(alkyle $C_1$-$C_6$), -NHC(O)-(alcényle $C_2$-$C_6$), -NHC(O)O-(alkyle $C_1$-$C_6$), et hétérocyclyle non aromatique à 5 ou 6 chaînons avec un ou deux hétéroatomes sélectionnés parmi N et 0, et où lesdits alkyle $C_1$-$C_6$, alcényle $C_2$-$C_6$ et phényle sont non substitués ou en outre substitués par un ou deux substituants indépendamment sélectionnés parmi le groupe consistant en halogène, -OH, $-NH_2$, -O-(alkyle $C_1$-$C_6$), -COOH, -C(O)O-(alkyle $C_1$-$C_6$), -NH-(alkyle $C_1$-$C_6$), et -N-(alkyle $C_1$-$C_6$)$_2$;

E est O, NH, ou S ;
G est S, carbonyle ou une liaison directe ;
$R_6$ est :

- alkyle $C_1$-$C_6$ non substitué ou substitué par un -O-(alkyle $C_1$-$C_6$), ou
- cycloalkyle $C_3$-$C_6$, non substitué ou substitué par un alkyle $C_1$-$C_6$,

$R_7$ est :

- un atome d'hydrogène,
- alkyle $C_1$-$C_3$ non substitué ou substitué par un substituant sélectionné parmi le groupe consistant en imidazole, tétrazole, et un hétérocyclyle non aromatique à 5 ou 6 chaînons comprenant un ou deux hétéroatomes sélectionnés parmi N et 0, où ledit hétérocyclyle non aromatique est non substitué ou substitué sur l'atome d'azote par un substituant sélectionné parmi le groupe consistant en -(alkyle $C_1$-$C_6$), -C(O)(alkyle $C_1$-$C_6$), et -C(O)N(alkyle $C_1$-$C_6$)$_2$,
- alcényle $C_1$-$C_6$,
- phényle non substitué ou substitué par un ou deux substituants indépendamment sélectionnés parmi le groupe consistant en halogène, -OH, $-NH_2$, $-NO_2$, -CN, alkyle $C_1$-$C_6$, alcényle $C_2$-$C_6$, -O-(alkyle $C_1$-$C_6$), -O-(alcényle $C_2$-$C_6$), -S-(alkyle $C_1$-$C_6$), -S-(alcényle $C_2$-$C_6$), $-CO_2H$, -C(O)O-(alkyle $C_1$-$C_6$), -C(O)O-(alcényle $C_2$-$C_6$), $-C(O)NH_2$, -C(O)NH(alkyle $C_1$-$C_6$), et où ledit alkyle $C_1$-$C_6$ est non substitué ou en outre substitué par -OH, -C(O)N(alkyle $C_1$-$C_6$)$_2$, -C(O)NH(alcényle $C_2$-$C_6$), - NH(alkyle $C_1$-$C_6$), -N(alkyle $C_1$-$C_6$)$_2$, -NH-phényl, -NHC(O)-(alkyle $C_1$-$C_6$), -NHC(O)-(alcényle $C_2$-$C_6$), -NHC(O)O-(alkyle $C_1$-$C_6$), $-SO_2$N(alkyle $C_1$-$C_6$), or $-SO_2$-(hétérocyclyle à 5 ou 6 chaînons avec un ou deux hétéroatomes sélectionnés parmi N et O),

  - (3S,4R)-3-methoxypiperidin-4-yle, ou

- 1-benzothiophène-3-yle, 2-oxo-2,3-dihydro-1H-1,3-benzodiazole-5-yle, 3-pyridyle, 4-pyridyle, 2H-1,3-benzodioxole-4-yle ou 2-tiophényle non substitués ou substitués par alkyle $C_1$-$C_6$, -O-(alkyle $C_1$-$C_6$) ou un halogène ;

X est N ou CH ;
à la condition que la 4-acétyl-3-hydroxy-1-méthylspiro[2,5-dihydropyrrol-5,3'-indole]-2,2'-dione soit exclu ;
et les sels, solvates, tautomères, et les stéréoisomères pharmaceutiquement acceptablesde celui-ci.

**2.** Le composé selon la revendication 1, représenté par la formule (IA).

**3.** Le composé selon la revendication 2, dans lequel G est le groupe carbonyle.

**4.** Le composé selon la revendication 2, dans lequel G est -S-.

**5.** Le composé selon la revendication 2, dans leqeul G est une liaison directe.

**6.** Le composé selon l'une quelconque des revendications 1 à 5, dans lequel
$R_1$ est :

- phényle ou 3-pyridyle non substitués ou substitués par un ou deux substituants indépendamment sélectionnés parmi le groupe consistant en halogène, -OH, $-NH_2$, $-NO_2$, -CN, alkyle $C_1$-$C_6$, $C_2$-$C_6$-alkenyle, -O-(alkyle $C_1$-$C_6$), -0-(alcényle $C_2$-$C_6$), -S-(alkyle $C_1$-$C_6$), -S-(alcényle $C_2$-$C_6$), -C(O)O-(alkyle $C_1$-$C_6$), -C(O)O-(alcényle $C_2$-$C_6$), $-C(O)NH_2$, -C(O)NH(alkyle $C_1$-$C_6$), -C(O)N(alkyle $C_1$-$C_6$)$_2$, -C(O)NH(alcényle $C_2$-$C_6$), -NH(alkyle $C_1$-$C_6$), -N(alkyle $C_1$-$C_6$)$_2$, -NH-phényle, -NHC(O)-(alkyle $C_1$-$C_6$), -NHC(O)-(alcényle $C_2$-$C_6$), -NHC(O)O-(alkyle $C_1$-$C_6$), et $NHSO_2$-(alkyle $C_1$-$C_6$), est où lesdits alkyle $C_1$-$C_6$, alcényle $C_2$-$C_6$ et phényle sont non substitués ou en outre substitués par un ou deux substituants indépendamment sélectionnés parmi le groupe consistant en halogène, -OH, -SH, -O-(alkyle $C_1$-$C_6$), -COOH, -C(O)O-(alkyle $C_1$-$C_6$), et $-SO_2$-(alkyle $C_1$-$C_6$),
- pyrazolyle non substitué ou substitué par un ou deux substituants alkyle $C_1$-$C_6$ indépendamment sélectionnés,
- 6-oxo-1,6-dihydropyridine-3-yle non substitué ou substitué par un ou deux substituants indépendamment sélectionnés parmi le groupe consistant en halogène et alkyle $C_1$-$C_6$, ou
- 2-oxo-1,2-dihydropyridine-3-yle non substitue ou substitue par un ou deux substituants indépendamment sélectionnés parmi le groupe consistant en halogène et alkyle $C_1$-$C_6$,

$R_4$ est H, et
$R_5$ est Cl ou F.

7. Le composé selon la revendication 1, représenté par la formule (IB).

8. Le composé selon la revendication 7, dans lequel X est N et le composé est représenté par la formule (IB-1)

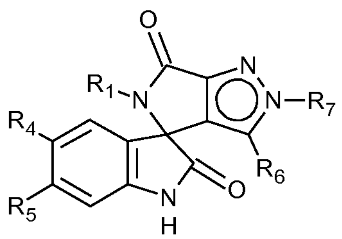

(IB-1).

9. Le composé selon la revendication 7, dans lequel X est CH et le composé est représenté par la formule (IB-2)

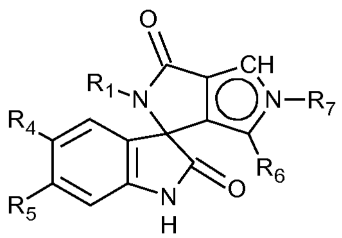

(IB-2).

10. Le composé selon l'une quelconque des revendications 7 à 9, dans lequel $R_1$ est :

- phényle ou 3-pyridyle non substitués ou substitués par un ou deux substituants indépendamment sélectionnés parmi le groupe consistant en halogène, -OH, $-NH_2$, $-NO_2$, -CN, alkyle $C_1$-$C_6$, $C_2$-$C_6$-alkenyl, -O-(alkyle $C_1$-$C_6$), -0-(alcényle $C_2$-$C_6$), -S-(alkyle $C_1$-$C_6$), -S-(alcényle $C_2$-$C_6$), -C(O)O-(alkyle $C_1$-$C_6$), -C(O)O-(alcényle $C_2$-$C_6$), $-C(O)NH_2$, -C(O)NH(alkyle $C_1$-$C_6$), -C(O)N(alkyle $C_1$-$C_6$)$_2$, -C(O)NH(alcényle $C_2$-$C_6$), -NH(alkyle $C_1$-$C_6$), -N(alkyle $C_1$-$C_6$)$_2$, -NH-phényl, -NHC(O)-(alkyle $C_1$-$C_6$), -NHC(O)-(alcényle $C_2$-$C_6$), -NHC(O)O-(alkyle $C_1$-$C_6$), et $NHSO_2$-(alkyle $C_1$-$C_6$), est où lesdits alkyle $C_1$-$C_6$, alcényle $C_2$-$C_6$ et phényle sont non substitués ou en outre substitués par un ou deux substituants indépendamment sélectionnés parmi le groupe consistant en halogène, -OH, -SH, -O-(alkyle $C_1$-$C_6$), -COOH, -C(O)O-(alkyle $C_1$-$C_6$), et $-SO_2$-(alkyle $C_1$-$C_6$),
- pyrazolyle non substitué ou substitué par un ou deux substituants alkyle $C_1$-$C_6$ indépendamment sélectionnés,
- 6-oxo-1,6-dihydropyridine-3-yle non substitué ou substitué par un ou deux substituants indépendamment sélectionnés parmi le groupe consistant en halogène et alkyle $C_1$-$C_6$, ou

- 2-oxo-1,2-dihydropyridine-3-yle non substitué ou substitué par un ou deux substituants indépendamment sélectionnés parmi le groupe consistant en halogène et alkyle $C_1$-$C_6$.

**11.** Le composé selon la revendication 10, dans lequel $R_1$ est :

- phényle substitué par un halogène à la position méta par rapport au lieu d'attachement à l'atome d'azote du cycle de pyrrolone, ou 3-pyridyle substitué par un halogène à la position 5 par rapport au lieu d'attachement à l'atome d'azote du cycle de pyrrolone, et où lesdits phényle et 3-pyridyle sont facultativement en outre substitués par un ou deux substituants indépendamment sélectionnés parmi le groupe consistant enhalogène, -OH, -$NH_2$, -$NO_2$, -CN, alkyle $C_1$-$C_6$, alcényle $C_2$-$C_6$, -O-(alkyle $C_1$-$C_6$), -O-(alcényle $C_2$-$C_6$), -S-(alkyle $C_1$-$C_6$), -S-(alcényle $C_2$-$C_6$), -C(O)O-(alkyle $C_1$-$C_6$), -C(O)O-(alcényle $C_2$-$C_6$), -C(O)$NH_2$, - C(O)NH(alkyle $C_1$-$C_6$), -C(O)N(alkyle $C_1$-$C_6$)$_2$, -C(O)NH(alcényle $C_2$-$C_6$), -NH(alkyle $C_1$-$C_6$), -N(alkyle $C_1$-$C_6$)$_2$, -NH-phényl, -NHC(O)-(alkyle $C_1$-$C_6$), -NHC(O)-(alcényle $C_2$-$C_6$), -NHC(O)O-(alkyle $C_1$-$C_6$), and $NHSO_2$-(alkyle $C_1$-$C_6$), et où lesdits alkyle $C_1$-$C_6$l, alcényle $C_2$-$C_6$ et phényl sont non substitués ou en outre substitués par un ou deux substituants indépendamment sélectionnés parmi le groupe consistant en halogène, -OH, -SH, -O-(alkyle $C_1$-$C_6$), -COOH, -C(O)O-(alkyle $C_1$-$C_6$), et -$SO_2$-(alkyle $C_1$-$C_6$).

**12.** Le composé selon l'une quelconque des revendications 7 à 11, dans lequel $R_4$ est H, et $R_5$ est Cl ou F.

**13.** Le composé selon la revendication 12, dans lequel $R_1$ est :

- meta-chlorophényle ou 5-chloro-3-pyridyle facultativement en outre substitués par un ou deux substituants indépendamment sélectionnés parmi le groupe consistant en halogène, -OH, -$NH_2$, -$NO_2$, -CN, alkyle $C_1$-$C_6$, alcényle $C_2$-$C_6$, -0-(alkyle $C_1$-$C_6$), -O-(alcényle $C_2$-$C_6$), -S-(alkyle $C_1$-$C_6$), -S-(alcényle $C_2$-$C_6$), -C(O)O-(alkyle $C_1$-$C_6$), -C(O)O-(alcényle $C_2$-$C_6$), -C(O)$NH_2$, -C(O)NH(alkyle $C_1$-$C_6$), - C(O)N(alkyle $C_1$-$C_6$)$_2$, -C(O)NH(alcényle $C_2$-$C_6$), -NH(alkyle $C_1$-$C_6$), -N(alkyle $C_1$-$C_6$)$_2$, -NH-phényl, -NHC(O)-(alkyle $C_1$-$C_6$), -NHC(O)-(alcényle $C_2$-$C_6$), -NHC(O)O-(alkyle $C_1$-$C_6$), and $NHSO_2$-(alkyle $C_1$-$C_6$), et où lesdits $C_1$-$C_6$-alkyl, alcényle $C_2$-$C_6$ et phényl sont non substitués ou encore substitués par un ou deux substituants indépendamment sélectionnés parmi le groupe consistant en halogène, -OH, -SH, -O-(alkyle $C_1$-$C_6$), -COOH, -C(O)O-(alkyle $C_1$-$C_6$), and -$SO_2$-(alkyle $C_1$-$C_6$).

**14.** Le composé selon l'une quelconque des revendications 7 à 13, dans lequel la configuration absolue à l'atome de carbone spiranique est S (configuration 3S).

**15.** Le composé selon la revendication 1 choisis dans le groupe suivant :

6-chloro-1'-(5-chloro-2-fluorophényl)-4'-hydroxy-3'-(1-méthylcyclopropanecarbonyl)-1,1',2,5'-tetrahydrospiro[indole-3,2'-pyrrole]-2,5'-dione,
6-chloro-1'-(5-chloro-2-méthylphényl)-4'-hydroxy-3'-(1-méthylcyclopropanecarbonyl)-1,1',2,5'-tetrahydrospiro[indole-3,2'-pyrrole]-2,5'-dione,
3'-benzoyl-6-chloro-1-(3-chlorophényl)-4'-hydroxy-1,1',2,5'-tetrahydrospiro[indole-3,2'-pyrrole]-2,5'-dione,
3'-benzoyl-6-chloro-1'-(1,5-diméthyl-1H-pyrazol-3-yl)-4'-hydroxy-1,1',2,5'-tetrahydrospiro[indole-3,2'-pyrrole]-2,5'-dione,
6-chloro-1'-(3-chlorophényl)-4'-hydroxy-3'-(1-méthylcyclopropanecarbonyl)-1,1',2,5'-tetrahydrospiro[indole-3,2'-pyrrole]-2,5'-dione,
6-chloro-1'-(3-chlorophényl)-3'-(2,2-tert-butanoyl)-4'-hydroxy-1,1',2,5'-tetrahydrospiro[indole-3,2'-pyrrole]-2,5'-dione,
6-chloro-1'-(5-chloro-2-méthoxyphényl)-4'-hydroxy-3'-(1-méthylcyclopropanecarbonyl)-1,1',2,5'-tetrahydrospiro[indole-3,2'-pyrrole]-2,5'-dione,
6-chloro-1'-(5-chloro-2-méthylphényl)-4'-hydroxy-3'-(iso-propanoyl)-1,1',2,5'-tetrahydrospiro[indole-3,2'-pyrrole]-2,5'-dione,
6-chloro-1'-(5-chloro-2-hydroxyphényl)-4'-hydroxy-3'-(iso-propanoyl)-1,1',2,5'-tetrahydrospiro[indole-3,2'-pyrrole]-2,5'-dione,
6-chloro-1'-(5-chloro-2-méthoxyphényl)-4'-hydroxy-3'-(iso-propanoyl)-1,1',2,5'-tetrahydrospiro[indole-3,2'-pyrrole]-2,5'-dione,
6-chloro-1'-(5-chloro-2-fluorophényl)-4'-hydroxy-3'-(iso-propanoyl)-1,1',2,5'-tetrahydrospiro[indole-3,2'-pyrrole]-2,5'-dione,
6-chloro-1'-(5-chloro-1-méthyl-6-oxo-1,6-dihydropyridin-3-yl)-4'-hydroxy-3'-(iso-propanoyl)-1,1',2,5'-tetrahydrospiro[indole-3,2'-pyrrole]-2,5'-dione,
6-chloro-1'-(3-chlorophényl)-3'-[2,2-diméthyl-3-(propane-2-yloxy)propanoyl]-4'-hydroxy-1,1',2,5'-tetrahydrospiro[indole-3,2'-pyrrole]-2,5'-dione,
2-{[6-chloro-1'-(5-chloro-2-méthylphényl)-3'-(1-méthylcyclopropanecarbonyl)-2,5'-dioxo-1,1',2,5'-tetrahydrospiro[indole-3,2'-pyrrole]-4'-yl]amino}acétamide,
éthyl 2-{[6-chloro-1'-(3-chlorophényl)-3'-(1-méthylcyclopropanecarbonyl)-2,5'-dioxo-1,1',2,5'-tetrahydrospiro[indole-3,2'-pyrrole]-4'-yl]amino}acétate,
2-{[6-chloro-1'-(3-chlorophényl)-3'-(1-méthylcyclopropanecarbonyl)-2,5'-dioxo-1,1',2,5'-tetrahydrospiro[indole-3,2'-pyrrole]-4'-yl]amino}acétamide,
éthyl (2E)-3-[(3-{[6-chloro-1'-(3-chlorophényl)-4'-hydroxy-2,5'-dioxo-1,1',2,5'-tetrahydrospiro[indole-3,2'-pyrrole]-3'-yl]carbonyl}phényl)carbamoyl]prop-2-énoate,
6-chloro-5'-(5-chloro-2-méthylphényl)-3'-(1-méthylcyclopropyl)-1,2,5',6'-tetrahydro-2'H-spiro[indole-3,4'-pyrrolo[3,4-c]pyrazole]-2,6'-dione,
6-chloro-5'-(3-chlorophényl)-3'-(1-méthylcyclopropyl)-1,2,5',6'-tetrahydro-2'H-spiro[indole-3,4'-pyrrolo[3,4-c]pyrazole]-2,6'-dione,
3'-tert-butyl-6-chloro-5'-(3-chlorophényl)-1,2,5',6'-tetrahydro-2'H-spiro[indole-3,4'-pyrrolo[3,4-c]pyrazole]-2,6'-dione,
3'-tert-butyl-6-chloro-5'-(5-chloro-2-méthoxyphényl)-1,2,5',6'-tetrahydro-2'H-spiro[indole-3,4'-pyrrolo[3,4-c]pyrazole]-2,6'-dione,
6-chloro-5'-(5-chloro-2-méthoxyphényl)-3'-(1-méthylcyclopropyl)-1,2,5',6'-tetrahydro-2'H-spiro[indole-3,4'-pyrrolo[3,4-c]pyrazole]-2,6'-dione,
6-chloro-5'-(5-chloro-2-méthylphényl)-3'-(propane-2-yl)-1,2,5',6'-tetrahydro-2'H-spiro[indole-3,4'-pyrrolo[3,4-c]pyrazole]-2,6'-dione,
6-chloro-5'-(5-chloro-2-hydroxyphényl)-3'-(propane-2-yl)-1,2,5',6'-tetrahydro-2'H-spiro[indole-3,4'-pyrrolo[3,4-c]pyrazole]-2,6'-dione,
6-chloro-5'-(5-chloro-2-méthoxyphényl)-3'-(propane-2-yl)-1,2,5',6'-tetrahydro-2'H-spiro[indole-3,4'-pyrrolo[3,4-c]pyrazole]-2,6'-dione,
6-chloro-5'-(3-chlorophényl)-2'-(2-méthoxyphényl)-3'-(1-méthylcyclopropyl)-1,2,5',6'-tetrahydro-2'H-spiro[indole-3,4'-pyrrolo[3,4-c]pyrazole]-2,6'-dione,
6-chloro-5'-(3-chlorophényl)-2'-(2-méthoxyphényl)-3'-(propane-2-yl)-1,2,5',6'-tetrahydro-2'H-spiro[indole-3,4'-pyrrolo[3,4-c]pyrazole]-2,6'-dione,
6-chloro-5'-(3-chlorophényl)-3'-(1-méthylcyclopropyl)-2'-(pyrrolidin-2-ylméthyl)-1,2,5',6'-tetrahydro-2'H-spiro[indole-3,4'-pyrrolo[3,4-c]pyrazole]-2,6'-dione,
2-[6-chloro-5'-(3-chlorophényl)-3'-(1-méthylcyclopropyl)-2,6'-dioxo-1,2,5',6'-tetrahydro-2'H-spiro[indole-3,4'-pyrrolo[3,4-c]pyrazole]-2'-ylméthyl]-N,N-diméthylcyclopentane-1-carboxamide,
6-chloro-5'-(5-chloro-2-méthoxyphényl)-2'-(2-méthoxyphényl)-3'-(propane-2-yl)-1,2,5',6'-tetrahydro-2'H-spiro[indole-3,4'-pyrrolo[3,4-c]pyrazole]-2,6'-dione,
6-chloro-5'-(5-chloro-2-méthylphényl)-2'-(2-méthoxyphényl)-3'-(propane-2-yl)-1,2,5',6'-tetrahydro-2'H-spiro[indole-3,4'-pyrrolo[3,4-c]pyrazole]-2,6'-dione,
6-chloro-5'-(5-chloro-2-méthylphényl)-1'-(2-méthoxyphényl)-3'-(propane-2-yl)-1,2,5',6'-tetrahydro-1'H-spiro[indole-3,4'-pyrrolo[3,4-c]pyrazole]-2,6'-dione,
2-[6-chloro-5'-(3-chlorophényl)-2,6'-dioxo-3'-(propane-2-yl)-1,2,5',6'-tetrahydro-2'H-spiro[indole-3,4'-pyrrolo[3,4-c]pyrazole]-2'-ylméthyl]-N,N-diméthylpyrrolidine-1-carboxamide,
6-chloro-1'-(2,2-diméthylpropyl)-4'-hydroxy-3'-phényl-1,1',2,5'-tetrahydrospiro[indole-3,2'-pyrrole]-2,5'-dione,
6-chloro-3'-(3-chlorophényl)-1'-(2,2-diméthylpropyl)-4'-hydroxy-1,1',2,5'-tetrahydrospiro[indole-3,2'-pyrrole]-2,5'-dione,
6-chloro-3'-(3-chlorophényl)-4'-hydroxy-1'-phényl-1,1',2,5'-tetrahydrospiro[indole-3,2'-pyrrole]-2,5'-dione,
5-chloro-1'-(3-chlorophényl)-4'-hydroxy-3'-phényl-1,1',2,5'-tetrahydrospiro[indole-3,2'-pyrrole]-2,5'-dione,
1'-(3-chlorophényl)-4'-hydroxy-3'-phényl-1,1',2,5'-tetrahydrospiro[indole-3,2'-pyrrole]-2,5'-dione,
6-chloro-1',3'-bis(3-chlorophényl)-4'-hydroxy-1,1',2,5'-tetrahydrospiro[indole-3,2'-pyrrole]-2,5'-dione,
6-chloro-1'-(3-chlorophényl)-4'-hydroxy-3'-phényl-1,1',2,5'-tetrahydrospiro[indole-3,2'-pyrrole]-2,5'-dione,
1'-(3-chlorophényl)-6-fluoro-4'-hydroxy-3'-phényl-1,1',2,5'-tetrahydrospiro[indole-3,2'-pyrrole]-2,5'-dione,
5,6-dichloro-1'-(3-chlorophényl)-4'-hydroxy-3'-phényl-1,1',2,5'-tetrahydrospiro[indole-3,2'-pyrrole]-2,5'-dione,
6-chloro-1'-(3-chlorophényl)-3'-cyclohexyl-4'-hydroxy-1,1',2,5'-tetrahydrospiro[indole-3,2'-pyrrole]-2,5'-dione,
6-chloro-1'-(5-chloro-2-fluorophényl)-4'-hydroxy-3'-phényl-1,1',2,5'-tetrahydrospiro[indole-3,2'-pyrrole]-2,5'-dione,

6-chloro-1'-(5-chloro-2-méthoxyphényl)-4'-hydroxy-3'-phényl-1,1',2,5'-tetrahydrospiro[indole-3,2'-pyrrole]-2,5'-dione,
6-chloro-1'-(5-chloro-2-méthylphényl)-4'-hydroxy-3'-phényl-1,1',2,5'-tetrahydrospiro[indole-3,2'-pyrrole]-2,5'-dione,
éthyl 2-{[6-chloro-1'-(3-chlorophényl)-2,5'-dioxo-3'-phényl-1,1',2,5'-tetrahydrospiro[indole-3,2'-pyrrole]-4'-yl]amino}acétate,
2-{[6-chloro-1'-(3-chlorophényl)-2,5'-dioxo-3'-phényl-1,1',2,5'-tetrahydrospiro[indole-3,2'-pyrrole]-4'-yl]amino}acide acétique,
méthyl 2-[6-chloro-1'-(3-chlorophényl)-2,5'-dioxo-3'-phényl-1,1',2,5'-tetrahydrospiro[indole-3,2'-pyrrole]-4'-ylsulfanyl]acétate,
2-[6-chloro-1'-(3-chlorophényl)-2,5'-dioxo-3'-phényl-1,1',2,5'-tetrahydrospiro[indole-3,2'-pyrrole]-4'-ylsulfanyl]acide acétique,
6-chloro-1'-(3-chlorophényl)-2,5'-dioxo-3'-phényl-1,1',2,5'-tetrahydrospiro[indole-3,2'-pyrrole]-4'-yl méthanesulfonate,
2-[6-chloro-1'-(3-chlorophényl)-2,5'-dioxo-3'-phényl-1,1',2,5'-tetrahydrospiro[indole-3,2'-pyrrole]-4'-ylsulfanyl]acetamide,
(3S)-6-chloro-1'-(3-chlorophényl)-3'-[(4-chlorophényl)sulfanyl]-4'-hydroxy-1,1',2,5'-tetrahydrospiro[indole-3,2'-pyrrole]-2,5'-dione,
6-chloro-1'-(3-chlorophényl)-3'-[(4-chlorophényl)sulfanyl]-4'-hydroxy-1,1',2,5'-tetrahydrospiro[indole-3,2'-pyrrole]-2,5'-dione,
3'-(butan-2-ylsulfanyl)-6-chloro-1'-(3-chlorophényl)-4'-hydroxy-1,1',2,5'-tetrahydrospiro[indole-3,2'-pyrrole]-2,5'-dione,
3'-(tert-butylsulfanyl)-6-chloro-1'-(3-chlorophényl)-4'-hydroxy-1,1',2,5'-tetrahydrospiro[indole-3,2'-pyrrole]-2,5'-dione,
6-chloro-1'-(3-chlorophényl)-4'-hydroxy-3'-{[(4-méthoxyphényl)méthyl]sulfanyl}-1,1',2,5'-tetrahydrospiro[indole-3,2'-pyrrole]-2,5'-dione,
6-chloro-1'-(3-chlorophényl)-4'-hydroxy-3'-{[5-(morpholin-4-yl)-1,3,4-thiadiazol-2-yl]sulfanyl}-1,1',2,5'-tetrahydrospiro[indole-3,2'-pyrrole]-2,5'-dione,
6-chloro-3'-[(5-chloro-1,3-diméthyl-1H-pyrazol-4-yl)sulfanyl]-1'-(3-chlorophényl)-4'-hydroxy-1,1',2,5'-tetrahydrospiro[indole-3,2'-pyrrole]-2,5'-dione,
méthyl (2E)-4-{4-chloro-2-[6-chloro-2'-(2-méthoxyphényl)-2,6'-dioxo-3'-(propane-2-yl)-1,2,5',6'-tetrahydro-2'H-spiro[indole-3,4'-pyrrolo[3,4-c]pyrazole]-5'-yl]phénoxy}but-2-énoate,
6-chloro-5'-(5-chloro-1-méthyl-6-oxo-1,6-dihydropyridin-3-yl)-2'-(2-méthoxyphényl)-3'-(propane-2-yl)-1,2,5',6'-tetrahydro-2'H-spiro[indole-3,4'-pyrrolo[3,4-c]pyrazole]-2,6'-dione,
6-chloro-5'-(5-chloro-2-méthylphényl)-2'-(3-méthoxypyridin-4-yl)-3'-(propane-2-yl)-1,2,5',6'-tetrahydro-2'H-spiro[indole-3,4'-pyrrolo[3,4-c]pyrazole]-2,6'-dione,
(3S)-6-chloro-5'-(5-chloro-2-méthylphényl)-2'-(2-méthoxyphényl)-3'-(propane-2-yl)-1,2,5',6'-tetrahydro-2'H-spiro[indole-3,4'-pyrrolo[3,4-c]pyrazole]-2,6'-dione,
6-chloro-5'-(5-chloro-2-méthylphényl)-2'-(3-éthylphényl)-3'-(propane-2-yl)-1,2,5',6'-tetrahydro-2'H-spiro[indole-3,4'-pyrrolo[3,4-c]pyrazole]-2,6'-dione,
6-chloro-5'-(5-chloro-2-méthylphényl)-2'-(2-éthylphényl)-3'-(propane-2-yl)-1,2,5',6'-tetrahydro-2'H-spiro[indole-3,4'-pyrrolo[3,4-c]pyrazole]-2,6'-dione,
6-chloro-5'-(5-chloro-2-méthylphényl)-2'-(2-méthylphényl)-3'-(propane-2-yl)-1,2,5',6'-tetrahydro-2'H-spiro[indole-3,4'-pyrrolo[3,4-c]pyrazole]-2,6'-dione,
6-chloro-5'-(5-chloro-2-méthylphényl)-2'-(3-méthylphényl)-3'-(propane-2-yl)-1,2,5',6'-tetrahydro-2'H-spiro[indole-3,4'-pyrrolo[3,4-c]pyrazole]-2,6'-dione,
6-chloro-5'-(5-chloro-2-méthylphényl)-2'-(2,6-diméthoxyphényl)-3'-(propane-2-yl)-1,2,5',6'-tetrahydro-2'H-spiro[indole-3,4'-pyrrolo[3,4-c]pyrazole]-2,6'-dione,
6-chloro-5'-(5-chloro-2-méthylpyridin-3-yl)-2'-(2-méthoxyphényl)-3'-(propane-2-yl)-1,2,5',6'-tetrahydro-2'H-spiro[indole-3,4'-pyrrolo[3,4-c]pyrazole]-2,6'-dione,
6-chloro-5'-(5-chloropyridin-3-yl)-2'-(2-méthoxyphényl)-3'-(propane-2-yl)-1,2,5',6'-tetrahydro-2'H-spiro[indole-3,4'-pyrrolo[3,4-c]pyrazole]-2,6'-dione,
6-chloro-5'-[5-chloro-2-(2-méthanesulfonyléthoxy)phényl]-2'-(2-méthoxyphényl)-3'-(propane-2-yl)-1,2,5',6'-tetrahydro-2'H-spiro[indole-3,4'-pyrrolo[3,4-c]pyrazole]-2,6'-dione,
2'-(1-benzothiophen-3-yl)-6-chloro-5'-(5-chloro-2-méthylphényl)-3'-(propane-2-yl)-1,2,5',6'-tetrahydro-2'H-spiro[indole-3,4'-pyrrolo[3,4-c]pyrazole]-2,6'-dione,
3-[6-chloro-5'-(5-chloro-2-hydroxyphényl)-2,6'-dioxo-3'-(propane-2-yl)-1,2,5',6'-tetrahydro-2'H-spiro[indole-3,4'-pyrrolo[3,4-c]pyrazole]-2'-yl]-4-méthoxybenzamide,

3-[6-chloro-5'-(5-chloro-2-hydroxyphényl)-2,6'-dioxo-3'-(propane-2-yl)-1,2,5',6'-tetrahydro-2'H-spiro[indole-3,4'-pyrrolo[3,4-c]pyrazole]-2'-yl]-4-methoxybenzoic acid,
6-chloro-5'-(5-chloro-2-méthylphényl)-2'-[2-(méthylsulfanyl)phényl]-3'-(propane-2-yl)-1,2,5',6'-tetrahydro-2'H-spiro[indole-3,4'-pyrrolo[3,4-c]pyrazole]-2,6'-dione,
méthyl 3-[6-chloro-5'-(5-chloro-2-méthylphényl)-2,6'-dioxo-3'-(propane-2-yl)-1,2,5',6'-tetrahydro-2'H-spiro[indole-3,4'-pyrrolo[3,4-c]pyrazole]-2'-yl]-4-méthoxybenzoate,
méthyl 3-[6-chloro-5'-(5-chloro-2-hydroxyphényl)-2,6'-dioxo-3'-(propane-2-yl)-1,2,5',6'-tetrahydro-2'H-spiro[indole-3,4'-pyrrolo[3,4-c]pyrazole]-2'-yl]-4-méthoxybenzoate,
(3S)-6-chloro-5'-(5-chloro-2-méthylpyridin-3-yl)-2'-(2-méthoxyphényl)-3'-(propane-2-yl)-1,2,5',6'-tetrahydro-2'H-spiro[indole-3,4'-pyrrolo[3,4-c]pyrazole]-2,6'-dione,
(3S)-6-chloro-5'-(5-chloropyridin-3-yl)-2'-(2-méthoxyphényl)-3'-(propane-2-yl)-1,2,5',6'-tetrahydro-2'H-spiro[indole-3,4'-pyrrolo[3,4-c]pyrazole]-2,6'-dione,
6-chloro-5'-(4-chloropyridin-2-yl)-3'-(propane-2-yl)-1,2,5',6'-tetrahydro-2'H-spiro[indole-3,4'-pyrrolo[3,4-c]pyrazole]-2,6'-dione,
6-chloro-5'-(5-chloropyridin-3-yl)-3'-(propane-2-yl)-1,2,5',6'-tetrahydro-2'H-spiro[indole-3,4'-pyrrolo[3,4-c]pyrazole]-2,6'-dione,
6-chloro-5'-(5-chloro-2-méthylpyridin-3-yl)-3'-(propane-2-yl)-1,2,5',6'-tetrahydro-2'H-spiro[indole-3,4'-pyrrolo[3,4-c]pyrazole]-2,6'-dione,
6-chloro-5'-(5-chloro-2-méthylphényl)-3'-éthyl-2'-(2-éthylphényl)-1,2,5',6'-tetrahydro-2'H-spiro[indole-3,4'-pyrrolo[3,4-c]pyrazole]-2,6'-dione,
6-chloro-5'-(cyclobutylméthyl)-2'-(2-méthoxyphényl)-3'-(propane-2-yl)-1,2,5',6'-tetrahydro-2'H-spiro[indole-3,4'-pyrrolo[3,4-c]pyrazole]-2,6'-dione,
6-chloro-5'-(2,2-diméthylpropyl)-2'-(2-méthoxyphényl)-3'-(propane-2-yl)-1,2,5',6'-tetrahydro-2'H-spiro[indole-3,4'-pyrrolo[3,4-c]pyrazole]-2,6'-dione,
(3S)-6-chloro-5'-(5-chloropyridin-3-yl)-2'-(2-méthoxyphényl)-3'-(propane-2-yl)-1,2,5',6'-tetrahydro-2'H-spiro[indole-3,4'-pyrrolo[3,4-c]pyrazole]-2,6'-dione,
6-chloro-2'-(3-chlorophényl)-5'-(2-méthoxyphényl)-6'-(propane-2-yl)-1,2,3',5'-tetrahydro-2'H-spiro[indole-3,1'-pyrrolo[3,4-c]pyrrole]-2,3'-dione,
6-chloro-2'-(5-chloro-2-nitrophényl)-5'-(2-méthoxyphényl)-6'-(propane-2-yl)-1,2,3',5'-tetrahydro-2'H-spiro[indole-3,1'-pyrrolo[3,4-c]pyrrole]-2,3'-dione,
2'-(2-amino-5-chlorophényl)-6-chloro-5'-(2-méthoxyphényl)-6'-(propane-2-yl)-1,2,3',5'-tetrahydro-2'H-spiro[indole-3,1'-pyrrolo[3,4-c]pyrrole]-2,3'-dione,
N-{4-chloro-2-[6-chloro-5'-(2-méthoxyphényl)-2,3'-dioxo-6'-(propane-2-yl)-1,2,3',5'-tetrahydro-2'H-spiro[indole-3,1'-pyrrolo[3,4-c]pyrrole]-2'-yl]phényl}methanesulfonamide,
6-chloro-2'-(5-chloro-2-méthylphényl)-5'-(2,4-diméthoxyphényl)-6'-(propane-2-yl)-1,2,3',5'-tetrahydro-2'H-spiro[indole-3,1'-pyrrolo[3,4-c]pyrrole]-2,3'-dione,
5'-(2H-1,3-benzodioxol-4-yl)-6-chloro-2'-(5-chloro-2-méthylphényl)-6'-(propane-2-yl)-1,2,3',5'-tetrahydro-2'H-spiro[indole-3,1'-pyrrolo[3,4-c]pyrrole]-2,3'-dione,
3-[6-chloro-2'-(5-chloro-2-méthylphényl)-2,3'-dioxo-6'-(propane-2-yl)-1,2,3',5'-tetrahydro-2'H-spiro[indole-3,1'-pyrrolo[3,4-c]pyrrole]-5'-yl]-4-méthoxybenzamide,
5'-(5-amino-2-méthoxyphényl)-6-chloro-2'-(5-chloro-2-méthylphényl)-6'-(propane-2-yl)-1,2,3',5'-tetrahydro-2'H-spiro[indole-3,1'-pyrrolo[3,4-c]pyrrole]-2,3'-dione,
N-{3-[6-chloro-2'-(5-chloro-2-méthylphényl)-2,3'-dioxo-6'-(propane-2-yl)-1,2,3',5'-tetrahydro-2'H-spiro[indole-3,1'-pyrrolo[3,4-c]pyrrole]-5'-yl]-4-méthoxyphényl}acetamide,
3-[6-chloro-2'-(5-chloro-2-méthylphényl)-2,3'-dioxo-6'-(propane-2-yl)-1,2,3',5'-tetrahydro-2'H-spiro[indole-3,1'-pyrrolo[3,4-c]pyrrole]-5'-yl]-N,N-diéthyl-4-méthoxybenzène-1-sulfonamide,
4-chloro-2-[6-chloro-5'-(2-méthoxyphényl)-2,3'-dioxo-6'-(propane-2-yl)-1,2,3',5'-tetrahydro-2'H-spiro[indole-3,1'-pyrrolo[3,4-c]pyrrole]-2'-yl]benzamide,
3-[6-chloro-2'-(5-chloro-2-méthylphényl)-2,3'-dioxo-6'-(propane-2-yl)-1,2,3',5'-tetrahydro-2'H-spiro[indole-3,1'-pyrrolo[3,4-c]pyrrole]-5'-yl]-N-(2-hydroxyéthyl)-4-méthoxybenzamide,
6-chloro-2'-(5-chloro-2-méthylphényl)-5'-(6-methoxy-2-oxo-2,3-dihydro-1H-1,3-benzodiazol-5-yl)-6'-(propane-2-yl)-1,2,3',5'-tetrahydro-2'H-spiro[indole-3,1'-pyrrolo[3,4-c]pyrrole]-2,3'-dione,
N-{4-chloro-2-[(3S)-6-chloro-5'-(2-méthoxyphényl)-2,3'-dioxo-6'-(propane-2-yl)-1,2,3',5'-tetrahydro-2'H-spiro[indole-3,1'-pyrrolo[3,4-c]pyrrole]-2'-yl]phényl}methanesulphonamide,
(3S)-6-chloro-2'-(5-chloro-2-méthylphényl)-5'-[(3S,4R)-3-methoxypiperidin-4-yl]-6'-(propane-2-yl)-1,2,3',5'-tetrahydro-2'H-spiro[indole-3,1'-pyrrolo[3,4-c]pyrrole]-2,3'-dione,
6-chloro-2'-(5-chloro-2-méthylpyridin-3-yl)-5'-(2,4-diméthoxyphényl)-6'-(propane-2-yl)-1,2,3',5'-tetrahydro-2'H-spiro[indole-3,1'-pyrrolo[3,4-c]pyrrole]-2,3'-dione,

4-chloro-2-[6-chloro-5'-(2-méthoxyphényl)-2,3'-dioxo-6'-(propane-2-yl)-1,2,3',5'-tetrahydro-2'H-spiro[indole-3,1'-pyrrolo[3,4-c]pyrrole]-2'-yl]benzoic acid,
éthyl 4-chloro-2-[6-chloro-5'-(2-méthoxyphényl)-2,3'-dioxo-6'-(propane-2-yl)-1,2,3',5'-tetrahydro-2'H-spiro[indole-3,1'-pyrrolo[3,4-c]pyrrole]-2'-yl]benzoate,
(3S)-6-chloro-2'-(5-chloro-2-méthylphényl)-5'-(2,4-diméthoxyphényl)-6'-(propane-2-yl)-1,2,3',5'-tetrahydro-2'H-spiro[indole-3,1'-pyrrolo[3,4-c]pyrrole]-2,3'-dione,
N-{3-[(3S)-6-chloro-2'-(5-chloro-2-méthylphényl)-2,3'-dioxo-6'-(propane-2-yl)-1,2,3',5'-tetrahydro-2'H-spiro[indole-3,1'-pyrrolo[3,4-c]pyrrole]-5'-yl]-4-méthoxyphényl}acetamide,
(3S)-5'-(5-amino-2-méthoxyphényl)-6-chloro-2'-(5-chloro-2-méthylphényl)-6'-(propane-2-yl)-1,2,3',5'-tetrahydro-2'H-spiro[indole-3,1'-pyrrolo[3,4-c]pyrrole]-2,3'-dione,
3-[(3S)-6-chloro-2'-(5-chloro-2-méthylphényl)-2,3'-dioxo-6'-(propane-2-yl)-1,2,3',5'-tetrahydro-2'H-spiro[indole-3,1'-pyrrolo[3,4-c]pyrrole]-5'-yl]-4-méthoxybenzamide,
(3S)-6-chloro-2'-(5-chloro-2-méthylpyridin-3-yl)-5'-(2,4-diméthoxyphényl)-6'-(propane-2-yl)-1,2,3',5'-tetrahydro-2'H-spiro[indole-3,1'-pyrrolo[3,4-c]pyrrole]-2,3'-dione,
6-chloro-2'-(5-chloro-2-méthylphényl)-5'-[2-methoxy-5-(morpholine-4-sulfonyl)phényl]-6'-(propane-2-yl)-1,2,3',5'-tetrahydro-2'H-spiro[indole-3,1'-pyrrolo[3,4-c]pyrrole]-2,3'-dione,
6-chloro-2'-(5-chloro-2-méthylphényl)-5'-(2-methoxythiophen-3-yl)-6'-(propane-2-yl)-1,2,3',5'-tetrahydro-2'H-spiro[indole-3,1'-pyrrolo[3,4-c]pyrrole]-2,3'-dione, et
6-chloro-5'-(5-chloro-2-méthylphényl)-2'-(2,6-diméthoxypyridin-3-yl)-3'-(propan-2-yl)-1,2,5',6'-tetrahydro-2'H-spiro[indole-3,4'-pyrrolo[3,4-c]pyrazole]-2,6'-dione.

**16.** Le composé selon la revendication15 choisis dans le groupe suivant :

(3S)-6-chloro-2'-(5-chloro-2-méthylphényl)-5'-(2,4-diméthoxyphényl)-6'-(propane-2-yl)-1,2,3',5'-tetrahydro-2'H-spiro[indole-3,1'-pyrrolo[3,4-c]pyrrole]-2,3'-dione,
N-{3-[(3S)-6-chloro-2'-(5-chloro-2-méthylphényl)-2,3'-dioxo-6'-(propane-2-yl)-1,2,3',5'-tetrahydro-2'H-spiro[indole-3,1'-pyrrolo[3,4-c]pyrrole]-5'-yl]-4-méthoxyphényl}acetamide,
(3S)-5'-(5-amino-2-méthoxyphényl)-6-chloro-2'-(5-chloro-2-méthylphényl)-6'-(propane-2-yl)-1,2,3',5'-tetrahydro-2'H-spiro[indole-3,1'-pyrrolo[3,4-c]pyrrole]-2,3'-dione,
3-[(3S)-6-chloro-2'-(5-chloro-2-méthylphényl)-2,3'-dioxo-6'-(propane-2-yl)-1,2,3',5'-tetrahydro-2'H-spiro[indole-3,1'-pyrrolo[3,4-c]pyrrole]-5'-yl]-4-méthoxybenzamide,
(3S)-6-chloro-2'-(5-chloro-2-méthylpyridin-3-yl)-5'-(2,4-diméthoxyphényl)-6'-(propane-2-yl)-1,2,3',5'-tetrahydro-2'H-spiro[indole-3,1'-pyrrolo[3,4-c]pyrrole]-2,3'-dione,
(3S)-6-chloro-2'-(5-chloro-2-méthylphényl)-5'-[(3S,4R)-3-methoxypiperidin-4-yl]-6'-(propane-2-yl)-1,2,3',5'-tetrahydro-2'H-spiro[indole-3,1'-pyrrolo[3,4-c]pyrrole]-2,3'-dione,
(3S)-6-chloro-5'-(5-chloropyridin-3-yl)-2'-(2-méthoxyphényl)-3'-(propane-2-yl)-1,2,5',6'-tetrahydro-2'H-spiro[indole-3,4'-pyrrolo[3,4-c]pyrazole]-2,6'-dione,
(3S)-6-chloro-5'-(5-chloro-2-méthylpyridin-3-yl)-2'-(2-méthoxyphényl)-3'-(propane-2-yl)-1,2,5',6'-tetrahydro-2'H-spiro[indole-3,4'-pyrrolo[3,4-c]pyrazole]-2,6'-dione,
(3S)-6-chloro-5'-(5-chloropyridin-3-yl)-2'-(2-méthoxyphényl)-3'-(propane-2-yl)-1,2,5',6'-tetrahydro-2'H-spiro[indole-3,4'-pyrrolo[3,4-c]pyrazole]-2,6'-dione, et
(3S)-6-chloro-5'-(5-chloro-2-méthylphényl)-2'-(2-méthoxyphényl)-3'-(propane-2-yl)-1,2,5',6'-tetrahydro-2'H-spiro[indole-3,4'-pyrrolo[3,4-c]pyrazole]-2,6'-dione.

**17.** Le composé selon la revendication15, qui est le (3S)-6-chloro-1'-(3-chlorophényl)-3'-[(4-chlorophényl)sulfanyl]-4'-hydroxy-1,1',2,5'-tetrahydrospiro[indole-3,2'-pyrrole]-2,5'-dione.

**18.** Le composé selon la revendication15 choisis dans le groupe suivant :

6-chloro-5'-(5-chloro-2-méthylphényl)-3'-(1-méthylcyclopropyl)-1,2,5',6'-tetrahydro-2'H-spiro[indole-3,4'-pyrrolo[3,4-c]pyrazole]-2,6'-dione,
6-chloro-5'-(3-chlorophényl)-3'-(1-méthylcyclopropyl)-1,2,5',6'-tetrahydro-2'H-spiro[indole-3,4'-pyrrolo[3,4-c]pyrazole]-2,6'-dione,
3'-tert-butyl-6-chloro-5'-(3-chlorophényl)-1,2,5',6'-tetrahydro-2'H-spiro[indole-3,4'-pyrrolo[3,4-c]pyrazole]-2,6'-dione,
3'-tert-butyl-6-chloro-5'-(5-chloro-2-méthoxyphényl)-1,2,5',6'-tetrahydro-2'H-spiro[indole-3,4'-pyrrolo[3,4-c]pyrazole]-2,6'-dione,
6-chloro-5'-(5-chloro-2-méthoxyphényl)-3'-(1-méthylcyclopropyl)-1,2,5',6'-tetrahydro-2'H-spiro[indole-3,4'-pyrrolo[3,4-c]pyrazole]-2,6'-dione,
6-chloro-5'-(5-chloro-2-méthylphényl)-3'-(propane-2-yl)-1,2,5',6'-tetrahydro-2'H-spiro[indole-3,4'-pyrrolo[3,4-c]pyrazole]-2,6'-dione,
6-chloro-5'-(5-chloro-2-hydroxyphényl)-3'-(propane-2-yl)-1,2,5',6'-tetrahydro-2'H-spiro[indole-3,4'-pyrrolo[3,4-c]pyrazole]-2,6'-dione,
6-chloro-5'-(5-chloro-2-méthoxyphényl)-3'-(propane-2-yl)-1,2,5',6'-tetrahydro-2'H-spiro[indole-3,4'-pyrrolo[3,4-c]pyrazole]-2,6'-dione,
6-chloro-5'-(3-chlorophényl)-2'-(2-méthoxyphényl)-3'-(1-méthylcyclopropyl)-1,2,5',6'-tetrahydro-2'H-spiro[indole-3,4'-pyrrolo[3,4-c]pyrazole]-2,6'-dione,
6-chloro-5'-(3-chlorophényl)-2'-(2-méthoxyphényl)-3'-(propane-2-yl)-1,2,5',6'-tetrahydro-2'H-spiro[indole-3,4'-pyrrolo[3,4-c]pyrazole]-2,6'-dione,
6-chloro-5'-(3-chlorophényl)-3'-(1-méthylcyclopropyl)-2'-(pyrrolidin-2-ylméthyl)-1,2,5',6'-tetrahydro-2'H-spiro[indole-3,4'-pyrrolo[3,4-c]pyrazole]-2,6'-dione,
2-[6-chloro-5'-(3-chlorophényl)-3'-(1-méthylcyclopropyl)-2,6'-dioxo-1,2,5',6'-tetrahydro-2'H-spiro[indole-3,4'-pyrrolo[3,4-c]pyrazole]-2'-ylméthyl]-N,N-diméthylcyclopentane-1-carboxamide,
6-chloro-5'-(5-chloro-2-méthoxyphényl)-2'-(2-méthoxyphényl)-3'-(propane-2-yl)-1,2,5',6'-tetrahydro-2'H-spiro[indole-3,4'-pyrrolo[3,4-c]pyrazole]-2,6'-dione,
6-chloro-5'-(5-chloro-2-méthylphényl)-2'-(2-méthoxyphényl)-3'-(propane-2-yl)-1,2,5',6'-tetrahydro-2'H-spiro[indole-3,4'-pyrrolo[3,4-c]pyrazole]-2,6'-dione,
6-chloro-5'-(5-chloro-2-méthylphényl)-1'-(2-méthoxyphényl)-3'-(propane-2-yl)-1,2,5',6'-tetrahydro-1'H-spiro[indole-3,4'-pyrrolo[3,4-c]pyrazole]-2,6'-dione, et
2-[6-chloro-5'-(3-chlorophényl)-2,6'-dioxo-3'-(propane-2-yl)-1,2,5',6'-tetrahydro-2'H-spiro[indole-3,4'-pyrrolo[3,4-c]pyrazole]-2'-ylméthyl]-N,N-diméthylpyrrolidine-1-carboxamide.

**19.** Le composé selon la revendication 15 choisis dans le suivant :

méthyl (2E)-4-{4-chloro-2-[6-chloro-2'-(2-méthoxyphényl)-2,6'-dioxo-3'-(propane-2-yl)-1,2,5',6'-tetrahydro-2'H-spiro[indole-3,4'-pyrrolo[3,4-c]pyrazole]-5'-yl]phénoxy}but-2-énoate,
6-chloro-5'-(5-chloro-1-méthyl-6-oxo-1,6-dihydropyridin-3-yl)-2'-(2-méthoxyphényl)-3'-(propane-2-yl)-1,2,5',6'-tetrahydro-2'H-spiro[indole-3,4'-pyrrolo[3,4-c]pyrazole]-2,6'-dione,
6-chloro-5'-(5-chloro-2-méthylphényl)-2'-(3-méthoxypyridin-4-yl)-3'-(propane-2-yl)-1,2,5',6'-tetrahydro-2'H-spiro[indole-3,4'-pyrrolo[3,4-c]pyrazole]-2,6'-dione,
(3S)-6-chloro-5'-(5-chloro-2-méthylphényl)-2'-(2-méthoxyphényl)-3'-(propane-2-yl)-1,2,5',6'-tetrahydro-2'H-spiro[indole-3,4'-pyrrolo[3,4-c]pyrazole]-2,6'-dione,
6-chloro-5'-(5-chloro-2-méthylphényl)-2'-(3-éthylphényl)-3'-(propane-2-yl)-1,2,5',6'-tetrahydro-2'H-spiro[indole-3,4'-pyrrolo[3,4-c]pyrazole]-2,6'-dione,
6-chloro-5'-(5-chloro-2-méthylphényl)-2'-(2-éthylphényl)-3'-(propane-2-yl)-1,2,5',6'-tetrahydro-2'H-spiro[indole-3,4'-pyrrolo[3,4-c]pyrazole]-2,6'-dione,
6-chloro-5'-(5-chloro-2-méthylphényl)-2'-(2-méthylphényl)-3'-(propane-2-yl)-1,2,5',6'-tetrahydro-2'H-spiro[indole-3,4'-pyrrolo[3,4-c]pyrazole]-2,6'-dione,
6-chloro-5'-(5-chloro-2-méthylphényl)-2'-(3-méthylphényl)-3'-(propane-2-yl)-1,2,5',6'-tetrahydro-2'H-spiro[indole-3,4'-pyrrolo[3,4-c]pyrazole]-2,6'-dione,
6-chloro-5'-(5-chloro-2-méthylphényl)-2'-(2,6-diméthoxyphényl)-3'-(propane-2-yl)-1,2,5',6'-tetrahydro-2'H-spiro[indole-3,4'-pyrrolo[3,4-c]pyrazole]-2,6'-dione,
6-chloro-5'-(5-chloro-2-méthylpyridin-3-yl)-2'-(2-méthoxyphényl)-3'-(propane-2-yl)-1,2,5',6'-tetrahydro-2'H-spiro[indole-3,4'-pyrrolo[3,4-c]pyrazole]-2,6'-dione,
6-chloro-5'-(5-chloropyridin-3-yl)-2'-(2-méthoxyphényl)-3'-(propane-2-yl)-1,2,5',6'-tetrahydro-2'H-spiro[indole-3,4'-pyrrolo[3,4-c]pyrazole]-2,6'-dione,
6-chloro-5'-[5-chloro-2-(2-méthanesulfonyléthoxy)phényl]-2'-(2-méthoxyphényl)-3'-(propane-2-yl)-1,2,5',6'-tetrahydro-2'H-spiro[indole-3,4'-pyrrolo[3,4-c]pyrazole]-2,6'-dione,
2'-(1-benzothiophen-3-yl)-6-chloro-5'-(5-chloro-2-méthylphényl)-3'-(propane-2-yl)-1,2,5',6'-tetrahydro-2'H-spiro[indole-3,4'-pyrrolo[3,4-c]pyrazole]-2,6'-dione,
3-[6-chloro-5'-(5-chloro-2-hydroxyphényl)-2,6'-dioxo-3'-(propane-2-yl)-1,2,5',6'-tetrahydro-2'H-spiro[indole-3,4'-pyrrolo[3,4-c]pyrazole]-2'-yl]-4-méthoxybenzamide,
3-[6-chloro-5'-(5-chloro-2-hydroxyphényl)-2,6'-dioxo-3'-(propane-2-yl)-1,2,5',6'-tetrahydro-2'H-spiro[indole-3,4'-pyrrolo[3,4-c]pyrazole]-2'-yl]-4-methoxybenzoic acid
6-chloro-5'-(5-chloro-2-méthylphényl)-2'-[2-(méthylsulfanyl)phényl]-3'-(propane-2-yl)-1,2,5',6'-tetrahydro-2'H-spiro[indole-3,4'-pyrrolo[3,4-c]pyrazole]-2,6'-dione,

méthyl 3-[6-chloro-5’-(5-chloro-2-méthylphényl)-2,6’-dioxo-3’-(propane-2-yl)-1,2,5’,6’-tetrahydro-2’H-spiro[indole-3,4’-pyrrolo[3,4-c]pyrazole]-2’-yl]-4-méthoxybenzoate,
méthyl 3-[6-chloro-5’-(5-chloro-2-hydroxyphényl)-2,6’-dioxo-3’-(propane-2-yl)-1,2,5’,6’-tetrahydro-2’H-spiro[indole-3,4’-pyrrolo[3,4-c]pyrazole]-2’-yl]-4-méthoxybenzoate,
(3S)-6-chloro-5’-(5-chloro-2-méthylpyridin-3-yl)-2’-(2-méthoxyphényl)-3’-(propane-2-yl)-1,2,5’,6’-tetrahydro-2’H-spiro[indole-3,4’-pyrrolo[3,4-c]pyrazole]-2,6’-dione,
(3S)-6-chloro-5’-(5-chloropyridin-3-yl)-2’-(2-méthoxyphényl)-3’-(propane-2-yl)-1,2,5’,6’-tetrahydro-2’H-spiro[indole-3,4’-pyrrolo[3,4-c]pyrazole]-2,6’-dione,
6-chloro-5’-(4-chloropyridin-2-yl)-3’-(propane-2-yl)-1,2,5’,6’-tetrahydro-2’H-spiro[indole-3,4’-pyrrolo[3,4-c]pyrazole]-2,6’-dione,
6-chloro-5’-(5-chloropyridin-3-yl)-3’-(propane-2-yl)-1,2,5’,6’-tetrahydro-2’H-spiro[indole-3,4’-pyrrolo[3,4-c]pyrazole]-2,6’-dione,
6-chloro-5’-(5-chloro-2-méthylpyridin-3-yl)-3’-(propane-2-yl)-1,2,5’,6’-tetrahydro-2’H-spiro[indole-3,4’-pyrrolo[3,4-c]pyrazole]-2,6’-dione
6-chloro-5’-(5-chloro-2-méthylphényl)-3’-éthyl-2’-(2-éthylphényl)-1,2,5’,6’-tetrahydro-2’H-spiro[indole-3,4’-pyrrolo[3,4-c]pyrazole]-2,6’-dione,
6-chloro-5’-(cyclobutylméthyl)-2’-(2-méthoxyphényl)-3’-(propane-2-yl)-1,2,5’,6’-tetrahydro-2’H-spiro[indole-3,4’-pyrrolo[3,4-c]pyrazole]-2,6’-dione,
6-chloro-5’-(2,2-diméthylpropyl)-2’-(2-méthoxyphényl)-3’-(propane-2-yl)-1,2,5’,6’-tetrahydro-2’H-spiro[indole-3,4’-pyrrolo[3,4-c]pyrazole]-2,6’-dione,
(3S)-6-chloro-5’-(5-chloropyridin-3-yl)-2’-(2-méthoxyphényl)-3’-(propane-2-yl)-1,2,5’,6’-tetrahydro-2’H-spiro[indole-3,4’-pyrrolo[3,4-c]pyrazole]-2,6’-dione,
6-chloro-2’-(3-chlorophényl)-5’-(2-méthoxyphényl)-6’-(propane-2-yl)-1,2,3’,5’-tetrahydro-2’H-spiro[indole-3,1’-pyrrolo[3,4-c]pyrrole]-2,3’-dione,
6-chloro-2’-(5-chloro-2-nitrophényl)-5’-(2-méthoxyphényl)-6’-(propane-2-yl)-1,2,3’,5’-tetrahydro-2’H-spiro[indole-3,1’-pyrrolo[3,4-c]pyrrole]-2,3’-dione,
2’-(2-amino-5-chlorophényl)-6-chloro-5’-(2-méthoxyphényl)-6’-(propane-2-yl)-1,2,3’,5’-tetrahydro-2’H-spiro[indole-3,1’-pyrrolo[3,4-c]pyrrole]-2,3’-dione,
N-{4-chloro-2-[6-chloro-5’-(2-méthoxyphényl)-2,3’-dioxo-6’-(propane-2-yl)-1,2,3’,5’-tetrahydro-2’H-spiro[indole-3,1’-pyrrolo[3,4-c]pyrrole]-2’-yl]phényl}methanesulfonamide,
6-chloro-2’-(5-chloro-2-méthylphényl)-5’-(2,4-diméthoxyphényl)-6’-(propane-2-yl)-1,2,3’,5’-tetrahydro-2’H-spiro[indole-3,1’-pyrrolo[3,4-c]pyrrole]-2,3’-dione,
5’-(2H-1,3-benzodioxol-4-yl)-6-chloro-2’-(5-chloro-2-méthylphényl)-6’-(propane-2-yl)-1,2,3’,5’-tetrahydro-2’H-spiro[indole-3,1’-pyrrolo[3,4-c]pyrrole]-2,3’-dione,
3-[6-chloro-2’-(5-chloro-2-méthylphényl)-2,3’-dioxo-6’-(propane-2-yl)-1,2,3’,5’-tetrahydro-2’H-spiro[indole-3,1’-pyrrolo[3,4-c]pyrrole]-5’-yl]-4-méthoxybenzamide,
5’-(5-amino-2-méthoxyphényl)-6-chloro-2’-(5-chloro-2-méthylphényl)-6’-(propane-2-yl)-1,2,3’,5’-tetrahydro-2’H-spiro[indole-3,1’-pyrrolo[3,4-c]pyrrole]-2,3’-dione,
N-{3-[6-chloro-2’-(5-chloro-2-méthylphényl)-2,3’-dioxo-6’-(propane-2-yl)-1,2,3’,5’-tetrahydro-2’H-spiro[indole-3,1’-pyrrolo[3,4-c]pyrrole]-5’-yl]-4-méthoxyphényl}acetamide,
3-[6-chloro-2’-(5-chloro-2-méthylphényl)-2,3’-dioxo-6’-(propane-2-yl)-1,2,3’,5’-tetrahydro-2’H-spiro[indole-3,1’-pyrrolo[3,4-c]pyrrole]-5’-yl]-N,N-diéthyl-4-méthoxybenzène-1-sulfonamide,
4-chloro-2-[6-chloro-5’-(2-méthoxyphényl)-2,3’-dioxo-6’-(propane-2-yl)-1,2,3’,5’-tetrahydro-2’H-spiro[indole-3,1’-pyrrolo[3,4-c]pyrrole]-2’-yl]benzamide,
3-[6-chloro-2’-(5-chloro-2-méthylphényl)-2,3’-dioxo-6’-(propane-2-yl)-1,2,3’,5’-tetrahydro-2’H-spiro[indole-3,1’-pyrrolo[3,4-c]pyrrole]-5’-yl]-N-(2-hydroxyéthyl)-4-méthoxybenzamide,
6-chloro-2’-(5-chloro-2-méthylphényl)-5’-(6-methoxy-2-oxo-2,3-dihydro-1H-1,3-benzodiazol-5-yl)-6’-(propane-2-yl)-1,2,3’,5’-tetrahydro-2’H-spiro[indole-3,1’-pyrrolo[3,4-c]pyrrole]-2,3’-dione,
N-{4-chloro-2-[(3S)-6-chloro-5’-(2-méthoxyphényl)-2,3’-dioxo-6’-(propane-2-yl)-1,2,3’,5’-tetrahydro-2’H-spiro[indole-3,1’-pyrrolo[3,4-c]pyrrole]-2’-yl]phényl}methanesulphonamide,
(3S)-6-chloro-2’-(5-chloro-2-méthylphényl)-5’-[(3S,4R)-3-methoxypiperidin-4-yl]-6’-(propane-2-yl)-1,2,3’,5’-tetrahydro-2’H-spiro[indole-3,1’-pyrrolo[3,4-c]pyrrole]-2,3’-dione,
6-chloro-2’-(5-chloro-2-méthylpyridin-3-yl)-5’-(2,4-diméthoxyphényl)-6’-(propane-2-yl)-1,2,3’,5’-tetrahydro-2’H-spiro[indole-3,1’-pyrrolo[3,4-c]pyrrole]-2,3’-dione,
4-chloro-2-[6-chloro-5’-(2-méthoxyphényl)-2,3’-dioxo-6’-(propane-2-yl)-1,2,3’,5’-tetrahydro-2’H-spiro[indole-3,1’-pyrrolo[3,4-c]pyrrole]-2’-yl]benzoic acid,
éthyl 4-chloro-2-[6-chloro-5’-(2-méthoxyphényl)-2,3’-dioxo-6’-(propane-2-yl)-1,2,3’,5’-tetrahydro-2’H-spiro[indole-3,1’-pyrrolo[3,4-c]pyrrole]-2’-yl]benzoate,

(3S)-6-chloro-2'-(5-chloro-2-méthylphényl)-5'-(2,4-diméthoxyphényl)-6'-(propane-2-yl)-1,2,3',5'-tetrahydro-2'H-spiro[indole-3,1'-pyrrolo[3,4-c]pyrrole]-2,3'-dione,
N-{3-[(3S)-6-chloro-2'-(5-chloro-2-méthylphényl)-2,3'-dioxo-6'-(propane-2-yl)-1,2,3',5'-tetrahydro-2'H-spiro[indole-3,1'-pyrrolo[3,4-c]pyrrole]-5'-yl]-4-méthoxyphényl}acetamide,
(3S)-5'-(5-amino-2-méthoxyphényl)-6-chloro-2'-(5-chloro-2-méthylphényl)-6'-(propane-2-yl)-1,2,3',5'-tetrahydro-2'H-spiro[indole-3,1'-pyrrolo[3,4-c]pyrrole]-2,3'-dione,
3-[(3S)-6-chloro-2'-(5-chloro-2-méthylphényl)-2,3'-dioxo-6'-(propane-2-yl)-1,2,3',5'-tetrahydro-2'H-spiro[indole-3,1'-pyrrolo[3,4-c]pyrrole]-5'-yl]-4-méthoxybenzamide,
(3S)-6-chloro-2'-(5-chloro-2-méthylpyridin-3-yl)-5'-(2,4-diméthoxyphényl)-6'-(propane-2-yl)-1,2,3',5'-tetrahydro-2'H-spiro[indole-3,1'-pyrrolo[3,4-c]pyrrole]-2,3'-dione,
6-chloro-2'-(5-chloro-2-méthylphényl)-5'-[2-methoxy-5-(morpholine-4-sulfonyl)phényl]-6'-(propane-2-yl)-1,2,3',5'-tetrahydro-2'H-spiro[indole-3,1'-pyrrolo[3,4-c]pyrrole]-2,3'-dione,
6-chloro-2'-(5-chloro-2-méthylphényl)-5'-(2-methoxythiophen-3-yl)-6'-(propane-2-yl)-1,2,3',5'-tetrahydro-2'H-spiro[indole-3,1'-pyrrolo[3,4-c]pyrrole]-2,3'-dione, et
6-chloro-5'-(5-chloro-2-méthylphényl)-2'-(2,6-diméthoxypyridin-3-yl)-3'-(propan-2-yl)-1,2,5',6'-tetrahydro-2'H-spiro[indole-3,4'-pyrrolo[3,4-c]pyrazole]-2,6'-dione.

**20.** Un composé selon l'une quelconque des revendications 1 à 19 pour utilisation comme médicament.

**21.** Le composé pour utilisation selon la revendication 20, pour utilisation dans une méthode de prévention et/ou le traitement de maladies, choisis dans le groupe consistant en le cancer, les maladies immunitaires, les états inflammatoires, les maladies allergiques de la peau associées à une prolifération excessive et des infections virales.

**22.** Une composition pharmaceutique comprenant comme l'agent actif un composé tel que défini dans l'une quelconque des revendications 1 à 19, en combinaison avec au moins un excipient pharmaceutiquement acceptable.

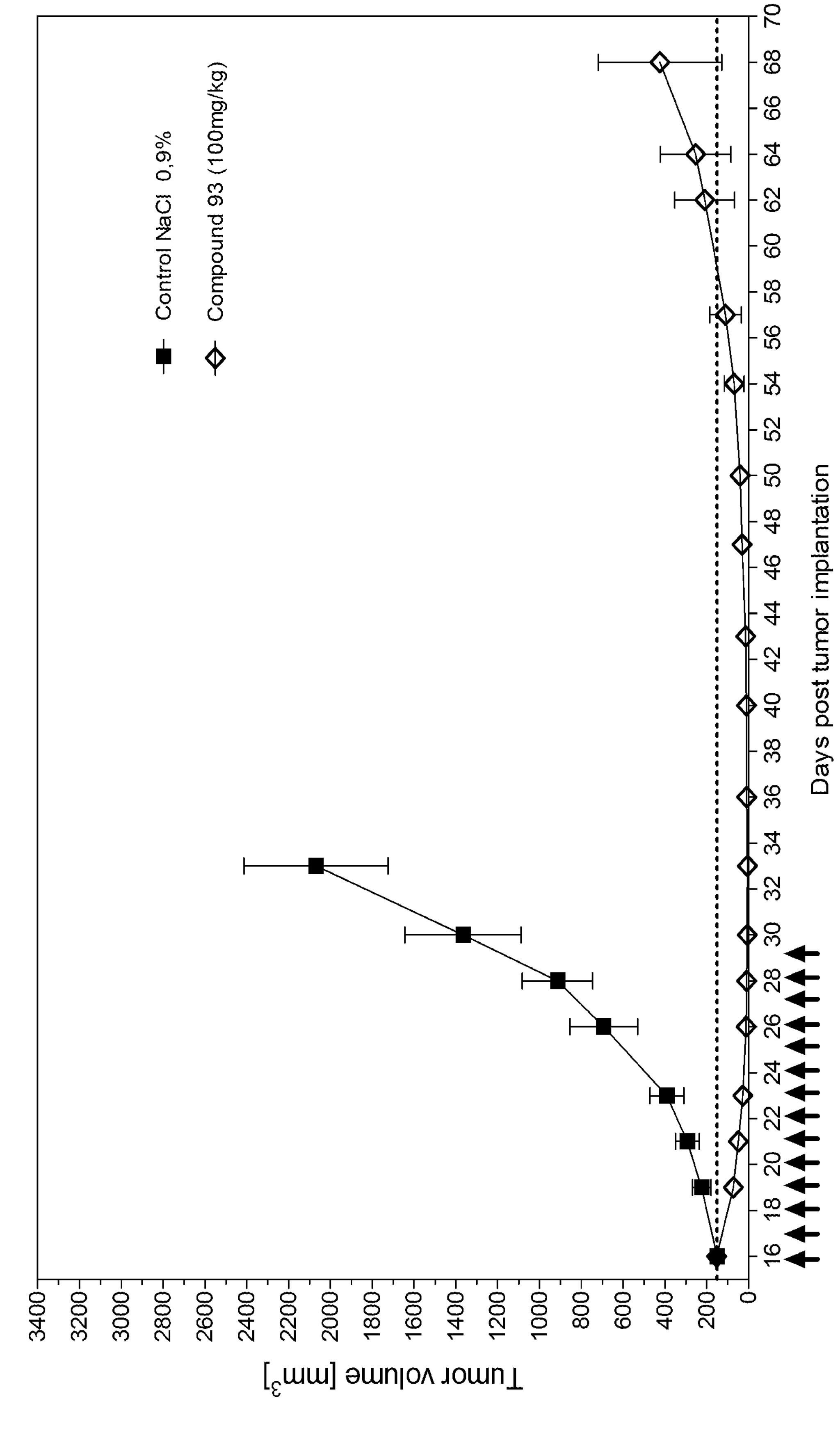
Tumor volume [mm3]
0
200
400
600
800
1000
1200
1400
1600
1800
2000
2200
2400
2600
2800
3000
3200
3400
Days post tumor implantation
16
18
20
22
24
26
28
30
32
34
36
38
40
42
44
46
48
50
52
54
56
58
60
62
64
66
68
70
Control NaCl 0,9%
Compound 93 (100mg/kg)

Fig. 1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2008060789 A
- WO 2008046049 A
- WO 2006110917 A
- WO 2012155066 A
- WO 2012121361 A
- WO 2011134925 A
- EP 2005957 A1
- US 200313656 A1, Wang, B.


### Non-patent literature cited in the description


- **V.L.GEIN et al.** *Chemistry of Heterocyclic Compounds,* 2008, vol. 44 (5), 626-627
- **ZHANG, J. et al.** *Bioorganic & Medicinal Chemistry Letters,* 2000, vol. 10, 2575-2578
- **PEI, Y. et al.** *Tetrahedron Letters,* 1993, vol. 34, 7509-7512
- **NAGARAPU, L. et al.** *European Journal of Medicinal Chemistry,* 2010, vol. 45, 4720-4725
- **SKINNER, PH. J. et al.** *Bioorganic and Medicinal Chemistry Letters,* 2007, vol. 17, 5620-5623
- **BALDUCCI, D.** *Tetrahedron,* 2012, vol. 68, 7374-7379
- **KIDWAI, M. ; MISHRAIN, N. K.** *Green Chemistry - Environmentally Benign Approaches, InTech,* 2012, vol. 23
- **MEIWES, J.** *Tetrahedron Asymmetry,* 1997, vol. 8, 527-536
- **HUTCHINSON, J.H. et al.** *Tetrahedron Letters,* 1992, vol. 33, 4713-4716
- **BECK. J.** *Tetrahedron,* 1994, vol. 50, 4691-4698
- **LAM, P.Y.S.** *Tetrahedron Lett.,* 1998, vol. 39, 2941-2944
- **POPP, F. D. et al.** *J. Pharm. Sci.,* 1980, vol. 69, 1235-1237
- **ASSELIN ; GUINOSSO.** *Soll. J. Org. Chem.,* 1988, vol. 53, 2844-2847
- **DONG, GUANG RI et al.** *Synlett,* 2013, vol. 24 (15), 1993-1997
- **DAN ZHU ; JING SUN ; CHAO-GUO YAN.** *RSC Adv.,* 2014, vol. 4, 62817
- **HAN, YING et al.** *Tetrahedron,* 2012, vol. 68, 8256-8260
- **SHAO, LI-XIONG et al.** *Org. Lett.,* 2013, vol. 15 (6), 1254-1257
- **BAILEY, D. M. ; DE GRAZIA C.G.** *Tetrahedron Lett.,* 1970, vol. 9, 633-636
- **GREEN, T.W. ; P.G.M. WUTS.** Greene's Protective Groups in Organic Synthesis. Wiley, 1999
- **GEIN, V. L. et al.** *Russian Journal of Organic Chemistry,* 2011, vol. 47 (1), 95-99